# EUROPEAN PATENT APPLICATION

(11) **EP 4 830 731 A1**
(43) Date of publication of application: **28.10.2026**
(21) Application number: 24906465.0
(22) Date of filing: 19.12.2024
(51) Int. Cl.: A61K 47/54, A61K 47/60, A61K 47/65, A61K 47/68, A61K 31/4745, A61K 38/08, A61P 35/00, A61P 29/00

(54) **TUMOR MICROENVIRONMENT-ACTIVATED DRUG CONJUGATE AND ANTIBODY-DRUG CONJUGATE**

(30) Priority: 22.12.2023 CN 202311791063
(71) Applicant: Affinity (Shanghai) Biopharmaceutical Co., Ltd., Shanghai 201203 (CN)
(72) Inventor: LIU, Yuan, Shanghai 201203 (CN); CHEN, Tao, Shanghai 201203 (CN); DING, Chengli, Shanghai 201203 (CN); ZHANG, Rui, Shanghai 201203 (CN); LIU, Chen, Shanghai 201203 (CN)
(74) Representative: Oryon NV
(86) International application number: PCT/CN2024/140704
(87) International publication number: WO 2025/130990

(57) **Abstract**

The present invention relates to a tumor microenvironment-activated drug conjugate structure and an antibody-drug conjugate. Specifically, the present invention provides a drug conjugate having a chemical structure represented by formula R₁-R₂-D or R₂'-D, a stereoisomer thereof, or a pharmaceutically acceptable salt thereof, wherein R₁ is a chemical structure conjugated to a biomolecule or is absent, R₂ and R₂' are each independently an adjustment chemical structure for enhancing the structure-activity relationship, and D is selected from structures represented by X1-X5. The present invention also provides an antibody-drug conjugate comprising the drug conjugate.

## Description

### Technical field

The present disclosure belongs to the field of medicinal chemistry, and specifically relates to a drug conjugate activatable by the tumor microenvironment and an antibody-drug conjugate.

### Background

To enhance the therapeutic window of antibodies or protein-conjugated drugs, significant efforts have been dedicated to improving the tumor specificity and release efficiency of these conjugated drugs. In recent years, propelled by advancements in tumor molecular biology and other fundamental disciplines, research and development in tumor-targeted therapeutic drugs have made considerable progress across various fronts.

The method of linking effector molecules to antibodies using natural cysteine residues in antibody sequences has been widely applied, such as for cytotoxins, immune agonists or immune antagonists, further expanding the use of antibodies. Generally, antibody-drug conjugates need to enter tumor cells through endocytosis to exert their efficacy, where the effector molecules with cell-killing effects are released through degradation inside the cells, thereby achieving tumor inhibition. After years of development of antibody-drug conjugates, only two types of linkers activatable by cathepsin B have been successful, and they must enter tumor cells through endocytosis. This has become a major limiting factor that hinders the druggability of various antibodies.

The tetrapeptide Gly-Gly-Phe-Gly (GGFG) is a cathepsin B-activatable linker, and the ADC drug Enhertu uses such a linker. Enhertu from Daiichi Sankyo is a plasma-stable ADC with a DAR of 7.7, which undergoes protease degradation in lysosomes to release a DX-8951f derivative, a potent topoisomerase I inhibitor derived from exatecan. Due to the specificity of the GGFG linker, achieving such a high DAR is remarkable, indicating that the structure-activity relationship between the linker and the toxin molecule is key to developing ADC drugs. However, the combination of the GGFG linker with the DX-8951f derivative causes toxicity of interstitial lung disease, and clinical interstitial lung disease has been observed for Enhertu (T-Dxd), Datopotamab deruxtecan (Dato-DXd), Raludotatug deruxtecan (R-DXd), and Ifinatamab deruxtecan (I-DXd). A comprehensive analysis of 8 monotherapy trials of T-DXd: among 879 patients with different tumor types, 139 (15.8%) were determined to have developed ILD. Among them, 108 had grade 1 or 2 events, and 21 had grade 5 events. This may be because cathepsin B is expressed in lung epithelial cells, and GGFG, being a cathepsin B-activatable linker, would cause some release in the lungs. A comprehensive analysis of 23 PD-1 monotherapy trials shows: among 3284 patients with different tumor types, 4% developed ILD. In the future, the combination of T-DXd with PD-1 therapy may further increase the incidence of ILD.

We have found that legumain is specifically highly expressed in tumors, and have developed a novel small molecule conjugate drug, Legubicin, activated by legumain. Through clinical trials involving hundreds of patients treated with this novel chemotherapy, no ILD occurred, indicating that legumain activation has greater tumor tissue specificity than cathepsin B. The DX-8951f derivative and the immune agonist T785 are far more toxic than conventional chemotherapeutic drugs such as doxorubicin and paclitaxel, making the development of broad-spectrum small molecule conjugate drugs more difficult. Through various connections and enzymatic activation of legumain-cleavable linkers and toxins, we have screened out an AAN-toxin combination suitable for development. Through the combination of R1 and R2 to match the screening of linkers and toxins, we have obtained small molecule conjugate drugs that are considered developable and currently have extremely high preclinical therapeutic indices. These small molecule compounds can be used as drugs alone or conjugated to antibodies via EMC or other conjugation methods to form antibody-drug conjugates.

### Summary

The object of the present disclosure is to provide a drug conjugate and an antibody-drug conjugate with high specificity and high stability, and use thereof in the treatment and/or prevention of cancer and/or inflammation. The drug conjugate and antibody-drug conjugate of the present disclosure are activated only in the pathological microenvironment (e.g., tumor microenvironment or an inflammatory site), thereby releasing active molecules, overcoming drug resistance, and reducing toxicity.

Specifically, the present disclosure provides a drug conjugate having a chemical structure of formula (I) or (II), a stereoisomer thereof, or a pharmaceutically acceptable salt thereof,

R₁-R₂-D (I)

R₂'-D (II)

wherein R₁ is a chemical structure for conjugation to a biomolecule or is absent; R₂ and R₂' are each independently an adjustment chemical structure for enhancing structure-activity relationship; D comprises -AAN- and a cytotoxic structure, and D is selected from X1-X5.

The present disclosure also provides an antibody-drug conjugate, a stereoisomer thereof, or a pharmaceutically acceptable salt thereof, wherein the antibody-drug conjugate is as shown in formula (III):

R₃-(R₁-R₂-D)ₓ Formula (III)

wherein R₃ is an antibody; R₁, R₂, and D are as defined in any embodiment herein; and x is an integer selected from 1 to 8.

The present disclosure also provides a pharmaceutical composition, comprising: (i) the drug conjugate and/or antibody-drug conjugate according to any embodiment herein, a stereoisomer thereof, or a pharmaceutically acceptable salt thereof; and (iii) a pharmaceutically acceptable carrier.

The present disclosure also provides use of the drug conjugate and/or antibody-drug conjugate according to any embodiment herein in the manufacture of a medicament for the treatment and/or prevention of tumors and/or inflammation.

### Description of Figures

Figure 1: Enzymatic cleavage spectrum of Control Compound Y.
Figure 2: Enzymatic cleavage spectrum of compound S5.
Figure 3: Enzymatic cleavage spectrum of compound S16.
Figure 4: Enzymatic cleavage spectrum of Deruxtecan in the presence of Cathepsin B.
Figure 5: Enzymatic cleavage spectrum of S16 in lung tissue.
Figure 6: Enzymatic cleavage spectrum of Deruxtecan in lung tissue.
Figure 7: Hydrophobic interaction chromatography (HIC) of Trastuzumab-PEG6-AAN-AM-Dxd (DAR8).
Figure 8: MS spectrum of Atezolizumab Ab-C5-AAN-PABC-T785 (DAR8).
Figure 9: HIC spectrum of Atezolizumab Ab-AAN-T785 (DAR8).
Figure 10: MS spectrum of Atezolizumab Ab-C5-AAN-T785 (DAR8).
Figure 11: Efficacy of a high dose of a TLR7/8 agonist-based (S5) small molecule conjugate drug alone and in combination with Legubicin.
Figure 12: Efficacy of a high dose of a TLR7/8 agonist-based (S5) small molecule conjugate drug alone and in combination with CTLA-4 antibody.
Figure 13: Inhibitory effect of an anti-HER2 antibody conjugated with Dxd drug molecule based on Affinity linker 2 on gastric cancer tumors.
Figure 14: Inhibitory effect of an anti-LY6G6D antibody conjugated with Dxd drug molecule based on Affinity linker 2 on a colon cancer tumor model.
Figure 15: Inhibitory effect of PDL1-Dxd in the MC38 tumor model.
Figure 16: Inhibitory effect of an anti-PD-L1 antibody conjugated with T785 drug based on Affinity linker 4 on large tumors.
Figure 17: Tumor growth inhibitory effect of 7B6-DXD in a subcutaneously transplanted HT-55 tumor model in mice.
Figure 18: Comparison of stability between S15 conjugated to albumin and after ring-opening.
Figure 19: Schematic structural diagram of a bispecific antibody-drug conjugate.
Figure 20: Inhibitory effect of EGFR/Cmet-T785 in the NCI-H292 tumor model.
Figure 21: Inhibitory effect of EGFR/Cmet-Dxd in the NCI-H292 tumor model.
Figure 22: Tumor volume after administration of S41 in a nude mouse xenograft model of human fibrosarcoma HT-1080.
Figure 23: Tumor volume after administration of S41 in a nude mouse xenograft model of human fibrosarcoma HT-1080.
Figure 24: Tumor volume after administration of S41 in a nude mouse model bearing transplanted CT-26 mouse colon carcinoma.
Figure 25: Tumor volume after administration of S41 and/or anti-PD1 antibody in a nude mouse model bearing transplanted CT-26 mouse colon carcinoma.
Figure 26: Tumor volume after administration of S41 in a nude mouse model bearing transplanted CT-26 mouse colon carcinoma.

### Detailed Description

It is understood that within the scope of the present disclosure, the above-mentioned technical features of the present disclosure and the technical features specifically described in the following (such as the examles) can be combined with each other to form a preferred technical solution.

It is noted that, as used herein and in the appended claims, the singular forms "a," "an," "as well as" and "the" comprise plural referents unless the context clearly dictates otherwise. Thus, for example, reference to "an antibody" includes a plurality of antibodies, and reference to "the antibody" includes reference to one or more antibodies and equivalents thereof known to those skilled in the art, etc. It is also noted that claims may be drafted to exclude any optional element. Therefore, this statement is intended to serve as an antecedent basis for the use of exclusive terms such as "alone", "only" in connection with the description of claim elements, or for the use of "negative" limitations.

The legumain-activated small molecule drugs previously developed by the inventors, Legubicin, which contains AANL-DOX, and Legutaxel, which contains AAN-PABC-taxel and paclitaxel, have been granted patents in China and are currently undergoing pivotal Phase II/III and Phase I clinical trials, respectively. However, the toxicity of doxorubicin and paclitaxel cannot meet the requirements of the more potent toxins used in ADCs, thus necessitating the innovation to produce conjugate structures with novel structure-activity relationships. Studies have found that legumain forms a complex with integrin and can efficiently activate specific substrate structures on the tumor cell surface. Therefore, the inventors used legumain as the activating enzyme, combined with various toxin and immune agonist chemical structures, as well as auxiliary structures and linking structures, to perform extensive structural screening. By considering the mechanism of action in biological systems and in vitro/in vivo efficacy, specific restricted conjugate structures were obtained. These conjugate structures are highly stable in human plasma, yet can be efficiently activated in the tumor microenvironment (extracellularly) to release the conjugated chemical molecules. Therefore, compared with traditional ADCs, this novel release mechanism, when paired with an immune agonist as the payload, can better exert the bystander effect to activate nearby immune cells, and exhibits a different mechanism of druggability and expandability from traditional ADCs.

### Drug Conjugates

This present disclosure provides a compound with a chemical structure represented by formula (I), a stereoisomers or pharmaceutically acceptable salts thereof:

R₁-R₂-D (I);

wherein the compound can be used as a drug conjugate for the preparation of ADC.

In formula (I), R₁ is a chemical structure for conjugation to a biomolecule or is absent; R₂ is an adjustment chemical structure for enhancing structure-activity relationship; and D is -AAN-linker-drug.

Herein, R₁ is preferably selected from: 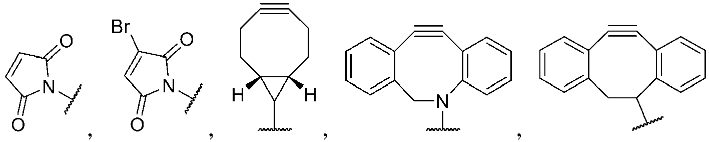 and ;
wherein the wavy line indicates the position of attachment of R₁ and R₂.

Preferably, R₁ is selected from:
and

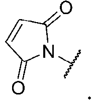

In some embodiments, R₁ is absent.

In some embodiments, R₂ is selected from:
-C₁₋₆ alkylene-CO-*;
-C₁₋₆ alkylene-O-*;
-C₁₋₆ alkylene-OCONH-C₁₋₆ alkylene-(O-CH₂CH₂-O)ₙ-C₁₋₆ alkylene-CO-*;
-C₁₋₆ alkylene-OCONH-C₁₋₆ alkylene-(O-CH₂CH₂-O)ₙ-C₁₋₆ alkylene-O-*;
-C₁₋₆ alkylene-OCONH-C₁₋₆ alkylene-(O-CH₂CH₂-O)ₙ-C₁₋₆ alkylene-NH-*;
-(O-CH₂CH₂-O)ₙ-C₁₋₆ alkylene-CO-*;
-C₁₋₆ alkylene-CONH-C₁₋₆ alkylene-(O-CH₂CH₂-O)ₙ-C₁₋₆ alkylene-CO-*;
-NH-C₁₋₆ alkylene-(O-CH₂CH₂-O)ₙ-C₁₋₆ alkylene-CO-*;
-NH-C₁₋₆ alkylene-(O-CH₂CH₂-O)ₙ-C₁₋₆ alkylene-NH-*;
-NH-C₁₋₆ alkylene-(O-CH₂CH₂-O)ₙ-C₁₋₆ alkylene-O-*;
-NH-C₁₋₅₅ alkylene-CO-*;
-NH-C₁₋₅₅ alkylene-NH-*;
-NH-C₁₋₅₅ alkylene-O-*;
-CO-C₁₋₅₅ alkylene-CO-*;
-CO-C₁₋₆ alkylene-CONH-C₁₋₆ alkylene-(O-CH₂CH₂-O)ₙ-C₁₋₆ alkylene-CO-*;
-CO-C₁₋₆ alkylene-CONH-C₁₋₆ alkylene-(O-CH₂CH₂-O)ₙ-C₁₋₆ alkylene-NH-*;
-CO-C₁₋₆ alkylene-CONH-C₁₋₆ alkylene-(O-CH₂CH₂-O)ₙ-C₁₋₆ alkylene-O-*;
-CO-C₁₋₆ alkylene-NH-*;
-CO-C₁₋₆ alkylene-NHCO-C₁₋₆ alkylene substituted with -SO₃H-NH-*; 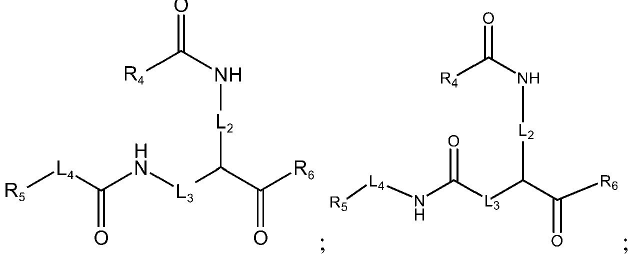 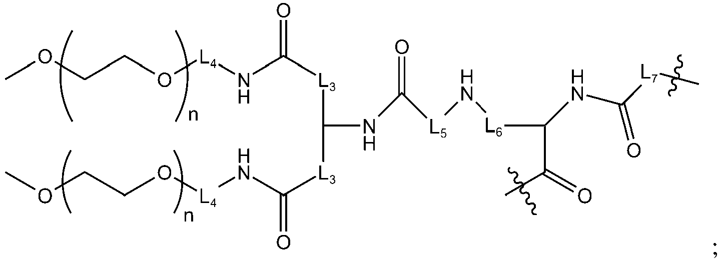 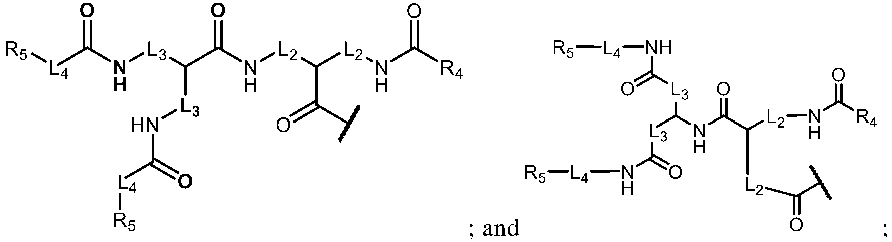
wherein:
* indicates the position of attachment to D;
R₄ is a monovalent or divalent group, wherein R₄ is a divalent group when it is connected to other parts of the compound, and R₄ is selected from: C₁₋₆ alkyl or C₁₋₆ alkylene; C₁₋₆ alkyl-C₃₋₈ cycloalkylene- or C₁₋₆ alkylene-C₃₋₈ cycloalkylene-; -CO-C₁₋₆ alkylene-; C₁₋₆ alkyl-(O-CH₂CH₂-O)ₘ-C₁₋₆ alkylene- or C₁₋₆ alkylene-(O-CH₂CH₂-O)ₘ-C₁₋₆ alkylene-; -NH-C₁₋₆ alkylene; C₁₋₆ alkylene-CONH-C₁₋₆ alkylene-(O-CH₂CH₂-O)ₘ-C₁₋₆ alkylene-; and -NH-C₁₋₆ alkylene-C₃₋₈ cycloalkylene;
R₅ is a monovalent or divalent group, wherein R₅ is a divalent group when it is connected to other parts of the compound, and R₅ is selected from: C₁₋₆ alkyl-(O-CH₂CH₂-O)ₘ-; C₁₋₆ alkylene-CONH-C₁₋₆ alkylene-(O-CH₂CH₂-O)ₘ-; -CO-C₁₋₆ alkylene-(O-CH₂CH₂-O)ₙ-;
one of R₄ and R₅ is a divalent group and the other is a monovalent group;
R₆ is absent, or is -NH-C₁₋₆ alkylene-triazolyl-C₁₋₆ alkylenecarbonyl or -NH-C₁₋₆ alkylene-triazolyl-C₁₋₆ alkylene-(O-CH₂CH₂-O)ₙ-C₁₋₆ alkylene-NHCO-C₁₋₆ alkyleneoxy-C₁₋₆ alkylenecarbonyl;
L₂ and L₃ are each independently a bond or C₁₋₆ alkylene;
L₄ is each independently C₁₋₆ alkylene;
L₅ is a bond, C₁₋₆ alkyleneoxy-C₁₋₆ alkylenecarbonyl, or C₁₋₆ alkylenecarbonyl;
L₆ is C₁₋₆ alkylene;
L₇ is C₁₋₆ alkylene or -C₃₋₈ cycloalkylene-C₁₋₆ alkylene; and
each n and m is independently an integer from 1 to 50.

In the present disclosure, alkylene has its well-known meaning in the art, referring to a divalent alkyl group, such as methylene (-CH₂-), ethylene (-CH₂CH₂-). The number of the carbon atoms in alkylene can be, for example, 1-55, 1-20, 1-10, 1-6, or 1-4.

In the present disclosure, cycloalkyl has its well-known meaning in the art, referring to a saturated carbocyclic ring. The number of the ring carbon atoms in cycloalkyl can be typically 3-8, such as 3-6. Exemplary cycloalkyl comprises cyclopropyl, cyclobutyl, cyclopentyl, and cyclohexyl. In the present disclosure, cycloalkylene refers to a divalent cycloalkyl group, i.e., having two ring carbon atoms for attachment to other parts of the compound.

In some embodiments, R₂ is: 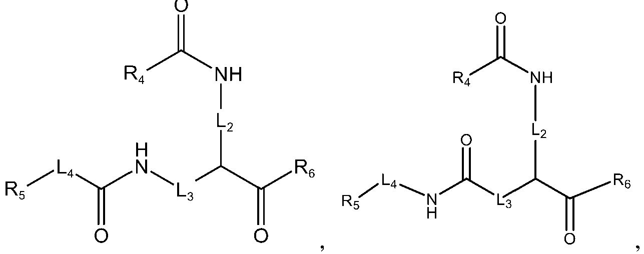 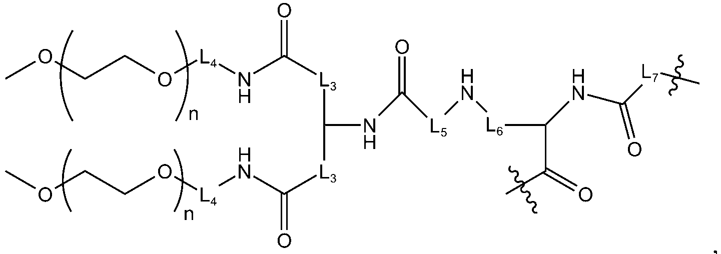 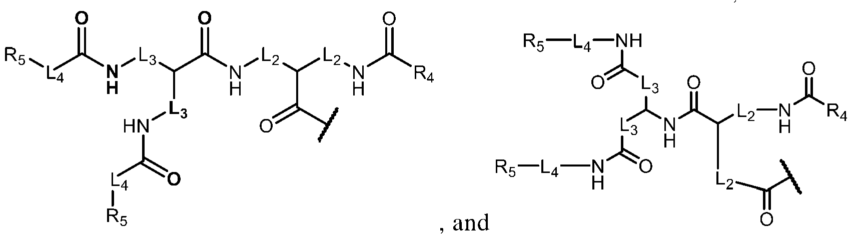 wherein:
R₄ is a monovalent or divalent group, wherein R₄ is a divalent group when it is connected to other parts of the compound, and R₄ is selected from: C₁₋₆ alkyl or C₁₋₆ alkylene; and C₁₋₆ alkyl-C₃₋₈ cycloalkylene- or C₁₋₆ alkylene-C₃₋₈ cycloalkylene-;
R₅ is a monovalent or divalent group, wherein R₅ is a divalent group when it is connected to other parts of the compound, and R₅ is selected from: C₁₋₆ alkyl-(O-CH₂CH₂-O)ₘ-; or C₁₋₆ alkylene-CONH-C₁₋₆ alkylene-(O-CH₂CH₂-O)ₘ-;
one of R₄ and R₅ is a divalent group and the other is a monovalent group;
R₆ is absent;
L₂ and L₃ are each independently a bond or C₁₋₆ alkylene;
L₄ is each independently C₁₋₆ alkylene;
L₅ is a bond, C₁₋₆ alkyleneoxy-C₁₋₆ alkylenecarbonyl or C₁₋₆ alkylenecarbonyl;
L₆ is C₁₋₆ alkylene;
L₇ is C₁₋₆ alkylene or -C₃₋₈ cycloalkylene-C₁₋₆ alkylene; and
each n and m is independently an integer from 1 to 50.

In some embodiments, R₂ is: 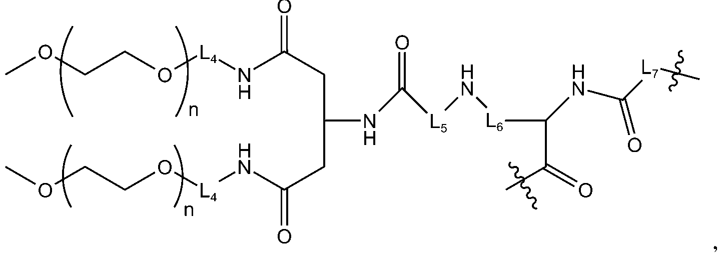 wherein L₄, L₅, L₆, L₇, and n are as defined in any of the preceding embodiments.

In formula (I), preferably, said R₂ is selected from: 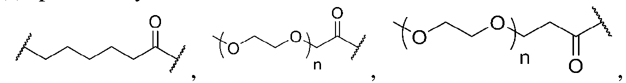 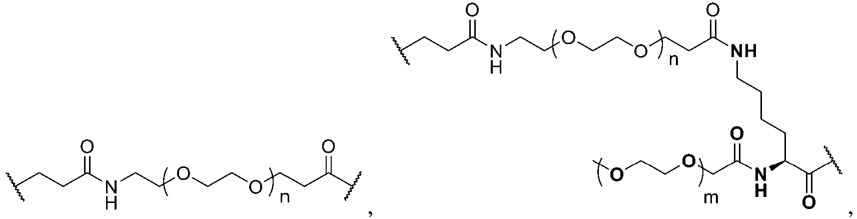 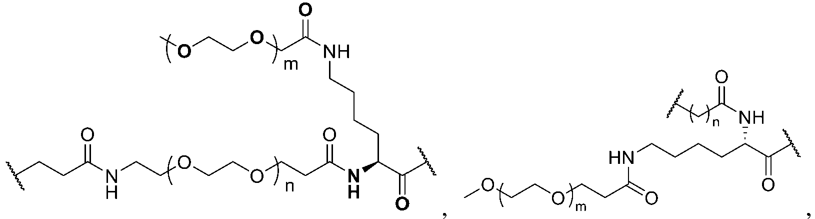 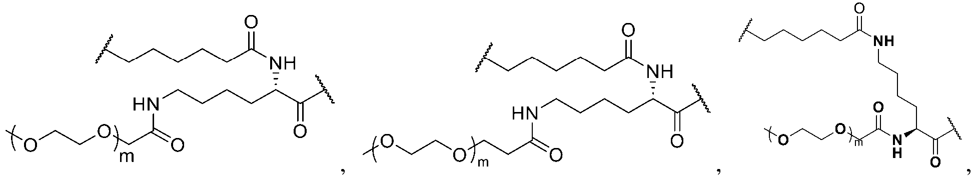 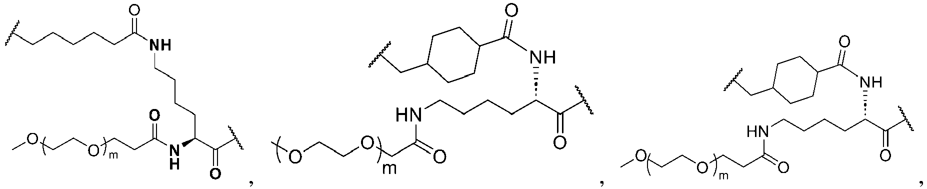 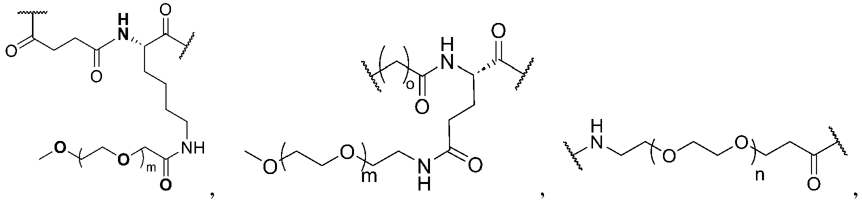 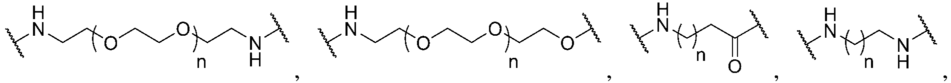 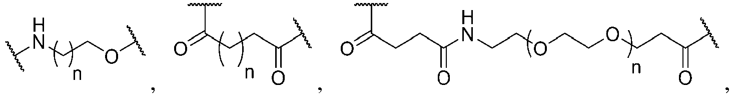 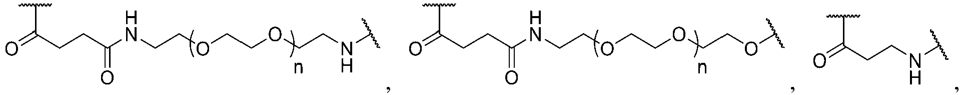 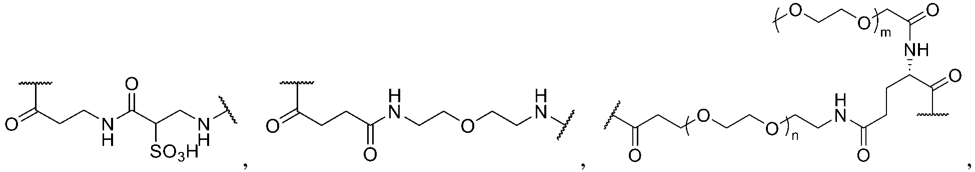 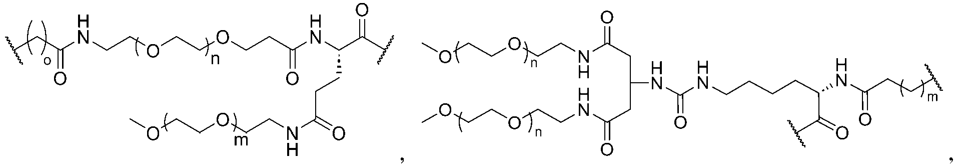 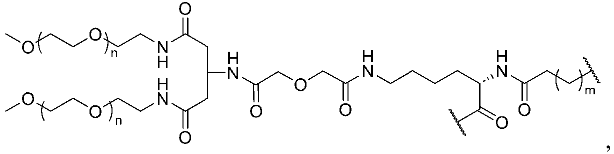 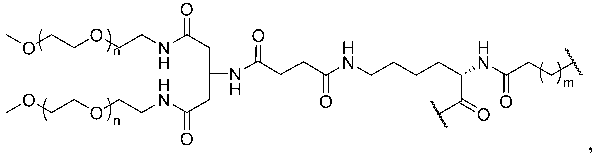 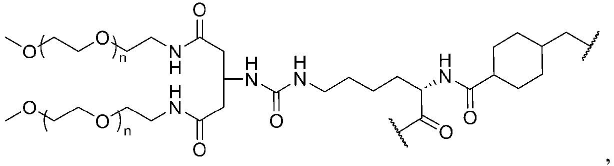 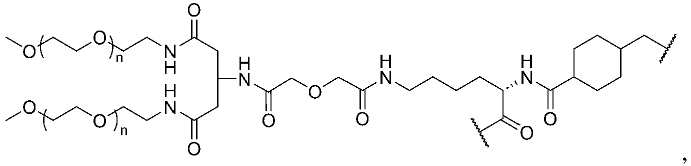 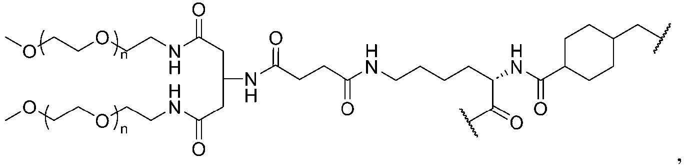 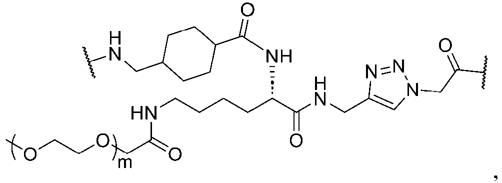 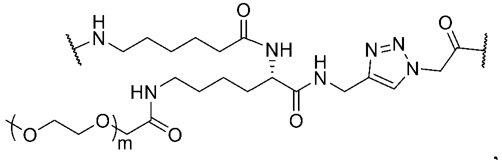 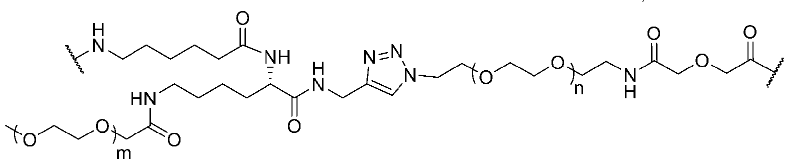 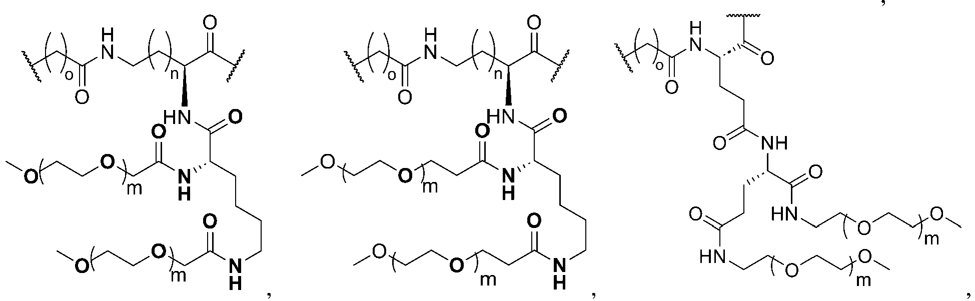 or 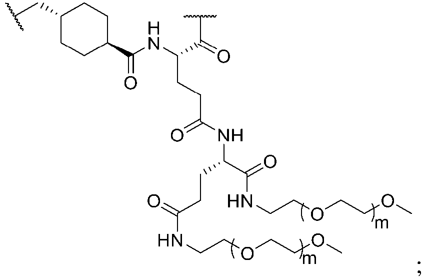 wherein o is independently an integer from 1 to 20, and m and n are each independently an integer from 1 to 50.

Preferably, in R₂, o is independently an integer from 1 to 10. Preferably, in R₂, m and n are each independently an integer from 1 to 40, or each independently an integer from 1 to 30, 2 to 40, 5 to 35, 10 to 25, 15 to 20, or 2 to 10.

Preferably, R₂ is selected from: 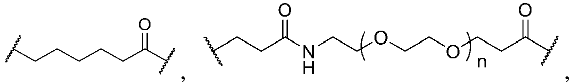 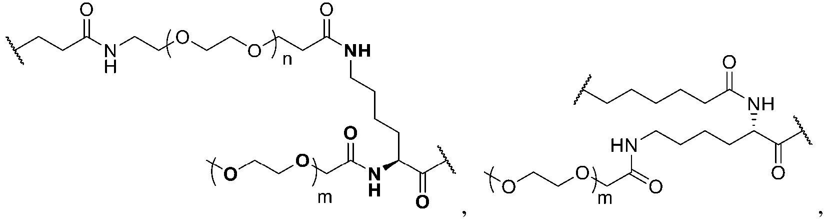 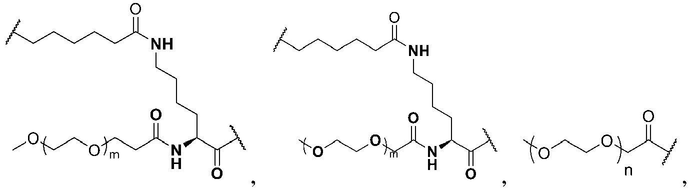 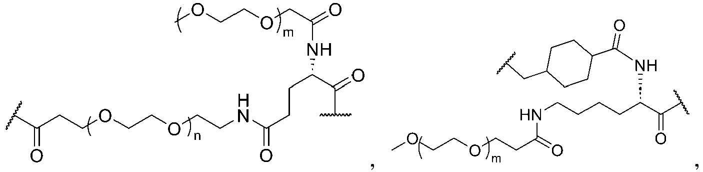 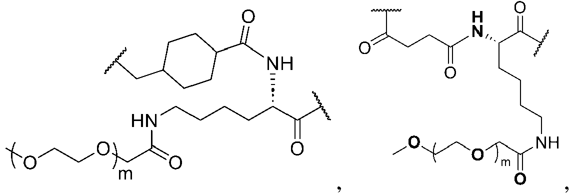 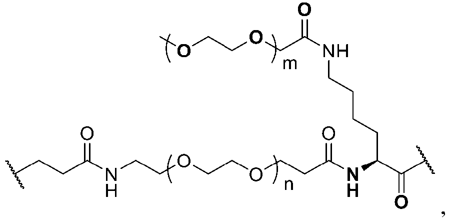 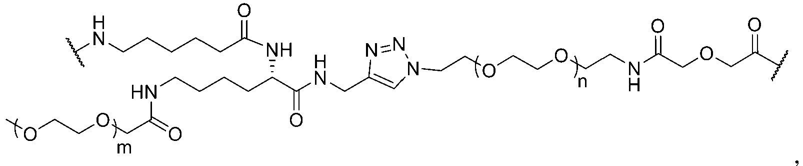 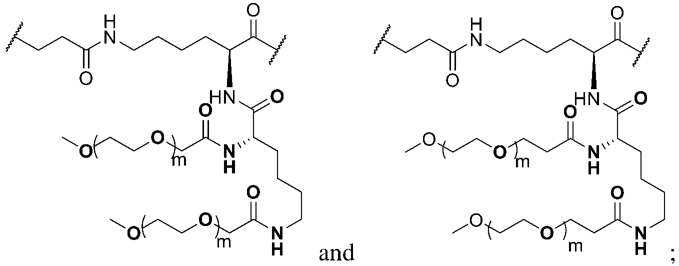 wherein o is independently an integer from 1 to 10, and n and m are each independently an integer from 2 to 20.

In formula (I), the drug can be selected from: camptothecin, camptothecin derivatives (such as 10-hydroxycamptothecin, irinotecan, and SN 38), topotecan, floxuridine, doxifluridine, cytarabine, etoposide, fludarabine, capecitabine, vincristine, epothilone B, daunorubicin, epirubicin, methotrexate, gemcitabine, melphalan, nimustine, mitoxantrone, paclitaxel, docetaxel, mitomycin, exatecan, exatecan derivative DXd, Sting agonists, resiquimod, and Toll-like receptor agonists.

In some embodiments, the drug is a TLR7/8 agonist, such as T785, resiquimod, and STING agonists, or a topoisomerase I inhibitor, such as exatecan and exatecan derivatives (DXd).

In some embodiments, the drug is selected from:

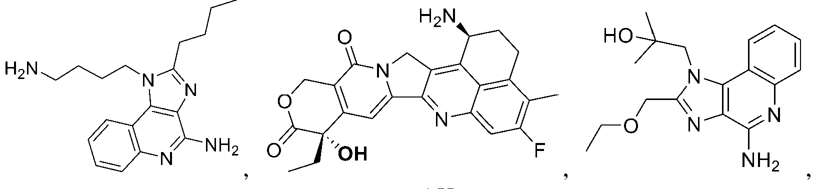

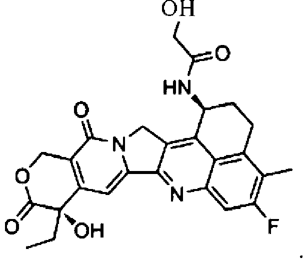

In formula (I), the drug can be attached to -AAN- directly or via a suitable linker. Typically, the drug and the -AAN- linker are attached via a suitable chemical bond, wherein the chemical bond can be a peptide bond (-NH-CO-) or -COO-. In some embodiments, the linker is a PABC (-NH-phenyl-CH₂-O-CO-) group or an AM (-NH-CH₂-) group.

In some embodiments, D in formula (I) is selected from the following X1 to X5: 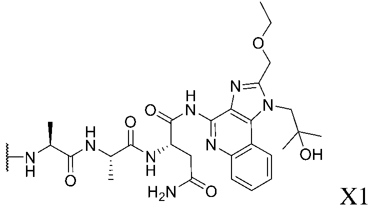 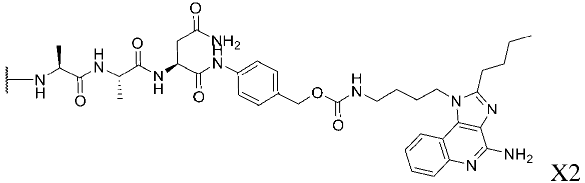 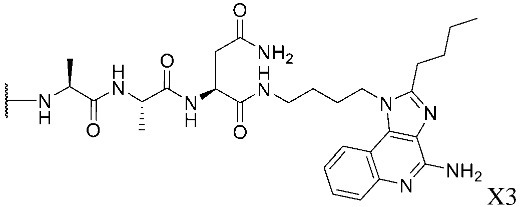 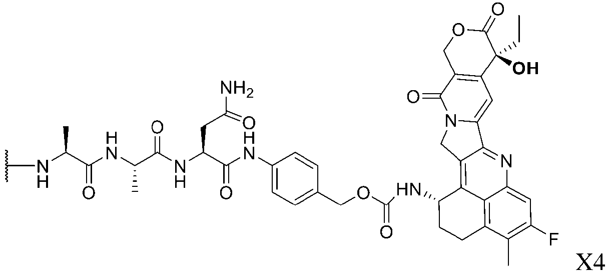 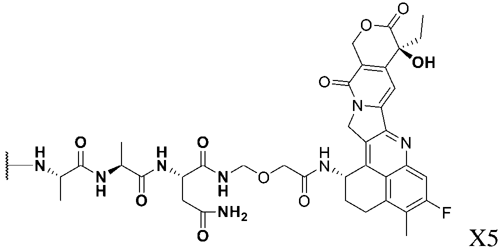 wherein the wavy line indicates the position of attachment of D to R₂.

In some embodiments, the drug conjugate of formula (I) is selected from: 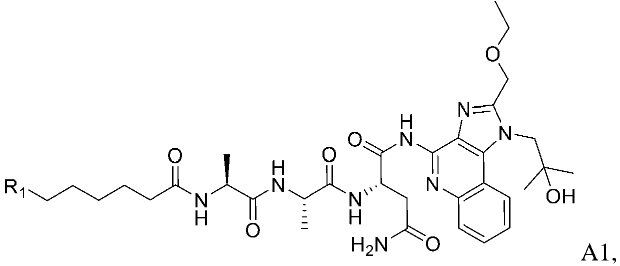 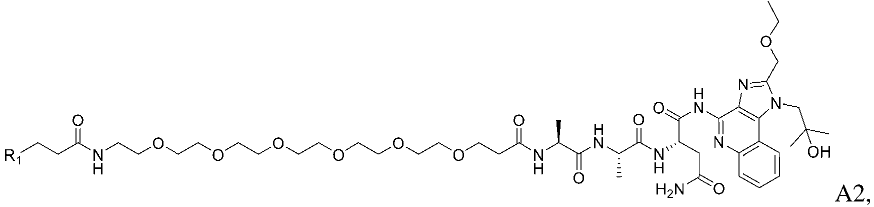 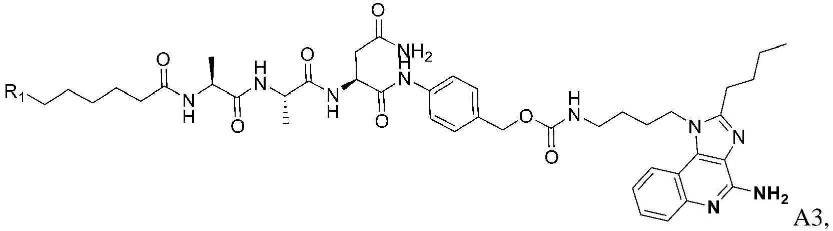 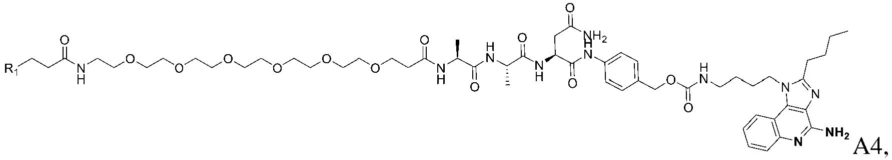 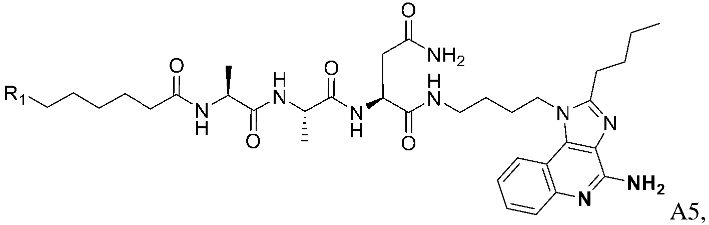 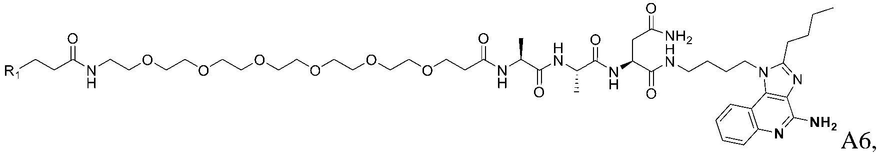 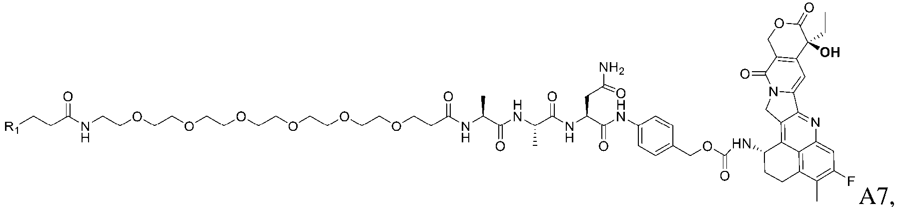 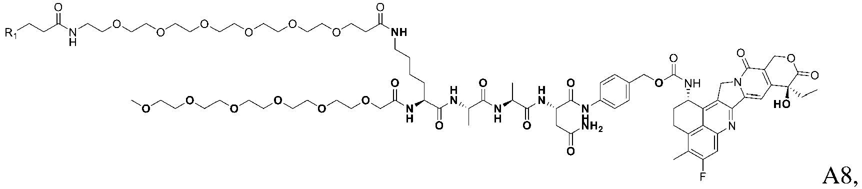 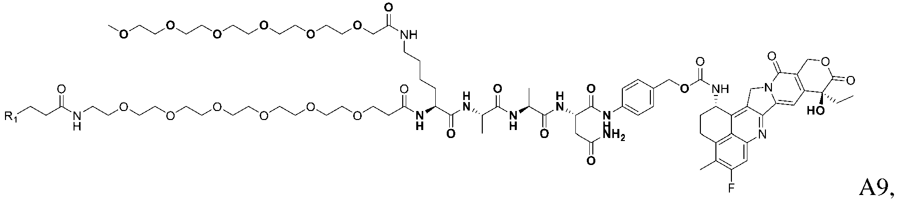 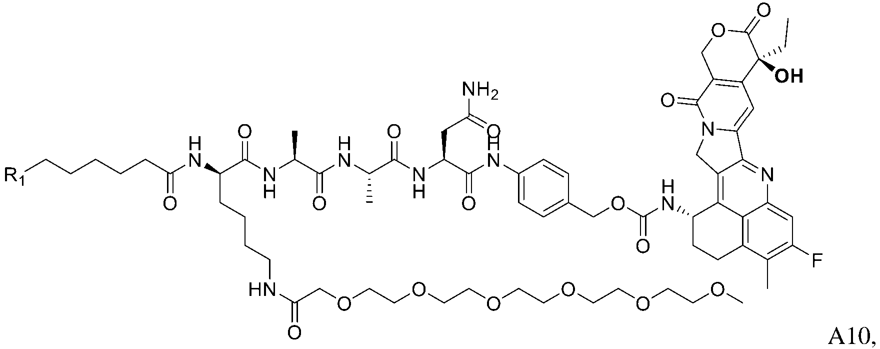 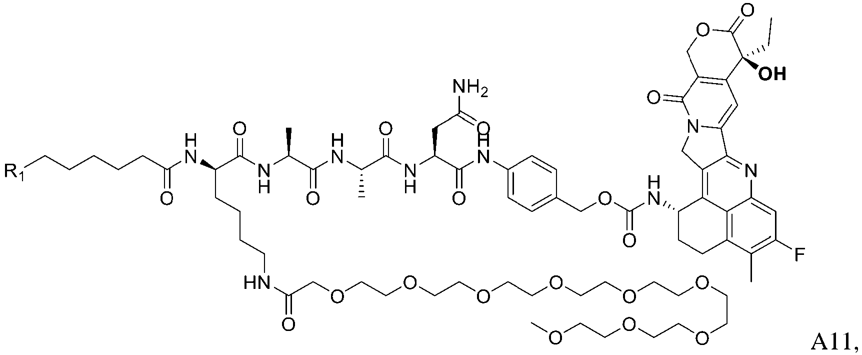 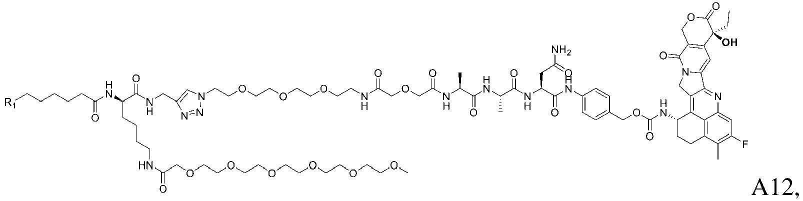 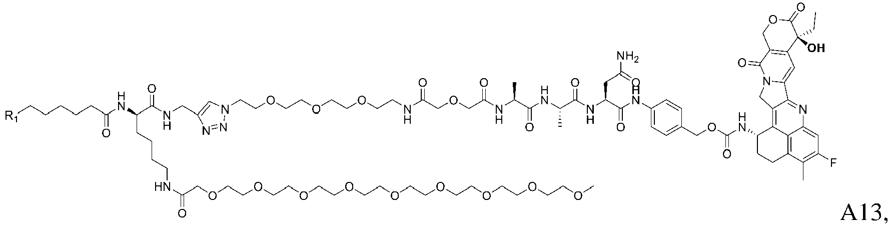 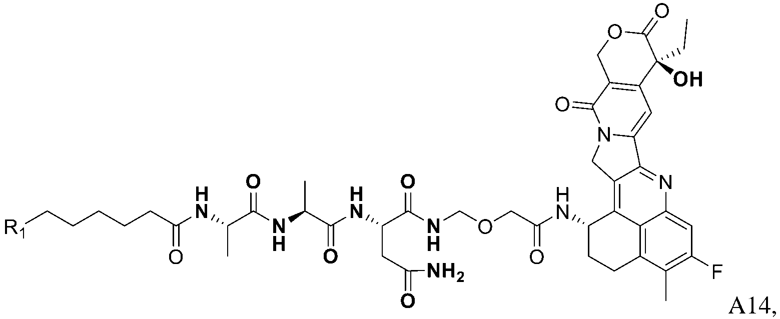 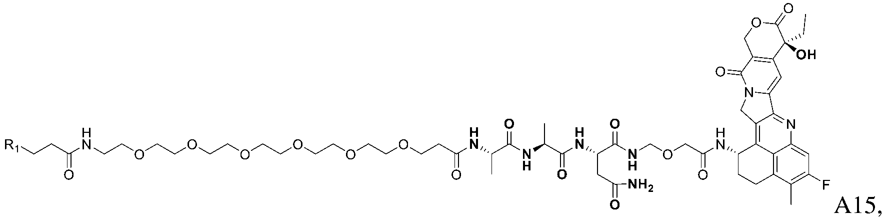 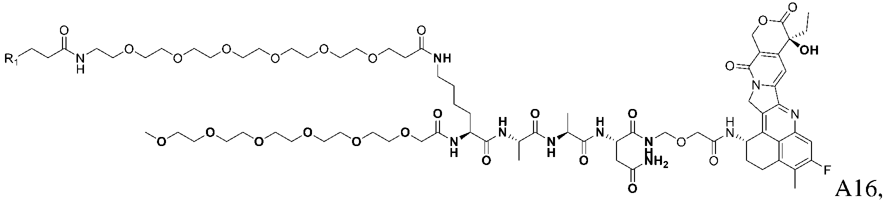 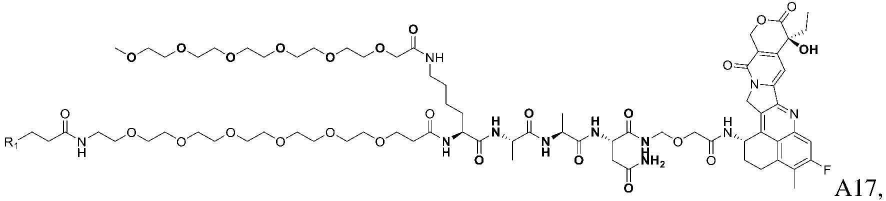 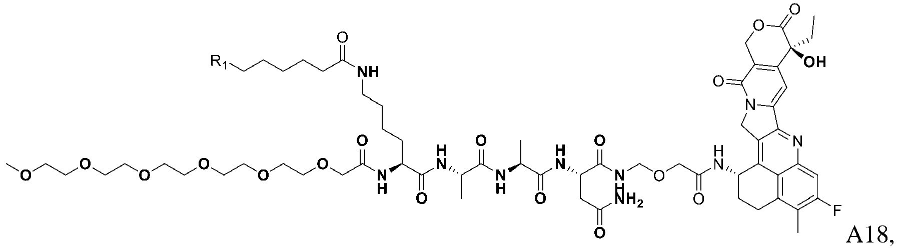 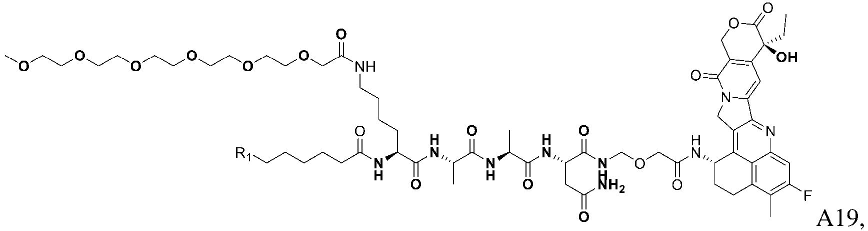 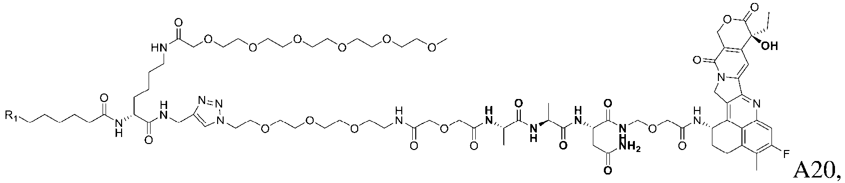 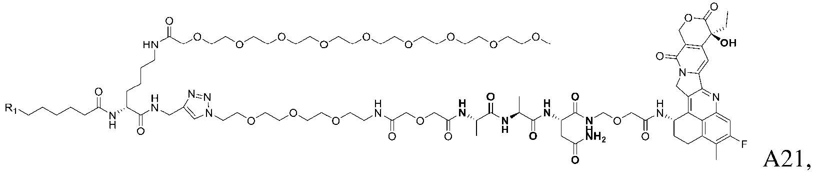 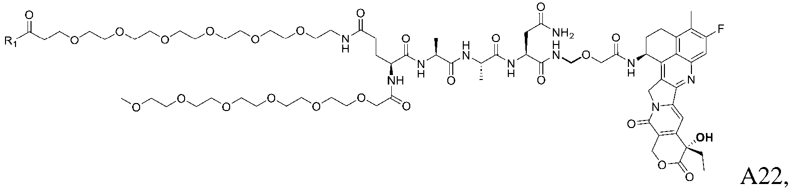 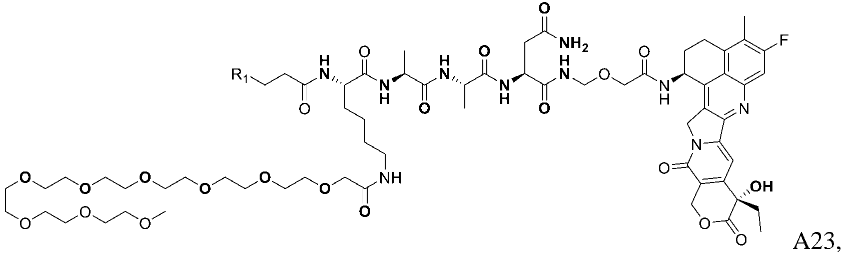 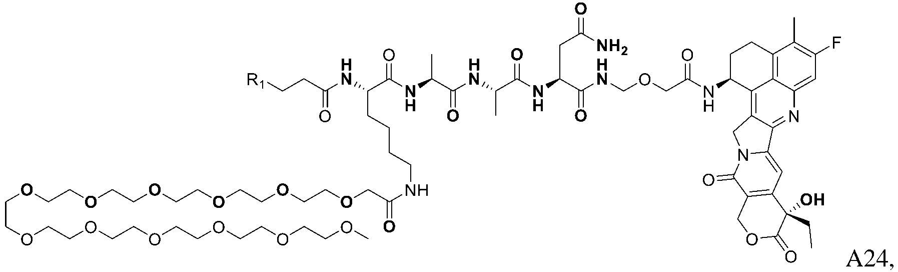 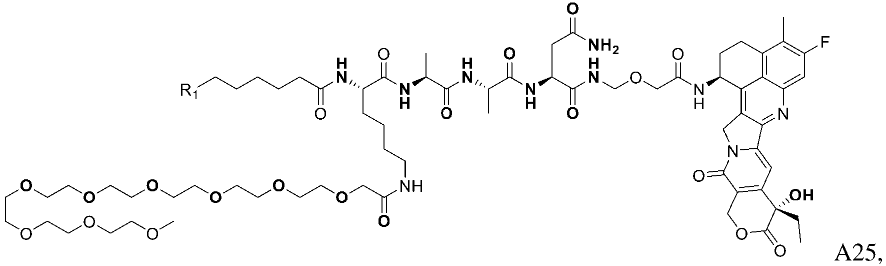 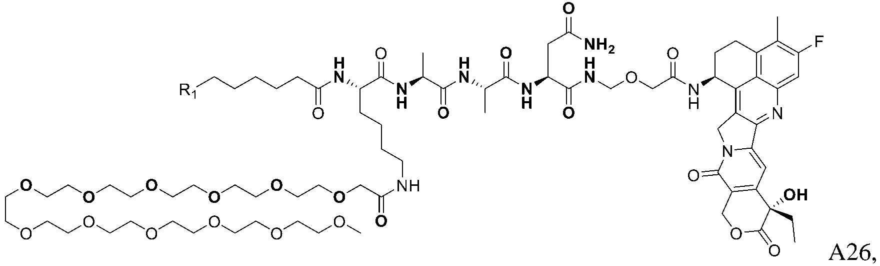 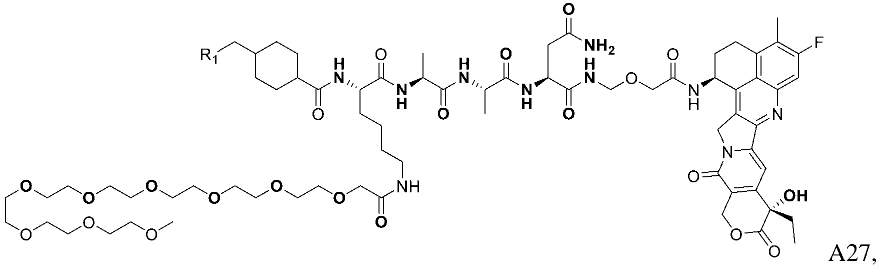 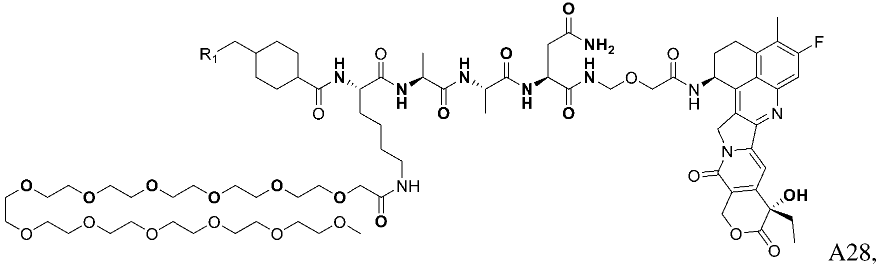 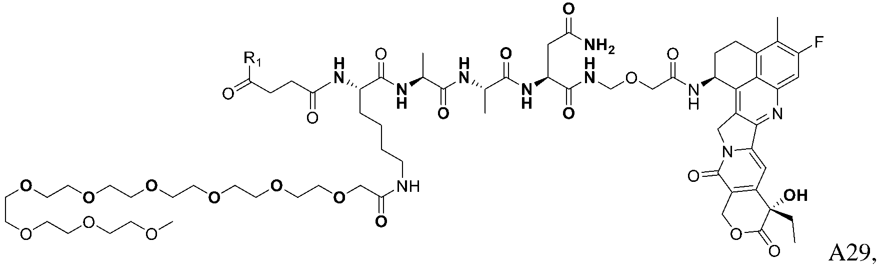 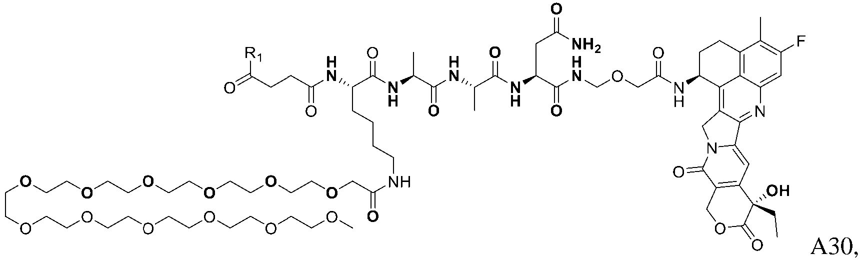 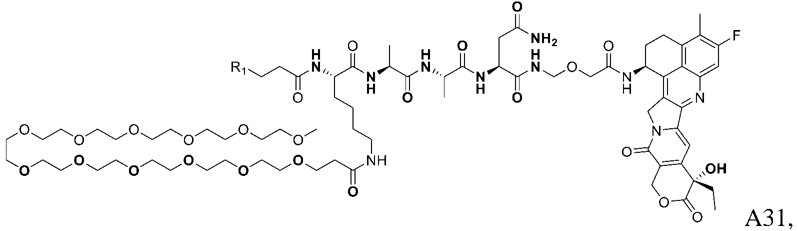 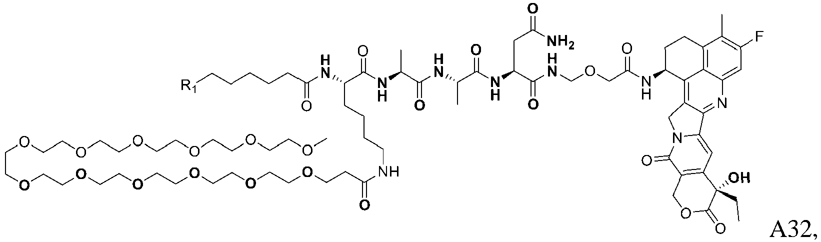 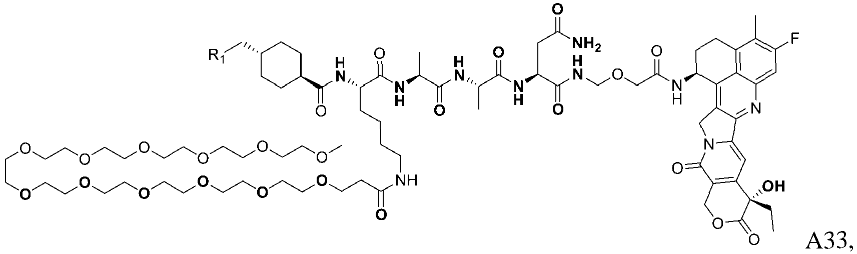 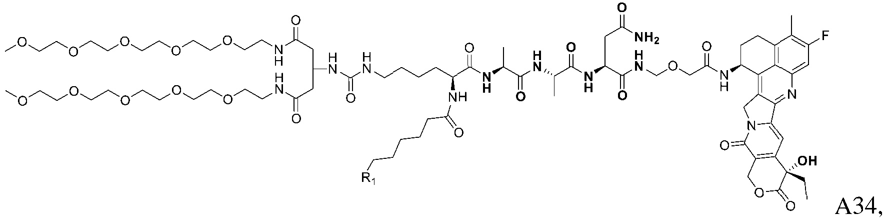 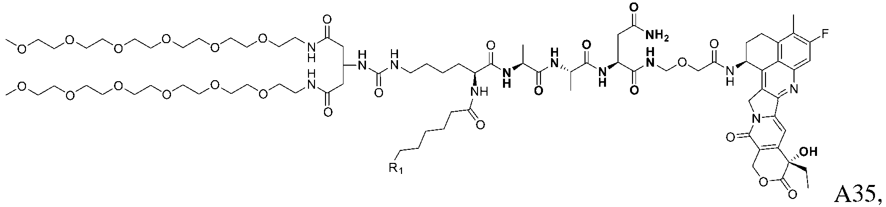 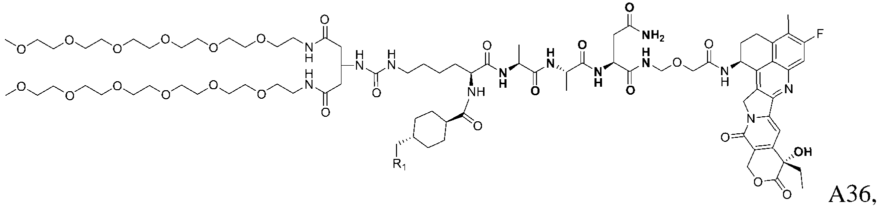 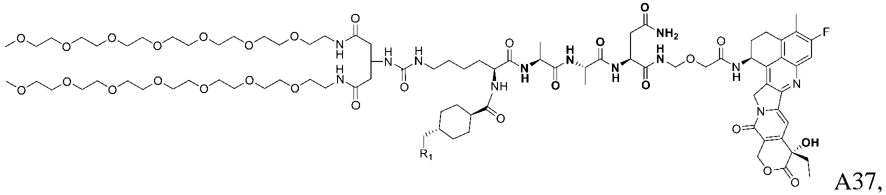 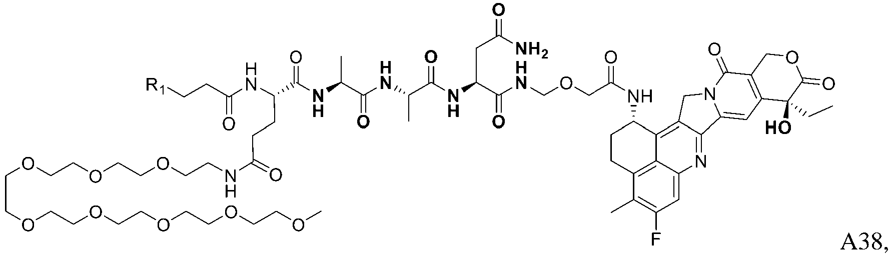 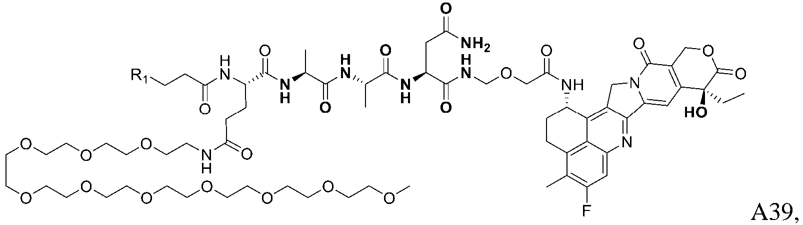 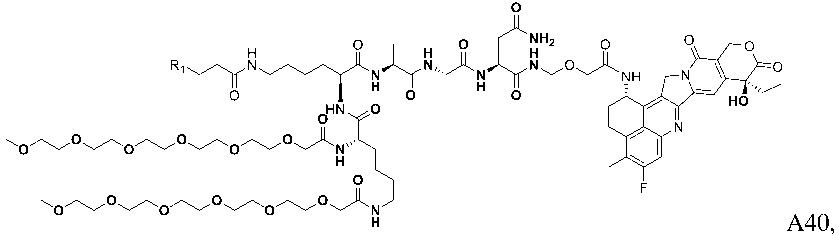 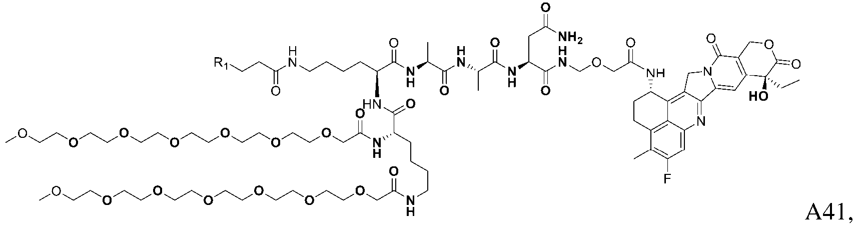 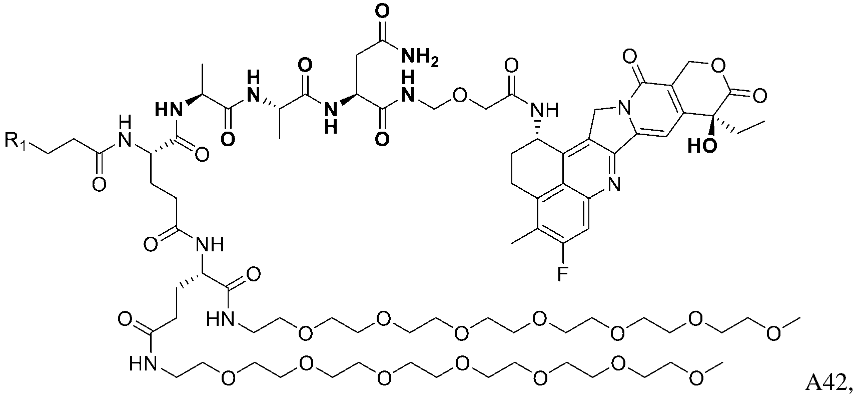 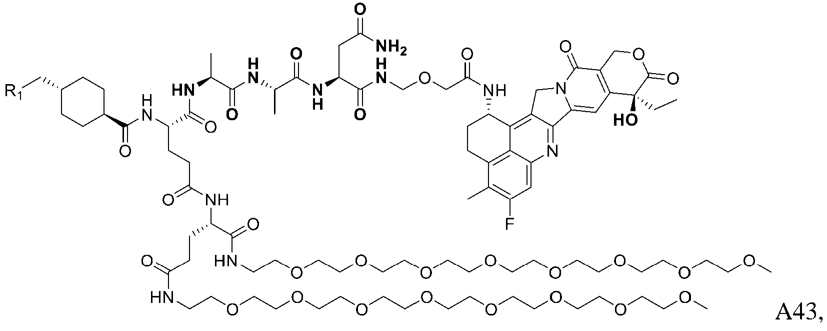 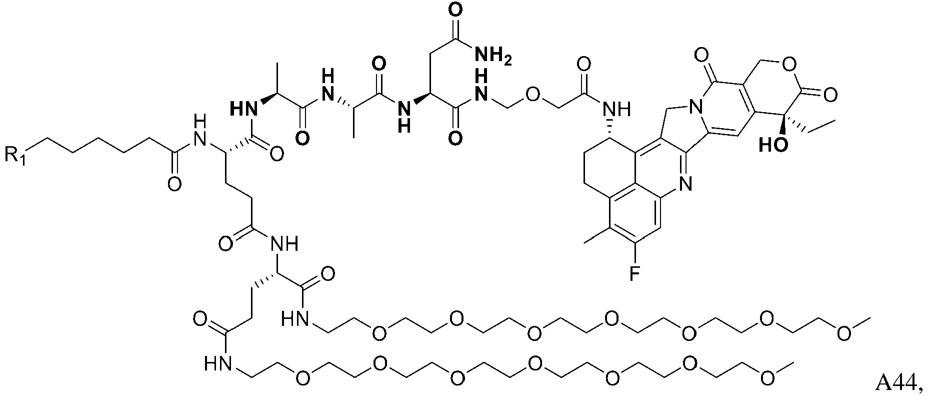 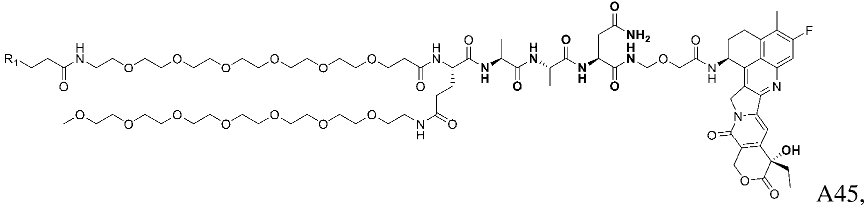 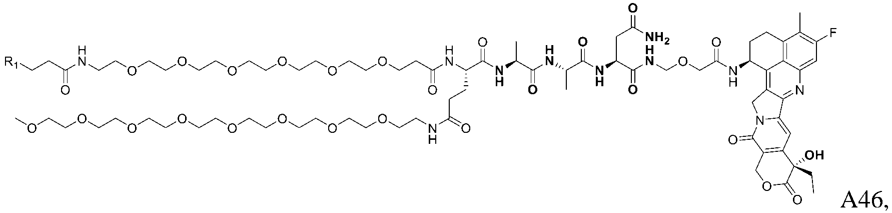 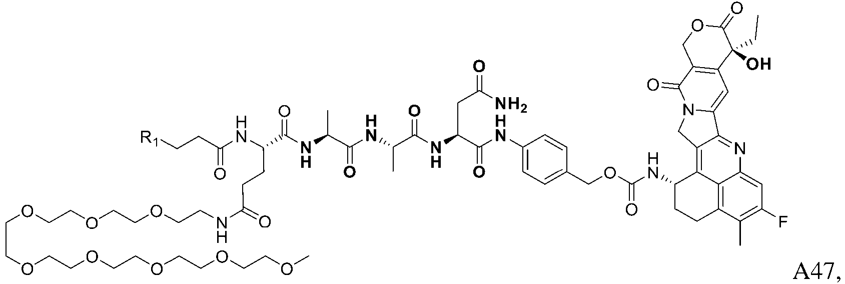 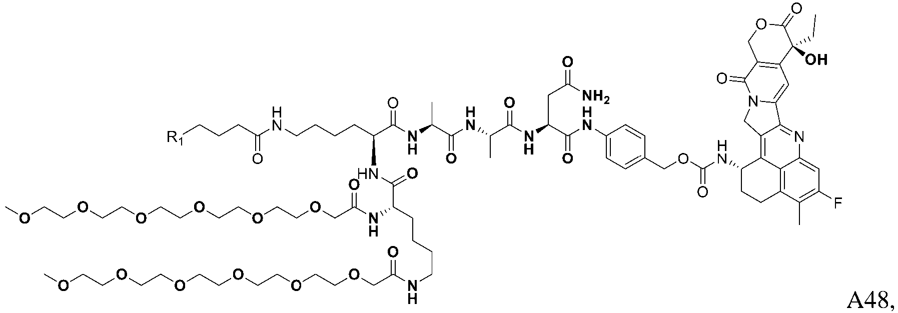 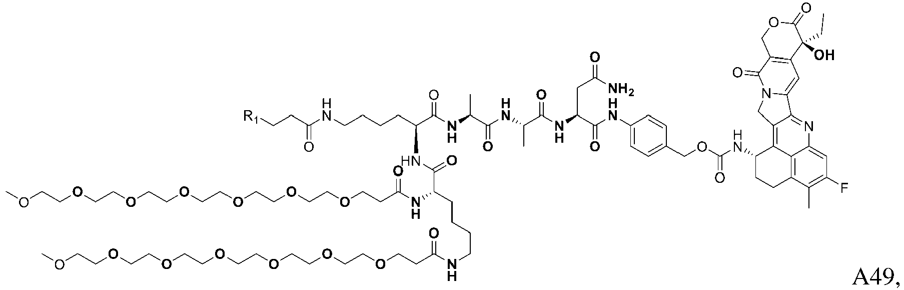 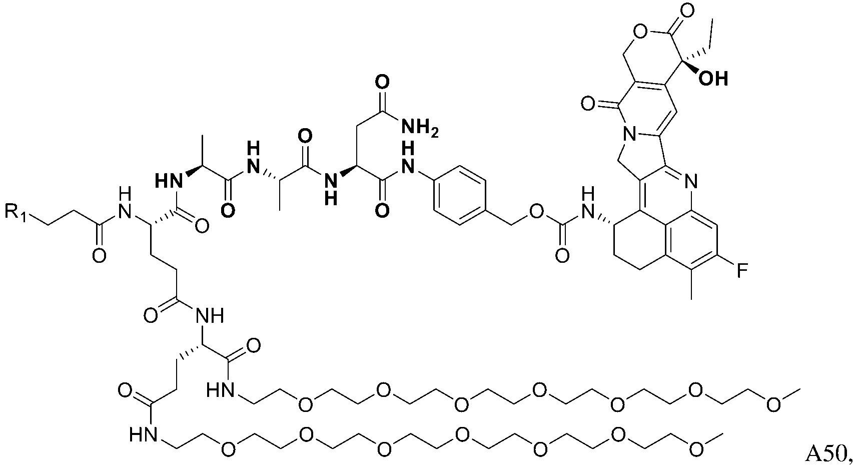 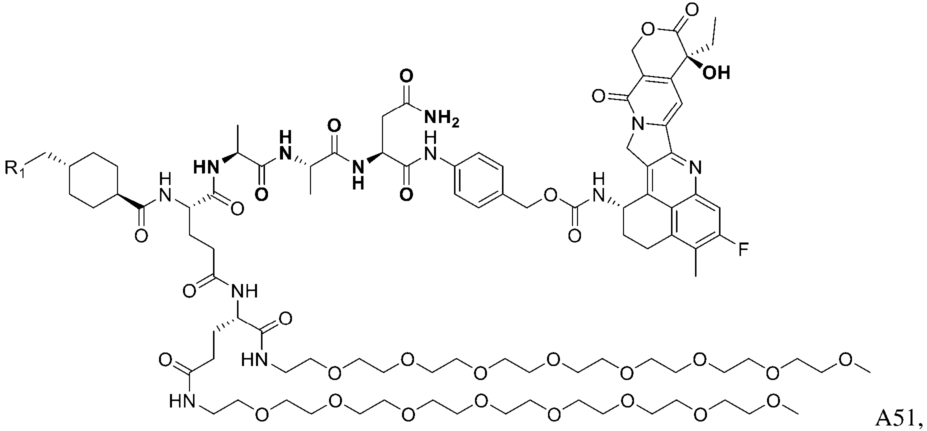 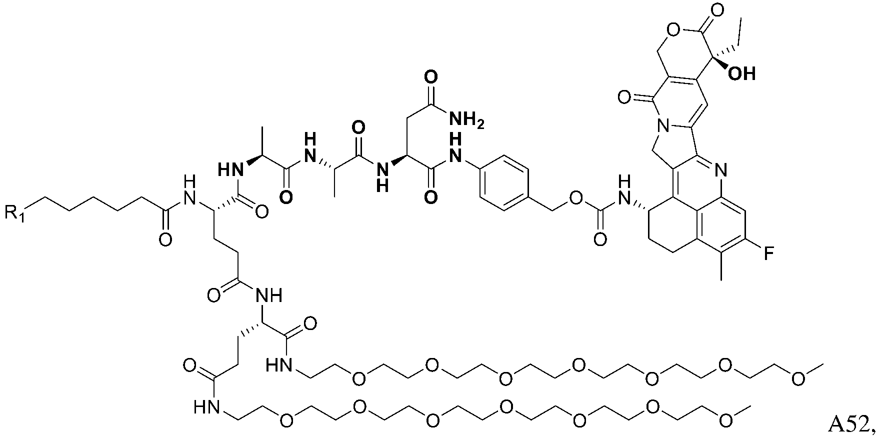 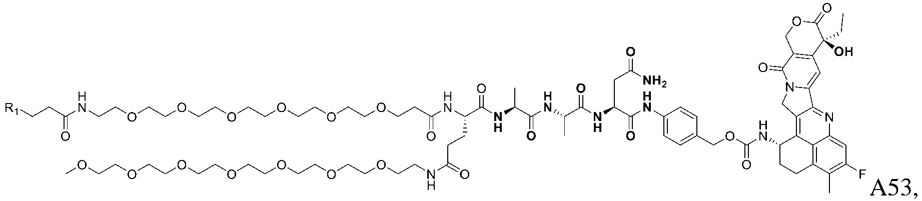 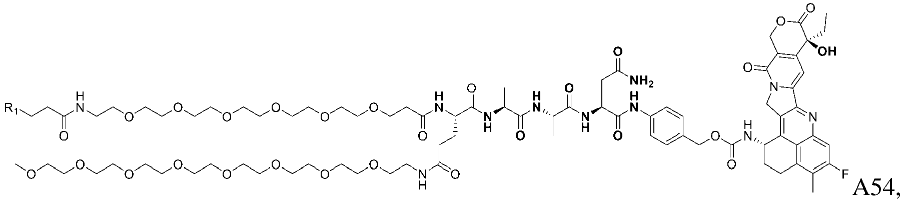 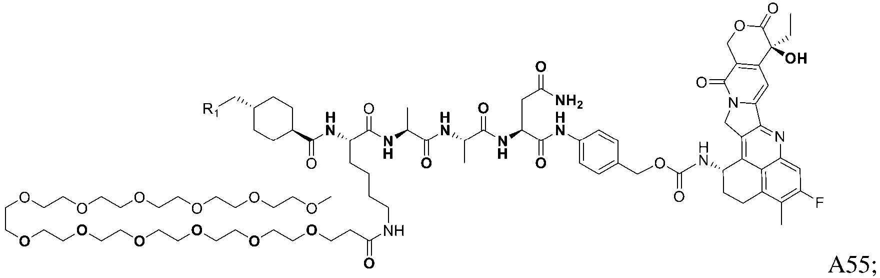 wherein in each formula, R₁ is as defined in any embodiment herein.

In some embodiments, the compound of formula (I) is selected from the following compounds S1 to S59:

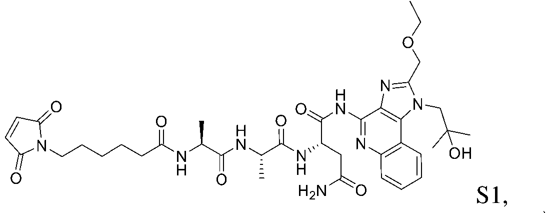

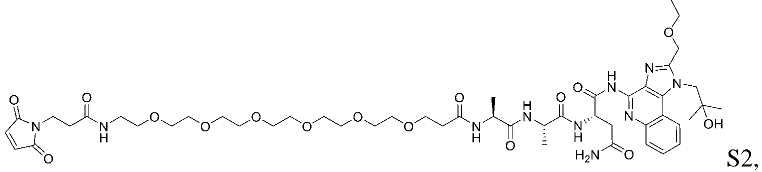

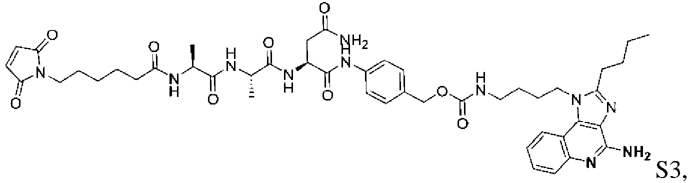

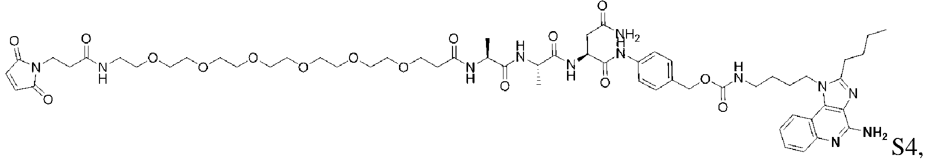

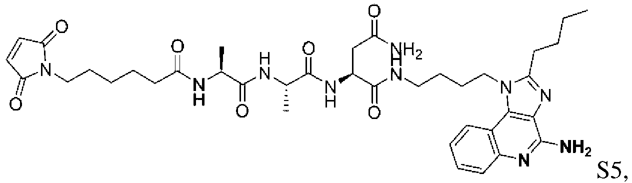

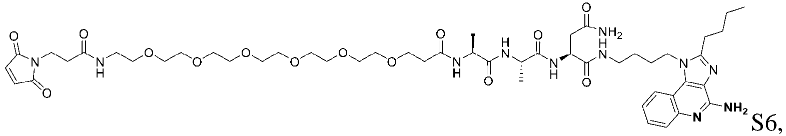

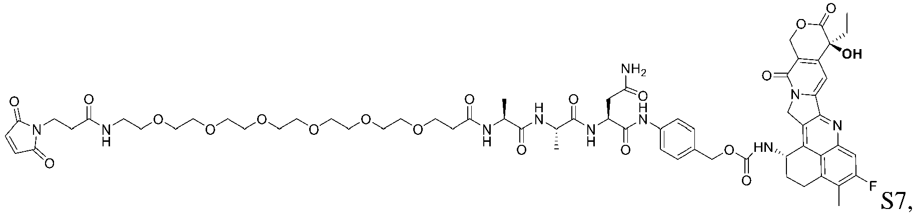

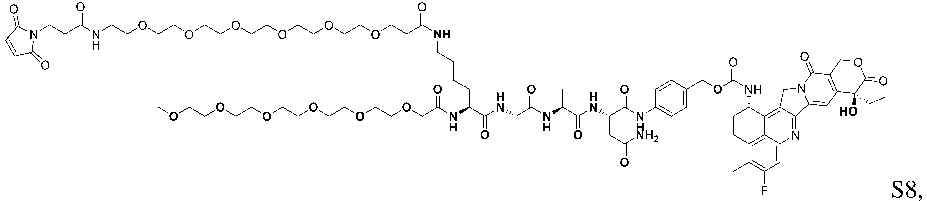

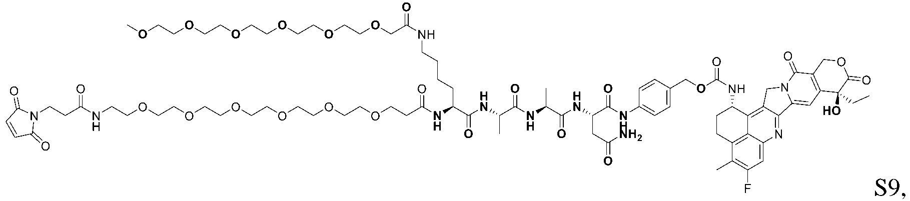

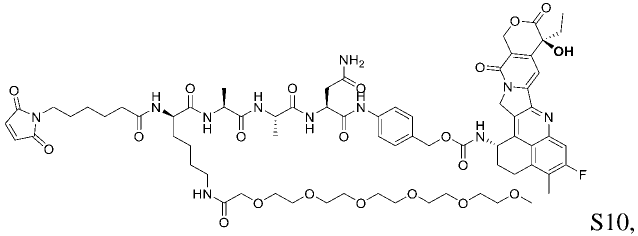

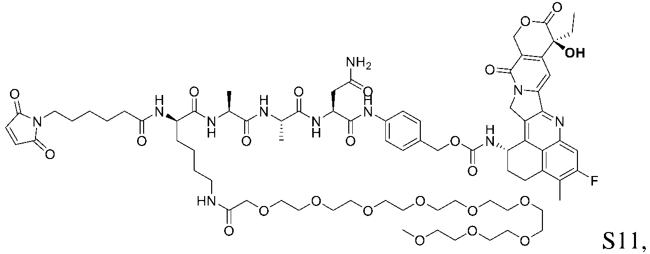

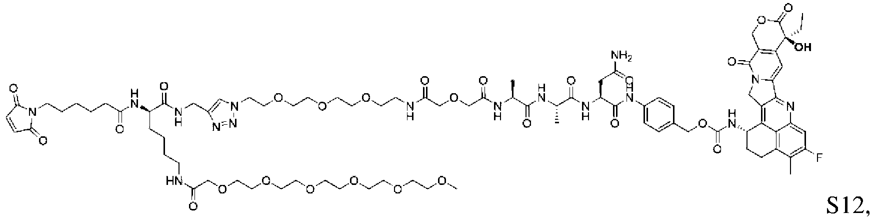

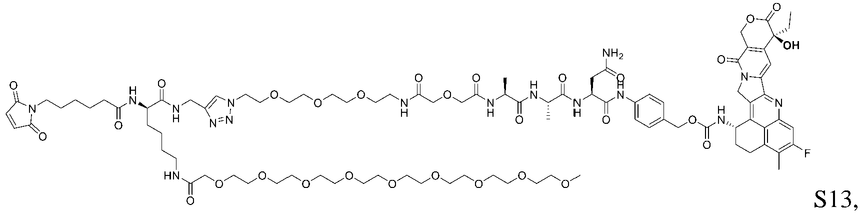

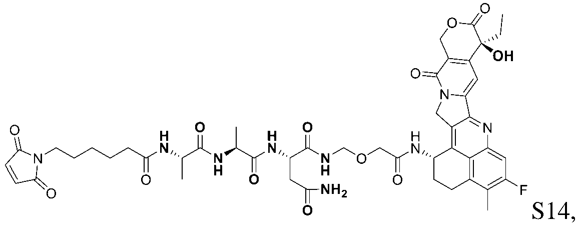

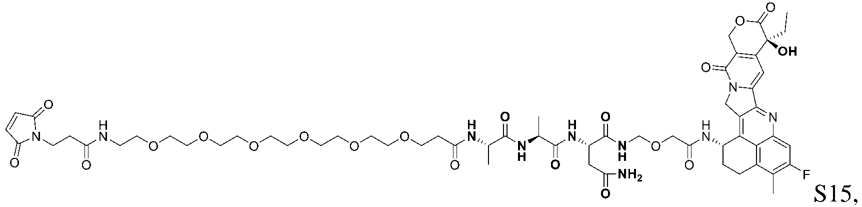

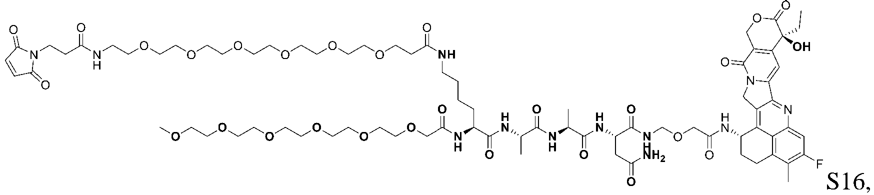

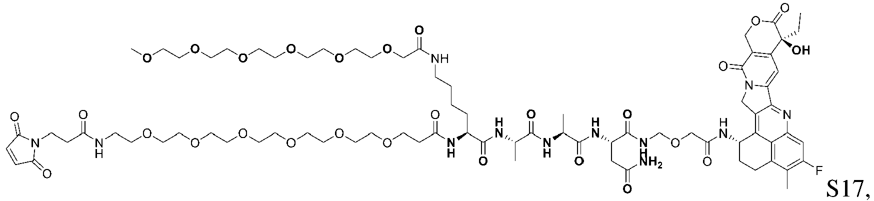

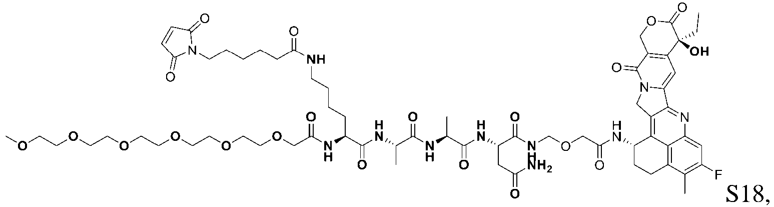

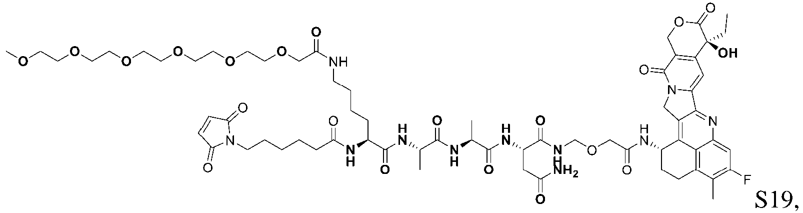

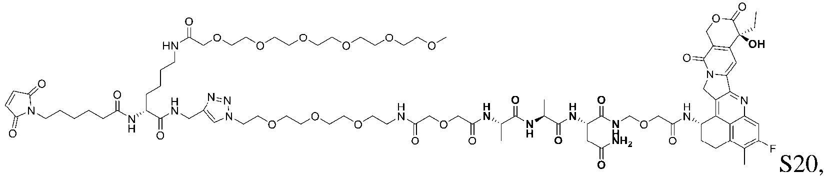

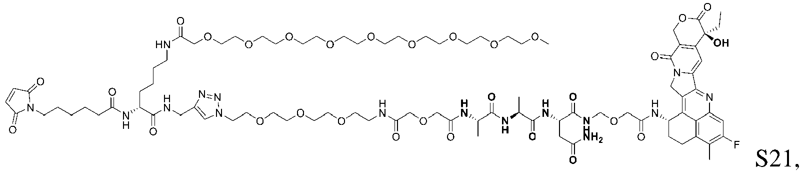

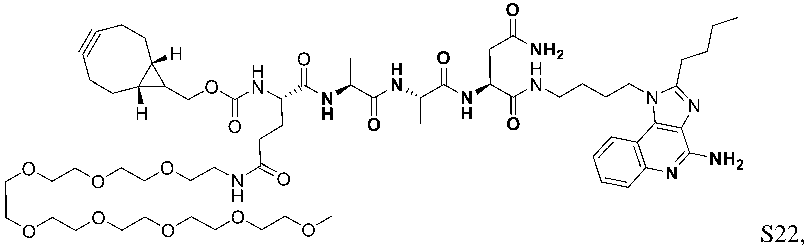

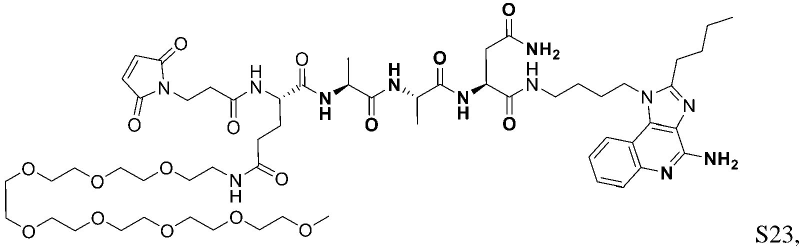

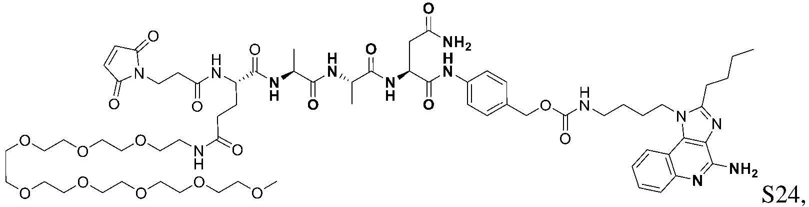

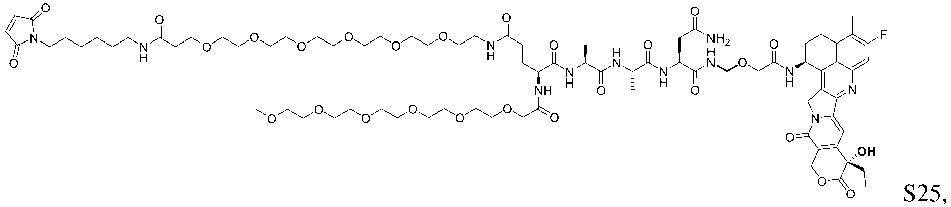

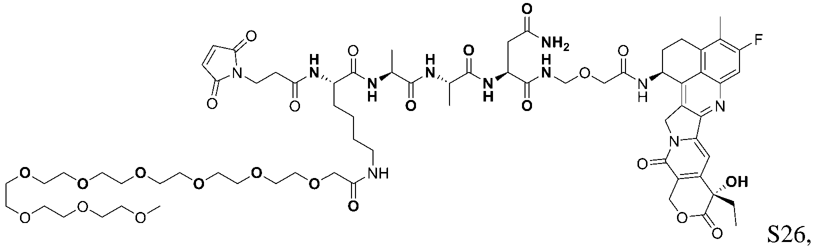

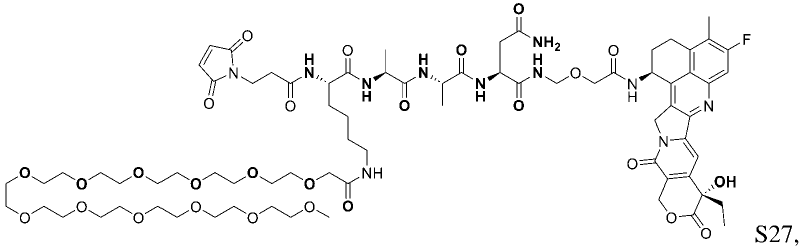

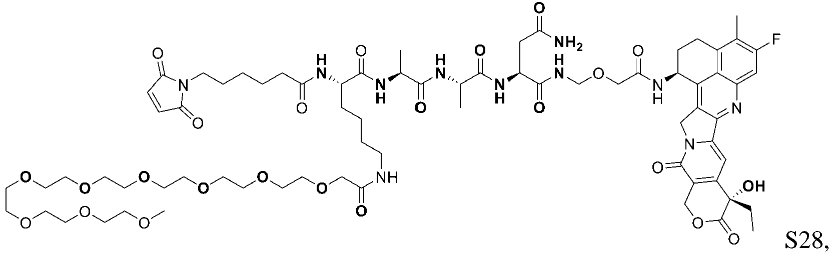

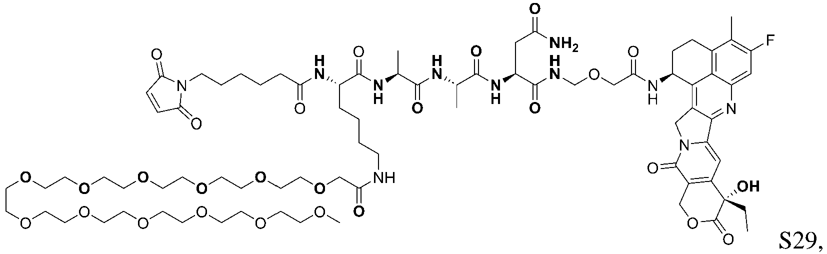

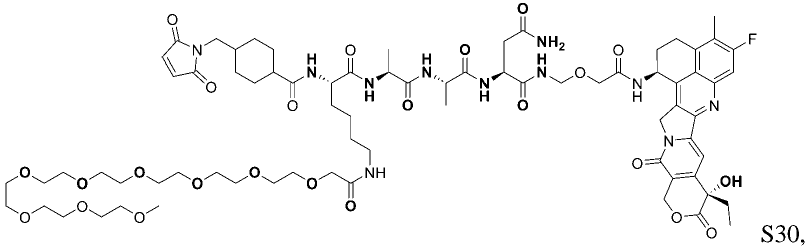

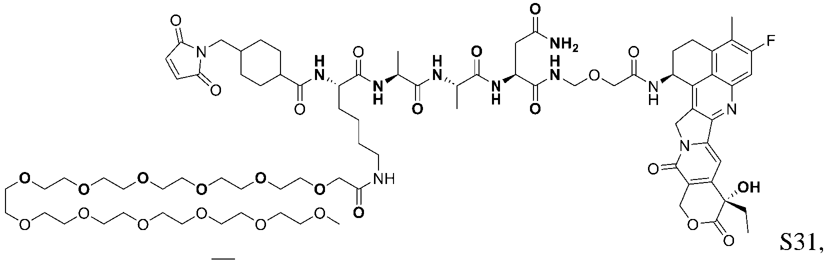

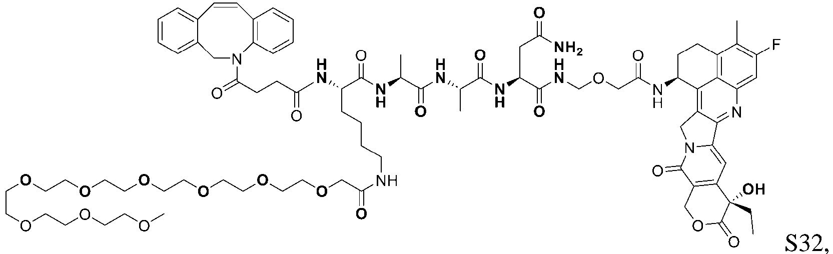

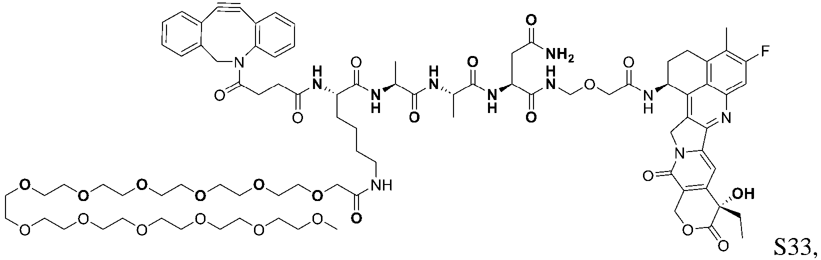

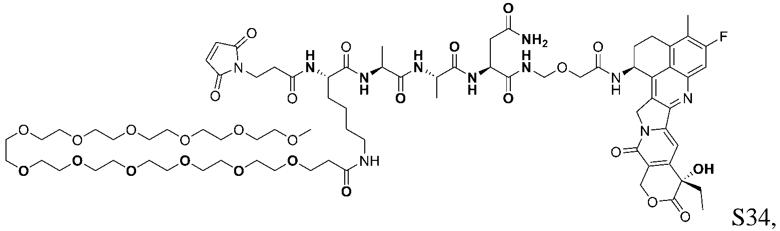

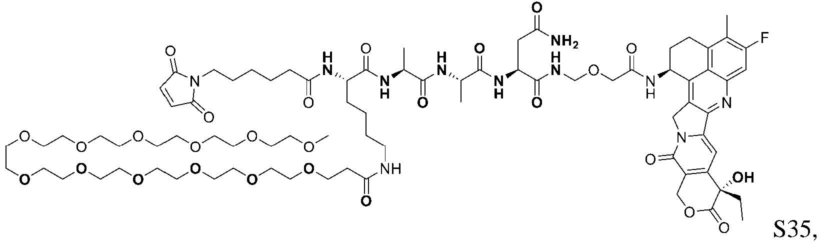

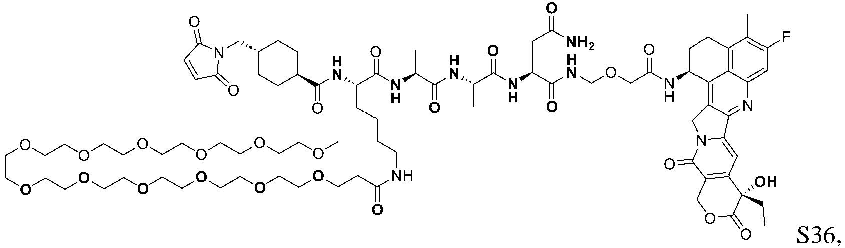

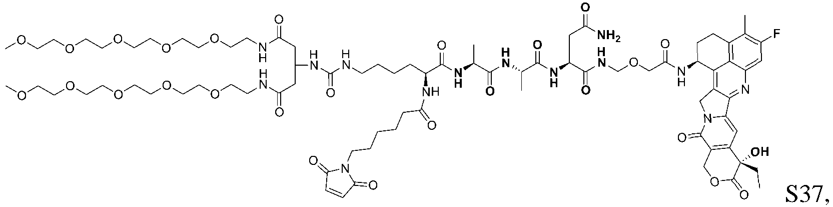

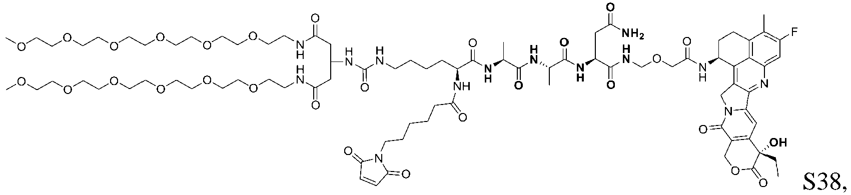

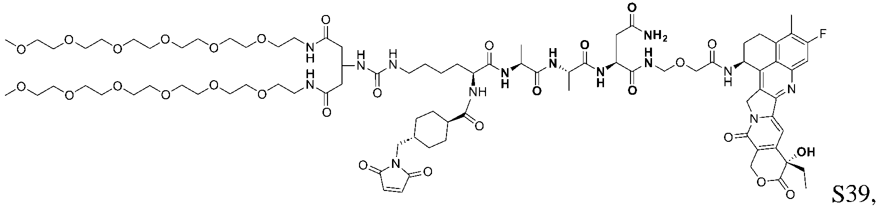

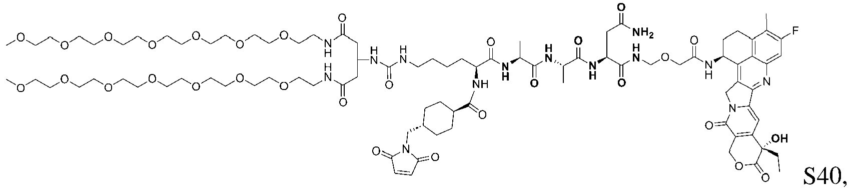

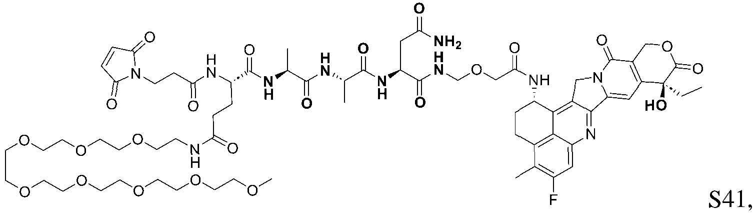

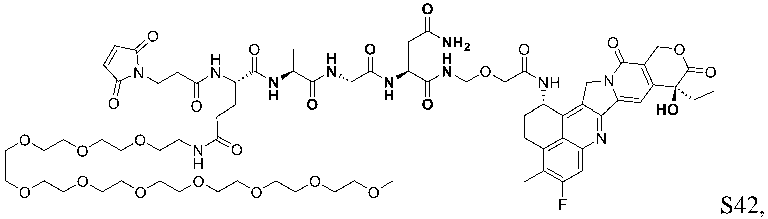

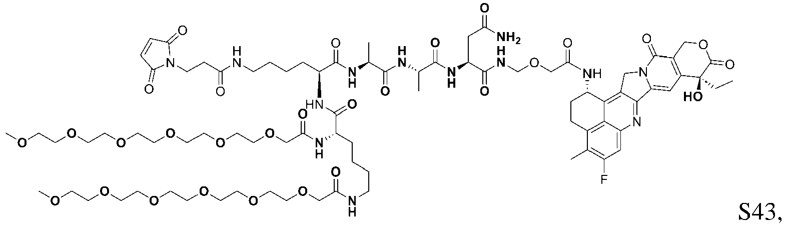

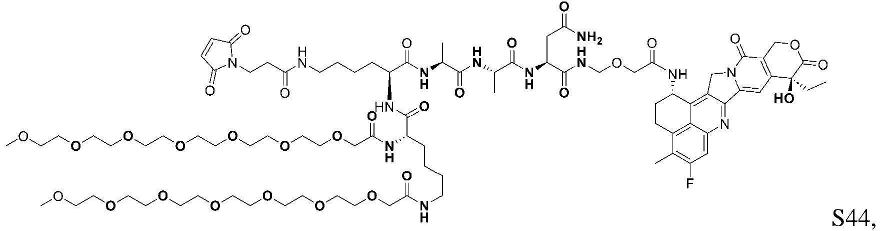

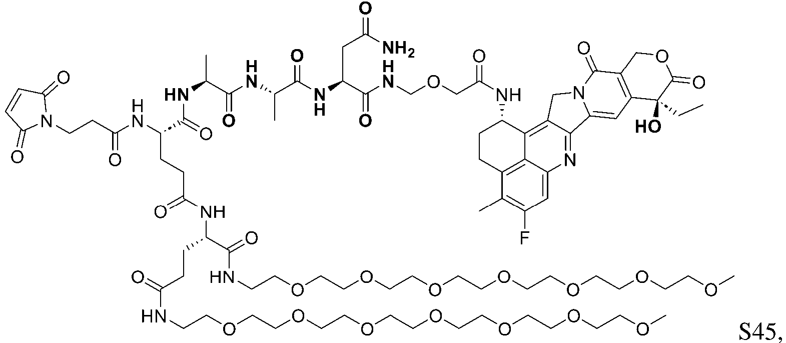

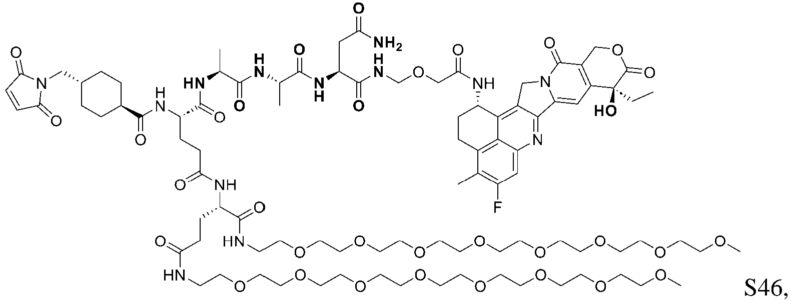

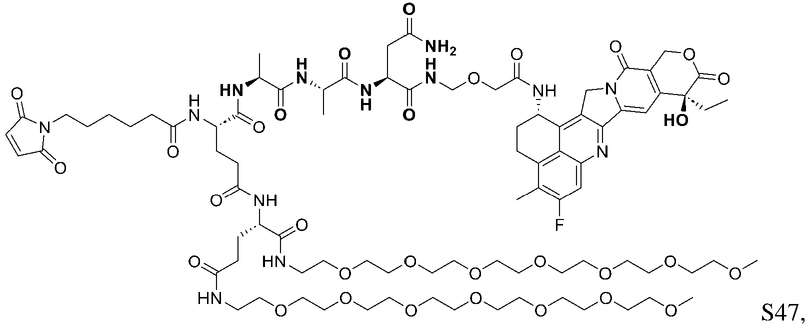

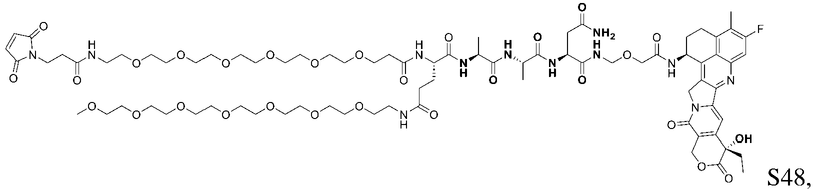

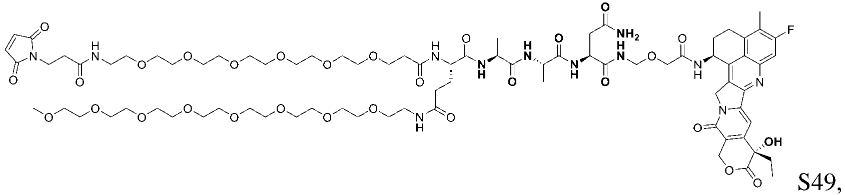

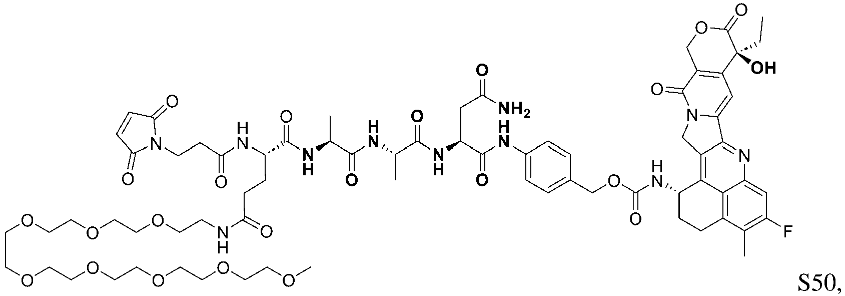

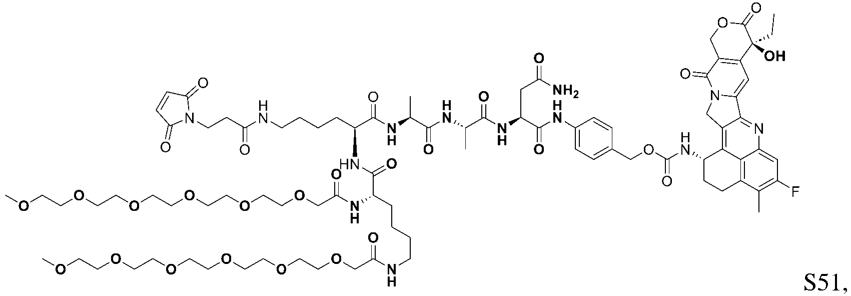

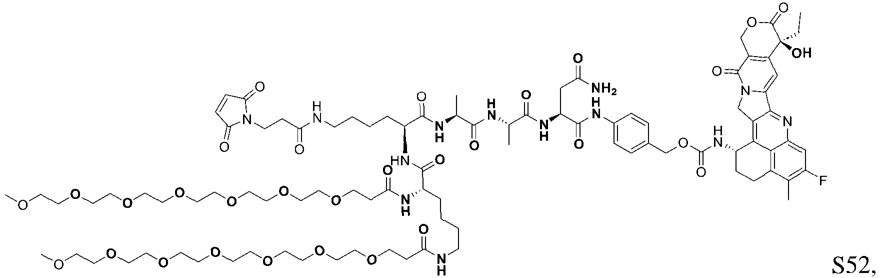

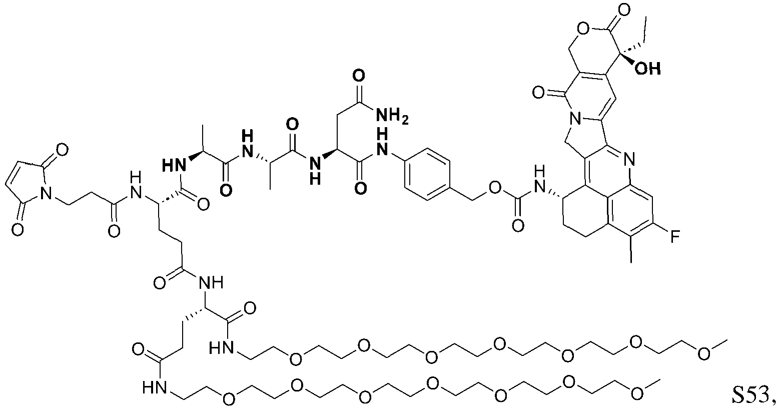

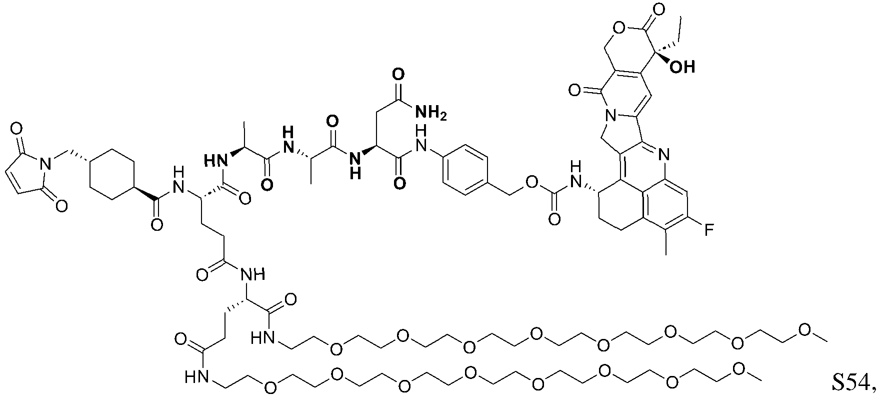

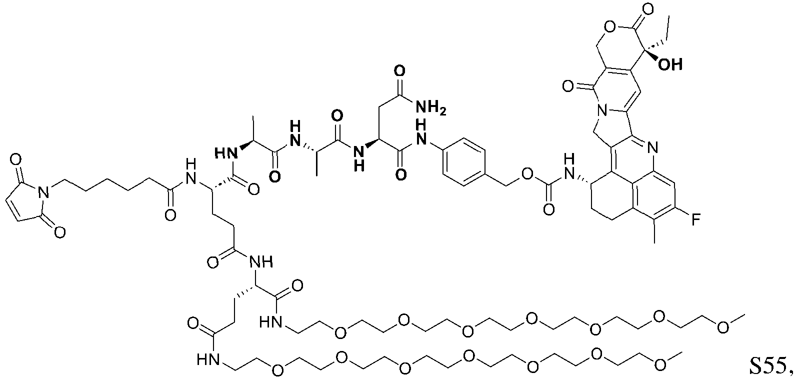

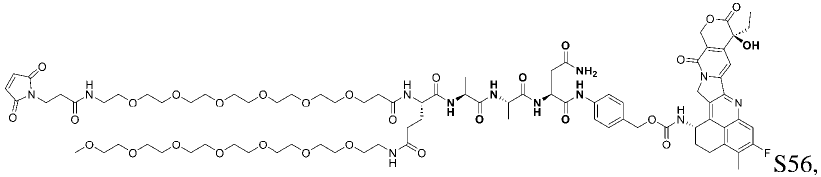

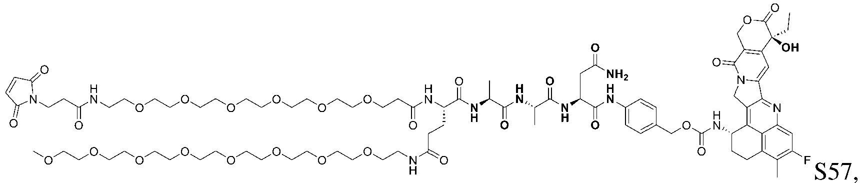

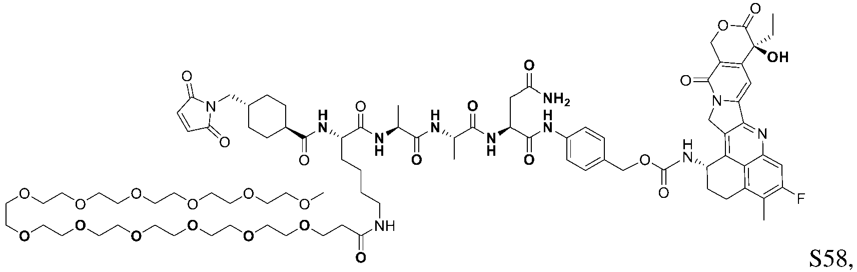

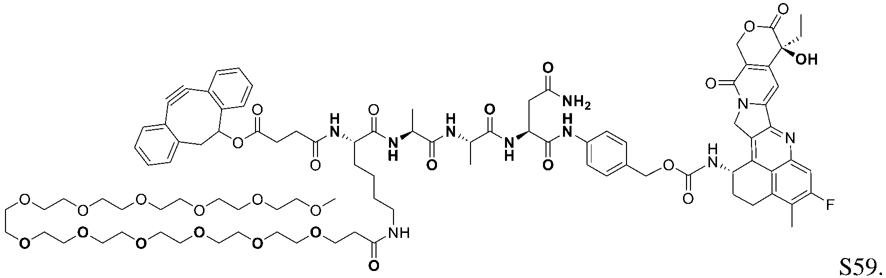

In some embodiments, the drug conjugate described herein has the chemical structure of formula (II): R₂'-D, wherein R₂' is an adjustment chemical structure for enhancing structure-activity relationship, and D is as shown in any one of X1-X5.

Preferably, said R₂' is selected from: C₁₋₆ alkyl-CO-*; C₁₋₆ alkyl-O-*; C₁₋₆ alkyl-OCONH-C₁₋₆ alkylene-(O-CH₂CH₂-O)ₙ-C₁₋₆ alkylene-CO-*; C₁₋₆ alkyl-OCONH-C₁₋₆ alkylene-(O-CH₂CH₂-O)ₙ-C₁₋₆ alkylene-O-*C₁₋₆ alkyl-OCONH-C₁₋₆ alkylene-(O-CH₂CH₂-O)ₙ-C₁₋₆ alkylene-NH-*(O-CH₂CH₂-O)ₙ-C₁₋₆ alkylene-CO-*; C₁₋₆ alkyl-CONH-C₁₋₆ alkylene-(O-CH₂CH₂-O)ₙ-C₁₋₆ alkylene-CO-*; NH₂-C₁₋₆ alkylene-(O-CH₂CH₂-O)ₙ-C₁₋₆ alkylene-CO-*; NH₂-C₁₋₆ alkylene-(O-CH₂CH₂-O)ₙ-C₁₋₆ alkylene-NH-*; NH₂-C₁₋₆ alkylene-(O-CH₂CH₂-O)ₙ-C₁₋₆ alkylene-O-*; NH₂-C₁₋₅₅ alkylene-CO-*; NH₂-C₁₋₅₅alkylene-NH-*; NH₂-C₁₋₅₅alkylene-O-*; HOOC-C₁₋₅₅ alkylene-CO-*; HOOC-C₁₋₆ alkylene-CONH-C₁₋₆ alkylene-(O-CH₂CH₂-O)ₙ-C₁₋₆ alkylene-CO-*; HOOC-C₁₋₆ alkylene-CONH-C₁₋₆ alkylene-(O-CH₂CH₂-O)ₙ-C₁₋₆ alkylene-NH-*; HOOC-C₁₋₆ alkylene-CONH-C₁₋₆ alkylene-(O-CH₂CH₂-O)ₙ-C₁₋₆ alkylene-O-*; HOOC-C₁₋₆ alkylene-NH-*; HOOC-C₁₋₆ alkylene-NHCO-C₁₋₆ alkylene substituted with SO₃H-NH-*; N₃-C₁₋₆ alkylene-CO-*; N₃-C₁₋₆ alkylene-(O-CH₂CH₂-O)ₙ-C₁₋₆ alkylene-NHCO-C₁₋₆ alkylene-O-C₁₋₆ alkylene-CO-*; HOOC-C₁₋₆ alkylene-O-C₁₋₆ alkylene-CO-*; 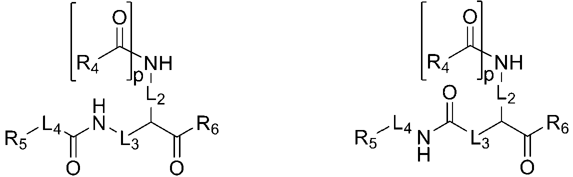 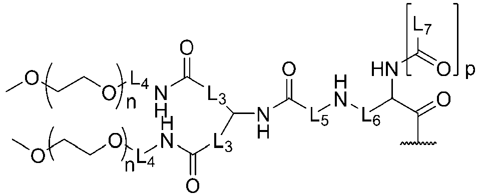 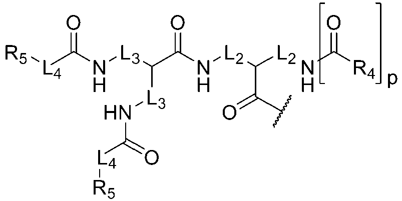 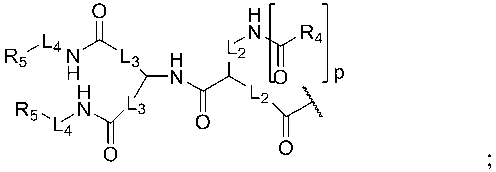 wherein:
* indicates the position of attachment to D;
R₄ is selected from: C₁₋₆ alkyl; C₁₋₆ alkyl-C₃₋₈ cycloalkylene-; COOH-C₁₋₆ alkylene-; C₁₋₆ alkyl-(O-CH₂CH₂-O)ₘ-C₁₋₆ alkylene-; NH₂-C₁₋₆ alkylene; C₁₋₆ alkyl-CONH-C₁₋₆ alkylene-(O-CH₂CH₂-O)ₘ-C₁₋₆ alkylene-; and NH₂-C₁₋₆ alkylene-C₃₋₈ cycloalkylene;
R₅ is selected from: C₁₋₆ alkyl-(O-CH₂CH₂-O)ₘ-; C₁₋₆ alkyl-CONH-C₁₋₆ alkylene-(O-CH₂CH₂-O)ₘ-; COOH-C₁₋₆ alkylene-(O-CH₂CH₂-O)ₙ-;
R₆ is absent, or is -NH-C₁₋₆ alkylene-triazolyl-C₁₋₆ alkylenecarbonyl, or -NH-C₁₋₆ alkylene-triazolyl-C₁₋₆ alkylene-(O-CH₂CH₂-O)ₙ-C₁₋₆ alkylene-NHCO-C₁₋₆ alkyleneoxy-C₁₋₆ alkylenecarbonyl;
L₂ and L₃ are each independently a bond or C₁₋₆ alkylene;
L₄ is each independently C₁₋₆ alkylene;
L₅ is a bond, C₁₋₆ alkyleneoxy-C₁₋₆ alkylenecarbonyl or C₁₋₆ alkylenecarbonyl;
L₆ is C₁₋₆ alkylene;
L₇ is C₁₋₆ alkylene or -C₃₋₈ cycloalkylene-C₁₋₆ alkylene;
each n and m is independently an integer from 1 to 50, and p is 0 or 1; wherein when p is 0, the -NH-[CO-R₄]ₚ group is -NH₂.

Preferably, R₂' is selected from: 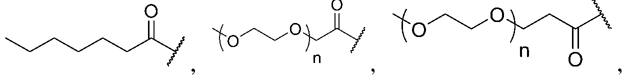 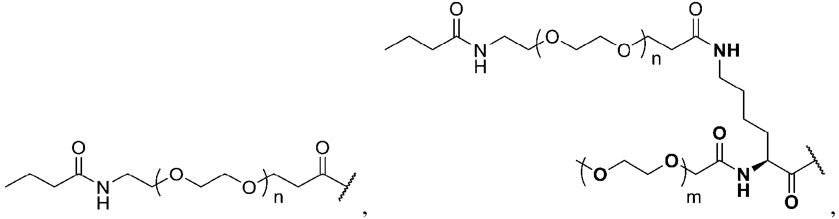 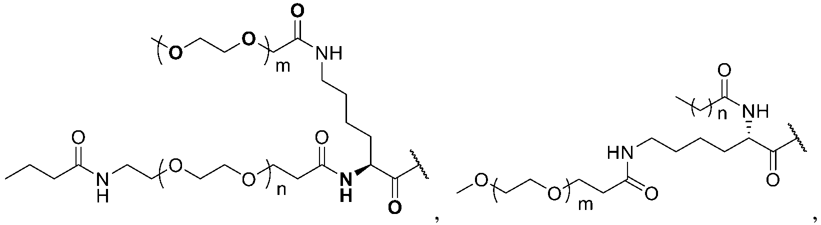 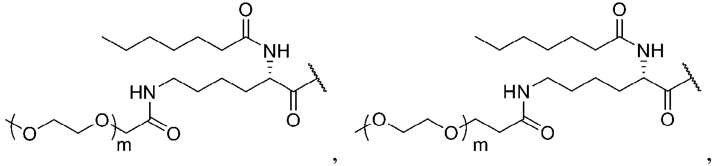 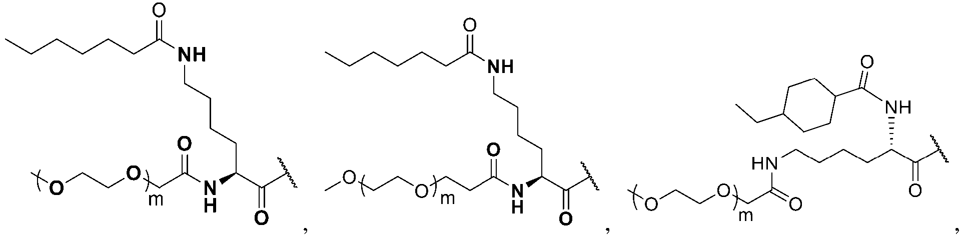 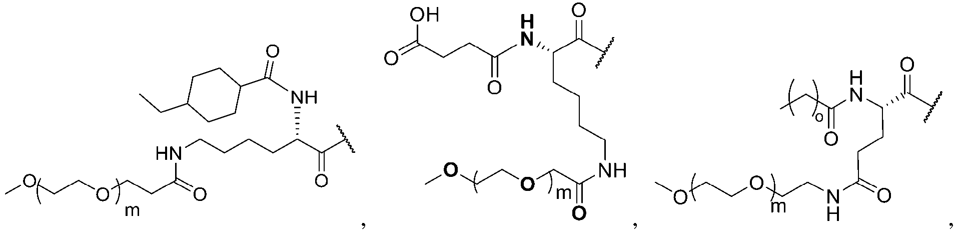 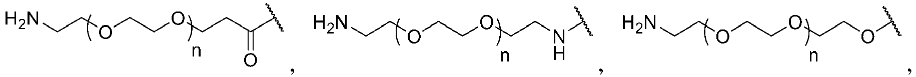 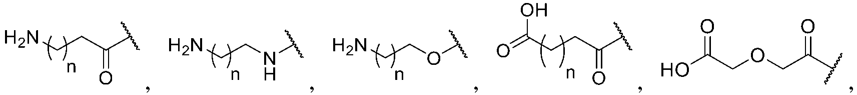 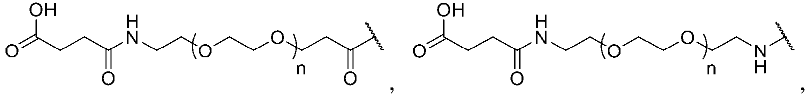 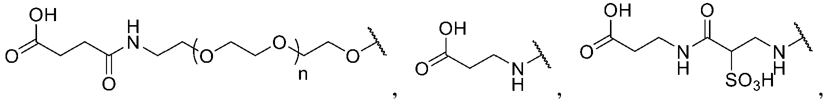 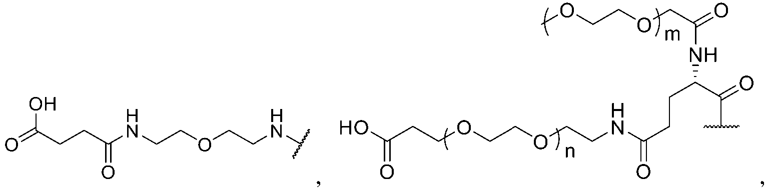 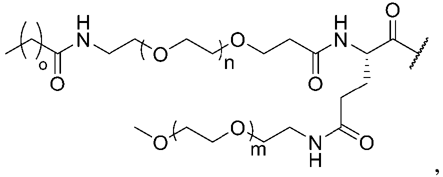 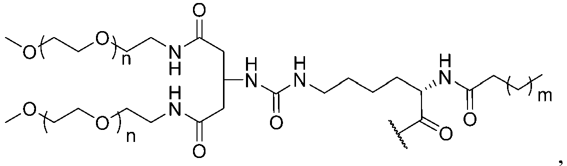 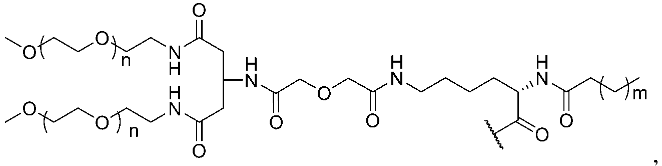 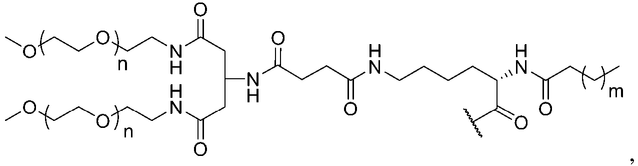 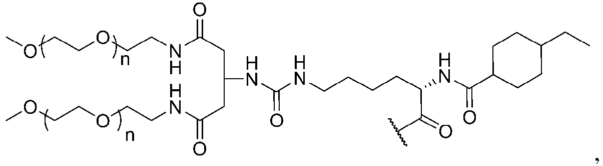 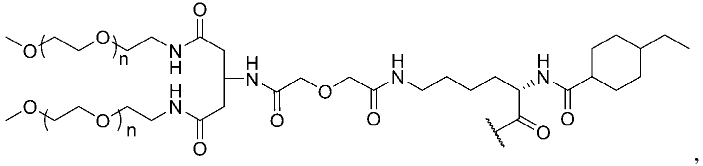 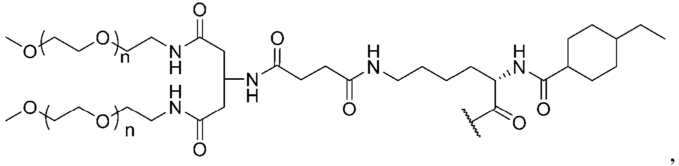 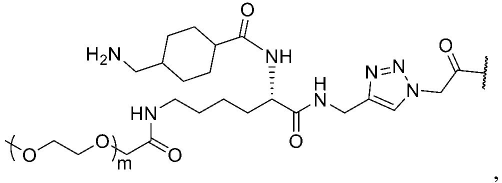 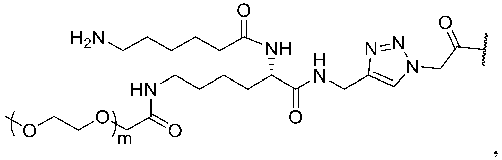 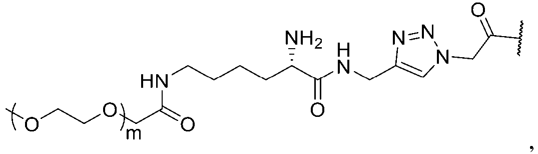 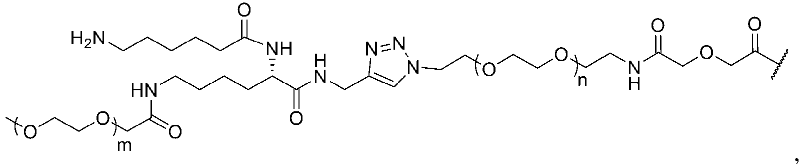 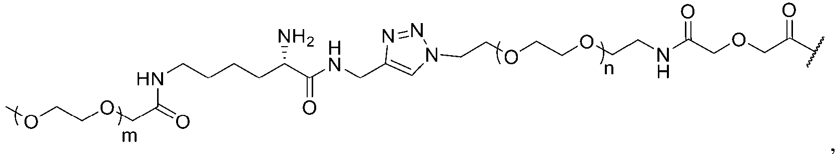 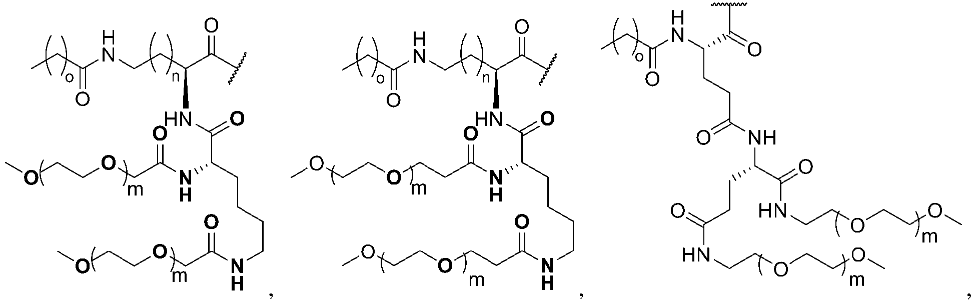 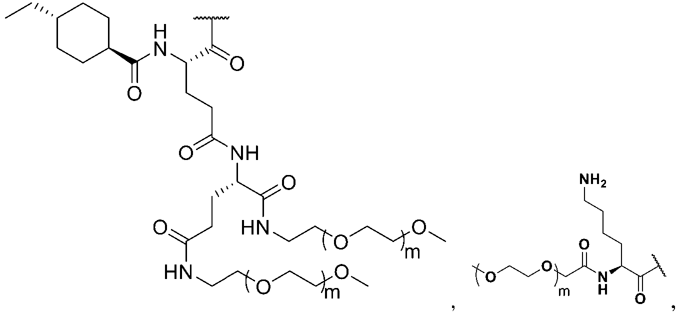 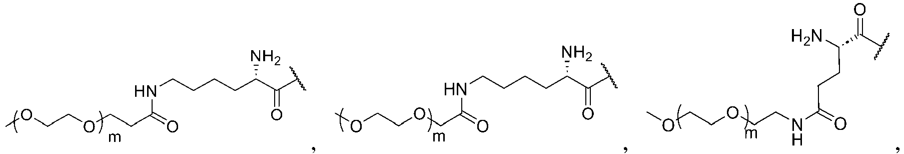 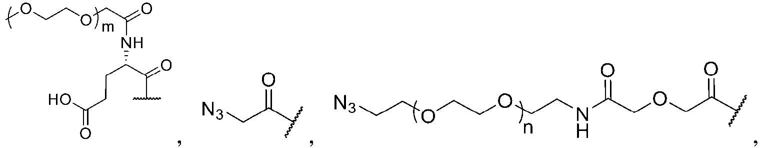 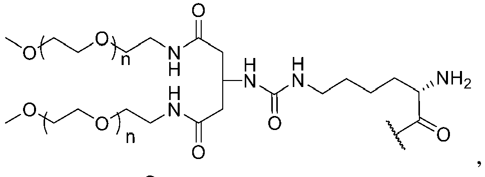 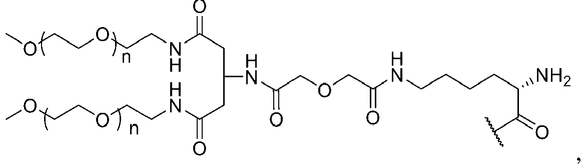 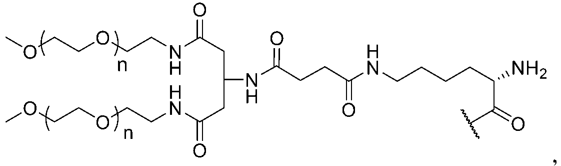 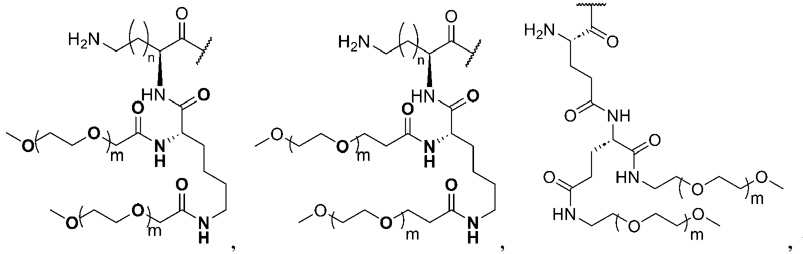 wherein o is independently an integer from 1 to 20, and m and n are each independently an integer from 1 to 50.

More preferably, R₂' is selected from: 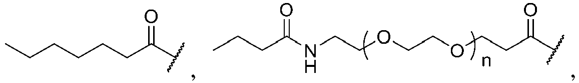 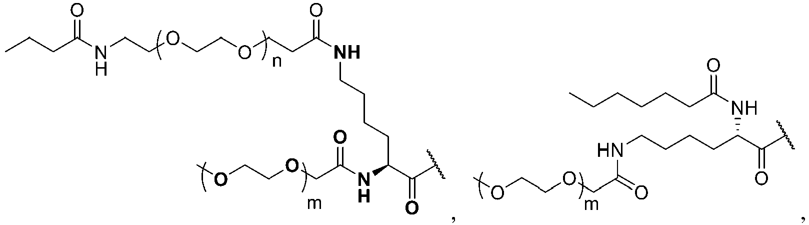 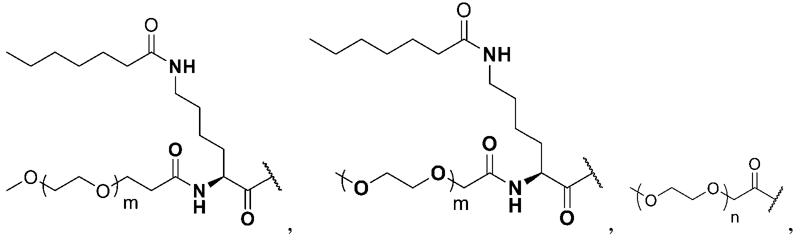 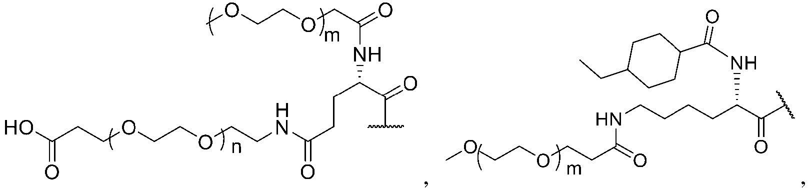 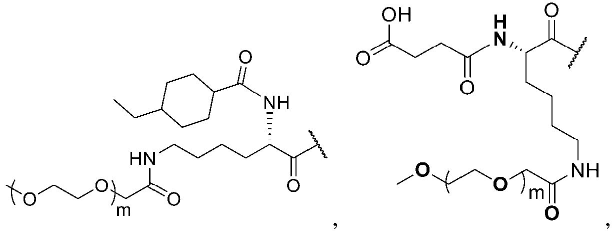 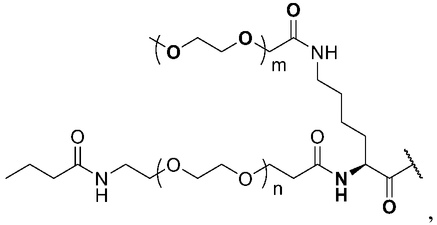 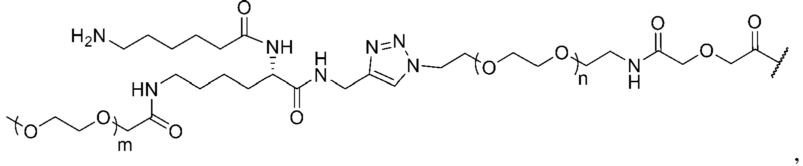 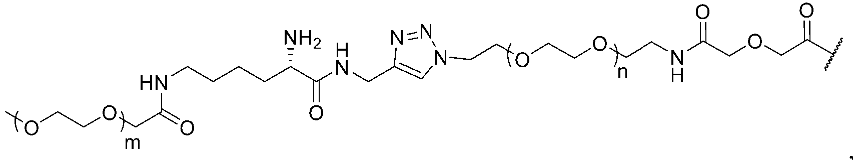 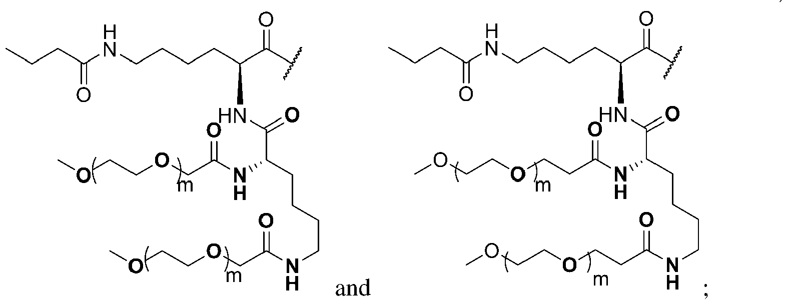 preferably, n and m are each independently an integer from 2 to 20.

In some embodiments, the drug conjugate of formula (II) is selected from:

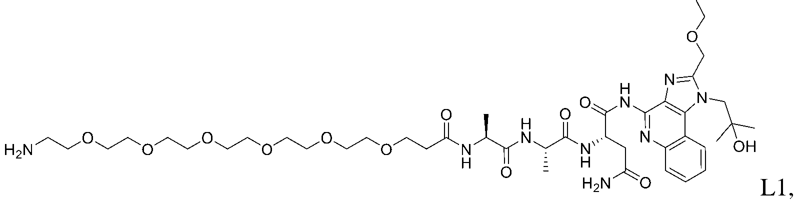

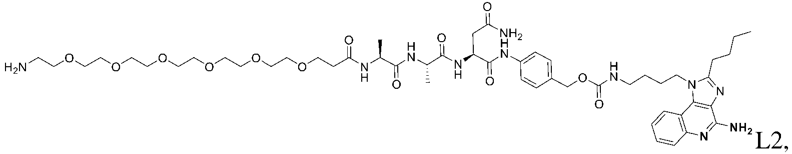

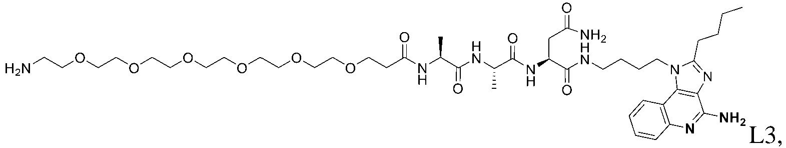

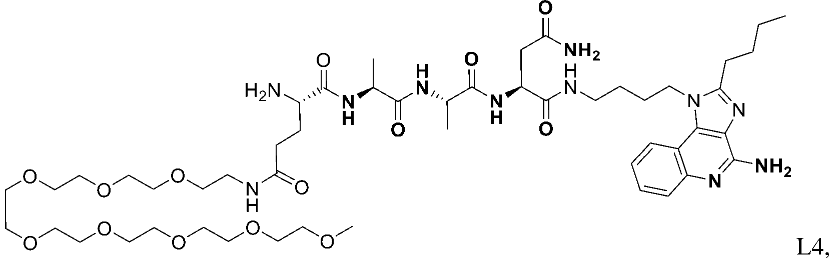

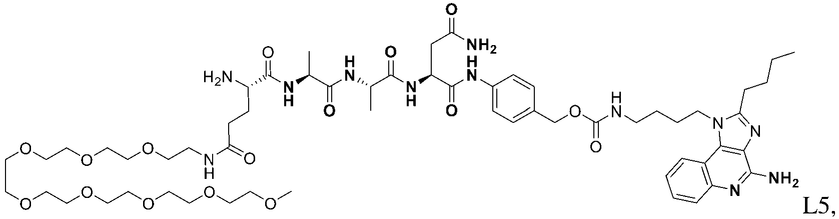

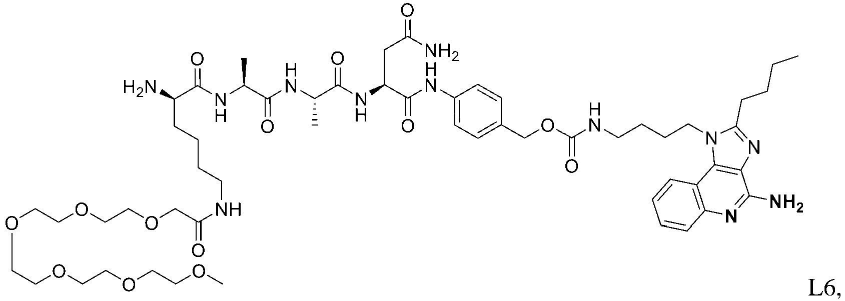

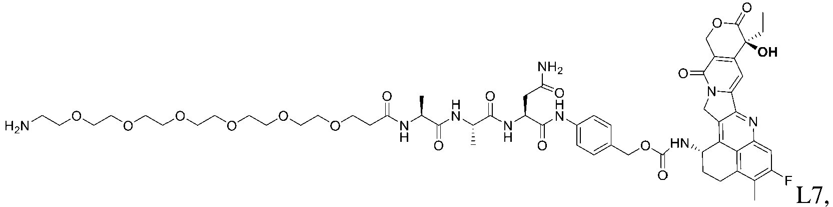

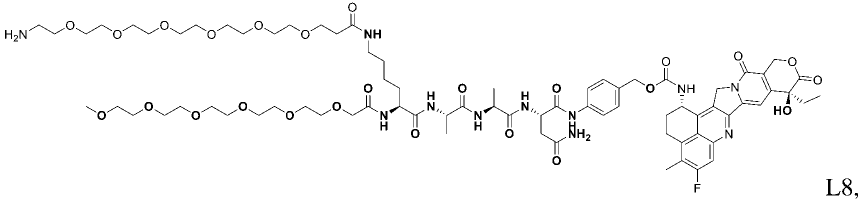

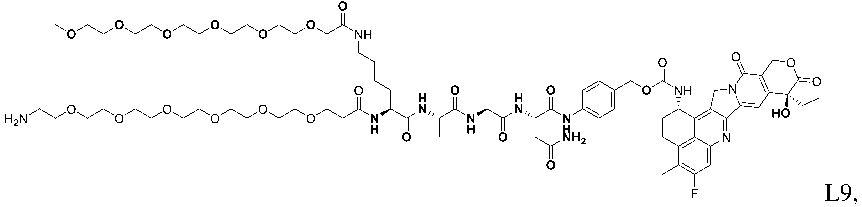

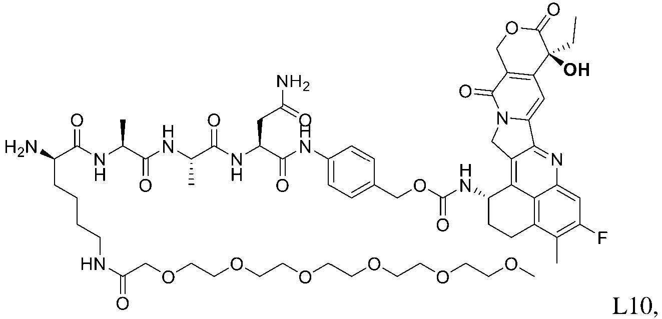

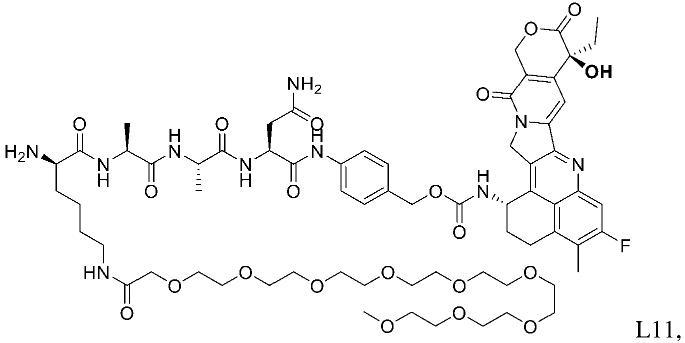

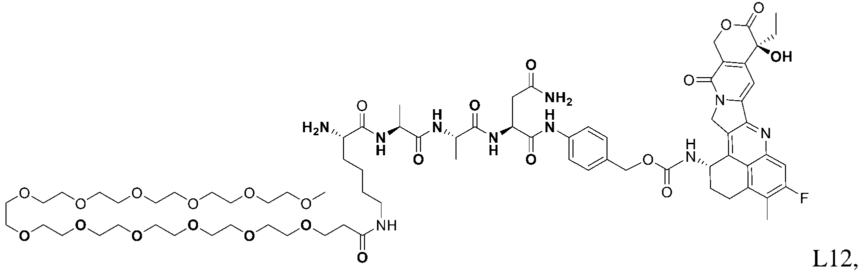

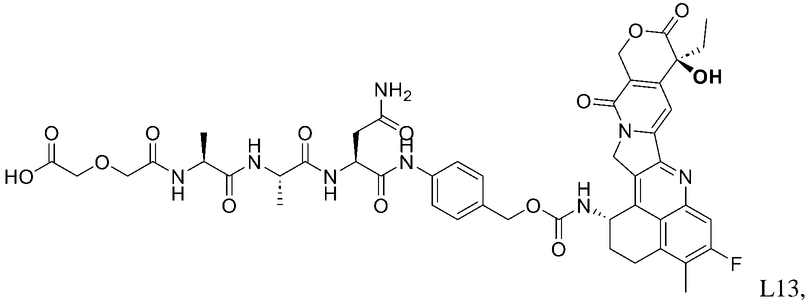

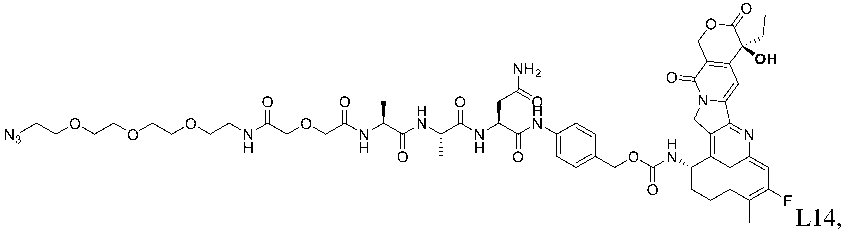

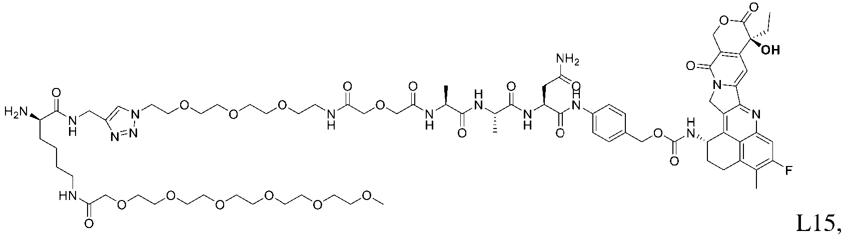

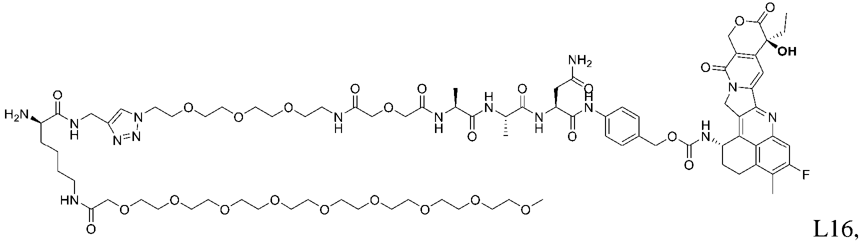

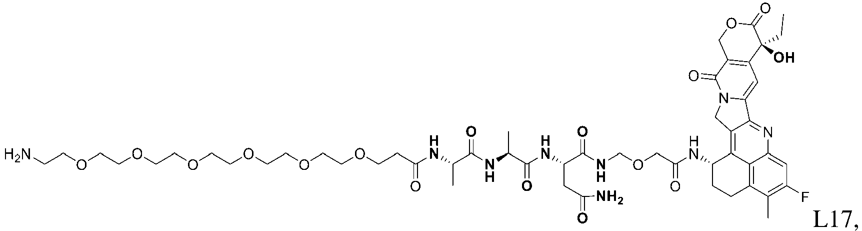

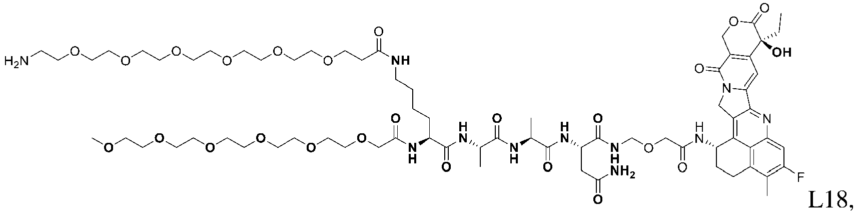

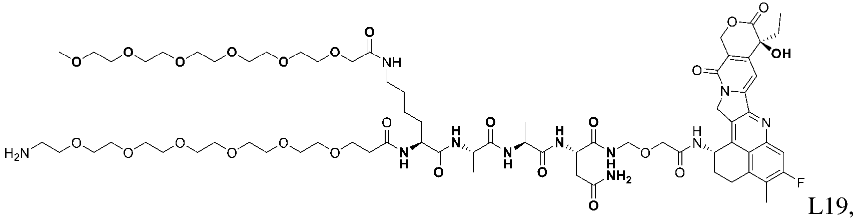

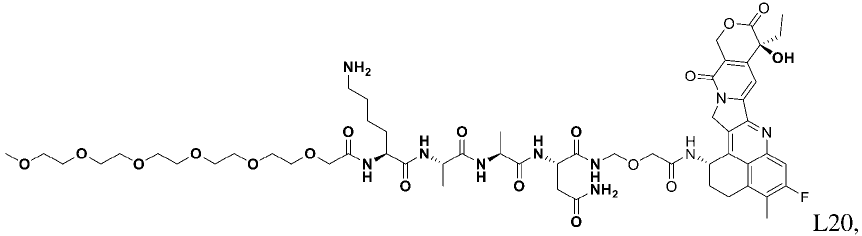

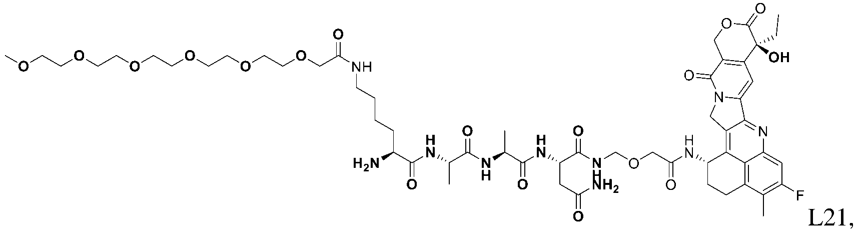

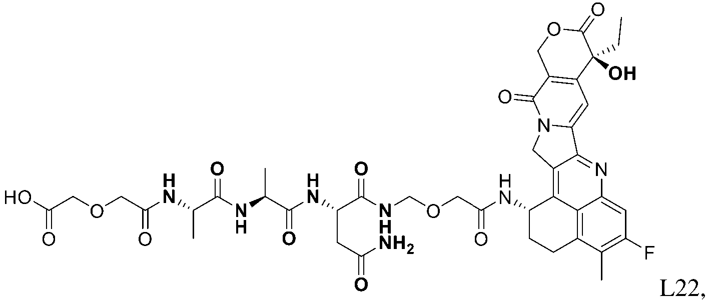

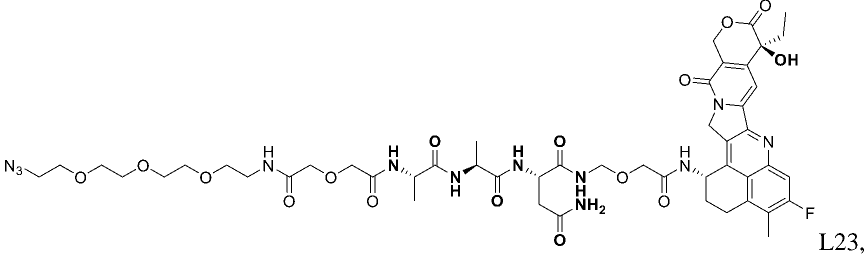

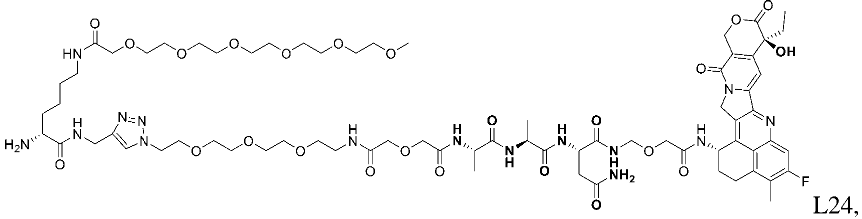

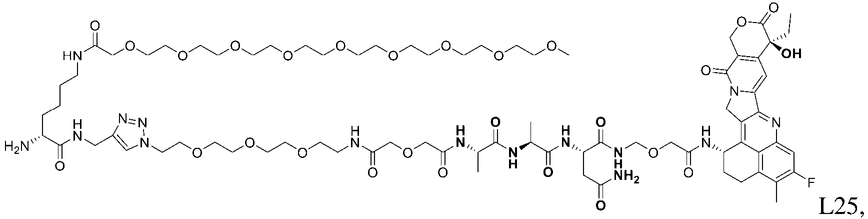

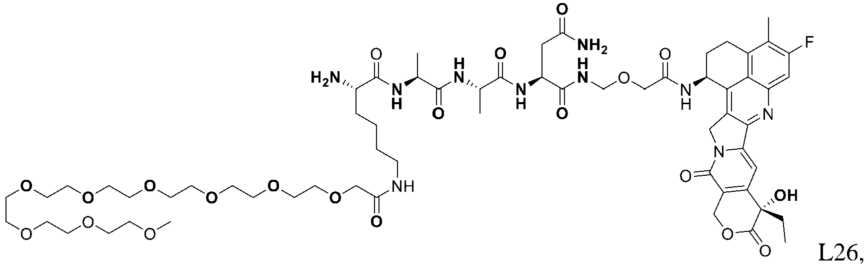

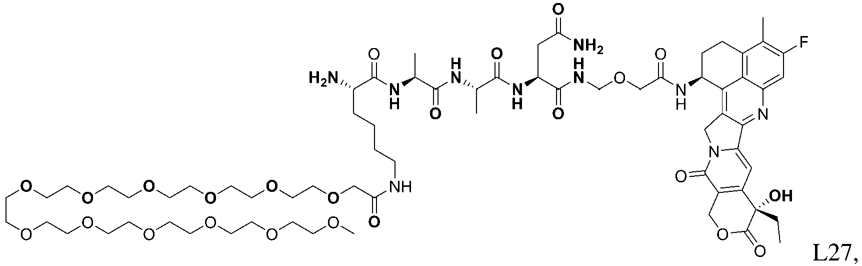

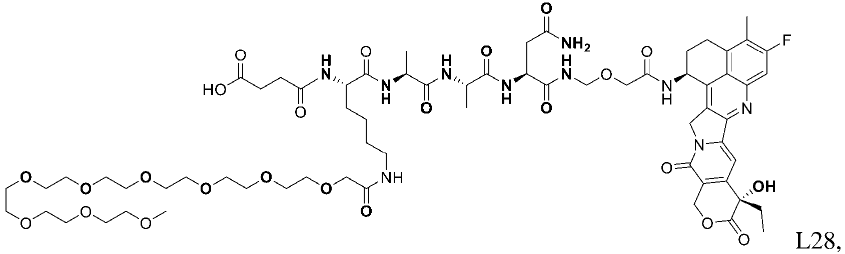

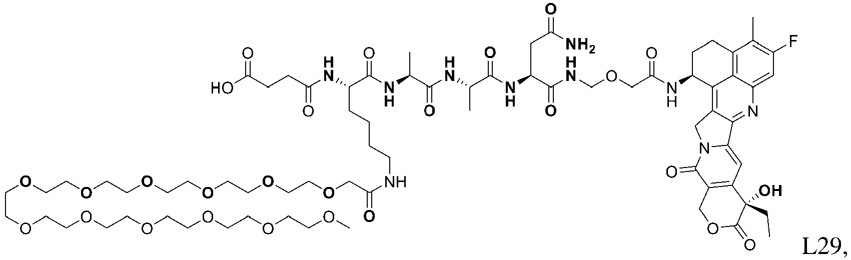

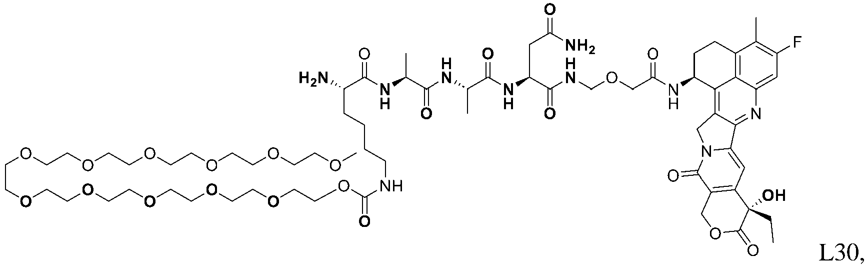

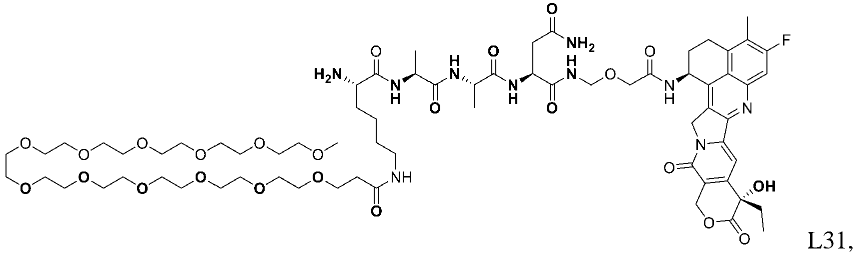

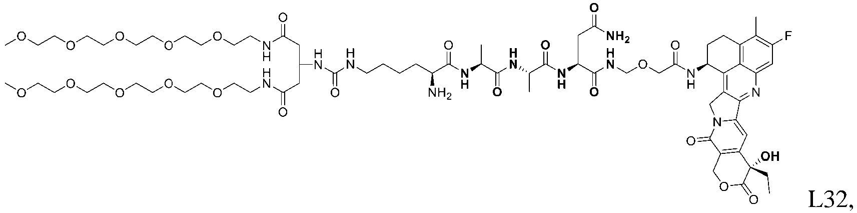

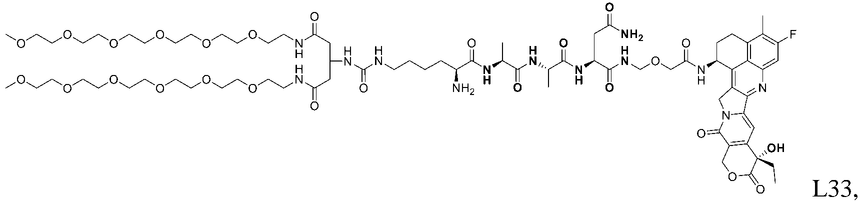

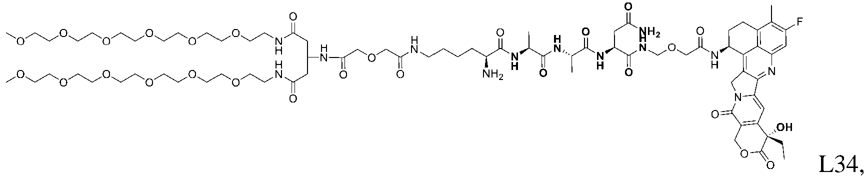

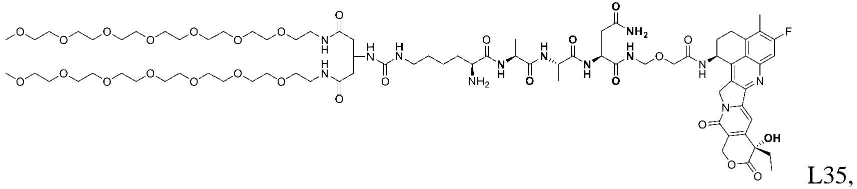

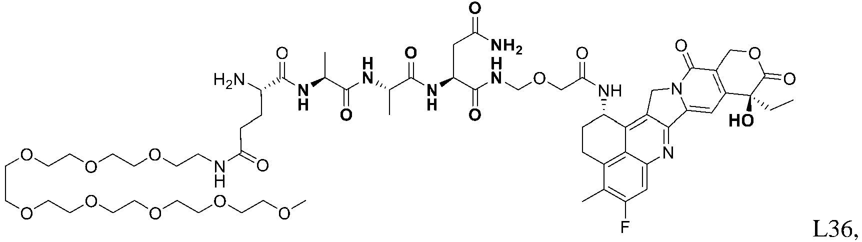

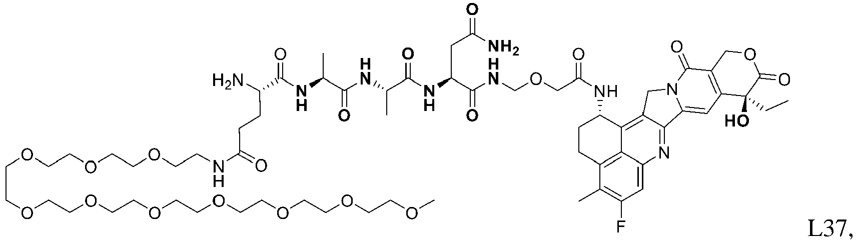

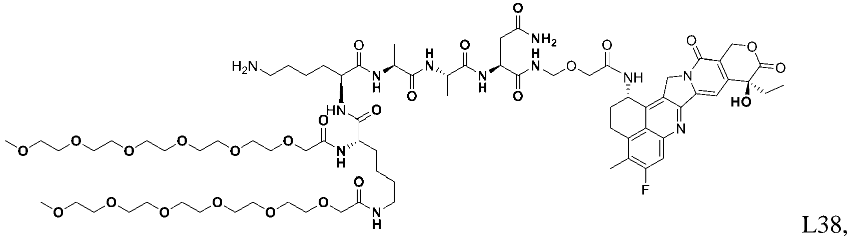

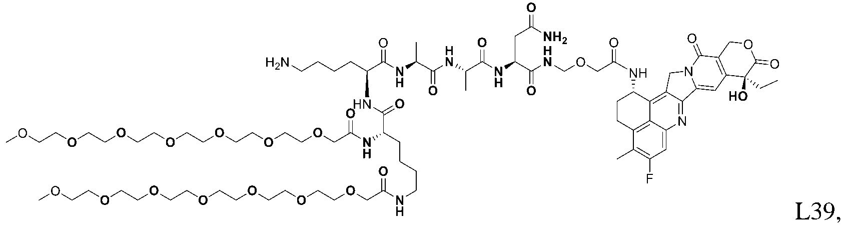

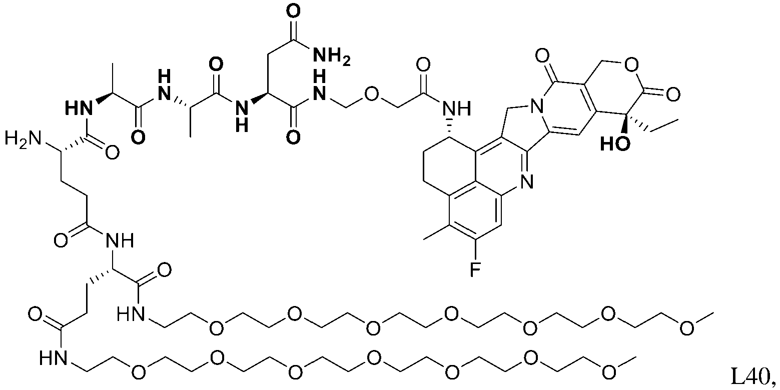

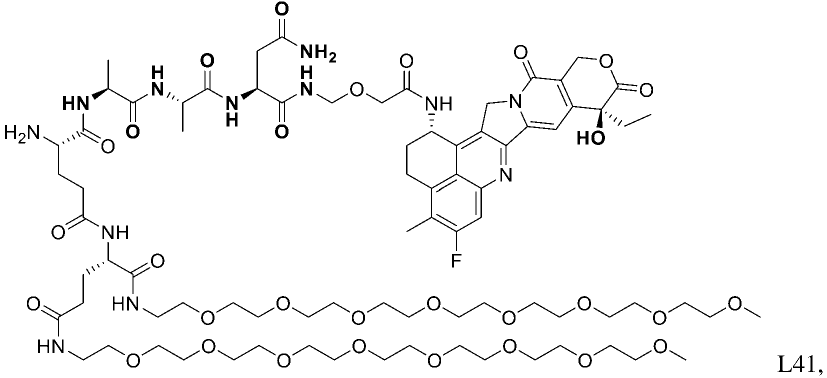

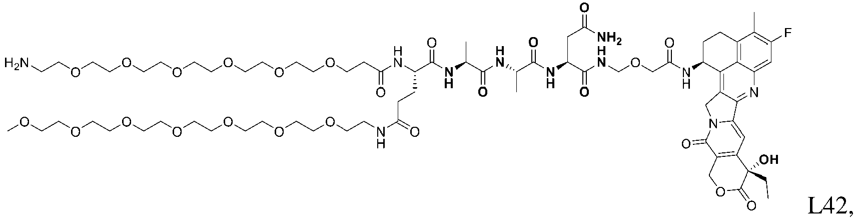

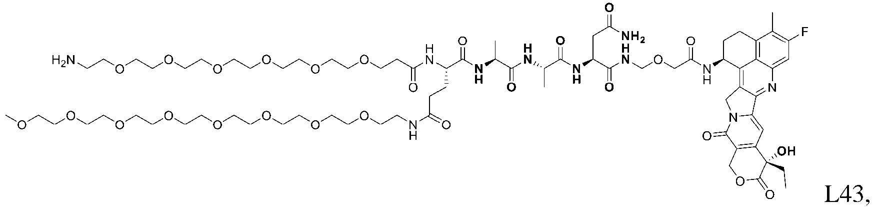

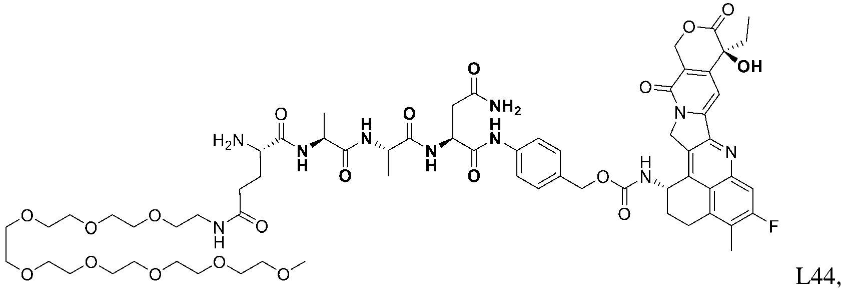

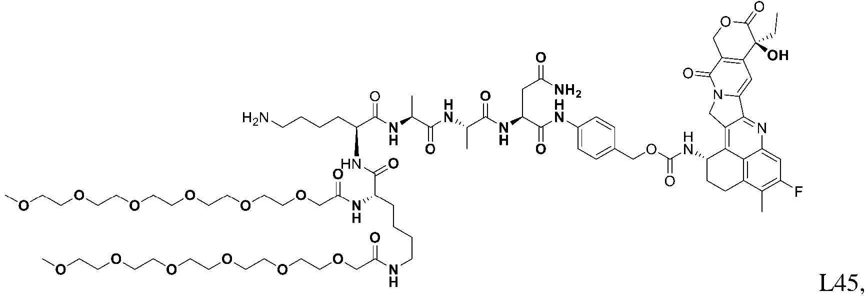

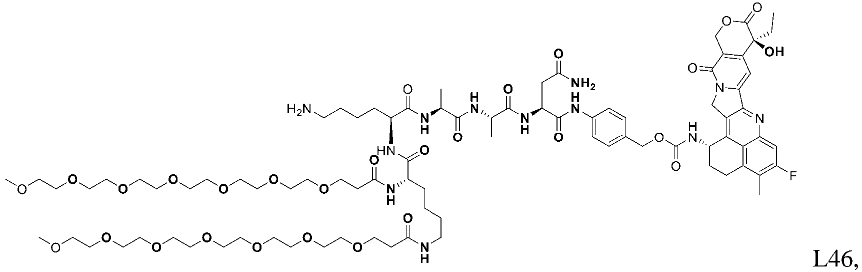

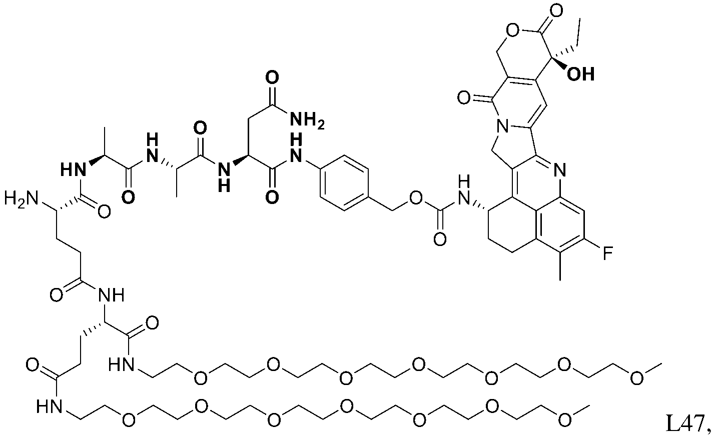

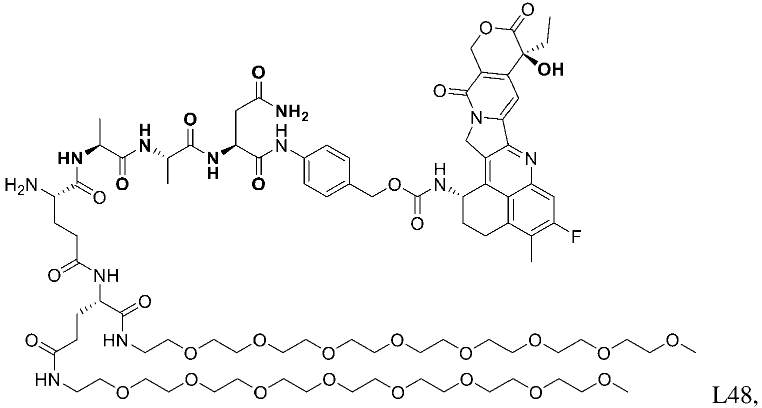

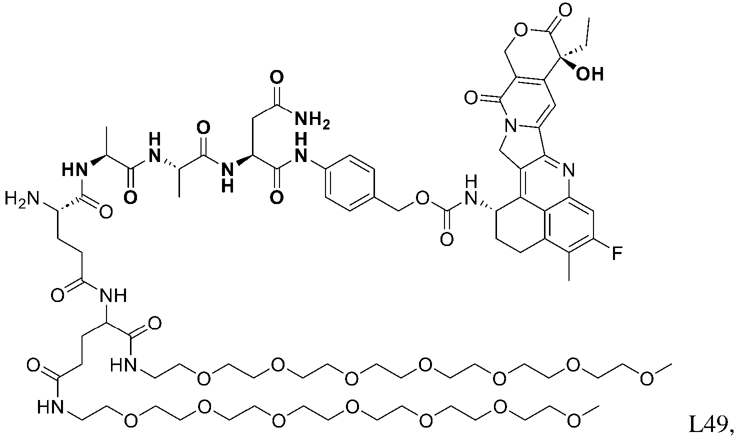

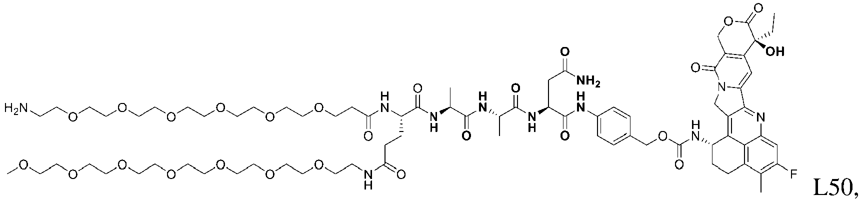

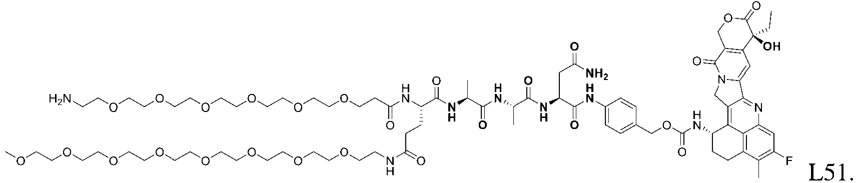

### Antibody-Drug Conjugates

Provided herein is an antibody-drug conjugate of formula (III) below, a stereoisomer thereof, or a pharmaceutically acceptable salt thereof:

R₃-(R₁-R₂-D)ₓ Formula (III);

wherein: R₃ is an antibody; R₁, R₂, and D are as defined in any of the preceding embodiments; and x is an integer selected from 1 to 8.

In formula III, when R₁ is: 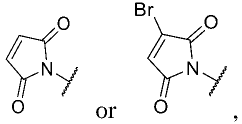 the attachment mode of R₃-R₁ is: 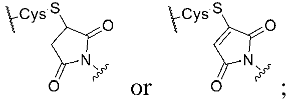 wherein S is the sulfur atom of a cysteine residue in R₃;
when R₁ is: 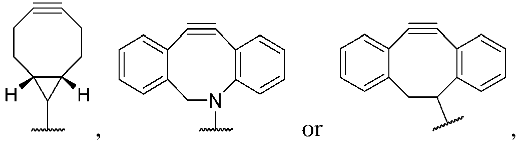 the attachment mode of R₃-R₁ is: 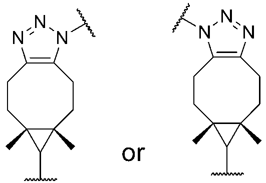 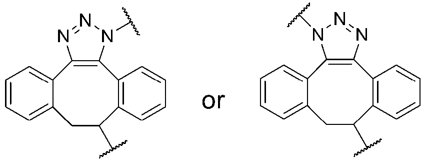 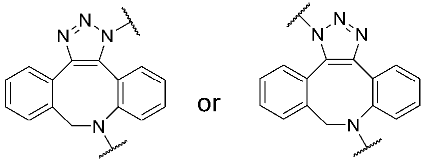 where R₃ is conjugated to the alkynyl group in the R₁ moiety.

An azido modification can be introduced into the glycans or reconstructed glycans of antibody, which can undergo a click reaction with DBCO (dibenzocyclooctyne) or BCN (bicyclo[6.1.0]nonyne) to form a triazole ring, thereby achieving the connection between R₁ and R₃.

The forms of the drug conjugate or the antibody-drug conjugate of the present disclosure comprise any one of the geometric isomers or mixtures thereof.

### Antibodies

Herein, the term "antibody" is used in its broadest sense and covers biological molecules such as monoclonal antibodies, polyclonal antibodies, dimers, multimers, multispecific antibodies (such as bispecific antibodies), and antibody fragments, as long as they exhibit the desired biological activity (Miller et al (2003) Jour. of Immunology 170:4854-4861).

Antibodies specifically bind to antigens and comprise at least two heavy (H) chains and two light (L) chains interconnected by disulfide bonds. Each heavy chain contains a variable region of heavy chain (VH) and a constant region of heavy chain (CH), with the constant region of heavy chain comprising three constant domains CH1, CH2, and CH3. Each light chain contains a variable region of light chain (VL) and a constant region of light chain (CL), with the constant region of light chain comprising one constant domain. The VH and the VL regions can be further subdivided into hypervariable regions known as complementarity determining regions (CDR), interspersed with more conserved regions known as framework regions (FR). Generally, from the N-terminal to the C-terminal, both the variable domains of light chain and the variable domains of heavy chain contain FR1, CDR1, FR2, CDR2, FR3, CDR3, and FR4. The assignment of amino acids to each domain is typically based on definitions such as those found in: Sequences of Proteins of Immunological Interest, Kabat et al.; National Institutes of Health, Bethesda, Md., 5th Edition; NIH Publication No. 91-3242 (1991): Kabat (1978) Adv. Prot. Chem. 32:1-75; Kabat et al., (1977) J. Biol. Chem. 252:6609-6616; Chothia et al., (1987) J Mol. Biol. 196:901-917; or Chothia et al., (1989) Nature 341:878-883.

The carboxyl-terminal portion of the heavy chain can define the constant region that is primarily responsible for effector function. Typically, humanized light chains are classified as κ chains and λ chains. Humanized heavy chains are generally classified as µ, δ, γ, α, or ε. The isotypes of antibodies are typically defined as IgM, IgD, IgG, IgA, and IgE, respectively. The IgG subclasse is well-known to those skilled in the art and includes, but is not limited to, IgG1, IgG2, IgG3, and IgG4.

Herein, antibodies can be naturally occurring antibodies or non-naturally occurring antibodies. An antibody can be a monoclonal antibody or a polyclonal antibody. An antibody can be a murine-derived antibody or a humanized antibody. An antibody can be a chimeric antibody.

A chimeric antibody refers to an antibody in which a portion of the heavy and/or light chain is identical or homologous to the corresponding sequence of an antibody from a specific species (e.g., human) or belongs to a specific antibody class or subclass, while the remainder of the chain is identical or homologous to the corresponding sequence of an antibody from another species (e.g., mouse) or belongs to another antibody class or subclass. A humanized antibody refers to an antibody form that contains sequences from non-human (e.g., murine) antibodies as well as human antibodies. Such an antibody contains minimal sequences derived from non-human immunoglobulins.

Herein, an antibody fragment or an antigen-binding fragment refers to the antigen-binding fragment of an antibody, which retains the specific binding ability of full-length antibodies to antigens, such as a fragment retaining one or more CDR regions. Examples of an antigen-binding fragment include, but are not limited to, Fab, Fab', F(ab')2, and Fv fragments; diabodies; linear antibodies; single chain antibodies (scFv); nanobodies; and multispecific antibodies formed from antibody fragments.

The antibody used in the present disclosure may be a known antibody or functional fragment thereof in the art. For example, an antibody or its functional fragment may be selected from a group consisting of: anti-Her2 antibody, anti-EGFR antibody, anti-VEGFR antibody, anti-CD20 antibody, anti-CD33 antibody, anti-PD-L1 antibody, anti-PD-1 antibody, anti-CTLA-4 antibody, anti-TNFα antibody, anti-CD28 antibody, anti-4-1BB antibody, anti-OX40 antibody, anti-GITR antibody, anti-CD27 antibody, anti b-CD40 antibody, or anti-ICOS antibody, anti-CD25 antibody, anti-CD30 antibody, anti-CD3 antibody, anti-CD22 antibody, anti-CCR6 antibody, anti-CD38 antibody, anti-CD52 antibody, anti-complement C5 antibody, anti-RSV F protein antibody, antibody-GD2 antibody, anti-GITR antibody, anti-glycoprotein receptor lib/Illa antibody, anti-ICOS antibody, anti-IL2R antibody, anti-LAG3 antibody, anti-α4 integrin antibody, anti-IgE antibody, anti-PDGFRα antibody, anti-RANKL antibody, anti-SLAMF7 antibody, anti-LTIGIT antibody, anti-TIM-3 antibody, anti-VEGFR2 antibody, anti-VISTA antibody, anti SSTR2 antibody, anti LY6G6D antibody, anti GCC antibody, anti Trop2 antibody, anti Cmet antibody.

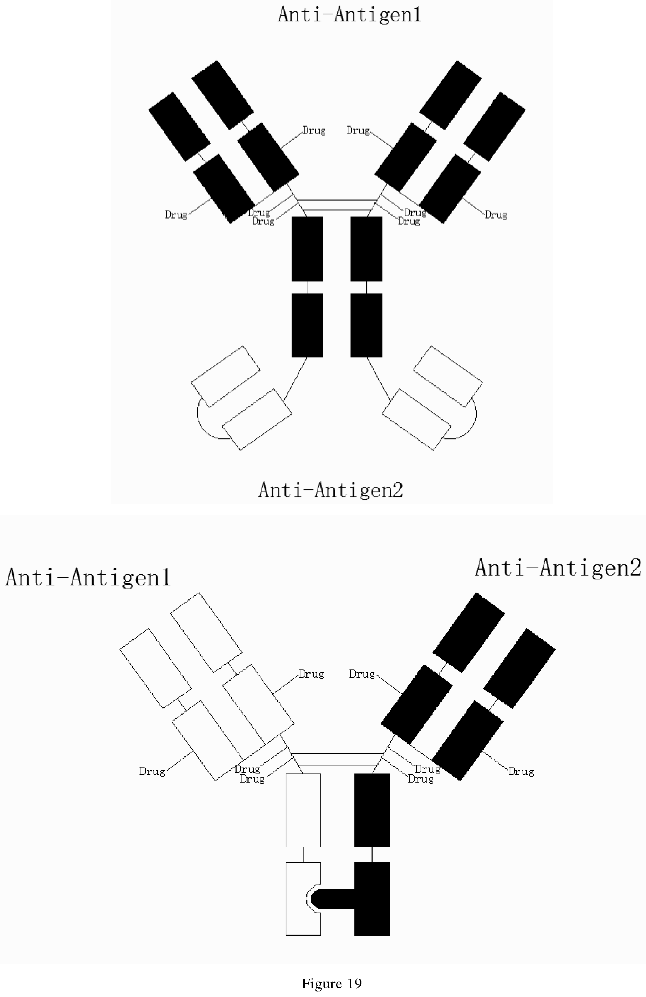

In some embodiments, an antibody used herein is a multispecific antibody, such as a bispecific antibody. A multispecific antibody refers to an artificial antibody containing two or more specific antigen-binding sites, which can bridge target cells and functional molecules (cells) to elicit directed immune responses. An antibody molecule containing at least two antigen binding domains can be referred to as a bispecific antibody molecule, where each antigen binding domain is capable of binding to a different target. The VH and the VL regions contain framework regions (FRs) on both sides of each CDR, which provide a scaffold for the CDRs. The structure of an exemplary bispecific antibody is shown in Figure 19. As shown in Figure 19 (left), from the N-terminus to the C-terminus, the VH region comprises the following structure: {N-terminus-[HFR1]-[HCDR1]-[HFR2]-[HCDR2]-[HFR3]-[HCDR3]-[HFR4]}-linker-{[HFR1]-[HCDR1]-[HFR2]-[HCDR2]-[HFR3]-[HCDR3]-[HFR4]-[LFR1]-[LCDR1]-[LFR2]-[LCDR2]-[L FR3]-[LCDR3]-[LFR4]-C-terminus}, and the VL region comprises the following structure: N-terminus-[LFR1]-[LCDR1]-[LFR2]-[LCDR2]-[LFR3]-[LCDR3]-[LFR4]-C-terminus. As shown in Figure 19 (right), from the N-terminus to the C-terminus, the VH region comprises the following structure: N-terminus-[HFR1]-[HCDR1]-[HFR2]-[HCDR2]-[HFR3]-[HCDR3]-[HFR4]-C-terminus, and the VL region comprises the following structure: N-terminus-[LFR1]-[LCDR1]-[LFR2]-[LCDR2]-[LFR3]-[LCDR3]-[LFR4]-C-terminus.

In a preferred embodiment, the antibody used in the present disclosure is selected from a group consisting of: Utomilumab, Urelumab, ADG106, Poteligeo^{™} (Mogamulizumab), Poteligeo^{™} (Mogamulizumab), Bexxar^{™} (tositumomab), Zevalin^{™} (ibritumomab tiuxetan), Rituxan^{™} (rituximab), Arzerra^{™} (Ofatumumab), Gazyva^{™} (Obinutuzumab), Besponsa^{™} (Inotuzumab ozogamicin), Zenapax^{™} (daclizumab), Varlilumab, Theralizumab, Adcetris^{™} (Brentuximab vedotin), Myelotarg^{™} (gemtuzumab), Darzalex^{™} (Daratumumab), CDX-1140, SEA-CD40, RO7009789, JNJ-64457107, APX-005M, Chi Lob 7/4, Campath^{™} (alemtuzumab), Raptiva^{™} (efalizumab), Soliris^{™} (eculizumab), Yervoy^{™}(ipilimumab), tremelimumab, Erbitux^{™} (cetuximab), Vectibix^{™} (panitumumab), Portrazza^{™} (Necitumumab), TheraCIM^{™} (Nimotuzumab), Synagis^{™} (palivizumab), Unituxin^{™} (Dinutuximab), TRX-518, MK-4166, MK-1248, GWN-323, INCAGN0186, BMS-986156, AMG-228, ReoPro^{™} (abiciximab), Herceptin^{™} (trastuzumab), Perjeta^{™} (Pertuzumab), Kadcyla^{™} (Ado-trastuzumab emtansine), GSK-3359609, JTX-2011, Simulect^{™} (basiliximab), Tysabri^{™} (natalizumab), BMS-986016, REGN3767, LAG525, Xolair^{™} (omalizumab), Tavolimab, PF-04518600, BMS-986178, MOXR-0916, GSK-3174998, INCAGN01949, IBI-101, Keytruda^{™} (Pembrolizumab), Opdivo^{™} (Nivolumab), Lartruvo^{™} (Olaratumab), Tencentriq^{™} (Atezolizumab), BMS-936559, Bavencio^{™} (Avelumab), Imfinzi^{™} (Duralumab), Prolia^{™} (Denosumab), Empliciti^{™} (Elotuzumab), MTIG7192A, TSR-022, MBG-453, Remicade^{™} (infliximab), Humira^{™} (adalimumab), Avastin^{™} (bevacizumab), Lucentis^{™} (ranibizumab), Cyramza^{™} (Ramucirumab) and JNJ-61610588.

In some embodiments, a bioactive macromolecule moiety (A) used in the ADC described herein is a fusion protein comprising an antigen-binding domain of an antibody and optionally a cytokine. The antigen-binding domain in the fusion protein may be selected from antigen-binding domains targeting the following antigens: HER2, CD19, CD20, EGFR, CD22, CD3, TROP2, Glycoprotein NMB, Guanylyl cyclase C, CEA, AXL, GCC, CD79b, PSMA, ENPP3, Mesothelin, CD138, NaPi2b, CD56, CD74, FOLR1, DLL3, CEACAM5, CD142, SLAMF7, CD25, SLTRK6, CD37, CD70, AGS-22, C4.4A, FGFR2, Ly6E, MUC16, BCMA, pCadherin, Ephrin-A, LAMP1, MUC1, PDL1, HER2, NY-ESO-1, BCMA, WT1, MUC1, CD20, CD23, ROR1, CD123, CD33, CD44v6, CD174, CD30, CD133, cMet, FAP, EphA2, GD2, GPC3, IL-13Ra2, LewisY, SS1, CD171, EGFR, EGFRvIII, VEGFR2, NY-ESO-1, MUC-1, SSTR2, LY6G6D, MAGE-A3, anti GCC antibody, anti Trop2 antibody, anti Cmet antibody, or may be selected from antigen-binding domains of any antibody mentioned herein (such as an antigen-binding fragment).

In some embodiments, the fusion protein may be a bispecific antibody comprising antigen-binding domains that specifically bind to the following antigens selected from: HER2, CD19, EGFR, CD22, CD3, TROP2, Glycoprotein NMB, Guanylyl cyclase C, CEA, AXL, GCC, CD79b, PSMA, ENPP3, Mesothelin, CD138, NaPi2b, CD56, CD74, FOLR1, DLL3, CEACAM5, CD142, SLAMF7, CD25, SLTRK6, CD37, CD70, AGS-22, C4.4A, FGFR2, Ly6E, MUC16, BCMA, pCadherin, Ephrin-A, LAMP1, MUC1, CD19, PDL1, NY-ESO-1, WT1, MUC1, CD20, CD23, ROR1, CD123, CD33, CD44v6, CD174, CD30, CD133, cMet, EGFR, FAP, EphA2, GD2, GPC3, IL-13Ra2, LewisY, Mesothelin, SS1, CD171, EGFR, EGFRvIII, VEGFR2, NY-ESO-1, MUC-1, MAGE-A3, anti GCC antibody, anti Trop2 antibody, anti Cmet antibody, or may be selected from the antigen-binding domains of any antibody mentioned in the present disclosure. Preferably, the bispecific antibody is a single-chain bispecific antibody comprising two scFvs derived from the same or different antibodies.

### Pharmaceutical compositions

A pharmaceutical composition of the present disclosure comprises: (i) a drug conjugate, or a stereoisomer or a pharmaceutically acceptable salt thereof, and/or an antibody-drug conjugate, or a stereoisomer or a pharmaceutically acceptable salt thereof; and (ii) a pharmaceutically acceptable carrier. The carrier may be any pharmaceutically acceptable carrier or excipient, varying with the dosage form and mode of administration. Pharmaceutically acceptable carriers are typically safe and non-toxic, and may include any known substances used in the pharmaceutical industry for formulating drug compositions, such as fillers, diluents, coagulants, binders, lubricants, glidants, stabilizers, colorants, wetting agents, disintegrants, and so on. Suitable pharmaceutically acceptable carriers comprise sugars, such as lactose or sucrose, mannitol or sorbitol; cellulose preparations and/or calcium phosphates, such as tricalcium phosphate or dicalcium phosphate; starch, including corn starch, wheat starch, rice starch, potato starch, gelatin, tragacanth, methylcellulose, hydroxypropylmethylcellulose, sodium carboxymethylcellulose and/or polyvinylpyrrolidone; silicon dioxide, talc, stearic acid or salts thereof, such as magnesium stearate or calcium stearate; and/or polyethylene glycol. When selecting a pharmaceutically acceptable carrier, a major consideration is the mode of administration of the pharmaceutical dosage form. This is well known in the art.

The pharmaceutical composition may comprise a therapeutically or prophylactically effective amount of a drug conjugate, or a stereoisomer or a pharmaceutically acceptable salt thereof, and/or an antibody-drug conjugate, or a stereoisomer or a pharmaceutically acceptable salt thereof. An "effective amount" refers to an amount of the component sufficient to produce the desired response. The specific effective amount depends on various factors, such as the specific disease to be treated, the patient's physical condition such as weight, age and sex, the duration of treatment, co-administered treatments (if any) and the specific formulation used. Generally, an "effective amount" as referred to herein is a conventional amount of the biomolecule. However, in some embodiments, the therapeutically or prophylactically effective amount of the conjugate in the pharmaceutical composition disclosed herein may be lower than conventional amounts of the biomolecule but may yield better therapeutic or prophylactic outcomes, as the biomolecule is protected by a masking group before reaching the pathological microenvironment for binding to its ligand or receptor.

The pharmaceutical compositions of this disclosure can be formulated into various suitable dosage forms, including but not limited to tablets, capsules, injections, etc., and can be administered via any appropriate route to achieve the intended purpose. For example, it can be administered parenterally, subcutaneously, intravenously, intramuscularly, intraperitoneally, transdermally, orally, intrathecally, intracranially, intranasally or topically. The dosage of the drug may depend on the age, health condition, and weight of the patient, concurrent therapies, treatment frequency, and other factors. The pharmaceutical compositions of the present disclosure can be administered to any subject in need, such as mammals, especially humans.

The conjugate of the present disclosure can release toxins (D) through endocytosis of biomolecules into lysosomes, or through hydrolysis by proteolytic enzymes in the tumor acidic microenvironment, especially Legumain or granzyme. For example, the biomolecules in the antibody-drug conjugate of the present disclosure are Trastuzumab, anti Trop-2 antibodies or anti PD-L1 antibodies or the like. In terms of toxin release, the proteolytic enzyme hydrolysis pathway in the tumor acidic microenvironment can more efficiently release toxins, thereby achieving better drug efficacy. Therefore, the conjugate of the present disclosure can effectively overcome the problem of release efficiency of bioconjugate drugs, and toxins are efficiently released in the pathological microenvironment.

### Uses

Each drug conjugate, a stereoisomer or a pharmaceutically acceptable salt thereof disclosed in the present disclosure, as well as each antibody-drug conjugate, a stereoisomer or pharmaceutically acceptable salt thereof, can be used for treating and/or preventing tumors or inflammations, or can be used as an active ingredient in the preparation of drugs for treating tumors or inflammations.

The diseases that can be treated by a drug conjugate, a stereoisomer or a pharmaceutically acceptable salt thereof, as well as an antibody-drug conjugate, a stereoisomer or a pharmaceutically acceptable salt thereof disclosed in the present disclosure, are related to the active ingredients comprised in the conjugate. The indications for these active ingredients are well known in the art.

In some embodiments, tumors described herein may comprise hematological tumors and solid tumors, including but not limited to sarcoma (e.g., fibrosarcoma), bladder cancer, brain cancer, breast cancer, cervical cancer, colorectal cancer, esophageal cancer, renal cancer, liver cancer, lung cancer, nasopharyngeal carcinoma, pancreatic cancer, prostate cancer, skin cancer, gastric cancer, uterine cancer, ovarian cancer, testicular cancer, and blood cancer.

The present disclosure also comprises a method for treating or preventing tumors or inflammation, comprising administering to a subject in need thereof a therapeutically or prophylactically effective amount of a drug conjugate, a stereoisomer or a pharmaceutically acceptable salt thereof, and an antibody-drug conjugate, a stereoisomer or a pharmaceutically acceptable salt thereof as described herein, or a pharmaceutical composition described herein. This method can be used in combination with any known radiotherapy or immunotherapy.

The present disclosure also provides a use of a drug conjugate, or a stereoisomer or a pharmaceutically acceptable salt thereof, and/or an antibody-drug conjugate, or a stereoisomer or a pharmaceutically acceptable salt thereof as described herein, in the preparation of drugs for anti-tumor purposes. Preferably, the tumor is selected from hematologic tumors and solid tumors. Preferably, the tumor includes but is not limited to sarcoma (e.g., fibrosarcoma), bladder cancer, brain cancer, breast cancer, cervical cancer, colorectal cancer, esophageal cancer, renal cancer, liver cancer, lung cancer, nasopharyngeal carcinoma, pancreatic cancer, prostate cancer, skin cancer, gastric cancer, uterine cancer, ovarian cancer, testicular cancer, and blood cancer.

The advantages of the present disclosure comprise:
Through continuous experimental screening, the inventors have prepared Trastuzumab-conjugated drugs, Atezolizumab-conjugated drugs, anti SSTR2 antibody-conjugated drugs and anti LY6G6D antibody-conjugated drugs, which are tumor-targeting macromolecular conjugated drugs that can be activated with high efficiency and possess innovative multifunctional targeting and activation properties: (1) The tumor microenvironment-targeted and activated antibody-drug conjugates of the present disclosure can accumulate, retain and be activated at tumor sites, and possess the characteristics of targeting the tumor microenvironment and efficiently releasing payloads. (2) The tumor microenvironment conjugated drugs of the present disclosure can be specifically activated or cleaved by tumor tissues, generating effective drugs locally. (3) Exatecan, Resiquimod or derivatives thereof containing an AAN sequence in the linker are conjugated to trastuzumab or certolizumab antibody to form macromolecules directly, thereby altering various properties of the drug, improving efficacy, and overcoming the limitation of drug indication. (4) During tumor metastasis, a large amount of proteolytic enzymes are usually secreted to degrade the intercellular matrix, therefore, conjugate drugs activated by proteolytic enzymes have a special therapeutic effect on the treatment of tumor metastasis, and the antibody-drug conjugates of the present disclosure has high activation efficiency.

The disclosure is further described below in conjunction with specific examples. It is understood that these examples are merely for illustrative purposes rather than intended to limit the scope of the disclosure. For the experimental methods without specific conditions indicated in the following examples, the conventional conditions or the conditions suggested by the manufacturer are usually followed. Percentages and parts are by weight unless otherwise indicated.

It is understood that the terms "comprise" and "include" or similar expressions used in the present disclosure also mean "consist of", etc. All weight or volume percentages should sum to 100%. Unless otherwise indicated, the various reagents and products used in the examples are commercial products. Unless otherwise indicated, the methods mentioned in the examples are carried out according to conventional techniques. The following examples are not intended to limit the scope of the present disclosure.

### Example 1: Preparation of Drug Conjugates S1-S21, and S26-S59

Typical preparation methods for the compounds are shown below:

### General formula (I) of the synthetic route

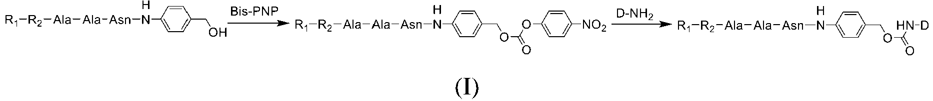

1) Compound R₁-R₂-Ala-Ala-Asn-PABOH (1.0 eq) was dissolved in an appropriate amount of DMF solvent. Then, compound Bis-PNP (2.0 eq) was added, followed by addition of DIPEA (2.0 eq) under an ice bath. The reaction mixture was slowly warmed to room temperature and stirred for another 10-15 h. Most of the solvent was removed from the obtained reaction solution under high vacuum. The residue was dissolved and diluted with an appropriate amount of dichloromethane. The organic phase was sequentially washed with water and saturated brine, dried over anhydrous sodium sulfate, and filtered. Column chromatography was then performed using dichloromethane/methanol as the eluent. The solvent was rotary evaporated to dryness to obtain R₁-R₂-Ala-Ala-Asn-PABC-PNP.
2) Compound R₁-R₂-Ala-Ala-Asn-PABC-PNP (1.0 eq) was dissolved in an appropriate amount of DMF solvent. Then, compound D-NH₂ (1.0 eq) was added, followed by addition of DIPEA (2.0 eq) under an ice bath. The reaction mixture was slowly warmed to room temperature and stirred for another 10-15 h. Most of the solvent was removed from the obtained reaction solution under high vacuum. The reaction mixture was then diluted with an appropriate amount of methanol. The mixture was purified by reverse-phase high-pressure preparative chromatography, and finally lyophilized to obtain the target product R₁-R₂-Ala-Ala-Asn-PABC-(C=O)-NHD.

Using the corresponding starting materials, the compounds shown in Table 1 were obtained by the above method, and the properties of each compound in Table 1 are shown in Table 2.

**Table 1**

| R₁ | R₂ | D | Compound No. |
|---|---|---|---|
| 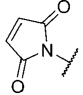 | 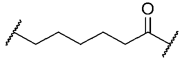 | X2 | S3 |
| 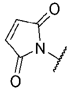 | 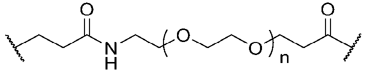 | X2 | S4 |
| 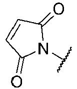 | 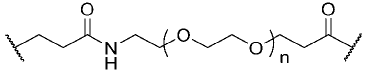 | X4 | S7 |
| 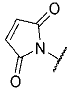 | 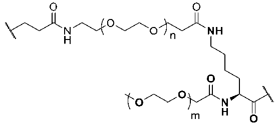 | X4 | S8 |
| 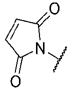 | 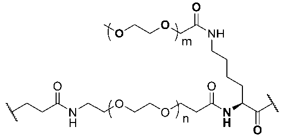 | X4 | S9 |
| 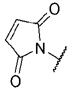 | 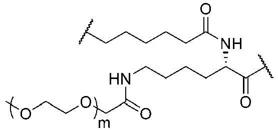 | X4 | S10 |
| 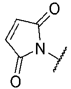 | 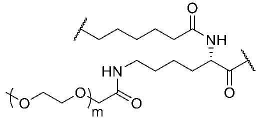 | X4 | S11 |
| 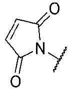 | 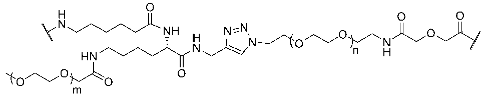 | X4 | S12 |
| 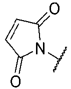 | 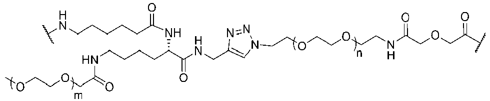 | X4 | S13 |
| 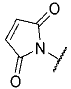 | 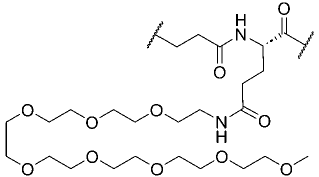 | X4 | S50 |
| 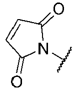 | 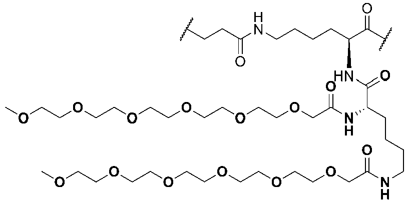 | X4 | S51 |
| 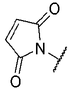 | 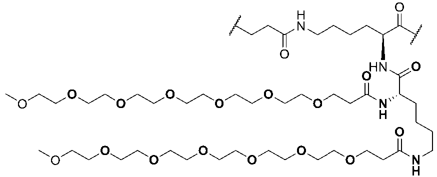 | X4 | S52 |
| 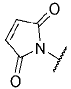 | 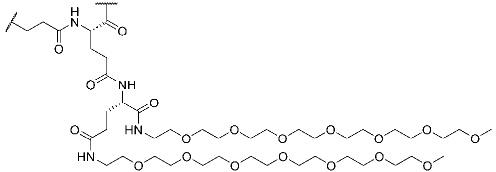 | X4 | S53 |
| 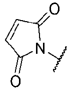 | 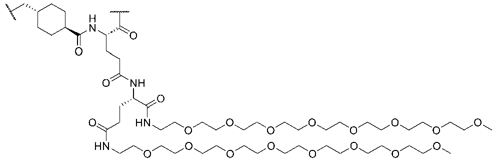 | X4 | S54 |
| 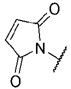 | 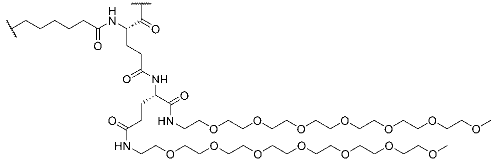 | X4 | S55 |
| 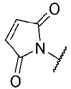 | 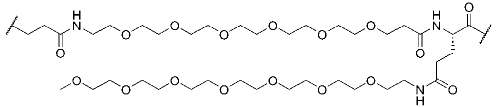 | X4 | S56 |
| 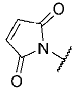 | 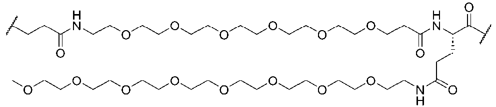 | X4 | S57 |
| 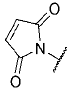 | 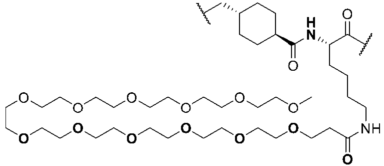 | X4 | S58 |
| 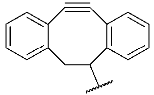 | 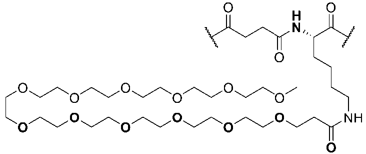 | X4 | S59 |

**Table 2**

| Compound No. | Appearance | Yield (mg) | Molecular weight | Mass spectrometry [M+H] |
|---|---|---|---|---|
| S3 | White solid | 60 | 910.05 | 910.45 |
| S4 | White solid | 50 | 1203.36 | 1203.60 |
| S7 | Yellow solid | 61 | 1327.38 | 1327.55 |
| S8 | Yellow solid | 55 | 1747.89 | 1747.79 |
| S9 | Yellow solid | 70 | 1747.89 | 1747.81 |
| S10 | Yellow solid | 60 | 1454.57 | 1454.65 |
| S11 | Yellow solid | 60 | 1586.73 | 1586.72 |
| S12 | Yellow solid | 45 | 1825.97 | 1825.83 |
| S13 | Yellow solid | 60 | 1958.12 | 1958.08 |
| S50 | Yellow solid | 50 | 1486.57 | 1486.65 |
| S51 | Yellow solid | 65 | 1832.99 | 1832.90 |
| S52 | Yellow solid | 47 | 1949.15 | 1949.01 |
| S53 | Yellow solid | 55 | 1893.05 | 1892.97 |
| S54 | Yellow solid | 45 | 2049.27 | 2049.10 |
| S55 | Yellow solid | 50 | 1935.13 | 1935.10 |
| S56 | Yellow solid | 41 | 1777.91 | 1777.80 |
| S57 | Yellow solid | 50 | 1821.97 | 1821.85 |
| S58 | Yellow solid | 45 | 1758.95 | 1758.90 |
| S59 | Yellow solid | 25 | 1842.04 | 1842.00 |

### General formula (II) of the synthetic route

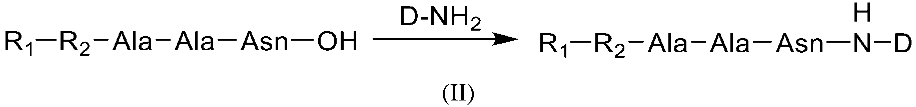

Compound R₁-R₂-Ala-Ala-Asn-OH (1.0 eq) was dissolved in an appropriate amount of DMF solvent. Then, compound D-NH₂ and the condensing agent HBTU were sequentially added. DIPEA (2.0 eq) was added to the reaction solution in an ice bath. Then, the reaction mixture was slowly warmed to room temperature and stirred for another 3-5 hours. Most of the solvent was removed from the obtained reaction solution under high vacuum. The reaction mixture was then diluted with an appropriate amount of methanol. The mixture was purified by reverse-phase high-pressure preparative chromatography, and finally lyophilized to obtain the target product.

Using the corresponding starting materials, the compounds shown in Table 3 were obtained by the above method, and the properties of each compound in Table 3 are shown in Table 4.

**Table 3**

| R₁ | R₂ | D | Compound No. |
|---|---|---|---|
| 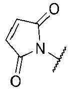 | 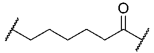 | X1 | S1 |
| 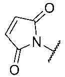 | 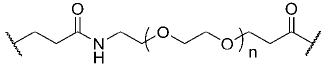 | X1 | S2 |
| 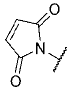 | 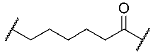 | X3 | S5 |
| 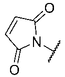 | 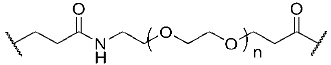 | X3 | S6 |

**Table 4**

| Compound No. | Appearance | Yield (mg) | Molecular Weight | Mass Spectrometry [M+H] |
|---|---|---|---|---|
| S1 | White powder | 20 | 763.85 | 764.47 |
| S2 | White powder | 15 | 1057.17 | 1057.51 |
| S5 | White powder | 18 | 760.90 | 761.40 |
| S6 | White powder | 22 | 1054.21 | 1054.55 |

### General formula (III) of the synthetic route

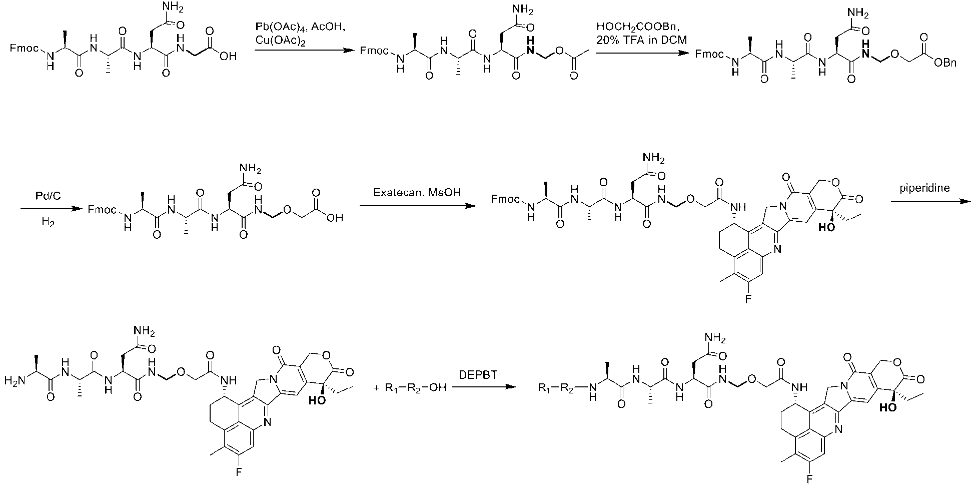

### 1) Synthesis of compound Fmoc-Ala-Ala-Asn-AM-OAc

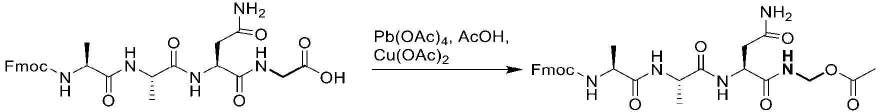

Fmoc-Ala-Ala-Asn(Trt)-Gly-OH (1.0 eq) was dissolved in dichloromethane/tetrahydrofuran (a volume ratio of 15/5), cooled to 0°C, and AcOH (1.0 eq), lead acetate (1.2 eq), and copper acetate (0.2 eq) were added under nitrogen protection. The reaction mixture was stirred at 40-50°C under nitrogen for 2-3 h, and monitored by TLC to show that the reaction was completed. The reaction solution was diluted with dichloromethane, sequentially washed with water, separated, and the aqueous phase was extracted with dichloromethane. The organic phases were combined, dried over anhydrous sodium sulfate, rotary evaporated, and purified by column chromatography to obtain Fmoc-Ala-Ala-Asn-AM-OAc.

### 2) Synthesis of compound Fmoc-Ala-Ala-Asn(Trt)-AM-Ac-OBn

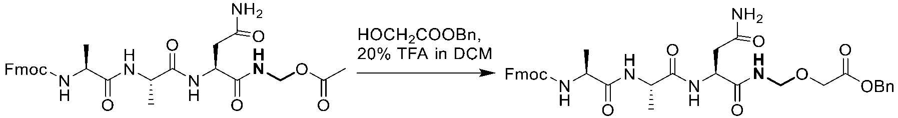

Compound Fmoc-Ala-Ala-Asn-AM-OAc (1.0 eq) was added to a three-necked flask, followed by addition of benzyl glycolate (2.0 eq). Under nitrogen protection and ice bath, trifluoroacetic acid/dichloromethane (a volume ratio of TFA/DCM = 1/5) was added, and the mixture was stirred at room temperature for 2-3 h. After the reaction was completed, the reaction mixture was concentrated, and the product Fmoc-Ala-Ala-Asn-AM-Ac-OBn was obtained as a white solid by high-pressure preparative chromatography.

### 3) Synthesis of compound Fmoc-Ala-Ala-Asn-AM-Ac-OH

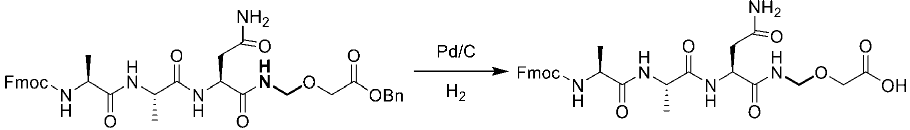

Compound Fmoc-Ala-Ala-Asn-AM-Ac-OBn (1.0 eq) was dissolved in an appropriate amount of MeOH. Pd/C (10 wt%, 0.1 eq) was slowly added, and the reaction mixture was stirred under hydrogen protection for 2-3 h. Compoud Fmoc-Ala-Ala-Asn-AM-Ac-OH was obtained by high-pressure preparation as a pale yellow product.

### 4) Synthesis of compound Fmoc-Ala-Ala-Asn-AM-DXd

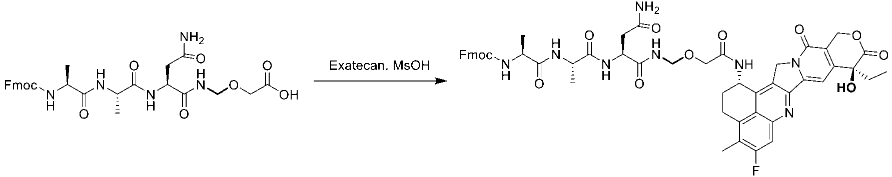

Compound Fmoc-Ala-Ala-Asn-AM-Ac-OH (1.0 eq) and exatecan methanesulfonate (1.0 eq) were dissolved in DMF, cooled to 0°C, and the condensing agent DEPBT (1.2 eq) and organic base DIPEA (2.0 eq) were sequentially added under nitrogen protection. The reaction mixture was stirred at room temperature for 2-3 h, and was monitored by TLC to show that the reaction was completed. The reaction solution was concentrated under vacuum, redissolved in DMF, filtered, and compound Fmoc-Ala-Ala-Asn-AM-DXd was obtained by high-pressure liquid preparation as a pale yellow product.

### 5) Synthesis of compound H-Ala-Ala-Asn-AM-DXd

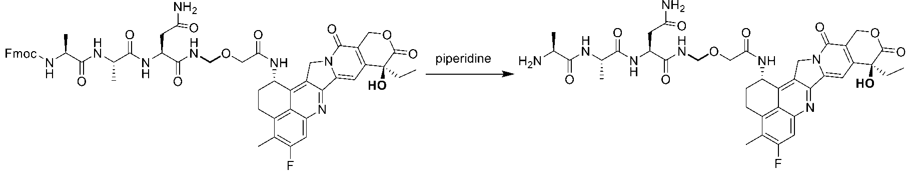

Compound Fmoc-Ala-Ala-Asn-AM-DXd (1.0 eq) was dissolved in DMF, piperidine (2.0 eq) was added, and the mixture was stirred at room temperature under nitrogen protection for 2-3 h. The reaction mixture was monitored by TLC to show that the reaction was completed. The reaction solution was redissolved, filtered, and compound H-Ala-Ala-Asn-AM-DXd was obtained by high-pressure liquid preparation as a pale yellow product.

### 6) Synthesis of compound R₁-R₂-Ala-Ala-Asn-AM-DXd

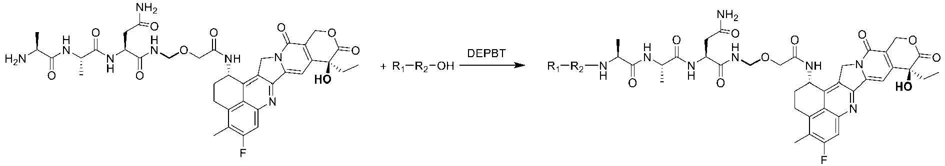

Compound R₁-R₂-OH (1.0 eq) was dissolved in an appropriate amount of DMF, H-Ala-Ala-Asn-AM-DXd (1.0 eq) was added, followed by addition of HBTU (1.2 eq) and DIPEA (2.0 eq) under nitrogen protection. The mixture was stirred at room temperature for 1-2 h. The reaction mixture was monitored by TLC to show that the reaction was completed. The crude reaction solution was filtered, and compound R₁-R₂-Ala-Ala-Asn-AM-DXd was then obtained by high-pressure liquid preparation as a pale yellow product.

Using the corresponding starting materials, the compounds shown in Table 5 were obtained by the above method, and the properties of each compound in Table 5 are shown in Table 6.

**Table 5**

| R₁ | R₂ | Compound No. |
|---|---|---|
| 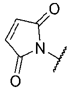 | 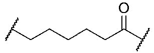 | S14 |
| 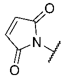 | 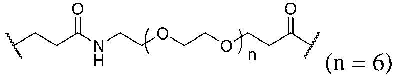 | S15 |
| 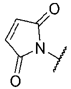 | 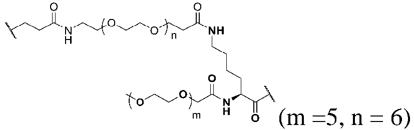 | S16 |
| 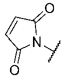 | 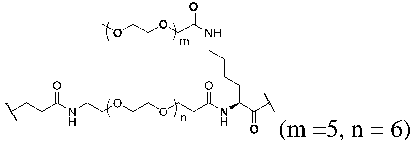 | S17 |
| 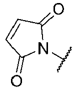 | 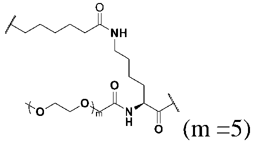 | S18 |
| 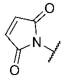 | 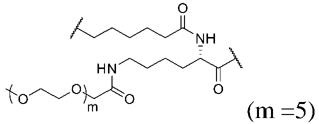 | S19 |
| 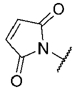 | 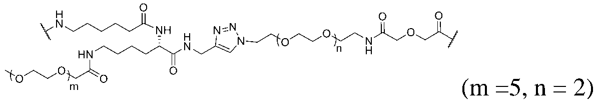 | S20 |
| 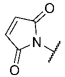 | 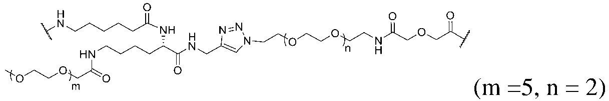 | S21 |
| 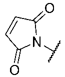 | 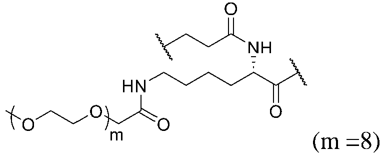 | S26 |
| 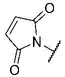 | 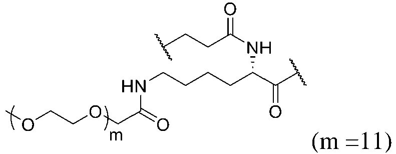 | S27 |
| 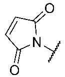 | 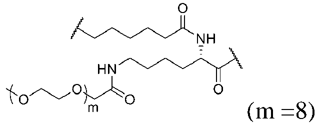 | S28 |
| 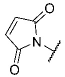 | 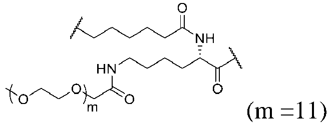 | S29 |
| 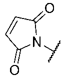 | 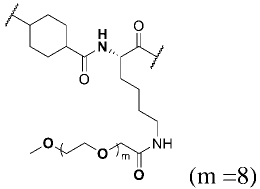 | S30 |
| 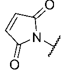 | 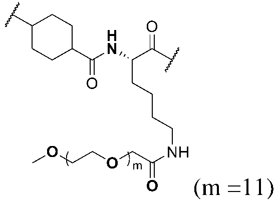 | S31 |
| 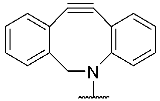 | 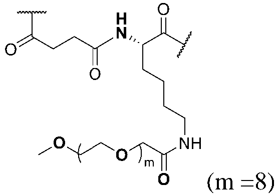 | S32 |
| 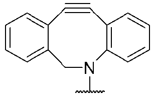 | 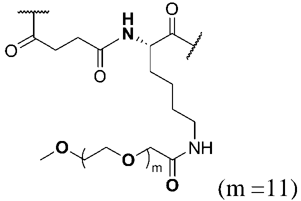 | S33 |
| 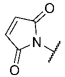 | 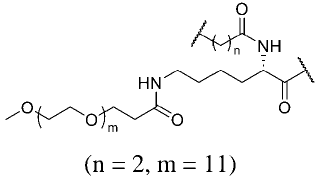 | S34 |
| 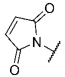 | 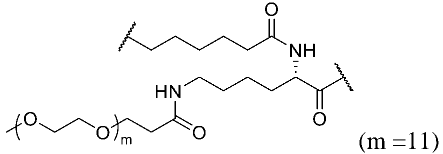 | S35 |
| 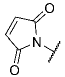 | 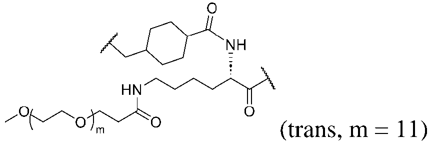 | S36 |
| 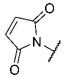 | 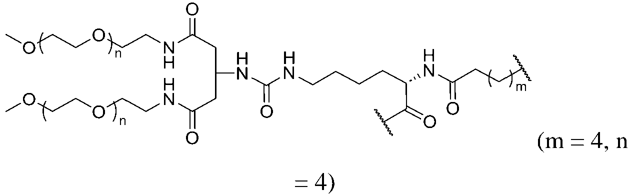 | S37 |
| 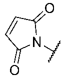 | 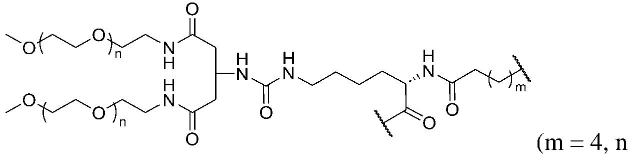 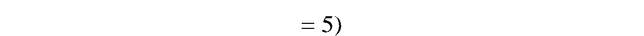 | S38 |
| 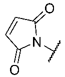 | 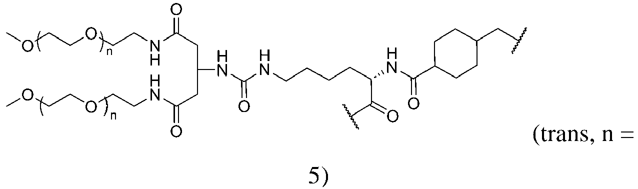 | S39 |
| 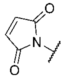 | 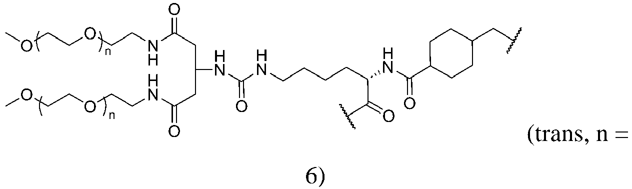 | S40 |
| 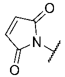 | 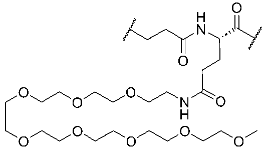 | S41 |
| 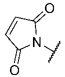 | 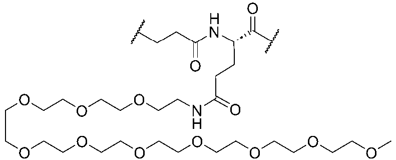 | S42 |
| 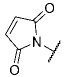 | 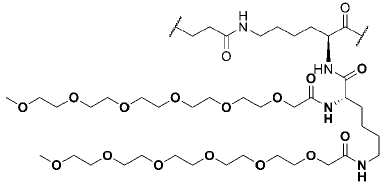 | S43 |
| 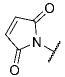 | 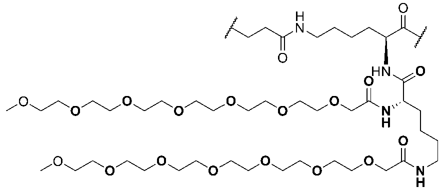 | S44 |
| 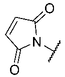 | 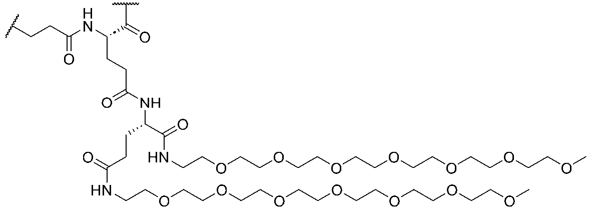 | S45 |
| 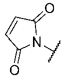 | 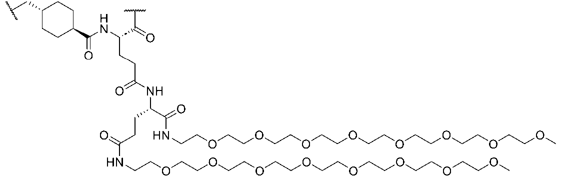 | S46 |
| 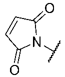 | 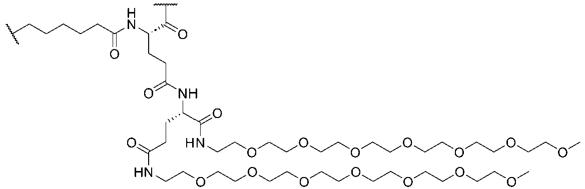 | S47 |
| 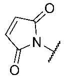 | 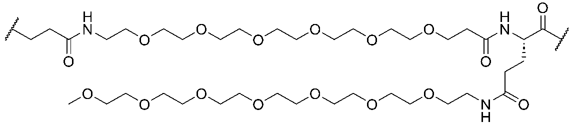 | S48 |
| 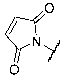 | 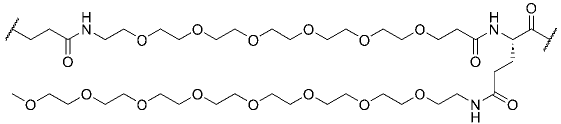 | S49 |

**Table 6**

| Compound No. | Appearance | Yield (mg) | Molecular Weight | Mass Spectrometry [M+H] |
|---|---|---|---|---|
| S14 | Pale yellow powder | 12 | 972.00 | 972.38 |
| S15 | Pale yellow powder | 10 | 1265.31 | 1265.53 |
| S16 | Pale yellow powder | 15 | 1685.82 | 1685.78 |
| S17 | Pale yellow powder | 8 | 1685.82 | 1685.88 |
| S18 | Pale yellow powder | 20 | 1392.50 | 1392.63 |
| S19 | Pale yellow powder | 13 | 1392.50 | 1392.63 |
| S20 | Pale yellow powder | 10 | 1763.89 | 1763.81 |
| S21 | Pale yellow powder | 25 | 1896.05 | 1896.10 |
| S26 | Pale yellow powder | 13 | 1482.58 | 1482.66 |
| S27 | Pale yellow powder | 10 | 1614.74 | 1614.74 |
| S28 | Pale yellow powder | 11 | 1524.66 | 1524.71 |
| S29 | Pale yellow powder | 12 | 1656.82 | 1656.79 |
| S30 | Pale yellow powder | 10 | 1550.70 | 1550.72 |
| S31 | Pale yellow powder | 13 | 1682.86 | 1682.80 |
| S32 | Pale yellow powder | 8 | 1618.78 | 1618.73 |
| S33 | Pale yellow powder | 10 | 1750.93 | 1750.81 |
| S34 | Pale yellow powder | 21 | 1628.76 | 1628.76 |
| S35 | Pale yellow powder | 15 | 1670.85 | 1670.80 |
| S36 | Pale yellow powder | 20 | 1696.88 | 1696.82 |
| S37 | Pale yellow powder | 17 | 1739.91 | 1739.84 |
| S38 | Pale yellow powder | 13 | 1828.02 | 1827.92 |
| S39 | Pale yellow powder | 18 | 1854.06 | 1853.90 |
| S40 | Pale yellow powder | 20 | 1942.16 | 1942.00 |
| S41 | Pale yellow powder | 30 | 1424.50 | 1424.62 |
| S42 | Pale yellow powder | 15 | 1512.60 | 1512.67 |
| S43 | Pale yellow powder | 27 | 1770.90 | 1770.83 |
| S44 | Pale yellow powder | 16 | 1859.03 | 1859.00 |
| S45 | Pale yellow powder | 32 | 1830.97 | 1830.85 |
| S46 | Pale yellow powder | 23 | 1987.20 | 1987.10 |
| S47 | Pale yellow powder | 40 | 1873.06 | 1873.10 |
| S48 | Pale yellow powder | 33 | 1715.84 | 1715.79 |
| S49 | Pale yellow powder | 21 | 1759.90 | 1759.81 |

### Example 2: Synthesis of Compound S22

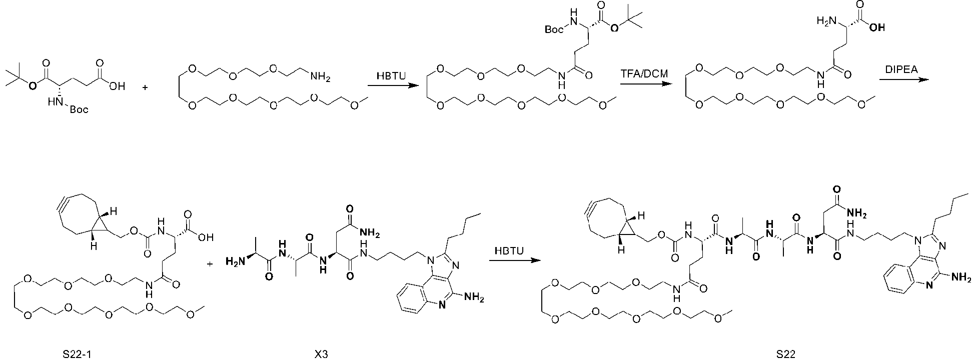

### 1) Synthesis of compound Boc-Glu(PEG8)-OtBu

Compound Boc-Glu(OH)-OtBu (300 mg, 0.989 mmol) and PEG8-NH₂ (379 mg, 0.988 mmol) were dissolved in super dry DMF (5 mL). Condensing agent HBTU (450 mg, 1.19 mmol) was added, and DIPEA (255 mg, 1.98 mmol) was added under ice bath. The mixture was stirred at room temperature for 2-3 h. The reaction solution was evaporated to dryness under reduced pressure, and the crude product was mixed with silica gel and subjected to column chromatography (petroleum ether:ethyl acetate = 10/1 to 1/1) to obtain compound Boc-Glu(PEG8)-OtBu as an oil (600 mg, yield: 91%).

### 2) Synthesis of compound H-Glu(PEG8)-OH

Compound Boc-Glu(PEG8)-OtBu (600 mg, 0.897 mmol) was dissolved in a mixed solvent of DCM/TFA with a volume ratio of 1:1 (10 mL) and stirred for 1 h. The reaction solution was evaporated to dryness under reduced pressure. The residue was dissolved in DCM and repeatedly concentrated to remove residual TFA to obtain compound H-Glu(PEG8)-OH as an oil (450 mg, yield 98%).

### 3) Synthesis of Compound S22-1

Compound H-Glu(PEG8)-OH (300 mg, 0.585 mmol) and BCN-PNP (185 mg, 0.587 mmol) were dissolved in super dry DMF (5 mL). DIPEA (151 mg, 1.17 mmol) was added dropwise, and the mixture was stirred at room temperature for 12-16 h. The reaction solution was evaporated to dryness under reduced pressure, and then triturated with the organic solvent MTBE to obtain compound S22-1 (350 mg, yield: 87%) as an oily liquid.

### 4) Synthesis of Compound S22

Compound S22-1 (100 mg, 0.145 mmol) and X3 (82 mg, 0.144 mmol) were dissolved in super dry DMF (5 mL). Condensing agent HBTU (66 mg, 0.174 mmol) was added, and DIPEA (38 mg, 0.29 mmol) was added dropwise under ice bath. The mixture was stirred at room temperature for 2-3 h. After the reaction was monitored as complete by LC-MS, the reaction solution was filtered. The filtrate was purified by high-pressure reverse-phase preparative chromatography to obtain S22 (69 mg, yield: 38%) as a white solid.

### Example 3: Synthesis of Compound S23

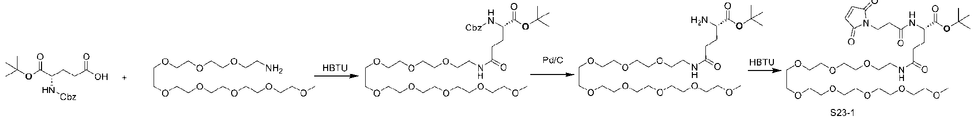

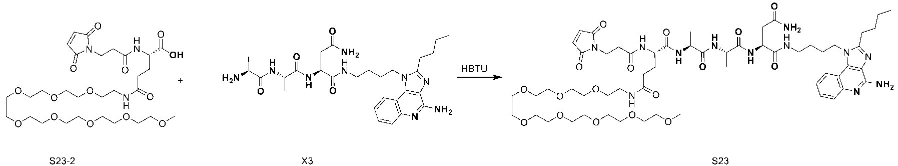

### 1) Synthesis of compound Cbz-Glu(PEG8)-OtBu

Compound Cbz-Glu(OH)-OtBu (334 mg, 0.991mmol) and PEG8-NH₂ (379 mg, 0.988 mmol) were dissolved in super dry DMF (5 mL). Condensing agent HBTU (450 mg, 1.19 mmol) was added, and DIPEA (255 mg, 1.98 mmol) was added to the reaction solution under ice bath. The mixture was stirred at room temperature for 2-3 h. The reaction solution was evaporated to dryness under reduced pressure, and the crude product was mixed with silica gel and subjected to column chromatography (petroleum ether : ethyl acetate = 10/1 to 1/1) to obtain compound Cbz-Glu(PEG8)-OtBu (610mg, yield: 88%) as an oil.

### 2) Synthesis of compound H-Glu(PEG8)-OtBu

Compound Cbz-Glu(PEG8)-OtBu (600 mg, 0.854 mmol) was dissolved in a mixed solvent of MeOH/THF with a volume ratio of 1/1 (10 mL) and stirred for 1 h. Then Pd/C (60 mg, 10 wt%) was added. The reaction solution was stirred for another 2-3 h. After the reaction was completed, the reaction solution was evaporated to dryness under reduced pressure to obtain compound H-Glu(PEG8)-OtBu (480 mg, yield 99%) as an oily liquid.

### 3) Synthesis of Compound S23-1

Compound H-Glu(PEG8)-OtBu (400 mg, 0.703 mmol) and MI-C2-COOH (119 mg, 0.704 mmol) were dissolved in super dry DMF (8 mL). Condensing agent HBTU (320 mg, 0.844 mmol) was added, and DIPEA (181 mg, 1.40 mmol) was added to the reaction solution under ice bath. The mixture was stirred at room temperature for 2-3 h. After the reaction solution was evaporated to dryness under reduced pressure, the crude product was mixed with silica gel and subjected to column chromatography (dichloromethane : methanol = 100/1 to 20/1) to obtain compound S23-1 (400 mg, yield: 79%) as an oil.

### 4) Synthesis of Compound S23-2

Compound S23-1 (400 mg, 0.556 mmol) was dissolved in a mixed solvent of DCM/TFA with a volume ratio of 1:1 (10 mL) and stirred for 2-3 h. The reaction solution was evaporated to dryness under reduced pressure. The residue was dissolved in DCM and repeatedly concentrated to remove residual TFA to obtain compound S23-2 as an oil (360mg, yield 97.5%).

### 5) Synthesis of Compound S23

Compound S23-2 (100 mg, 0.150 mmol) and X3 (80 mg, 0.141 mmol) were dissolved in super dry DMF (5 mL). Condensing agent HBTU (65 mg, 0.172 mmol) was added, and N,N-Diisopropylethylamine (36 mg, 0.28 mmol) was added dropwise under ice bath. The mixture was stirred at room temperature for 2-3 h. After the reaction was monitored as complete by LC-MS, the reaction solution was filtered, and the filtrate was purified by high-pressure reverse-phase preparative chromatography to obtain S23 (70 mg, yield: 41%) as a white solid. LCMS: [M+H]=1213.64.

### Example 4: Synthesis of Compound S24

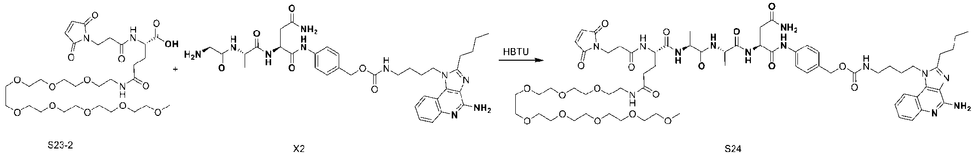

Compound S23-2 (100 mg, 0.151 mmol) was dissolved in an appropriate amount of DMF solvent. Then, compound X2 (100 mg, 0.140 mmol) and condensing agent HBTU (63 mg, 0.166 mmol) were sequentially added. DIPEA (40 mg, 0.31 mmol) was added to the reaction solution under ice bath. The reaction mixture was slowly warmed to room temperature and stirred for another 3-5 h. Most of the solvent was removed from the obtained reaction solution under high vacuum. The reaction mixture was then diluted with an appropriate amount of methanol. The mixture was purified by reverse-phase high-pressure preparative chromatography, and finally lyophilized to obtain S24 (50 mg, yield 26%) as a white powder. LCMS: [M+H]=1362.69.

### Example 5: Preparation Method of Compound S25

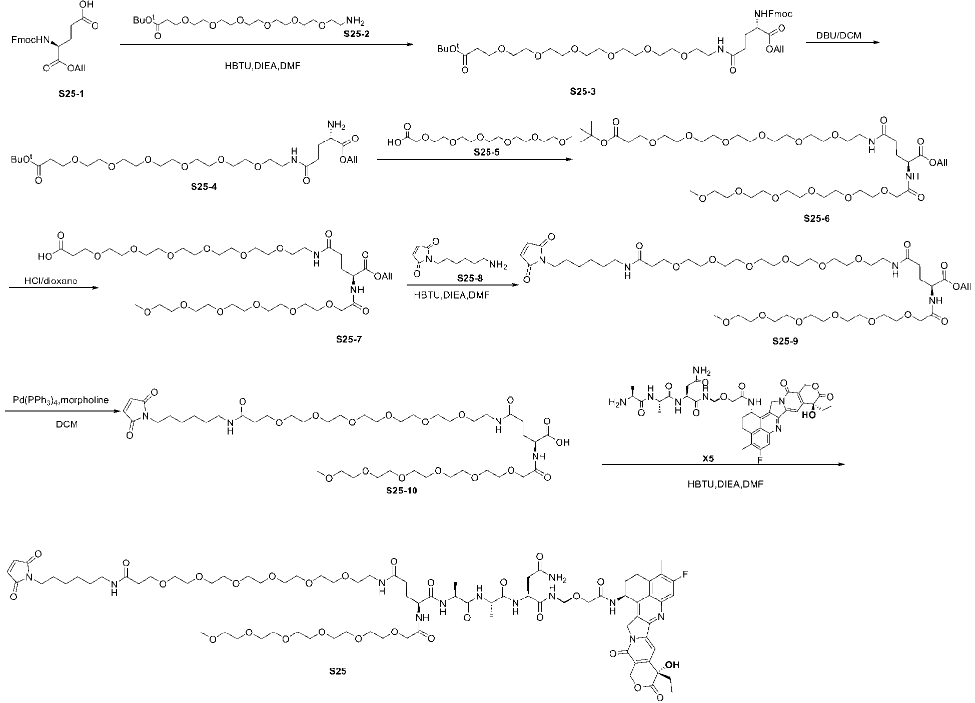

1) Compound S25-1 (2 g, 4.9 mmol), HBTU (2.24 g, 5.9 mmol), DIPEA (3.2 g, 24.5 mmol), and compound S25-2 (2.24 g, 5.9 mmol) were dissolved in DMF (30 mL). The mixture was stirred at room temperature for 3 h. The reaction mixture was monitored by TLC to show that the reaction was completed. Water (20 mL) was added to the reaction solution, and the reaction solution was extracted twice with ethyl acetate. The organic phase was washed with water, then with saturated brine, dried over anhydrous sodium sulfate, and the organic phase was rotary evaporated to dryness to afford the target compound S25-3 (3.5 g, yield 89.7%).
2) Compound S25-3 (3.5 g, 4.4 mmol) was dissolved in a solution of 1% DBU in DCM (40 mL). The mixture was stirred at room temperature for 1.5 h. The reaction mixture was monitored by TLC to show that the reaction was completed. The organic phase was rotary evaporated to dryness, mixed with silica gel, and separated through a normal-phase column to obtain the target product S25-4 (2.35 g, yield 92.1%).
3) Compound S25-4 (2.35 g, 4.06 mmol), DEPBT (1.7 g, 5.7 mmol), DIEA (1.5g, 12 mmol), and compound S25-5 (1.77 g, 5.7 mmol) were dissolved in DMF (30 mL). The mixture was stirred at room temperature for 3 h. The reaction mixture was monitored with a TLC plate to show that the reaction was completed. Water (20 mL) was added to the reaction solution, and the reaction solution was extracted twice with ethyl acetate. The organic phase was washed with water, then with saturated brine, dried over anhydrous sodium sulfate, and the organic phase was rotary evaporated to dryness and purified through a silica gel column to obtain the target product S25-6 (2.5 g, yield 71.4%).
4) Compound S25-6 (1.5 g, 1.72 mmol) was added to 15 mL of a solution of hydrogen chloride in dioxane and stirred at room temperature for 2 hours. The reaction solution was concentrated and rotary evaporated to dryness to obtain the product compound S25-7 (1.2 g, yield 85.7%).
5) DIPEA (379 mg, 2.94 mmol), HBTU (1.1 g, 2.94 mmol), and compound S25-8 (516 mg, 1.76 mmol, TFA salt) were added to a 100 mL DMF solution containing compound S25-7 (1.2 g, 1.47 mmol). The reaction was stirred at 25°C for 0.5 hour.The reaction solution was concentrated and purified by silica gel column chromatography (DCM/MeOH=20:1) to obtain the product compound S25-9 (800 mg, yield 54%).
6) Compound S25-9 (800 mg, 0.806 mmol) and tetrakis(triphenylphosphine)palladium (73 mg, 0.1 eq) were placed in a dry three-necked flask. Anhydrous dichloromethane was added under nitrogen protection and ice bath. The mixture was allowed to warm to room temperature and reacted for 1 h. The mixture was filtered. The reaction solution was concentrated, and purified by silica gel column chromatography (DCM/MeOH=20:1) to obtain the product compound S25-10 (500 mg, yield 65%).
7) Compound S25-10 (120 mg, 0.126 mmol) and compound X5 (76 mg, 0.098 mmol) were dissolved in DMF (3 mL). HBTU (57 mg, 0.150 mmol) and DIPEA (49 mg, 0.38 mmol) were added, and the reaction was allowed to proceed for 2 hours. Compound S25 (57 mg, yield 26.3%) was obtained by reverse-phase preparation as a pale yellow solid. LCMS: [M+H]⁺=1713.81.

### Example 6: Enzymatic Cleavage Test of Drug Conjugates S1-S59

The stability and enzymatic cleavage efficiency of the drug conjugates was detected using the following method, and the test results are shown in Table 7.

Each drug conjugate compound was accurately weighed and dissolved in a certain amount of DMSO to prepare a stock solution at a concentration of 10 µmol/mL. Then, 10 µL of the stock solution was added to 80 µL of DMSO to dilute to a concentration of 1 µmol/mL, and then further diluted with pure water at a ratio of 1:4 to achieve a sample solution concentration of 0.2 µmol/mL. After the sample became clear, it was placed in a water bath at 25 °C / 37 °C. Samples were taken at 0 hour and 24 hours and analyzed by HPLC (Agilent 1260, column: Eclipse Plus C18, 4.6×250 mm, 5 µm; mobile phase A: 0.1% TFA + H₂O; mobile phase B: CH3CN; flow rate: 1.0 mL/min; column temperature: 30 °C; detection wavelength: 214 nm) to determine the content of the drug conjugate relative to that at 0 hour, thereby obtaining the solution stability data of the different compounds.

An appropriate amount of sample was weighed and dissolved in DMSO to prepare a 1 mM solution. Human plasma was added at a volume ratio of 1:9, and the mixture was incubated in an incubator at 37 °C. Aliquots were taken at 0, 2, 4, 6, and 24 hours, and DMSO/MeOH (1:1) was added at a volume ratio of 1:3 to remove proteins. The mixture was centrifuged at 12000 rpm for 5 minutes, and the supernatant was collected. The HPLC analysis was performed using the method described above (Agilent 1260, column: Eclipse Plus C18, 4.6 * 250 mm, 5 µm; mobile phase A: 0.1% TFA + H₂O; mobile phase B: CH₃CN; flow rate: 1.0 mL/min; column temperature: 30 °C; detection wavelength: 214 nm).

A certain amount of the drug conjugates were weighed, dissolved, and diluted tenfold to a concentration of 0.1 mM/ml. The drug conjugates were added at a concentration of 1 mg/ml to 100 µg of acidified CT-26 tumor tissue homogenate (pH 5.0) at 37°C. The enzymes in the tumor tissue homogenate were able to release the drug, and the released drug was detected by HPLC to compare the activation efficiency of the linker in the tumor tissue.

**Table 7**

| Compound No. | Aqueous solution stability % (24 h) | Plasma stability % (24 h) | Enzymatic cleavage efficiency % (2 h) |
|---|---|---|---|
| Control Compound C | 80 | 70 | 80 |
| S1 | 95 | 80 | 91 |
| S2 | 95 | 85 | 90 |
| S3 | 95 | 85 | 90 |
| S4 | 95 | 88 | 90 |
| S5 | 100 | 99 | 95 |
| S6 | 99 | 100 | 95 |
| S7 | 95 | 88 | 95 |
| S8 | 95 | 90 | 95 |
| S9 | 95 | 88 | 95 |
| S10 | 99 | 95 | 90 |
| S11 | 99 | 95 | 92 |
| S12 | 95 | 95 | 95 |
| S13 | 99 | 98 | 95 |
| S14 | 99 | 100 | 95 |
| S15 | 100 | 99 | 98 |
| S16 | 100 | 99 | 95 |
| S17 | 100 | 98 | 97 |
| S18 | 100 | 98 | 97 |
| S19 | 100 | 98 | 98 |
| S20 | 100 | 99 | 100 |
| S21 | 100 | 99 | 100 |
| S22 | 98 | 98 | 95 |
| S23 | 99 | 99 | 98 |
| S24 | 99 | 95 | 100 |
| S25 | 99 | 97 | 95 |
| S26 | 98 | 98 | 100 |
| S27 | 99 | 98 | 100 |
| S28 | 100 | 98 | 100 |
| S29 | 100 | 98 | 99 |
| S30 | 100 | 99 | 100 |
| S31 | 100 | 99 | 99 |
| S32 | 98 | 95 | 99 |
| S33 | 100 | 99 | 100 |
| S34 | 98 | 98 | 100 |
| S35 | 98 | 99 | 100 |
| S36 | 98 | 98 | 100 |
| S37 | 95 | 95 | 95 |
| S38 | 95 | 97 | 99 |
| S39 | 93 | 95 | 97 |
| S40 | 94 | 95 | 95 |
| S41 | 100 | 99 | 100 |
| S42 | 98 | 99 | 100 |
| S43 | 95 | 95 | 100 |
| S44 | 95 | 97 | 100 |
| S45 | 95 | 98 | 100 |
| S46 | 95 | 99 | 100 |
| S47 | 95 | 99 | 100 |
| S48 | 95 | 99 | 100 |
| S49 | 95 | 99 | 100 |
| S50 | 98 | 98 | 100 |
| S51 | 98 | 98 | 100 |
| S52 | 95 | 95 | 100 |
| S53 | 95 | 95 | 100 |
| S54 | 97 | 95 | 99 |
| S55 | 100 | 95 | 100 |
| S56 | 99 | 95 | 100 |
| S57 | 99 | 95 | 100 |
| S58 | 100 | 99 | 100 |
| S59 | 99 | 98 | 100 |

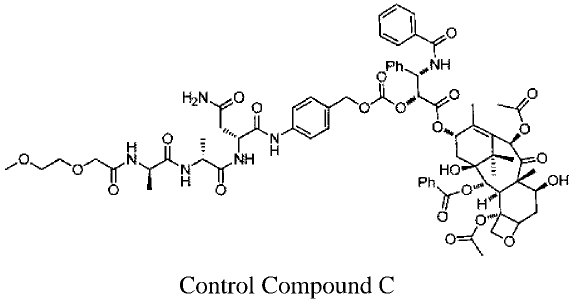

As can be seen from Table 7, compounds S1-S59 of the present disclosure exhibited high stability, with aqueous solution stability at 24 h all above 85%, plasma stability at 24 h all above 75%, and enzymatic cleavage efficiency at 2 h all above 90%. Control Compound C (compound S1 disclosed in CN104262455B) showed lower aqueous solution stability and plasma stability at 24 h, and lower enzymatic cleavage efficiency at 2 h compared to compounds S1-S59 of the present disclosure. The reason is that the stability of the amide bond and urethane bond between the linker and the payload in the present disclosure is much higher than the carbonate bond in Control Compound C, and the ester bond is more prone to hydrolysis.

The above results indicate that compounds S1-S59 provided by the present disclosure have higher stability in aqueous solution and plasma, and the corresponding compounds are not easily degraded or transformed into other compounds after entering the body, and the targeted therapeutic effect after entering tumor cells or tumor tissues is stable. In addition, after entering tumor cells, compounds S1-S59 provided by the present disclosure are cleaved by specific enzymes within 2 hours, releasing toxic drugs to achieve higher killing rate of tumor cells.

### Example 7: Comparison of activation and release of Dxd by S5, S16 and Control Compound Y

A certain amount of the drug conjugates (S5, S16, Deruxtecan, Control Compound Y; wherein Control Compound Y is compound S1 in CN103011521B) were weighed, dissolved, and diluted tenfold to a concentration of 0.1 mmol/ml. The drug conjugates were added at a concentration of 1 mg/ml to 100 µg of acidified CT-26 tumor tissue homogenate (pH 5.0) at 37°C. The enzymes in the tumor tissue homogenate were able to release the drug, and the released drug was detected by HPLC to compare the activation efficiency of the linker in the tumor tissue.

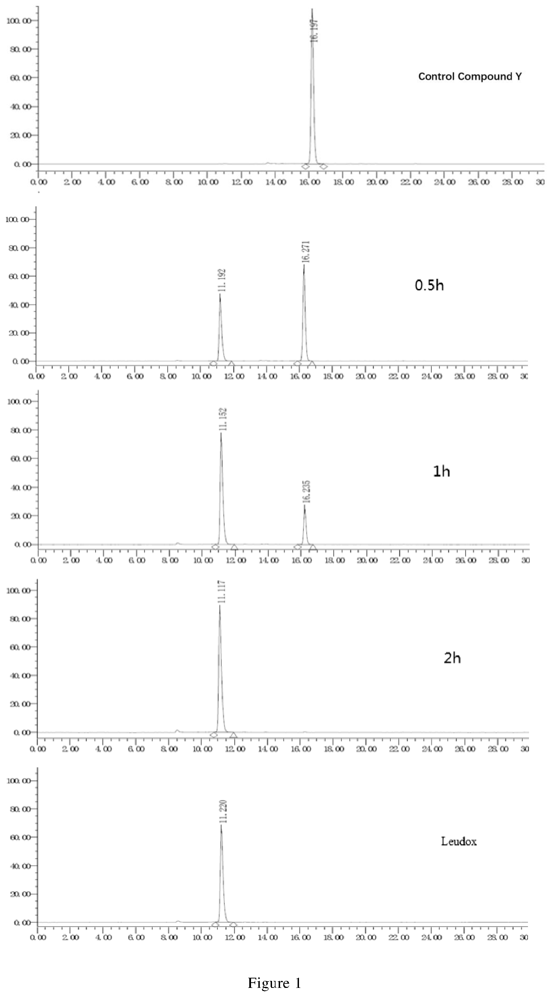

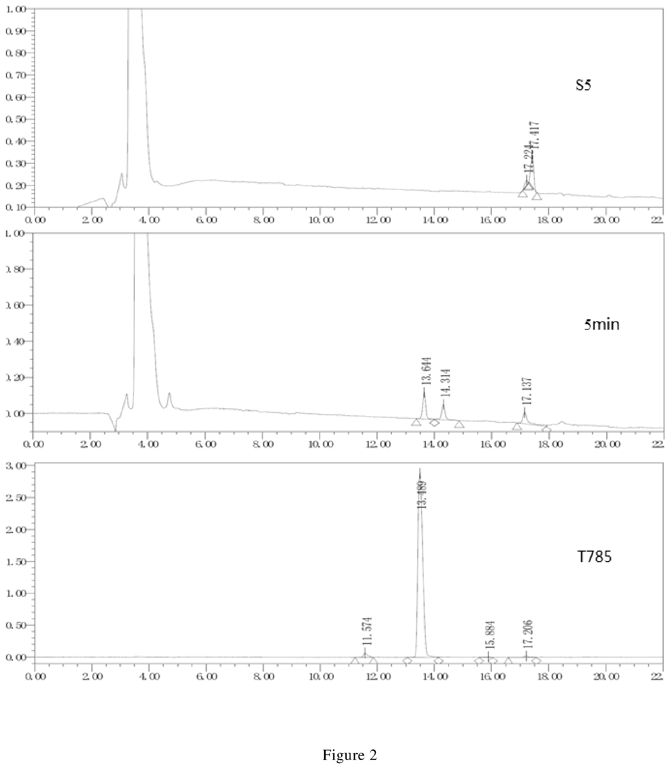

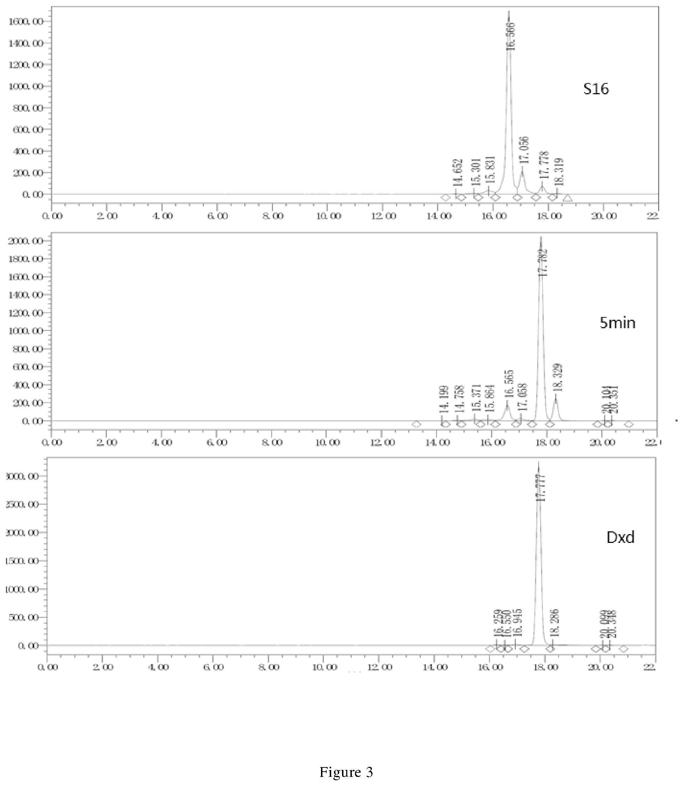

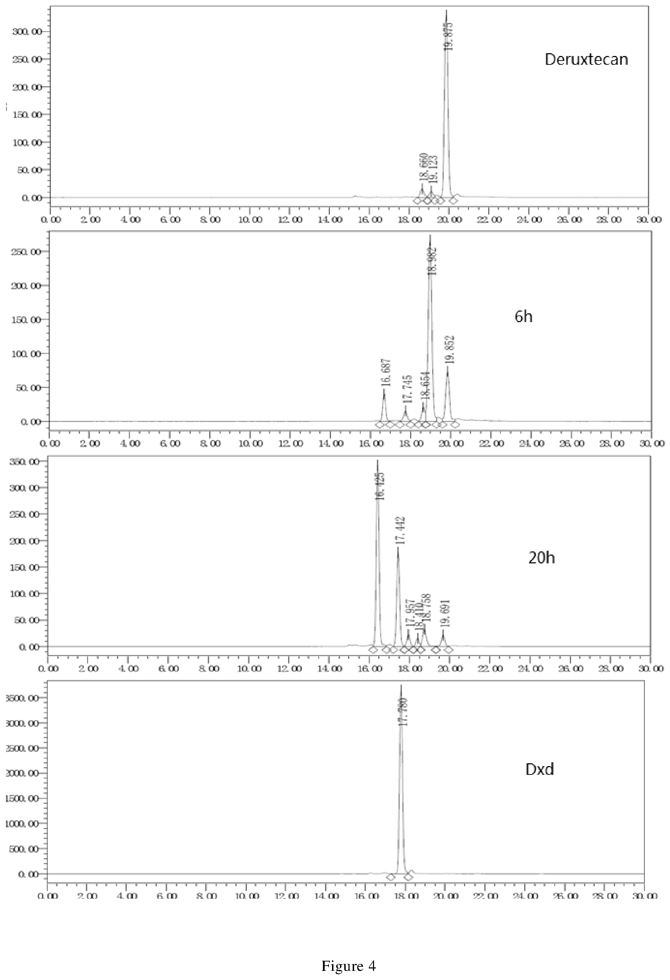

The results are shown in Figure 1 to 4. Figure 1 shows that Control Compound Y started to release Leudox at 0.5 h under the action of the enzyme (no release of Leudox was detected at 5 min), and the release was complete in about 2 h. Figure 2 shows that compound S5 started to release T785 at 5 min. Figure 3 shows that compound S16 started to release Dxd at 5 min. Figure 4 shows that Deruxtecan started to release Dxd at 6 h under the action of Cathepsin B enzyme.

Therefore, the activation efficiency of compounds S5 and S16 was significantly better than that of Control Compound Y and Deruxtecan.

### Example 8: Comparison of Lung Tissue Stability between S16 and Deruxtecan

### Method:

### Preparation of experimental solutions:

Detection buffer: 25 mM MES, pH 5.0.

Protein precipitant: DMSO/MeOH = 1:1.

Preparation of sample solution: Appropriate amounts of 6PEG-lys(MI-6PEG)-AAN-AM-Dxd and EMC-GGFG-AM-Dxd were weighed and a certain amount of DMSO was added to prepare a stock solution at a concentration of 10 µmol/mL. Then, 10 µL of the stock solution was taken and diluted with 80 µL of DMSO to a concentration of 1 µmol/mL, and then further diluted with pure water at a ratio of 1:4 to achieve a sample solution concentration of 0.2 µmol/mL.

### Preparation of lung tissue homogenate:

An appropriate amount of flash-frozen lung in liquid nitrogen was placed in a 4 mL EP tube, weighed, and the detection buffer (pH 5.0) was added at a mass-to-volume ratio of 1:5. The tissue was homogenized for 3 min using a tissue grinder, and then the lung tissue homogenate was centrifuged at 12000 rpm for 5 min. The supernatant was collected and stored in a -40°C refrigerator for later use.

### Stability of lung tissue homogenate:

The sample solution and the lung tissue homogenate were shaken thoroughly at a volume ratio of 1:9. Another sample solution and the buffer (pH 5.0) were shaken thoroughly at a volume ratio of 1:9 as a blank control solution. The mixtures were placed in a 37°C constant temperature water bath. The sample was taken at 0 h, 2 h, 4 h, and 20 h, 30 µL, respectively, and 90 µL of the protein precipitant was added, the mixture was shaken thoroughly, centrifuged at 12000 rpm for 5 min, the supernatant was transferred to a sampling vial, and HPLC injection was performed.

The percentage of DXD released after the drug entered the lung tissue homogenate was calculated using the following formula: 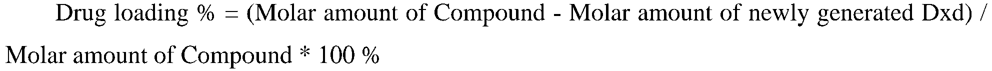 Drug loading % = (Molar amount of Compound - Molar amount of newly generated Dxd) / Molar amount of Compound * 100 %

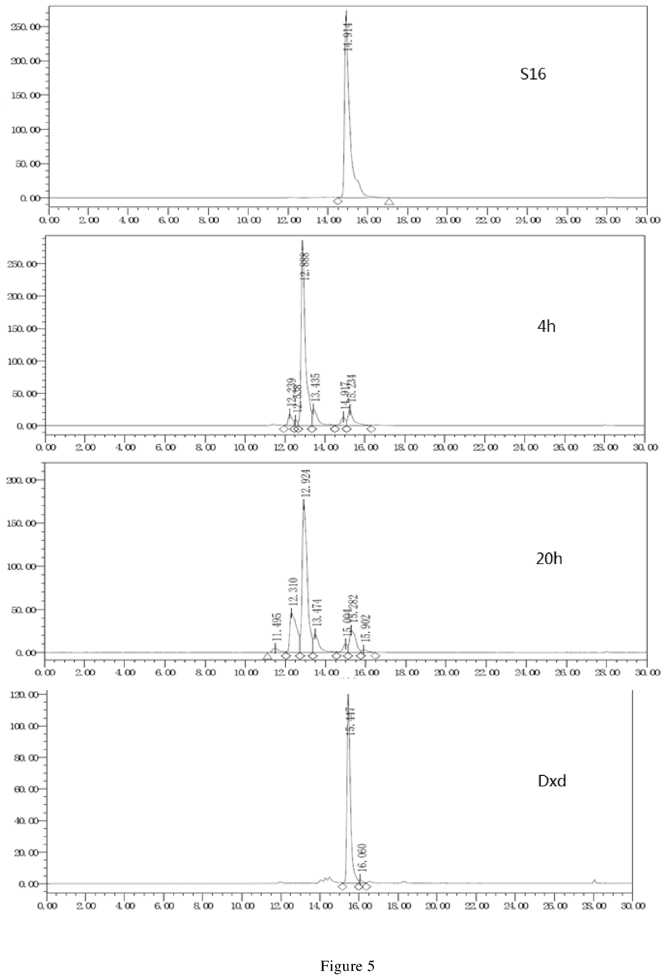

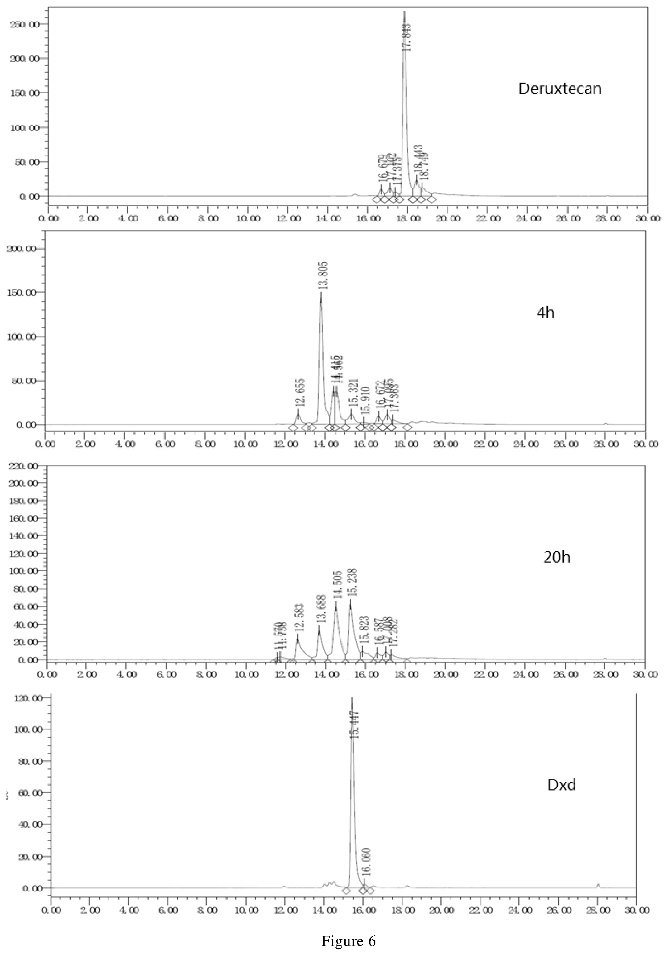

The results are shown in Figures 5, 6 and Table 8 below.

**Table 8**

| | Time (h) | EMC-GGFG-AM-Dxd | S16 |
|---|---|---|---|
| | | Dxd (%) | |
| Lung tissue homogenate | 0 | 1.47 | 0.00 |
| | 2 | 3.39 | 3.02 |
| | 4 | 14.58 | 3.79 |
| | 20 | 30.93 | 10.03 |

The results showed that S16 produced less Dxd in lung tissue compared to Deruxtecan, exerting a smaller impact on normal lung tissue.

### Example 9: Preparation of Conjugate Intermediates L1-L51

Some compounds were prepared using the following reaction scheme: 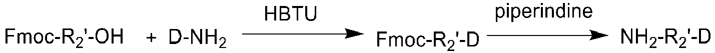
1) Compound Fmoc-R₂'-OH (1.0 eq) was dissolved in an appropriate amount of DMF solvent. Then, compound D-NH₂ and condensing agent HBTU were sequentially added. DIPEA was added to the reaction solution under ice bath. The reaction mixture was slowly warmed to room temperature and stirred for another 3-5 h. Most of the solvent was removed from the obtained reaction solution under high vacuum. The reaction mixture was then diluted with an appropriate amount of methanol. The mixture was purified by reverse-phase high-pressure preparative chromatography, and finally lyophilized to obtain the target product.
2) Compound Fmoc-R₂'-D (1.0 eq) was dissolved in DMF solvent and stirred for 30 min, then piperidine (3.0 eq) was added. The reaction solution was stirred at room temperature for another 2-3 h. The reaction solution was evaporated to dryness under reduced pressure, and the residue was triturated with a mixed solvent of MTBE to obtain the target product NH₂-R₂'-D.

Using the corresponding starting materials, the compounds shown in Table 9 were obtained by the above method, and the properties of each compound in Table 9 are shown in Table 10.

**Table 9**

| R₂' | D | Compound No. |
|---|---|---|
| 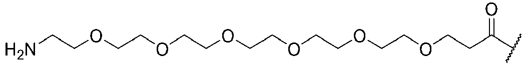 | X1 | L1 |
| 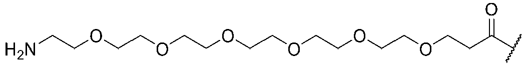 | X2 | L2 |
| 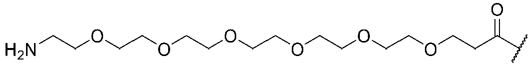 | X3 | L3 |
| 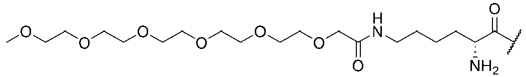 | X2 | L6 |
| 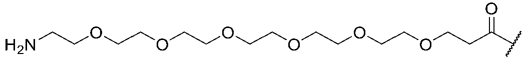 | X4 | L7 |
| 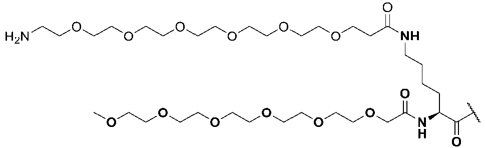 | X4 | L8 |
| 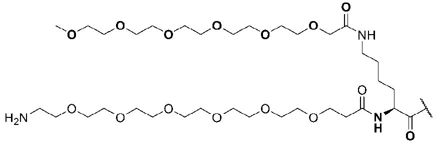 | X4 | L9 |
| 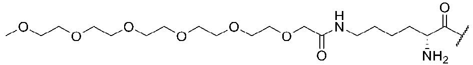 | X4 | L10 |
| 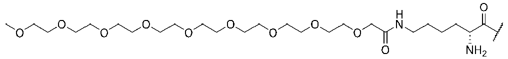 | X4 | L11 |
| 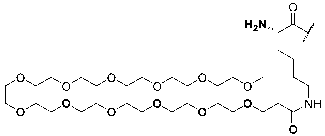 | X4 | L12 |
| 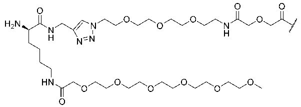 | X4 | L15 |
| 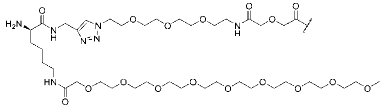 | X4 | L16 |
| 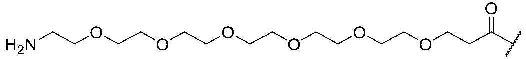 | X5 | L17 |
| 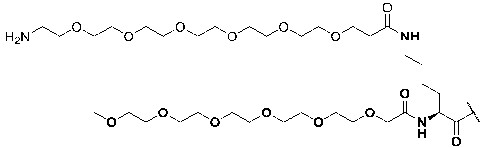 | X5 | L18 |
| 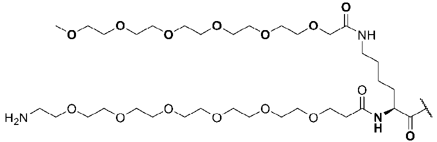 | X5 | L19 |
| 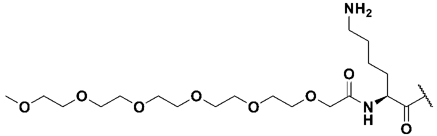 | X5 | L20 |
| 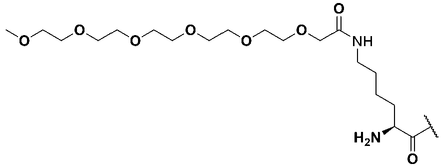 | X5 | L21 |
| 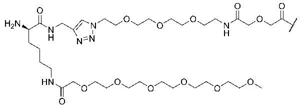 | X5 | L24 |
| 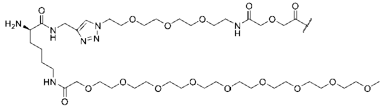 | X5 | L25 |
| 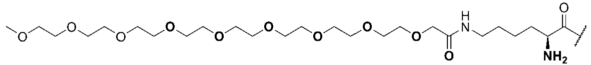 | X5 | L26 |
| 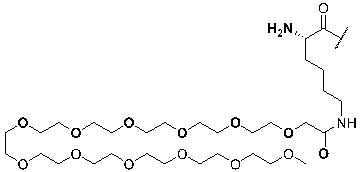 | X5 | L27 |
| 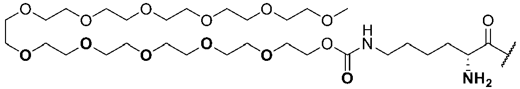 | X5 | L30 |
| 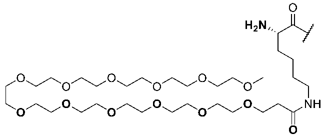 | X5 | L31 |
| 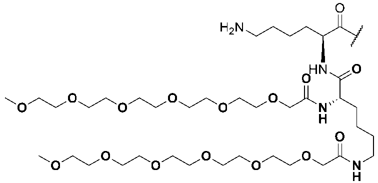 | X5 | L38 |
| 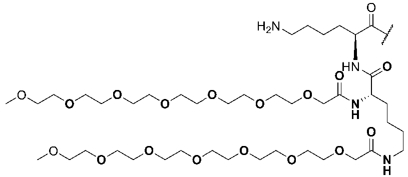 | X5 | L39 |
| 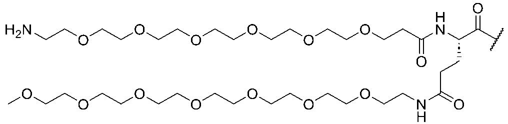 | X5 | L42 |
| 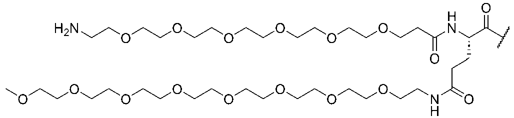 | X5 | L43 |
| 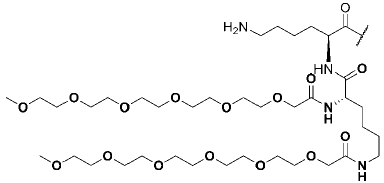 | X4 | L45 |
| 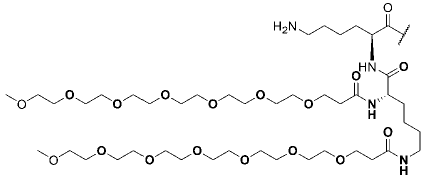 | X4 | L46 |
| 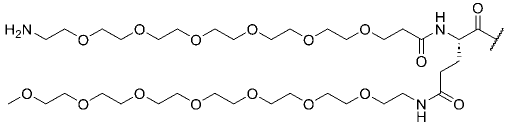 | X4 | L50 |
| 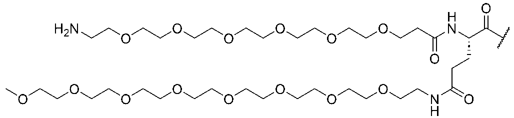 | X4 | L51 |

**Table 10**

| Compound No. | Appearance | Yield (mg) | Molecular Weight | Mass Spectrometry[M+H] |
|---|---|---|---|---|
| L1 | White powder | 30 | 906.05 | 906.40 |
| L2 | White powder | 45 | 1052.24 | 1052.57 |
| L3 | White powder | 38 | 903.09 | 903.52 |
| L6 | White powder | 42 | 1137.35 | 1137.62 |
| L7 | Light yellow powder | 40 | 1176.26 | 1176.52 |
| L8 | Light yellow powder | 35 | 1596.77 | 1596.78 |
| L9 | Light yellow powder | 28 | 1596.77 | 1596.78 |
| L10 | Light yellow powder | 32 | 1261.37 | 1261.57 |
| L11 | Light yellow powder | 35 | 1393.53 | 1393.65 |
| L12 | Light yellow powder | 47 | 1539.71 | 1539.74 |
| L15 | Light yellow powder | 42 | 1632.76 | 1632.75 |
| L16 | Light yellow powder | 40 | 1764.92 | 1764.83 |
| L17 | Light yellow powder | 27 | 1114.19 | 1114.50 |
| L18 | Light yellow powder | 47 | 1534.70 | 1534.75 |
| L19 | Light yellow powder | 37 | 1534.70 | 1534.75 |
| L20 | Light yellow powder | 42 | 1199.30 | 1199.56 |
| L21 | Light yellow powder | 31 | 1199.30 | 1199.56 |
| L24 | Light yellow powder | 24 | 1570.69 | 1570.74 |
| L25 | Light yellow powder | 28 | 1702.85 | 1702.81 |
| L26 | Light yellow powder | 33 | 1331.46 | 1331.63 |
| L27 | Light yellow powder | 18 | 1463.62 | 1463.71 |
| L30 | Light yellow powder | 25 | 1449.59 | 1449.70 |
| L31 | Light yellow powder | 15 | 1477.64 | 1477.73 |
| L38 | Light yellow powder | 20 | 1619.80 | 1619.81 |
| L39 | Light yellow powder | 27 | 1707.91 | 1707.86 |
| L42 | Light yellow powder | 35 | 1564.72 | 1564.72 |
| L43 | Light yellow powder | 20 | 1608.77 | 1608.79 |
| L45 | Light yellow powder | 18 | 1681.87 | 1681.82 |
| L46 | Light yellow powder | 22 | 1798.03 | 1798.00 |
| L50 | Light yellow powder | 20 | 1626.79 | 1626.78 |
| L51 | Light yellow powder | 15 | 1670.85 | 1670.80 |

Some compounds were prepared using the following reaction scheme: 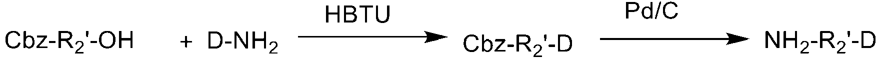
1) Compound Cbz-R₂'-OH (1.0 eq) was dissolved in an appropriate amount of DMF solvent. Then, compound D-NH₂ and condensing agent HBTU were sequentially added. DIPEA was added to the reaction solution under ice bath. The reaction mixture was slowly warmed to room temperature and stirred for another 3-5 h. Most of the solvent was removed from the obtained reaction solution under high vacuum. The reaction mixture was then diluted with an appropriate amount of methanol. The mixture was purified by reverse-phase high-pressure preparative chromatography, and finally lyophilized to obtain the target product.
2) Compound Cbz-R₂'-D (1.0 eq) was dissolved in methanol solvent and stirred for 30 min, then Pd/C (0.1 eq) was added. The reaction solution was stirred at room temperature for another 2-3 h.The reaction solution was evaporated to dryness under reduced pressure, and the residue was triturated with a mixed solvent of MTBE and ethyl acetate to obtain the target product NH₂-R₂'-D.

Using the corresponding starting materials, the compounds shown in Table 11 were obtained by the above method, and the properties of each compound in Table 11 are shown in Table 12.

**Table 11**

| R₂' | D | Compound No. |
|---|---|---|
| 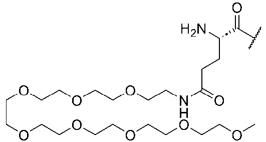 | X3 | L4 |
| 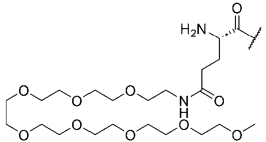 | X2 | L5 |
| 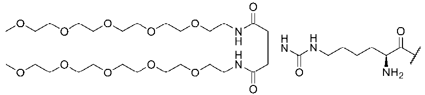 | X5 | L32 |
| 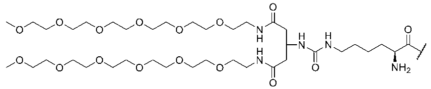 | X5 | L33 |
| 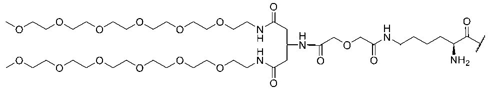 | X5 | L34 |
| 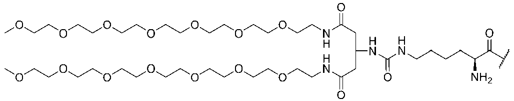 | X5 | L35 |
| 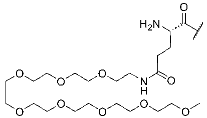 | X5 | L36 |
| 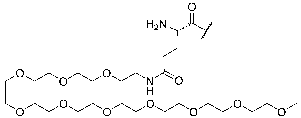 | X5 | L37 |
| 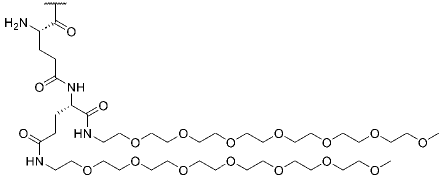 | X5 | L40 |
| 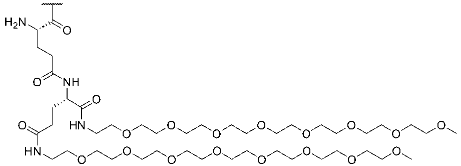 | X5 | L41 |
| 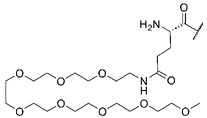 | X4 | L44 |
| 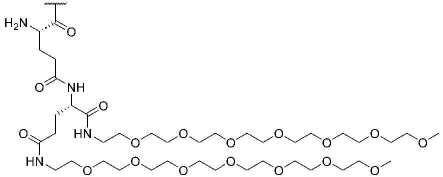 | X4 | L47 |
| 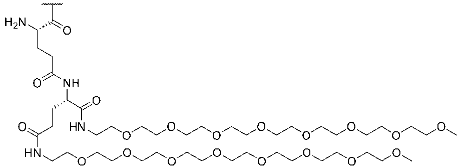 | X4 | L48 |
| 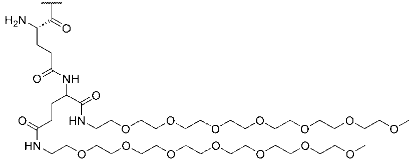 | X4 | L49 |

**Table 12**

| Compound No. | Appearance | Yield (mg) | Molecular Weight | Mass Spectrometry[M+H] |
|---|---|---|---|---|
| L4 | White powder | 50 | 1062.28 | 1062.61 |
| L5 | White powder | 65 | 1211.43 | 1211.66 |
| L32 | Light yellow powder | 70 | 1546.71 | 1546.76 |
| L33 | Light yellow powder | 48 | 1634.82 | 1634.81 |
| L34 | Light yellow powder | 61 | 1706.88 | 1706.83 |
| L35 | Light yellow powder | 50 | 1722.92 | 1722.87 |
| L36 | Light yellow powder | 63 | 1273.38 | 1273.59 |
| L37 | Light yellow powder | 47 | 1361.48 | 1364.48 |
| L40 | Light yellow powder | 38 | 1679.85 | 1679.82 |
| L41 | Light yellow powder | 40 | 1767.96 | 1767.90 |
| L44 | Light yellow powder | 45 | 1335.45 | 1335.61 |
| L47 | Light yellow powder | 37 | 1741.92 | 1741.84 |
| L48 | Light yellow powder | 62 | 1830.03 | 1829.99 |
| L49 | Light yellow powder | 48 | 1741.92 | 1741.84 |

Compounds L14 and L23 were prepared using the following method:

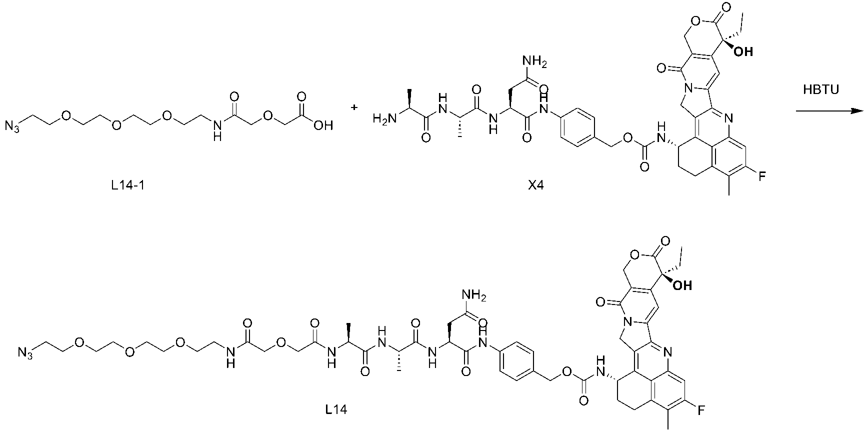

Compound L14-1 (100 mg, 0.299mmol) was dissolved in an appropriate amount of DMF solvent. Then, compound X4 (250 mg, 0.297mmol) and condensing agent HBTU (136 mg, 0.359mmol) were sequentially added. DIPEA (116 mg, 0.899mmol) was added to the reaction solution under ice bath. The reaction mixture was slowly warmed to room temperature and stirred for another 3-5 h. Most of the solvent was removed from the obtained reaction solution under high vacuum. The reaction mixture was then diluted with an appropriate amount of methanol. The mixture was purified by reverse-phase high-pressure preparative chromatography, and finally lyophilized to obtain L14 (100 mg, yield 29%) as a light yellow powder. LCMS: [M+H]=1157.46.

Compound L23 was prepared under the same reaction conditions by replacing X4 with X5.

Compounds L13 and L22 were prepared using the following method:

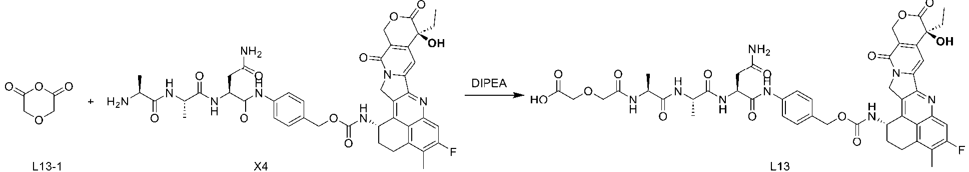

Compound diglycolic anhydride (14 mg, 0.12mmol) was dissolved in an appropriate amount of DMF solvent. Then, compound X4 (100 mg, 0.119mmol) were sequentially added. DIPEA (31 mg, 0.24mmol) was added to the reaction solution under ice bath. The reaction mixture was slowly warmed to room temperature and stirred for another 3-5 h. Most of the solvent was removed from the obtained reaction solution under high vacuum. The reaction mixture was then diluted with an appropriate amount of methanol. The mixture was purified by reverse-phase high-pressure preparative chromatography, and finally lyophilized to obtain L14 (80 mg, yield 70%) as a light yellow powder. LCMS: [M+H]=957.34.

Compound L22 was prepared under the same reaction conditions by replacing X4 with X5.

Compounds L28 and L29 were prepared using the following method:

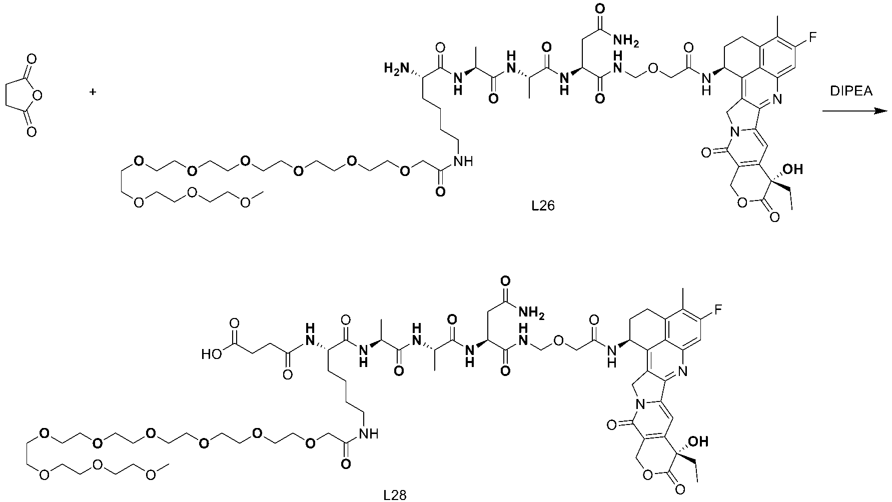

Compound succinic anhydride (3 mg, 0.03mmol) was dissolved in an appropriate amount of DMF solvent. Then, compound L26 (20 mg, 0.015mmol) were sequentially added. DIPEA (31 mg, 0.24mmol) was added to the reaction solution under ice bath. The reaction mixture was slowly warmed to room temperature and stirred for another 3-5 h. Most of the solvent was removed from the obtained reaction solution under high vacuum. The reaction mixture was then diluted with an appropriate amount of methanol. The mixture was purified by reverse-phase high-pressure preparative chromatography, and finally lyophilized to obtain L28 (20 mg, yield 95%) as a light yellow powder. LCMS: [M+H]=1431.65.

Compound L29 was prepared under the same reaction conditions by replacing L26 with L27.

### Example 10: Preparation of Compounds X1-X5

### Synthesis of compound X1:

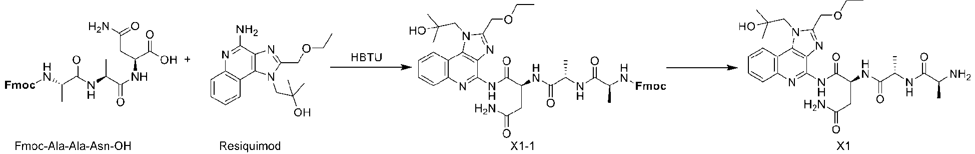

### 1) Synthesis of compound X1-1

Compound Fmoc-Ala-Ala-Asn-OH (500 mg, 1.01 mmol) and Resiquimod (314 mg, 1.00 mmol) were dissolved in super dry DMF (5 mL).Condensing agent HBTU (455 mg, 1.20 mmol) and N,N-diisopropylethylamine (387 mg, 3.00 mmol) were added, and the mixture was stirred at room temperature for 16 h. The reaction solution was evaporated to dryness under reduced pressure, and then triturated with a mixed solvent of ethanol and MTBE, filtered to obtain compound X1-1 (500 mg, yield: 63%) as a white solid.

### 2) Synthesis of Compound X1

Compound X1-1 (500 mg, 0.631 mmol) was dissolved in DMF solvent (10 mL) and stirred for 30 min, then piperidine (160 mg, 1.88 mmol) was added. The reaction solution was stirred at room temperature for another 2-3 h. The reaction solution was evaporated to dryness under reduced pressure, and the residue was triturated with a mixed solvent of MTBE to obtain compound X1 (300 mg, yield 83%) as a white solid.

### Synthesis of compound X2:

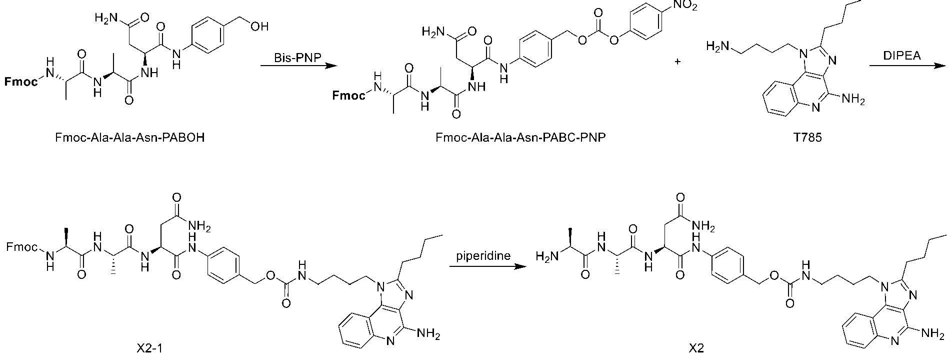

### 1) Synthesis of compound Fmoc-Ala-Ala-Asn-PABC-PNP

Compound Fmoc-Ala-Ala-Asn-PABC (800mg, 1.33mmol) and Bis-PNP (808mg, 2.66mmol) were dissolved in super dry DMF (5 mL). N,N-diisopropylethylamine (343mg, 2.66mmol) was added, and the mixture was stirred at room temperature for 16 h.The reaction solution was evaporated to dryness under reduced pressure, and then triturated with a mixed solvent of ethanol and MTBE, and filtered to obtain compound Fmoc-Ala-Ala-Asn-PABC-PNP (820mg, yield: 80%) as a white solid.

### 2) Synthesis of compound X2-1

Compound Fmoc-Ala-Ala-Asn-PABC-PNP (200 mg, 0.261 mmol) was dissolved in DMF solvent (20 mL) and stirred for 1 h. Then compound T785 (80 mg, 0.257 mmol) and N,N-diisopropylethylamine (60 mg, 0.465 mmol) were added. The reaction solution was evaporated to dryness under reduced pressure, and the residue was triturated with a mixed solvent of MTBE to obtain compound X2-1 (200mg, yield 83%) as a white solid.

### 3) Synthesis of compound X2:

Compound X2-1 (200 mg, 0.213 mmol) was dissolved in DMF solvent (5 mL) and stirred for 1 h. Then piperidine (55 mg, 0.647 mmol) was added. The reaction solution was evaporated to dryness under reduced pressure, and the residue was triturated with a mixed solvent of MTBE to obtain compound X2 (110 mg, yield 72%) as a white solid.

### Synthesis of compound X3:

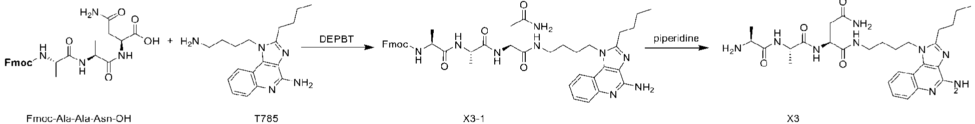

### 1) Synthesis of compound X3-1

Compound Fmoc-Ala-Ala-Asn-OH (450mg, 0.907mmol) and T785 (188mg, 0.605mmol) were dissolved in super dry DMF (5 mL). Condensing agent DEPBT (217mg, 0.726mmol) and N,N-diisopropylethylamine (234mg, 1.82mmol) were added, and the mixture was stirred at room temperature for 16 h. The reaction solution was evaporated to dryness under reduced pressure, and then triturated with a mixed solvent of ethanol and MTBE, and filtered to obtain compound X3-1 (300mg, yield: 63%) as a white solid.

### 2) Synthesis of Compound X3

Compound X3-1 (300 mg, 0.380 mmol) was dissolved in DMF solvent (20 mL) and stirred for 1 h. Then piperidine (100 mg, 1.18 mmol) was added. The reaction solution was evaporated to dryness under reduced pressure, and the residue was triturated with a mixed solvent of MTBE to obtain compound X3 (200mg, yield 93%) as a white solid.

### Synthesis of compound X4:

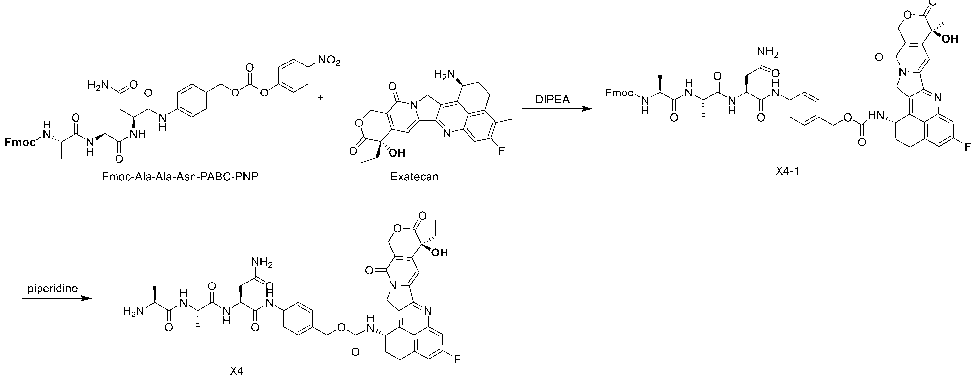

### 1) Synthesis of compound X4-1

Compound Fmoc-Ala-Ala-Asn-PABC-PNP (500 mg, 0.652mmol) and exatecan methanesulfonate (346mg, 0.652mmol) were dissolved in super dry DMF (8 mL). Organic base N,N-diisopropylethylamine (168mg, 1.30mmol) was added, and the mixture was stirred at room temperature for 16 h. The reaction solution was evaporated to dryness under reduced pressure, and then triturated with a mixed solvent of ethyl acetate and MTBE, and filtered to obtain compound X4-1 (600mg, yield: 86.6%) as a yellow solid.

### 2) Synthesis of Compound X4

Compound X4-1 (600 mg, 0.564mmol) was dissolved in DMF solvent (10 mL) and stirred for 1 h. Then piperidine (144 mg, 1.69 mmol) was added. The reaction solution was stirred for another 2 h. The reaction was monitored by TLC and stopped upon completion. The solution was evaporated to dryness under reduced pressure, and the residue was sequentially triturated with mixed solvents of MTBE and ethyl acetate to obtain compound X4 (450 mg, yield 95%) as a light yellow solid.

### Synthesis of compound X5:

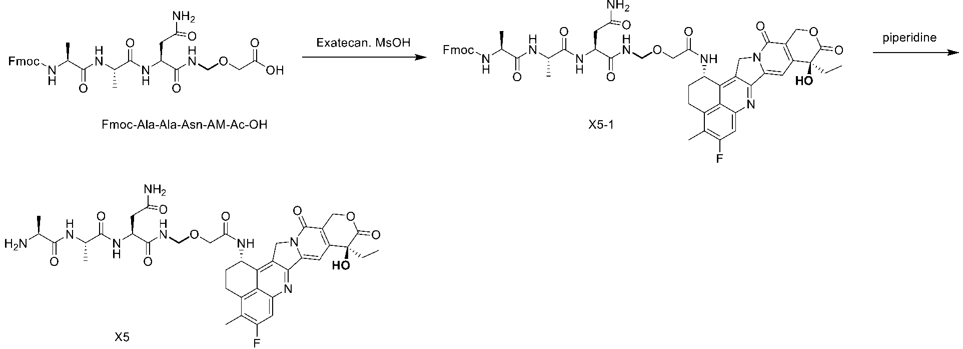

### 1) Synthesis of compound X5-1:

1) Compound Fmoc-Ala-Ala-Asn-AM-Ac-OH (500 mg, 0.857 mmol) and exatecan methanesulfonate (455 mg, 0.857 mmol) were dissolved in DMF. The reaction mixture was cooled to 0°C, and condensing agent DEPBT (307 mg, 1.03 mmol) and organic base N,N-diisopropylethylamine (332 mg, 2.57 mmol) were sequentially added under nitrogen protection. The mixture was stirred at room temperature for 2-3 h. The reaction mixture was monitored by TLC to show that the reaction was completed. The reaction solution was concentrated under vacuum, redissolved in DMF, filtered, and compound X5-1 was obtained by high-pressure liquid preparation as a pale yellow product (510 mg, yield 60%).

### 2) Synthesis of compound X5:

Compound X5-1 (500 mg, 0.50 mmol) was dissolved in DMF, piperidine (128 mg, 1.51 mmol) was added, and the mixture was stirred at room temperature under nitrogen protection for 2-3 h. The reaction mixture was monitored by TLC to show that the reaction was completed. The reaction solution was redissolved, filtered, and compound X5 was obtained by high-pressure liquid preparation as a pale yellow product (300 mg, yield 77%).

### Example 11: Preparation of Antibody-Drug Conjugates

The preparation method for antibody-drug conjugates was as follows: Tris(2-carboxyethyl)phosphine (TCEP) (1-40 eq) was added to an antibody buffer solution for reduction (0.5-16 h), and then the above drug conjugate (2-40 eq) was added and allowed to react (0.5-16 h) to form the antibody-drug conjugate. The pure antibody-drug conjugate was then obtained by purification via column chromatography and filtration.

The following ADCs were prepared using the above method: Trastuzumab-S15 (HER2-S15), anti LY6G6D antibody-S15 (LY6G6D-PEG6-AAN-AM-DXD), Atezolizumab-S5 (PD-L1-TLR7/8), Atezolizumab-S16 (PD-L1-PEG-K(MI-PEG)-AAN-AM-DXd), anti GCC antibody-S16. The DAR value of each ADC was approximately 8. Note: The names in parentheses correspond to the names in the figures.

In the above ADCs, "HER2" refers to Trastuzumab (Kaihui Ruizhi Biotechnology (Shanghai) Co., Ltd.); "PD-L1" refers to Atezolizumab (the amino acid sequences of its variable region of light chain and variable region heavy chain being shown in SEQ ID NO: 1 and 2, respectively; the light chain and heavy chain being shown in SEQ ID NO: 3 and 4, respectively); "Anti-SSTR2" refers to anti SSTR2 antibody; "LY6G6D" refers to anti LY6G6D antibody (the amino acid sequences of its variable region of light chain and variable region of heavy chain being shown in SEQ ID NO: 5 and 6, respectively); and the amino acid sequences of the variable region of light chain and variable region of heavy chain of the anti GCC antibody are shown in SEQ ID NO: 7 and 8, respectively.

In the above ADCs, the drug conjugates used were as follows:
Affinity linker2 = EMC-PEG6-AAN-AM-Dxd (S15);
Affinity linker4 = EMC-C5-AAN-T785 (S5).

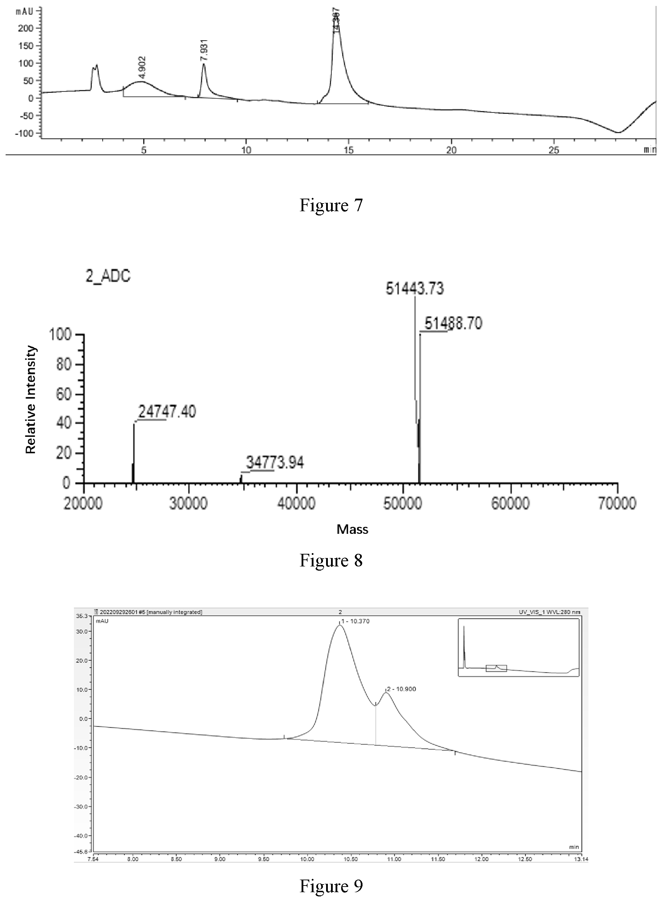

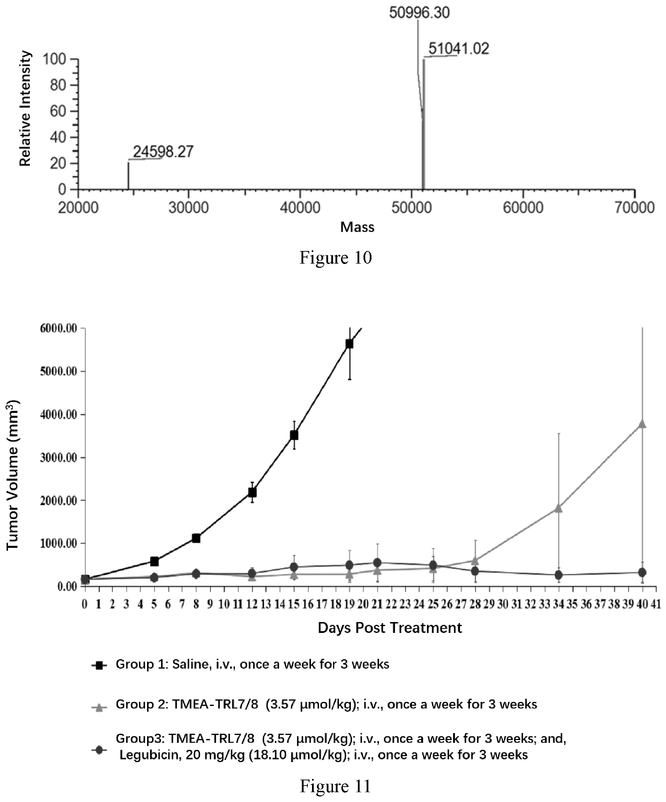

Affinity linker5 = PEG-K(MI-PEG)-AAN-AM-DXd (S16). Figure 7 shows the hydrophobic interaction chromatography (HIC) of Trastuzumab-S15; Figure 8 shows the MS spectrum of Atezolizumab-S5; Figure 9 shows the HIC spectrum of Atezolizumab-S5; and Figure 10 shows the MS spectrum of Atezolizumab-S5.

### Example 12

1. Animals: BALB/c mice, 8-week-old females (Shanghai Lingchang Biotechnology Co., Ltd.).
2. Cells: Cell Bank of Chinese Academy of Sciences. Cells were cultured in RPMI Medium 1640 (1x) medium containing 10% fetal bovine serum at 37°C in 5% CO₂, passaged every 3 days, and cells within 15 passages were used.
3. Tumor induction: 0.3×10⁶ CT26 cells were subcutaneously injected into the back of BALB/c mice. When the tumor grew to about 160 mm³, the mice were randomly grouped and treatment was started.
4. Treatment process: administration via tail vein once a week for a total of three weeks.

Grouping and result measurements are shown in Table 13 and Figure 11.

**Table 13**

| Group | Number of animals | Tumor volume on day 40(mm³) | TGI (%) on day 40 |
|---|---|---|---|
| PBS | 3 | >6000 | 0 |
| TMEA-TLR7/8 (3.57 µmol/kg) | 3 | 3779.98 | 76.74 |
| TMEA-TLR7/8 (3.57 µmol/kg)+Control Compound Y, 20 mg/kg(18.10 µmol/kg) | 3 | 320.89 | 98.03 |

| | | | |
|---|---|---|---|
| TGI% in the present disclosure refers to the tumor growth inhibition rate of the drug. TMEA-TLR7/8 is compound S5. | | | |

### Example 13

1. Animals: BALB/c mice, 8-week-old females (Shanghai Lingchang Biotechnology Co., Ltd.).
2. Cells: Cell Bank of Chinese Academy of Sciences. Cells were cultured in RPMI Medium 1640 (1x) medium containing 10% fetal bovine serum at 37°C in 5% CO₂, passaged every 3 days, and cells within 15 passages were used.
3. Tumor induction: 0.3×10⁶ CT26 cells were subcutaneously injected into the back of BALB/c mice. When the tumor grew to about 160 mm³, the mice were randomly grouped and treatment was started.
4. Treatment process: CTLA-4 was administered twice a week, and TMEA-TRL7/8 was administered once a week, for three weeks, by tail vein administration.

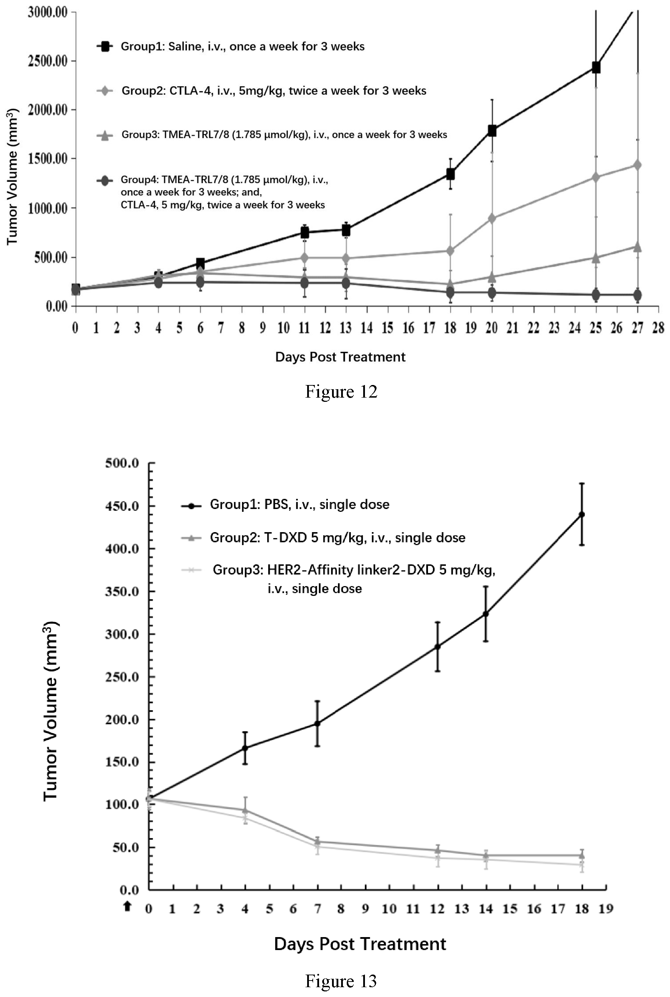

Grouping and result measurements are shown in Table 14 and Figure 12.

**Table 14**

| Group | Number of animals | Tumor volume on day 27 (mm³) | TGI (%) on day 27 |
|---|---|---|---|
| PBS | 3 | >3000 | 0 |
| CTLA-4, 5mg/kg | 3 | 1435.38 | 53.32 |
| TMEA-TRL7/8 (1.785 µmol/kg) | 3 | 604.86 | 80.33 |
| TMEA-TRL7/8 (1.785 µmol/kg) + CTLA-4, 5mg/kg | 3 | 108.82 | 96.46 |

### Example 14

1. Animals: NCG mice, 8-week-old females (Jiangsu GemPharmatech Co., Ltd.).
2. Cells: Cell Bank of Chinese Academy of Sciences. Cells were cultured in RPMI Medium 1640 (1x) medium containing 10% fetal bovine serum at 37°C in 5% CO₂, passaged every 7 days, and cells within 15 passages were used.
3. Tumor induction: 5×10⁶ NCI-N87 cells were subcutaneously injected into the back of NCG mice. When the tumor grew to about 100 mm³, the mice were randomly grouped and treatment was started.
4. Treatment process: administration via tail vein with a single dose.

Grouping and result measurements are shown in Table 15 and Figure 13.

**Table 15**

| Group | Number of animals | Tumor volume on day 18 (mm³) | TGI (%) on day 18 |
|---|---|---|---|
| PBS | 6 | 439.54 | 0 |
| T-DXD 5mg/kg | 6 | 40.23 | 90.85 |
| HER2-S15, 5mg/kg | 6 | 29.40 | 93.31 |

### Example 15

1. Animals: BALB/c mice, 8-week-old females (Shanghai Lingchang Biotechnology Co., Ltd.).
2. Cells: Wuhan Huamei Biotech. Cells were cultured in RPMI Medium 1640 (1x) medium containing 10% fetal bovine serum and 10 µg/ml Puromycin (10 mg/ml) at 37°C in 5% CO₂, passaged every 3 days, and cells within 15 passages were used.
3. Tumor induction: 0.3×10⁶ CT26 cells were subcutaneously injected into the back of BALB/c mice. When the tumor grew to about 100 mm³, the mice were randomly grouped and treatment was started.
4. Treatment process: intraperitoneal administration, twice a week for a total of three weeks.

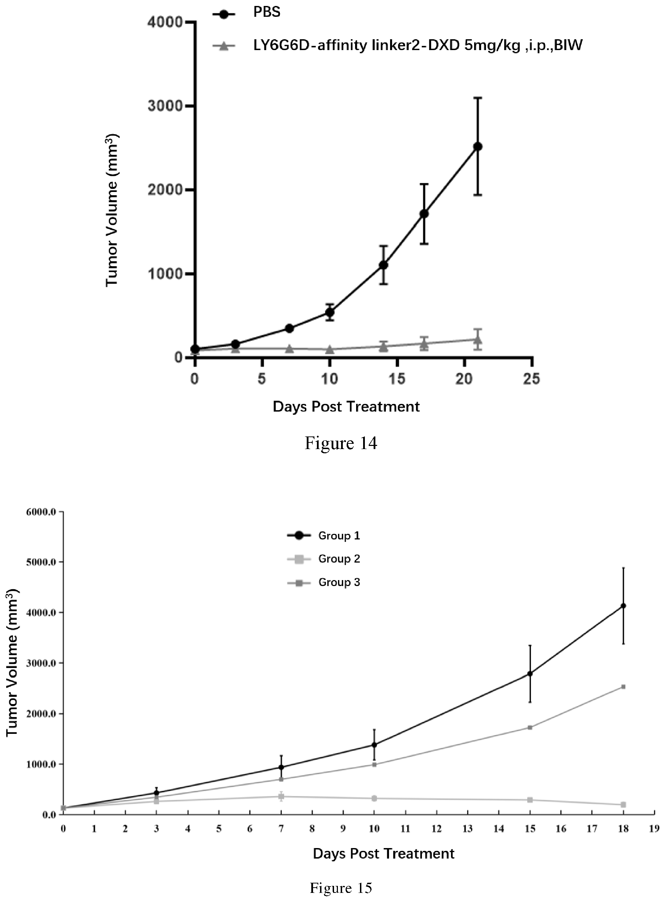

Grouping and result measurements are shown in Table 16 and Figure 14.

**Table 16**

| Group | Number of animals | Tumor volume on day 21(mm³) | TGI (%) on day 21 |
|---|---|---|---|
| PBS | 5 | 2517.62 mm³ | 0 |
| LY6G6D-PEG6-AAN-AM-DXD, 5mg/kg | 5 | 218.96 mm³ | 91.30 |

### Example 16

Female C57BL/6J-Pdedleml(hPDCD1)/Smoc mice aged 6-8 weeks were purchased from Shanghai Model Organisms Center. Upon arrival and unpacking, the mice were housed at a density of 5 mice per cage in an SPF-grade animal facility. General conditions of the animals (mental state, activity, fur color) were observed, and body weights were recorded in the first week. MC38 (mPD-L1 knockout; hPD-L1 knock-in) cells were harvested in the logarithmic growth phase and placed in sterile saline before animal inoculation. 1x10⁶ cells were subcutaneously inoculated into the upper inguinal region of the lateral hind limb of the mice. The administration regimens were as follows: Gr1 - control PBS (twice a week for a total of 2 doses); Gr2 - PDL1-PEG-K(MI-PEG)-AAN-AM-DXd (10 mg/kg, twice a week for a total of 2 doses); Gr3 - Atezolizumab (10 mg/kg, twice a week for a total of 2 doses). Tumor volume and mouse weight were measured twice a week. Tumor volume was calculated using the formula (0.5 × L × W²), wherein L and W represent the length and width of the tumor, respectively. When tumors reached 50 to 150 mm³, the mice were randomly grouped according to a random number table. At the end of the experiment, the mice were euthanized by cervical dislocation.

The results are shown in Figure 15 and Table 17 below.

**Table 17**

| Group | Treatment | TGI on day 18 |
|---|---|---|
| 1 | Vehicle | |
| 2 | PDL1-PEG-K(MI-PEG)-AAN-AM-DXd | 98.6% |
| 3 | Atezolizumab | 38.5% |

From the results in Figure 15 and Table 17, on day 18, the TGI of the drug PDL1-PEG-K(MI-PEG)-AAN-AM-DXd in the MC38 tumor model was 98.6%, and PDL1-PEG-K(MI-PEG)-AAN-AM-DXd was able to completely inhibit tumor growth from day 7 to 18. Therefore, the drug PDL1-Dxd exhibited good anti-tumor efficacy in the MC38 tumor model.

### Example 17

1. Animals: NCG mice, 8-week-old females (Jiangsu GemPharmatech Co., Ltd.).
2. Cells: Cell Bank of Chinese Academy of Sciences. Cells were cultured in DMEM (1x) medium containing 10% fetal bovine serum at 37°C in 5% CO₂, passaged every 3 days, and cells within 15 passages were used.
3. Tumor induction: 5×10⁶ MDA-MB-231 cells were subcutaneously injected into the back of NCG mice. When the tumor grew to about 1000 mm³, the mice were randomly grouped and treatment was started.
4. Treatment process: administration via tail vein with a single dose.

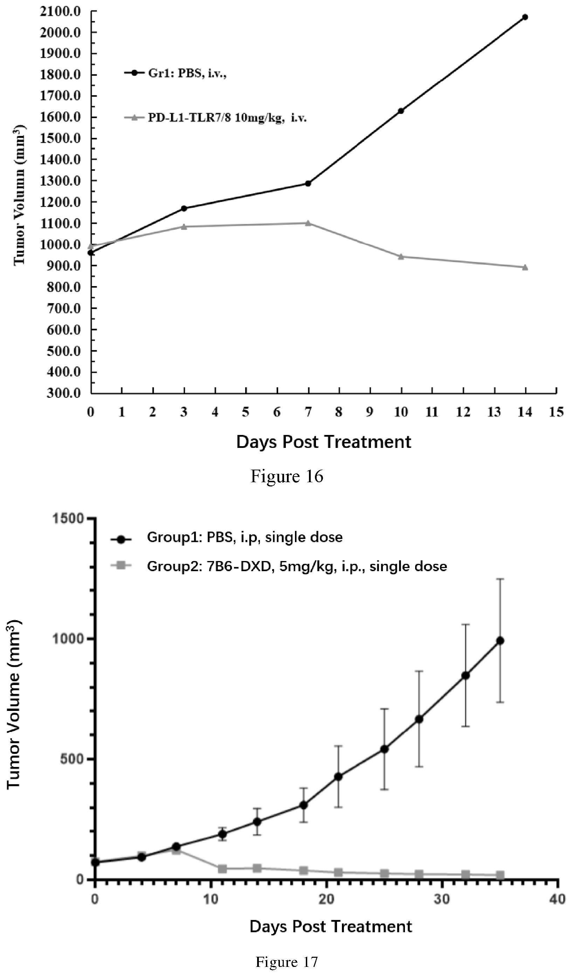

Grouping and result measurements are shown in Table 18 and Figure 16.

**Table 18**

| Group | Number of animals | Tumor volume on day 32 (mm³) | TGI (%) on day 14 |
|---|---|---|---|
| PBS | 3 | 2069.36 | 0 |
| PD-L1-TLR7/8, 10 mg/kg | 3 | 891.03 | 56.94 |

### Example 18: In Vivo Characterization of GCC TMEAbody in Mouse Tumor Models

Human colon cancer HT-55 cells were subcutaneously transplanted into NDG mice to establish an in vivo model. HT-55 cells were cultured according to culturing conditions, harvested during the exponential growth phase, and counted. Each mouse was inoculated on the right side with 1×10⁶ human colon cancer HT-55 cells (suspended in 0.1 mL of basal medium). After inoculation, when tumors grew to 56 to 86 mm³ (7 days post-inoculation), the mice were randomly grouped based on mouse weight and tumor volume. The experiment was divided into a PBS group and a 7B6-S16 (5 mg/kg) group.

Treatment started on the day of grouping. Mouse weight and tumor volume were measured twice a week. Tumor volume was calculated using the formula: tumor volume = 1/2 × length × width × width (mm³). Tumor growth inhibition TGI (%) = [1 - (Tt - T0) / (Ct - C0)] × 100%. Tumor inhibition rate T/C (%) = (Tt/T0) / (Ct/C0) × 100%. T0 is the tumor volume of the experimental group at grouping; Tt is the tumor volume of the experimental group at each measurement; C0 is the mean tumor volume of the PBS group at grouping; Ct is the mean tumor volume of the PBS group at each measurement.

The results are shown in Figure 17. 7B6-S16 exerted a prominent inhibitory effect on tumor growth in the subcutaneous HT-55 xenograft tumor model in mice. After 35 days of treatment, the inhibition rate TGI of the 7B6-S16 (5 mg/kg) group was 106%, and T/C was 2%. These results indicate that 7B6-S16 can be activated in the tumor microenvironment and stimulate anti-tumor immune responses

### Example 19

1. Animals: Nude mice, 6-8 weeks old, all female (Shanghai Lingchang Biotechnology Co., Ltd.).
2. Cells: The corresponding cells were purchased from American Type Culture Collection (ATCC) and authenticated according to the instructions provided by ATCC. The cells were cultured in Dulbecco's Modified Eagle Medium (DMEM) supplemented with 10% fetal bovine serum at 37°C under 5% CO₂. The cells were passaged every 3 days, and cells within 15 passages were used.
3. Tumor induction: 1.5×10⁶ HT1080 cells were subcutaneously injected into the back of nude mice. When the tumor volume reached approximately 100 mm³, the mice were randomly grouped, and treatment was initiated. The day treatment began was designated as day 1.
4. Treatment process
Dose (5 µmol/kg), administered once a week for a total of 3 weeks.
The grouping and result measurements are shown in Table 19.

**Table 19: Effects of Corresponding Compounds and Control Groups on Tumor Inhibition**

| Group | Number of animals | Tumor volume (mm³) | TGI % |
|---|---|---|---|
| | | Day 20 | Day 20 |
| Saline (blank group) | 6 | >1000 | 0 |
| Exatecan derivative (control group) | 6 | 420 | 58% |
| S26 | 6 | 45 | 95% |
| S41 | 6 | 34 | 97% |
| S43 | 6 | 45 | 96% |
| S57 | 6 | 35 | 97% |
| S58 | 6 | 40 | 96% |

5. Results and Discussion: The compounds S26, S41, S43, S49, S57, and S58 of the present disclosure showed greatly improved therapeutic effects compared to the exatecan derivative, nearly achieving complete tumor eradication.

### Example 20

1. Objective: To understand the acute toxicity of the drugs of the present disclosure in vivo by determining the MTD after intravenous administration in mice.
2. Test drugs: Exatecan derivative, and compounds S26, S41, S43, S49, S57, and S58 were dissolved in DMSO and diluted to the corresponding doses with physiological saline before testing.
3. Animals: Grade 1 BALB/C mice (purchased from Shanghai SLAC Laboratory Animal Co., Ltd.), weighing 19-21 g, all female.
4. Methods and Results: 48 test BALB/C mice, weighing 19-21 g, all female, were randomly divided into 7 groups according to body weight, with 6 mice in each group. The exatecan derivative, compounds S26, S41, S43, S49, S57, and S58 were respectively administered by single intravenous injection at the doses as shown in Table 20. A saline control group was also tested. The administration volume per mouse was 0.2 mL. The mice were continuously observed for 17 days. Daily observations were made for signs of piloerection, messy and dull hair, lethargy, hunched posture, hyperreactivity, etc. Body weight and mortality were recorded. Blood samples were collected on days 3, 5, and 14 for complete blood count. On day 14, the animals were dissected, and the heart, liver, kidney, lung, spleen, and pancreas were taken for HE staining and observation.

**Table 20: Comparison of mortality rates in test mice receiving different doses of compound injections and saline**

| Group | | Dose (µmol/kg) | Animals (No.) | Number of deaths (No.) |
|---|---|---|---|---|
| 1 | Saline | 0 | 6 | 0 |
| 2 | Exatecan derivative | 20 | 6 | 3 |
| 3 | S26 | 20 | 6 | 0 |
| 4 | S41 | 20 | 6 | 0 |
| 5 | S43 | 20 | 6 | 0 |
| 6 | S49 | 20 | 6 | 0 |
| 7 | S57 | 20 | 6 | 0 |
| 8 | S58 | 20 | 6 | 0 |

5. Results and Discussion: Compared to the exatecan derivative, when administering S26, S41, S43, S49, S57, and S58 of the present disclosure, no signs of piloerection, messy and dull hair, lethargy, hunched posture, hyperreactivity, or mortality were observed in the animals, indicating that the toxicity of the small molecule conjugated drugs was significantly lower than that of the unconjugated drugs.

### Example 21: Ring-opening method for S15 conjugated to albumin and antibodies

Conjugation process: 1.8 mL of an albumin solution (1.43 µmol/mL) was placed into a 5 mL PEEK tube, and 2 mL of S15 (2 µmol/mL) was added, mixed thoroughly. The mixture was allowed to stand at room temperature for 2 hours, and then dialyzed overnight against 10 mM NaH₂PO₄ buffer (pH 5.0) using a 10K dialysis cassette to obtain the albumin-conjugated compound.

Ring-opening process: After conjugation to albumin, the compound was incubated in 30-80 mM sodium bicarbonate buffer at pH 5.0 to 10.0 at 25-50°C for 24 to 60 hours. After incubation, a 3K ultrafiltration tube was used, and ring-opening was assessed by LC-MS high resolution mass spectrometry. The molecular weight of the compound after conjugation to albumin was 67702.78, and the molecular weight after ring-opening was 67721.57.

Plasma stability test procedure for compounds after conjugation and ring-opening process:
1. 20 µL of HSA-Compound and HSA-Compound-ring-opened samples (hereinafter HSA-Compound-OR) (0.5 µmol/mL) were added to 380 µL of human plasma, mixed thoroughly, and placed in a 37°C water bath.
2. At 0 h, 2 h, 4 h, 24 h, 48 h, and 96 h, 50 µL of the mixed solution was taken respectively, and 150 µL of protein precipitant (CH₃OH/DMSO=1:1) was added. The mixture was centrifuged at 12000 rpm for 5 min, and the supernatant was taken for HPLC injection.

The percentage of DXD released after the drug entered the lung tissue homogenate was calculated using the following formula: $Drug loading % = \frac{\left(Molar amount of HSA-Compound\right) - \left(Molar amount of newly generated DxD\right)}{Molar amount of HSA - Compound} * 100 %$

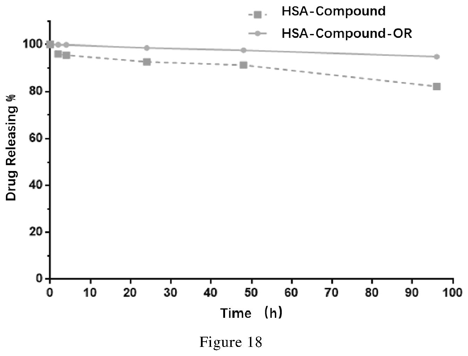

The results are shown in Figure 18.

### Example 22: Preparation of bispecific antibody-drug conjugates

The bispecific antibody conjugated drugs can simultaneously target two antigens. Their targeting modes are shown in two forms in Figure 19, wherein Anti-Antigen1 is selected from any one of EGFR, Trop2, and Cmet; and Anti-Antigen2 is selected from any one of EGFR, Trop2, and Cmet.

In the prepared bispecific antibody-drug conjugates, the heavy chain amino acid sequence of the bispecific antibody in EGFR/Cmet-T785 is shown in SEQ ID NO: 9, and the light chain amino acid sequence is shown in SEQ ID NO: 11; the heavy chain amino acid sequence of the bispecific antibody in EGFR/Cmet-Dxd is shown in SEQ ID NO: 10, and the light chain amino acid sequence is shown in SEQ ID NO: 12; the light chain amino acid sequence of the EGFR/Trop2 bispecific antibody is shown in SEQ ID NO: 13, and the heavy chain amino acid sequence is shown in SEQ ID NO: 14.

### 1. EGFR/Cmet-S5:

2.0×10⁶ NCI H292 cells expressing human HGF were mixed with Matrigel in PBS in a ration of 1:1 to a total volume of 100 µL, and subcutaneously inoculated into the right side of SCID Beige mice. Treatment began when the mean tumor size reached approximately 139 mm³. Dosing and animal numbers for each study group are shown in Table 21. The day of tumor cell inoculation was designated as day 0.

**Table 21**

| Group | Treatment | Dose (mg/kg) | Route of administration | Regimen |
|---|---|---|---|---|
| Gr1 | Vehicle | 0 | i.v. | BIW |
| Gr 2 | EGFR/Cmet-S5 | 10 | i.v. | BIW |

Before treatment, all animals were weighed and tumor volumes were measured. Due to the fact that tumor volume can affect the effectiveness of any given treatment, the mice were grouped using a randomized block design based on tumor volume. This ensured comparability of all groups on the baseline. The randomized block design was used to group the experimental animals. First, the experimental animals were divided into uniform blocks based on their initial tumor volume. Second, within each block, the experimental animals were randomized. A randomized block design was adopted for experimental animal allocation to ensure an equal probability of each animal being assigned to a specific treatment, thereby reducing systematic errors.

During routine monitoring, any effects of the animals' tumor growth and treatment on normal behavior were examined, such as mobility, visual estimation of food and water consumption, body weight gain/loss (measured twice a week), eye/hair matting, and any other abnormal effects.

The study endpoints were whether tumor growth could be delayed or whether tumor-bearing mice could be cured. Tumor size was measured twice a week using a caliper in two dimensions, and volume was expressed in mm³ using the formula: V = 0.5 × a × b², wherein a and b are the long and short diameters, respectively.

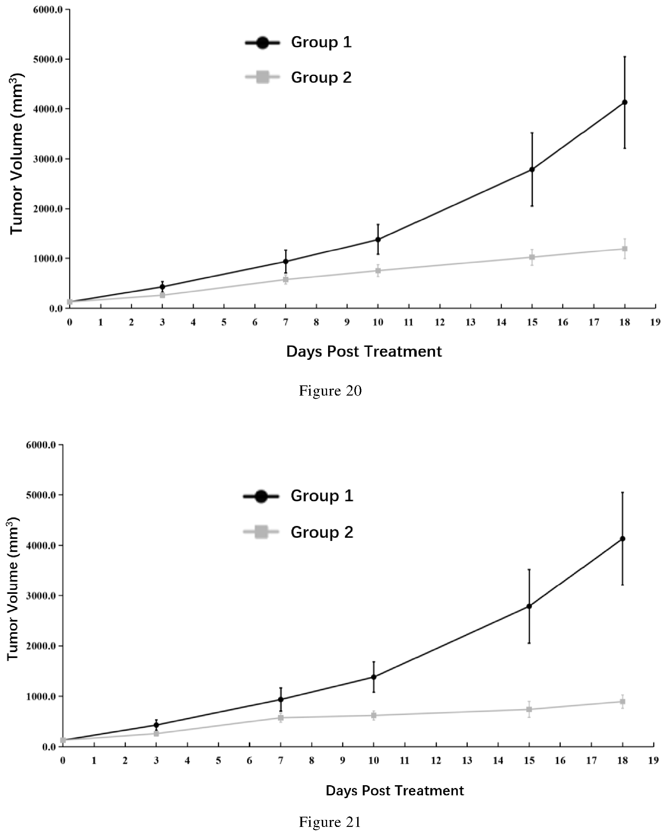

The results are shown in Figure 20. The results showed that on day 18, the TGI of the drug EGFR/Cmet-T785 in the NCI-H292 tumor model was 76.9%, indicating that EGFR/Cmet-S5 could significantly slow the tumor growth rate and thereby effectively prolong the survival of the mice. Therefore, the drug EGFR/Cmet-S5 showed good tumor inhibition efficacy in the NCI-H292 tumor model.

### 2. EGFR/Cmet-S16:

2.0×10⁶ NCI H292 cells expressing human HGF were mixed with Matrigel in PBS in a ratio of 1:1 to a total volume of 100 µL, and subcutaneously inoculated into the right side of SCID Beige mice. Treatment began when the mean tumor size reached approximately 139 mm³. Dosing and animal numbers for each study group are shown in Table 22. The day of tumor cell inoculation was designated as day 0.

**Table 22**

| Group | Treatment | Dose (mg/kg) | Route of administration | Regimen |
|---|---|---|---|---|
| Gr1 | Vehicle | 0 | i.v. | BIW |
| Gr2 | EGFR/Cmet-S 16 | 5 | i.v. | BIW |

Before treatment, all animals were weighed and tumor volumes were measured. Due to the fact that tumor volume can affect the effectiveness of any given treatment, the mice were grouped using a randomized block design based on tumor volume. This ensured comparability of all groups on the baseline. The randomized block design was used to group the experimental animals. First, the experimental animals were divided into uniform blocks based on their initial tumor volume. Second, within each block, the experimental animals were randomized. A randomized block design was adopted for experimental animal allocation to ensure an equal probability of each animal being assigned to a specific treatment, thereby reducing systematic errors.

During routine monitoring, any effects of the animals' tumor growth and treatment on normal behavior were examined, such as mobility, visual estimation of food and water consumption, body weight gain/loss (measured twice a week), eye/hair matting, and any other abnormal effects.

The study endpoints were whether tumor growth could be delayed or whether tumor-bearing mice could be cured. Tumor size was measured twice a week using a caliper in two dimensions, and volume was expressed in mm³ using the formula: V = 0.5 × a × b², wherein a and b are the long and short diameters, respectively.

The results are shown in Figure 21. The results showed that on day 18, TGI of the drug EGFR/Cmet-S16 in the NCI H292 tumor model was 87.2%, and EGFR/Cmet-S16 completely suppressed tumor growth from day 7 to day 18. Therefore, the drug EGFR/Cmet-S16 showed good tumor inhibition efficacy in the NCI H292 tumor model.

### 3. EGFR/Trop2-S5:

2.0×10⁶ NCI-H292 (TROP2^{high}EGFR^{high}) cells were mixed with Matrigel in PBS in a ratio of 1:1 to a total volume of 100 µL, and subcutaneously inoculated into the right side of SCID Beige mice. Treatment began when the mean tumor size reached approximately 100 mm³. Dosing and animal numbers for each study group are shown in Table 23. The day of tumor cell inoculation was designated as day 0.

**Table 23**

| Group | Treatment | Dose (mg/kg) | Route of administration | Regimen |
|---|---|---|---|---|
| 1 | Vehicle | 0 | i.v. | BIW |
| 2 | EGFR/Trop2 | 10 | i.v. | BIW |
| 3 | EGFR/Trop2-S5 | 10 | i.v. | BIW |

Before treatment, all animals were weighed and tumor volumes were measured. Due to the fact that tumor volume can affect the effectiveness of any given treatment, the mice were grouped using a randomized block design based on tumor volume. This ensured comparability of all groups on the baseline. The randomized block design was used to group the experimental animals. First, the experimental animals were divided into uniform blocks based on their initial tumor volume. Second, within each block, the experimental animals were randomized. A randomized block design was adopted for experimental animal allocation to ensure an equal probability of each animal being assigned to a specific treatment, thereby reducing systematic errors.

During routine monitoring, any effects of the animals' tumor growth and treatment on normal behavior were examined, such as mobility, visual estimation of food and water consumption, body weight gain/loss (measured twice a week), eye/hair matting, and any other abnormal effects.

The study endpoints were whether tumor growth could be delayed or whether tumor-bearing mice could be cured. Tumor size was measured twice a week using a caliper in two dimensions, and volume was expressed in mm³ using the formula: V = 0.5 × a × b², wherein a and b are the long and short diameters, respectively.

The results are shown in Table 24.

**Table 24**

| Group | Treatment | Average tumor volume on day | TGI on day 26 |
|---|---|---|---|
| | | 26 | |
| 1 | Vehicle | 3284mm³ | |
| 2 | EGFR/Trop2 | 1928mm³ | 42.6% |
| 3 | EGFR/Trop2-S5 | 435mm³ | 89.5% |

### 4. EGFR/Trop2-S16:

2.0×10⁶ NCI-H292 (TROP2^{high}EGFR^{high}) cells were mixed with Matrigel in PBS in a ratio of 1:1 to a total volume of 100 µL, and subcutaneously inoculated into the right side of SCID Beige mice. Treatment began when the mean tumor size reached approximately 100 mm³. Dosing and animal numbers for each study group are shown in Table 25. The day of tumor cell inoculation was designated as day 0.

**Table 25**

| Group | Treatment | Dose (mg/kg) | Route of administration | Regimen |
|---|---|---|---|---|
| 1 | Vehicle | 0 | i.v. | BIW |
| 2 | EGFR/Trop2 | 10 | i.v. | BIW |
| 3 | EGFR/Trop2-S16 | 10 | i.v. | BIW |

Before treatment, all animals were weighed and tumor volumes were measured. Due to the fact that tumor volume can affect the effectiveness of any given treatment, the mice were grouped using a randomized block design based on tumor volume. This ensured comparability of all groups on the baseline. The randomized block design was used to group the experimental animals. First, the experimental animals were divided into uniform blocks based on their initial tumor volume. Second, within each block, the experimental animals were randomized. A randomized block design was adopted for experimental animal allocation to ensure an equal probability of each animal being assigned to a specific treatment, thereby reducing systematic errors.

During routine monitoring, any effects of the animals' tumor growth and treatment on normal behavior were examined, such as mobility, visual estimation of food and water consumption, body weight gain/loss (measured twice a week), eye/hair matting, and any other abnormal effects.

The study endpoints were whether tumor growth could be delayed or whether tumor-bearing mice could be cured. Tumor size was measured twice a week using a caliper in two dimensions, and volume was expressed in mm³ using the formula: V = 0.5 × a × b², wherein a and b are the long and short diameters, respectively.

The results are shown in Table 26.

**Table 26**

| Group | Treatment | Average tumor volume on day 35 | TGI on day 35 |
|---|---|---|---|
| 1 | Vehicle | 3639mm³ | |
| 2 | EGFR/Trop2 | 2378mm³ | 35.6% |
| 3 | EGFR/Trop2-S 16 | 235mm³ | 96.2% |

From the above experiments, it was found that small molecule drugs or protein conjugated drugs containing the AAN sequence in the linker could release the payload more efficiently, thereby improving therapeutic efficacy.

### Example 23: Pharmacodynamics and safety evaluation of S41 (QHL 1618) against human fibrosarcoma cells HT-1080 on a subcutaneous tumor model in BALB/c nude mice

In this study, 32 female Balb/c nude mice were subcutaneously inoculated with human fibrosarcoma cells HT 1080 to establish a transplanted tumor model. When the mean volume of the transplanted tumor reached 100 mm³, the mice were randomly divided into 4 groups, with 8 mice in each group. The 4 groups received Vehicle, CPT-780 (positive control, S41 payload Dxd) at 2.47 mg/kg or 5 µmol/kg, S41 at 7.12 mg/kg or 5 µmol/kg, or S41 at 14.25 mg/kg or 10 µmol/kg, respectively. All doses were administered by tail vein injection at a volume of 10 mL/kg, once a week for 3 weeks.

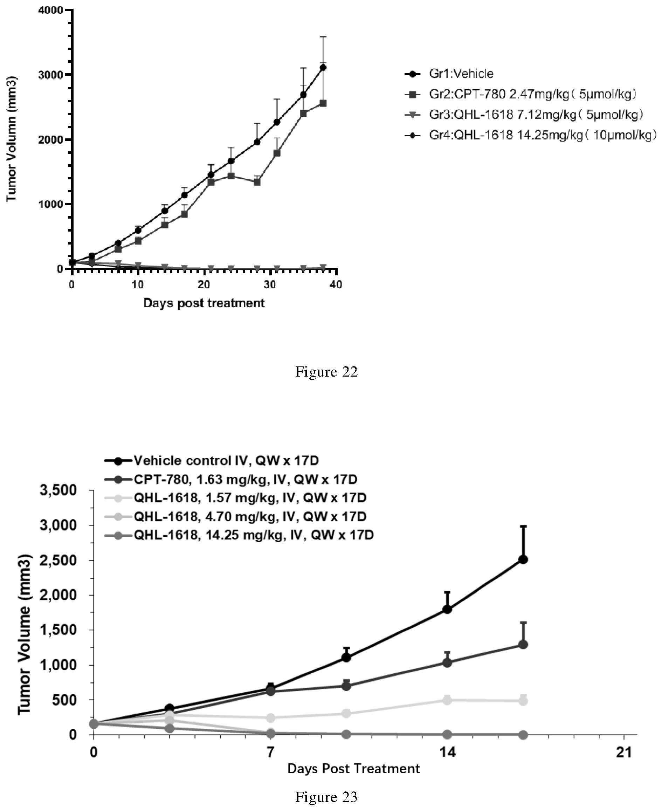

The results are shown in Figure 22. S41 exhibited a prominent inhibitory effect on the growth of transplanted tumor of HT-1080 tumor cells. On Day 38 of the study, S41 at 10 µmol/kg was identified as an effective dose, with no obvious drug-related toxicity observed in mice.

### Example 24: Pharmacodynamics study in vivo of test compounds against human fibrosarcoma cells HT-1080 on a subcutaneous tumor model in BALB/c nude mice

In this study, 30 female Balb/c mice were subcutaneously inoculated with human fibrosarcoma HT-1080 cells to establish a transplanted tumor model. When the mean volume of the transplanted tumor reached 161 mm³, the mice were randomly divided into 5 groups, with 6 mice in each group. The 5 groups received Vehicle, CPT-780 (positive control, S41 payload Dxd) at 1.63 mg/kg or 3.3 µmol/kg, S41 at 1.57mg/kg or 1.1 µmol/kg, S41 at 4.70mg/kg or 3.3 µmol/kg, or S41 at 14.25 mg/kg or 10 µmol/kg, respectively. All doses were administered by tail vein injection at a volume of 10 µl/g, once a week for 17 days.

The results are shown in Figure 23. The results showed that S41 exhibited a prominent inhibitory effect on the growth of transplanted tumor of HT-1080 tumor cells. On day 17 of the test, tumor regression was observed in all mice (N=6) in the treatment groups of S41 4.70 mg/kg and 14.25 mg/kg, with the proportions of mice showing complete tumor regression to clearance being 80% in each of the two treatment groups, respectively.

### Example 25: Pharmacodynamics and safety evaluation of S41 against mouse colon cancer cells CT-26 on a subcutaneous tumor model in BALB/c mice

In this study, 30 female Balb/c mice were subcutaneously inoculated with mouse colon cancer cells CT-26 to establish a transplanted tumor model. When the mean volume of the transplanted tumor reached 127 mm³, the mice were randomly divided into 5 groups, with 6 mice in each group. The 5 groups received Vehicle, CPT-780 (positive control, S41 payload Dxd) at 3 mg/kg or 6 µmol/kg, S41 at 3 mg/kg or 6 µmol/kg, or S41 at 9 mg/kg or 18 µmol/kg, respectively. All doses were administered by tail vein injection at a volume of 10 mL/kg, once a week for 4 weeks.

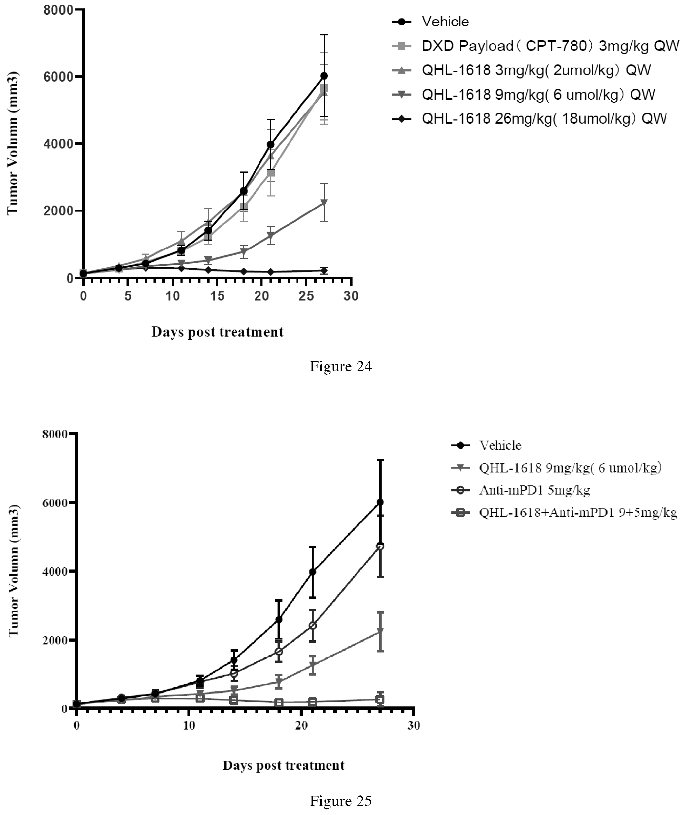

The results are shown in Figure 24. The results showed that S41 exhibited a prominent inhibitory effect on the growth of transplanted tumor of CT-26 tumor cells. On day 27 of the test, S41 at 18 µmol/kg was identified as an effective dose, with no obvious drug-related toxicity observed in mice.

### Example 26: Pharmacodynamics and safety evaluation of combined treatment with S41 and anti-mPD-1 against mouse colon cancer cells CT-26 on a subcutaneous tumor model in BALB/c mice

In this study, 24 female Balb/c mice were subcutaneously inoculated with mouse colon cancer cells CT-26 to establish a transplanted tumor model. When the mean volume of the transplanted tumor reached 127 mm³, the mice were randomly divided into 4 groups, with 6 mice in each group. The 4 groups received Vehicle, S41 at 9 mg/kg or 18 µmol/kg, anti-mPD-1 antibody at 5 mg/kg, or the combination of S41 at 9 mg/kg with anti-mPD-1 antibody at 5 mg/kg, respectively. All doses were administered by tail vein injection at a volume of 10 ml/kg. S41 was administered once a week, and anti-mPD-1 antibody was administered twice a week, for 4 weeks.

The results are shown in Figure 25. The results demonstrat that S41 and anti-mPD-1 exhibited synergistic inhibition effect on the growth of transplanted tumor of CT-26 tumor cells.

### Example 27: Pharmacodynamics and safety evaluation of S41 against mouse colon cancer cells CT-26 on a subcutaneous tumor model in BALB/c mice

In this study, 24 female Balb/c mice were subcutaneously inoculated with mouse colon cancer cells CT-26 to establish a transplanted tumor model. When the mean volume of the transplanted tumor reached 150-200 mm³, the mice were randomly divided into 4 groups, with 6 mice in each group. The 4 groups received Vehicle, T-DXD analog (positive control) at 10 mg/kg, S41 at 16 mg/kg, or S41 at 24 mg/kg (HSNTD dose), respectively. All doses were administered by tail vein injection at a volume of 10 ml/kg, once a week for 2 weeks.

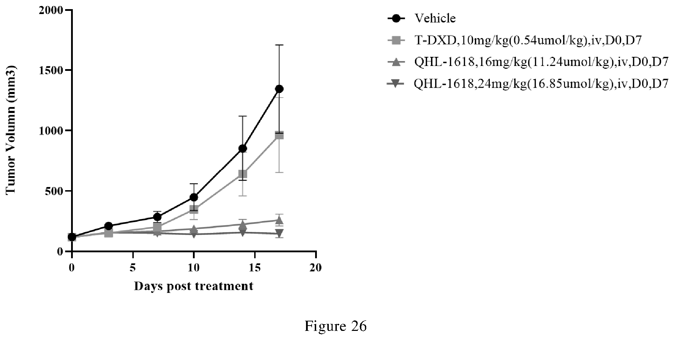

The results are shown in Figure 26. The results showed that S41 exhibited a prominent inhibitory effect on the growth of transplanted tumor of CT-26 tumor cells. Based on the rat toxicity study results, S41 at 16 mg/kg showed similar toxicity to T-DXD at 10 mg/kg, and at the high dose, the efficacy of S41 was significantly superior to that of T-DXD.

### Example 28: Toxicity study of S41 administered intravenously to SD rats in a 4-week repeated dosing regimen followed by a 4-week recovery period

In this study, 160 rats were randomly divided into 4 groups, with 40 rats per group, half male and half female. Each group comprised 30 rats as the main experimental group and 10 rats as the TK group. The groups received saline or S41 at 3, 6, or 12 mg/kg, respectively, once a week for 4 consecutive weeks, followed by a 4-week recovery period without treatment. The following examinations were performed during the study: general observation, body weight, food consumption, clinical pathology (including hematology, blood biochemistry, and coagulation), body temperature, electrocardiogram, ophthalmological examination, urinalysis, gross anatomy, organ weights, bone marrow smear, histopathology examination, and toxicokinetics. The results showed that the HNSTD dose was 12 mg/kg.

### Example 29: Toxicity study of S41 administered intravenously to Beagle dogs in a 4-week repeated dosing regimen followed by a 4-week recovery period

In this study, 40 Beagle dogs were randomly divided into 4 groups, with 10 dogs per group, half male and half female. The groups received saline or S41 at 1, 2.5, or 5 mg/kg, respectively, once a week for 4 consecutive weeks, followed by a 4-week recovery period without treatment. The following examinations were performed during the study: general observation, body weight, food consumption, clinical pathology (including hematology, blood biochemistry, and coagulation), body temperature, electrocardiogram, ophthalmological examination, urinalysis, gross anatomy, organ weights, bone marrow smear, histopathology examination, and toxicokinetics. The results showed that the HNSTD dose was 2.5 mg/kg.

In summary, the antibody conjugated drugs provided by the present disclosure can targetedly recognize and accumulate around tumor cells, and can only be activated on the tumor cell surface, thereby efficiently killing tumors while reducing toxicity to a certain extent. Therefore, they have excellent application prospects.

## Claims

1. A drug conjugate, a stereoisomer thereof, or a pharmaceutically acceptable salt thereof, wherein the drug conjugate has a chemical structure represented by formula (I) or (II):
R₁-R₂-D (I)
R₂'-D (II)
wherein:
R₁ is a chemical structure for conjugation to a biomolecule, or R₁ is absent;
R₂ and R₂' are each independently an adjustment chemical structure for enhancing structure-activity relationship;
D is selected from the structures shown in X1-X5 below: 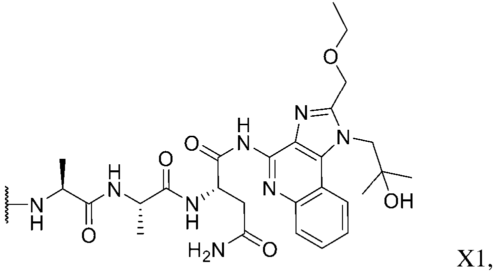 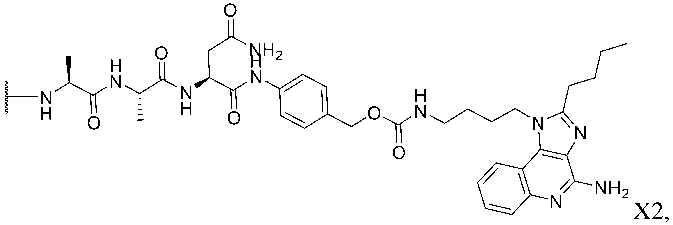 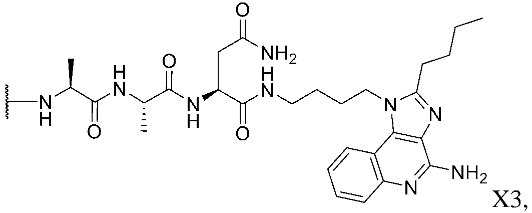 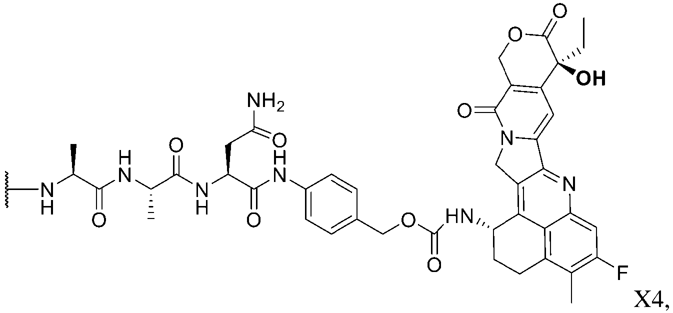 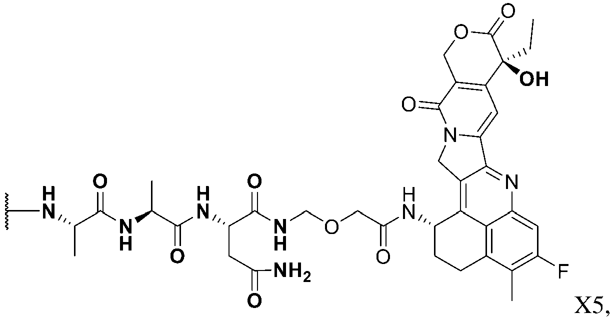 wherein the wavy line indicates the position of attachment of D to R₂ or R₂'.

2. The drug conjugate of claim 1, a stereoisomer thereof, or a pharmaceutically acceptable salt thereof, wherein R₁ is absent, or is selected from: 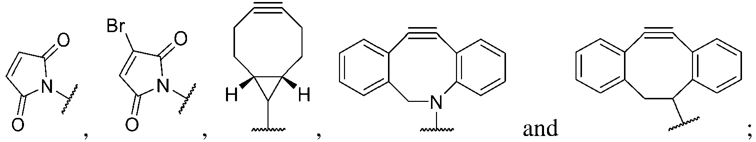
preferably, R₁ is selected from: 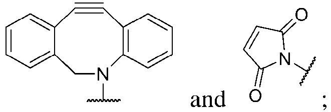
wherein the wavy line indicates the position of attachment of R₁ to R₂.

3. The drug conjugate of claim 1, a stereoisomer thereof, or a pharmaceutically acceptable salt thereof, wherein R₂ is selected from:
-C₁₋₆ alkylene-CO-*;
-C₁₋₆ alkylene-O-*;
-C₁₋₆ alkylene-OCONH-C₁₋₆ alkylene-(O-CH₂CH₂-O)ₙ-C₁₋₆ alkylene-CO-*;
-C₁₋₆ alkylene-OCONH-C₁₋₆ alkylene-(O-CH₂CH₂-O)ₙ-C₁₋₆ alkylene-O-*;
-C₁₋₆ alkylene-OCONH-C₁₋₆ alkylene-(O-CH₂CH₂-O)ₙ-C₁₋₆ alkylene-NH-*;
-(O-CH₂CH₂-O)ₙ-C₁₋₆ alkylene-CO-*;
-C₁₋₆ alkylene-CONH-C₁₋₆ alkylene-(O-CH₂CH₂-O)ₙ-C₁₋₆ alkylene-CO-*;
-NH-C₁₋₆ alkylene-(O-CH₂CH₂-O)ₙ-C₁₋₆ alkylene-CO-*;
-NH-C₁₋₆ alkylene-(O-CH₂CH₂-O)ₙ-C₁₋₆ alkylene-NH-*;
-NH-C₁₋₆ alkylene-(O-CH₂CH₂-O)ₙ-C₁₋₆ alkylene-O-*;
-NH-C₁₋₅₅ alkylene-CO-*;
-NH-C₁₋₅₅ alkylene-NH-*;
-NH-C₁₋₅₅ alkylene-O-*;
-CO-C₁₋₅₅ alkylene-CO-*;
-CO-C₁₋₆ alkylene-CONH-C₁₋₆ alkylene-(O-CH₂CH₂-O)ₙ-C₁₋₆ alkylene-CO-*;
-CO-C₁₋₆ alkylene-CONH-C₁₋₆ alkylene-(O-CH₂CH₂-O)ₙ-C₁₋₆ alkylene-NH-*;
-CO-C₁₋₆ alkylene-CONH-C₁₋₆ alkylene-(O-CH₂CH₂-O)ₙ-C₁₋₆ alkylene-O-*;
-CO-C₁₋₆ alkylene-NH-*;
-CO-C₁₋₆ alkylene-NHCO-C₁₋₆ alkylene substituted with-SO₃H-NH-*; 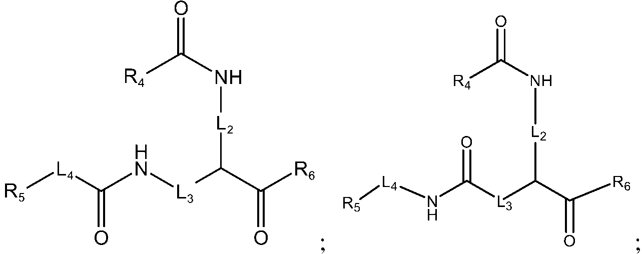 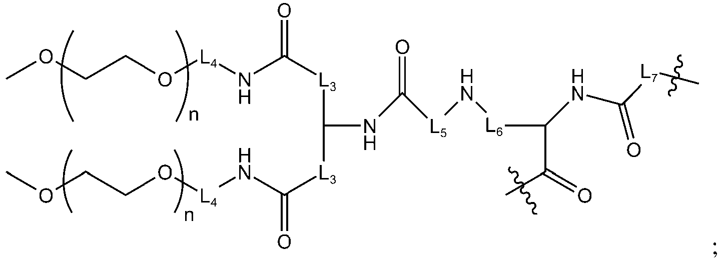 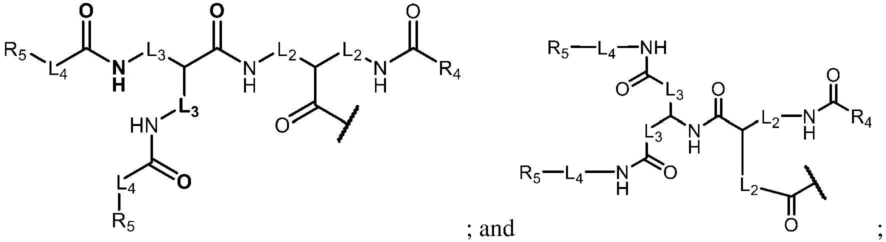
wherein:
* indicates the position of attachment to D;
R₄ is a monovalent or divalent group, R₄ is a divalent group when it is connected to other parts of the compound, and R₄ is selected from: C₁₋₆ alkyl or C₁₋₆ alkylene; C₁₋₆ alkyl-C₃₋₈ cycloalkylene- or C₁₋₆ alkylene-C₃₋₈ cycloalkylene-; -CO-C₁₋₆ alkylene-; C₁₋₆ alkyl-(O-CH₂CH₂-O)ₘ-C₁₋₆ alkylene- or C₁₋₆ alkylene-(O-CH₂CH₂-O)ₘ-C₁₋₆ alkylene-; -NH-C₁₋₆ alkylene; C₁₋₆ alkylene-CONH-C₁₋₆ alkylene-(O-CH₂CH₂-O)ₘ-C₁₋₆ alkylene-; and -NH-C₁₋₆ alkylene-C₃₋₈ cycloalkylene;
R₅ is a monovalent or divalent group, R₅ is a divalent group when it is connected to other parts of the compound, and R₅ is selected from: C₁₋₆ alkyl-(O-CH₂CH₂-O)ₘ-; C₁₋₆ alkylene-CONH-C₁₋₆ alkylene-(O-CH₂CH₂-O)ₘ-; -CO-C₁₋₆ alkylene-(O-CH₂CH₂-O)ₙ-;
one of R₄ and R₅ is a divalent group and the other is a monovalent group;
R₆ is absent, or is -NH-C₁₋₆ alkylene-triazolyl-C₁₋₆ alkylenecarbonyl or -NH-C₁₋₆ alkylene-triazolyl-C₁₋₆ alkylene-(O-CH₂CH₂-O)ₙ-C₁₋₆ alkylene-NHCO-C₁₋₆ alkyleneoxy-C₁₋₆ alkylenecarbonyl;
L₂ and L₃ are each independently a bond or C₁₋₆ alkylene;
L₄ is each independently C₁₋₆ alkylene;
L₅ is a bond, C₁₋₆ alkyleneoxy-C₁₋₆ alkylenecarbonyl, or C₁₋₆ alkylenecarbonyl;
L₆ is C₁₋₆ alkylene;
L₇ is C₁₋₆ alkylene or -C₃₋₈ cycloalkylene-C₁₋₆ alkylene;
each n and m is independently an integer from 1 to 50;
preferably, R₂ is selected from: 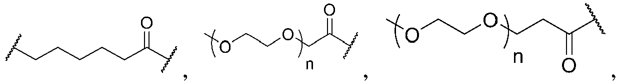 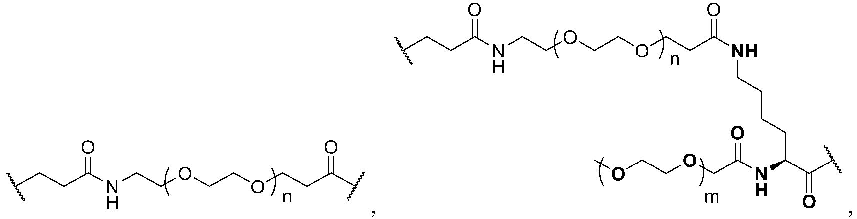 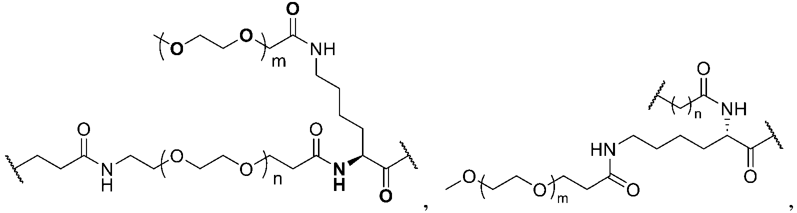 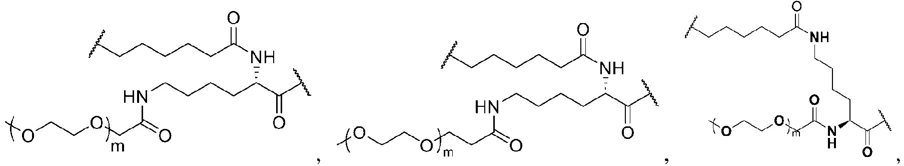 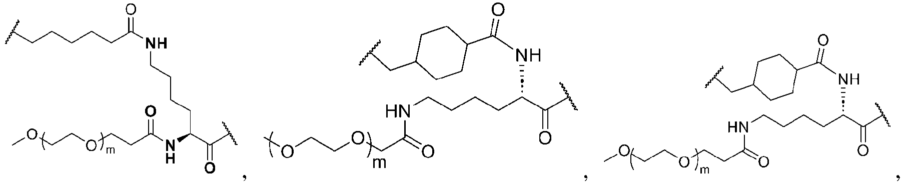 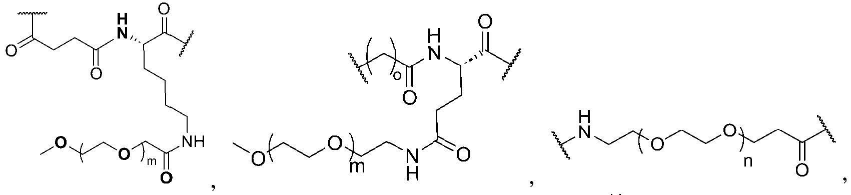 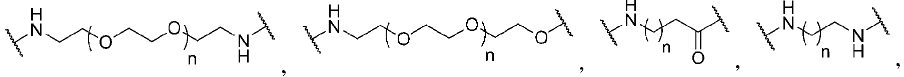 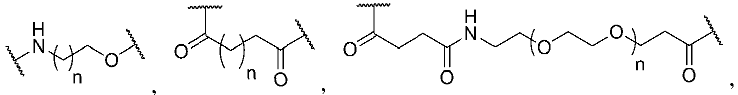 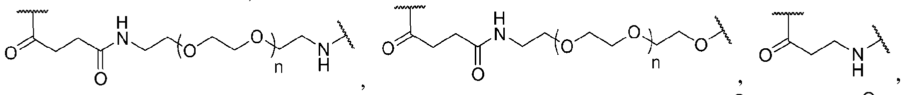 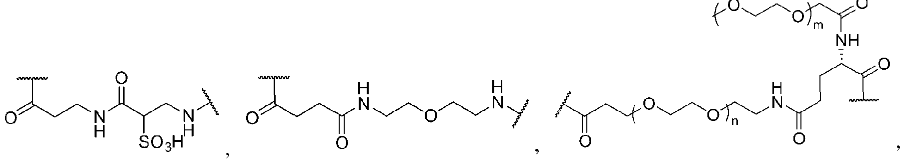 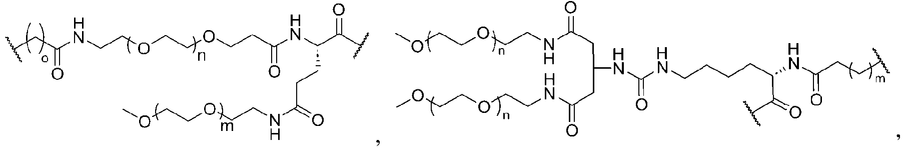 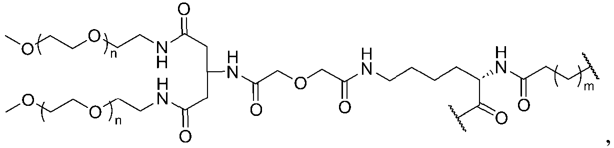 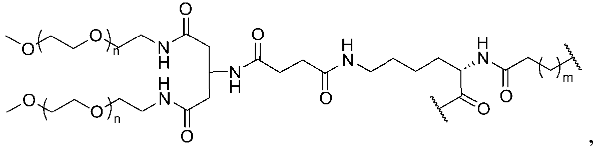 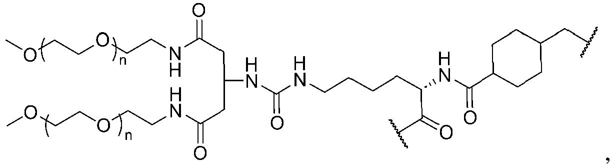 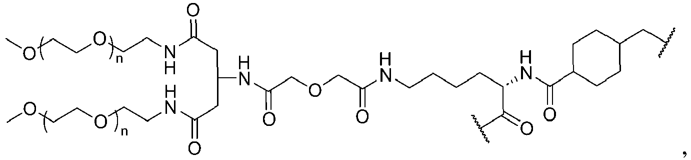 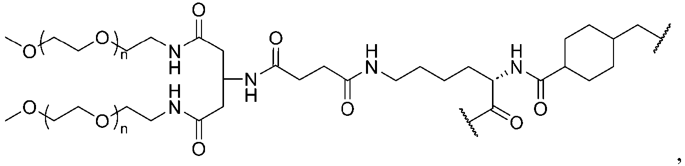 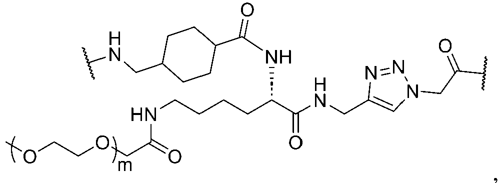 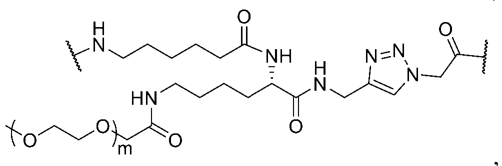 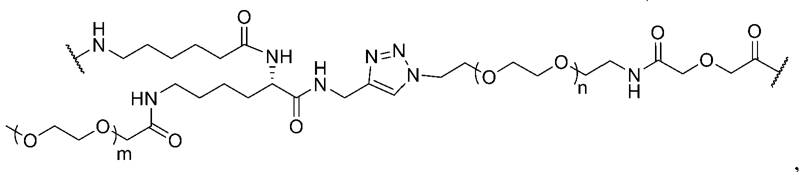 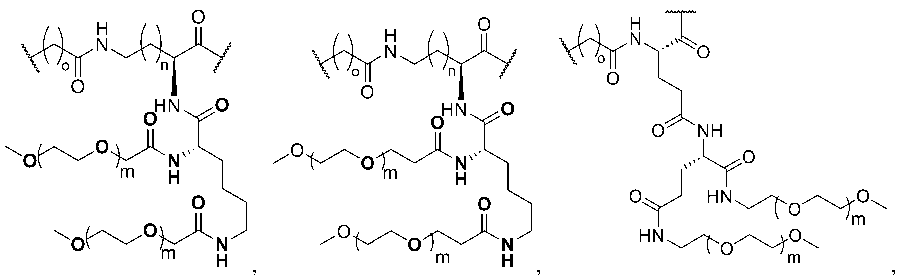 and 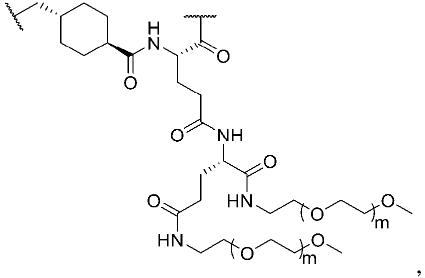 wherein o is independently an integer from 1 to 20, and m and n are each independently an integer from 1 to 50;
preferably, R₂ is selected from: 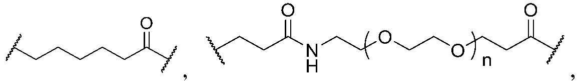 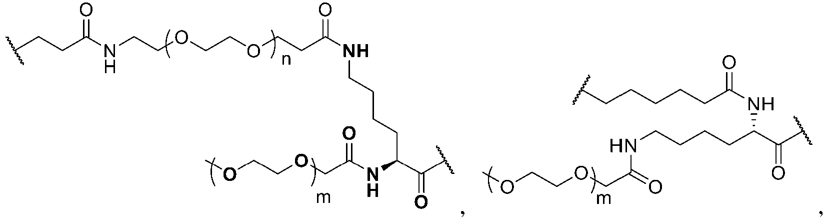 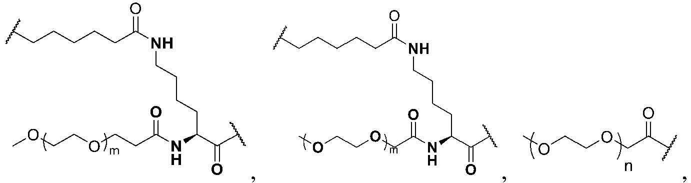 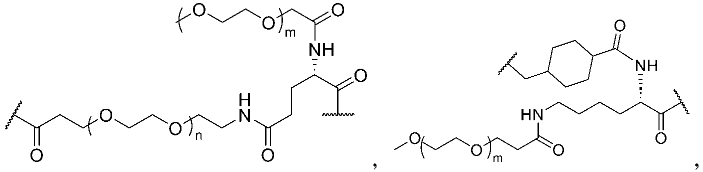 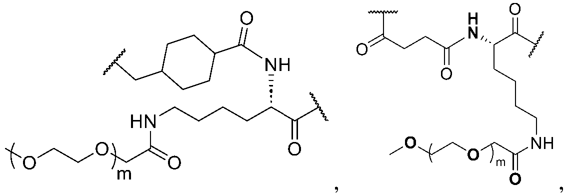 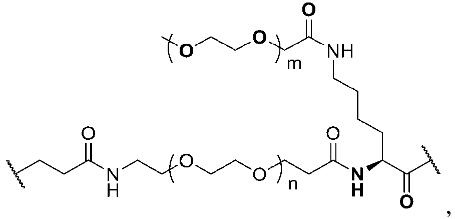 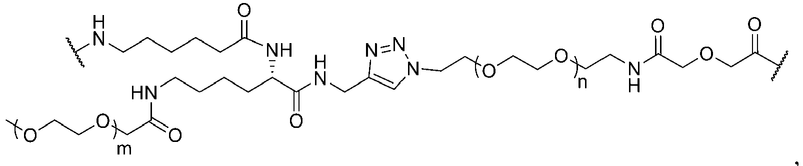 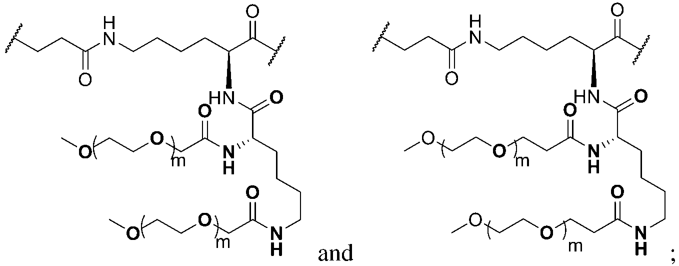 preferably, n and m are each independently an integer from 2 to 20.

4. The drug conjugate of claim 1, a stereoisomer thereof, or a pharmaceutically acceptable salt thereof, wherein the drug conjugate of formula (I) is selected from: 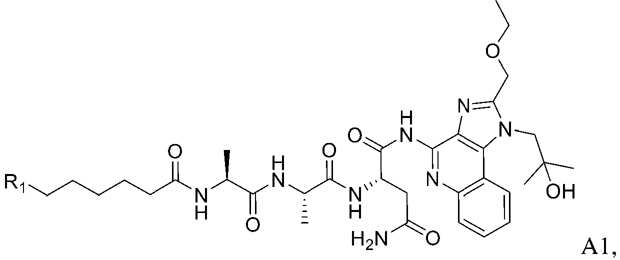 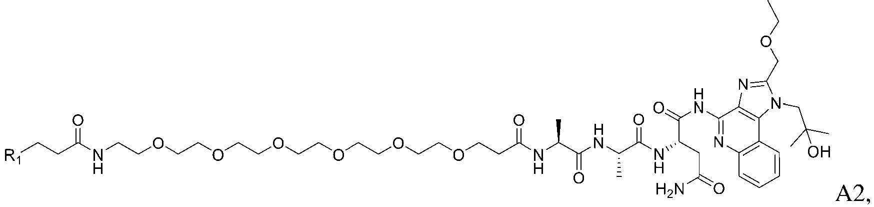 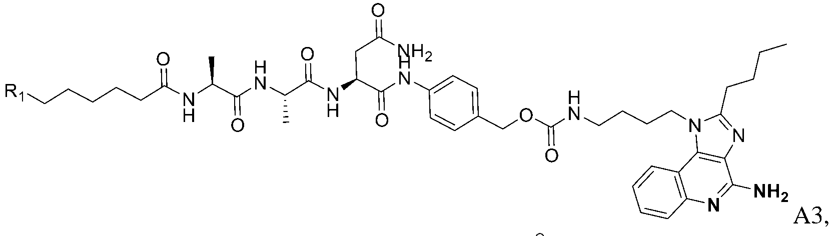 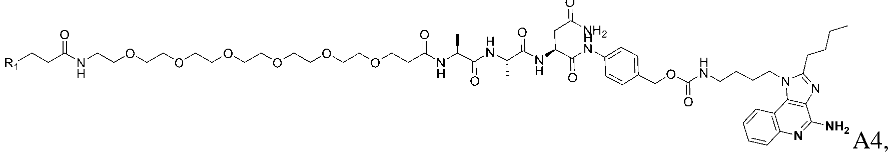 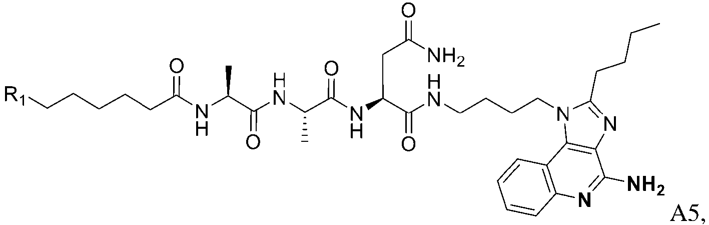 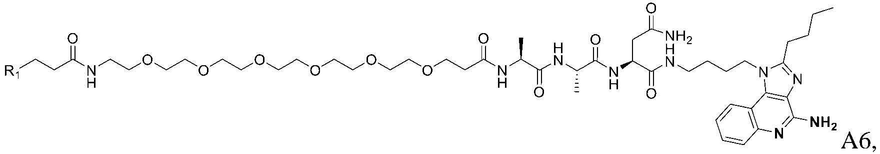 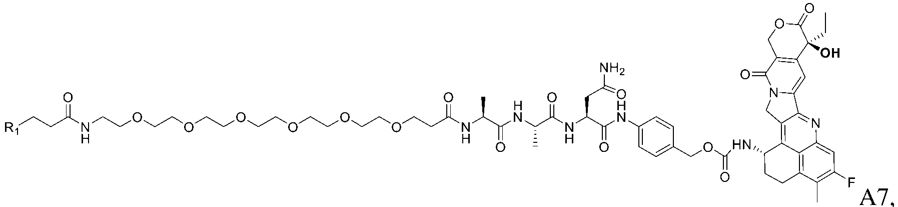 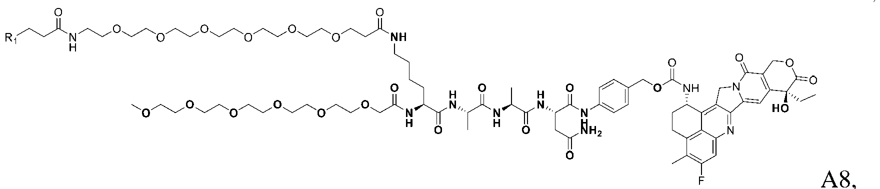 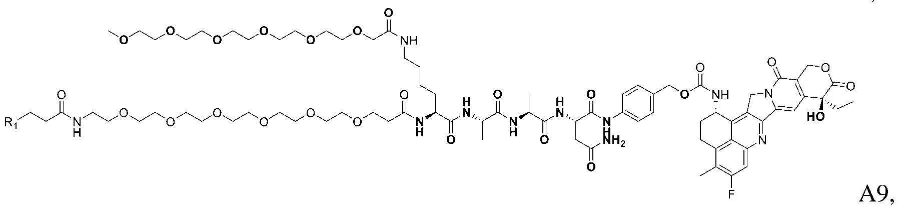 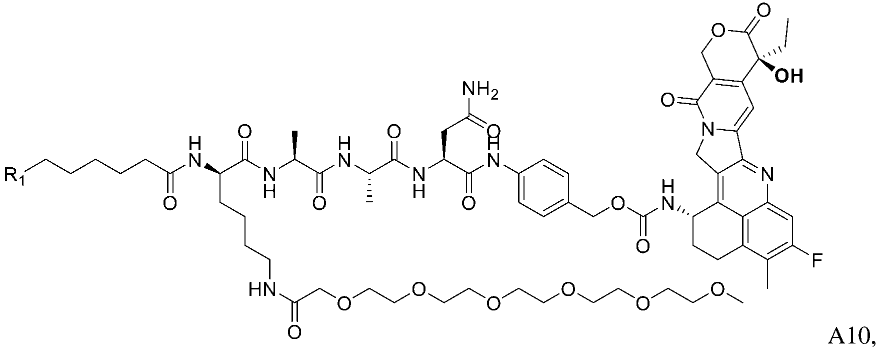 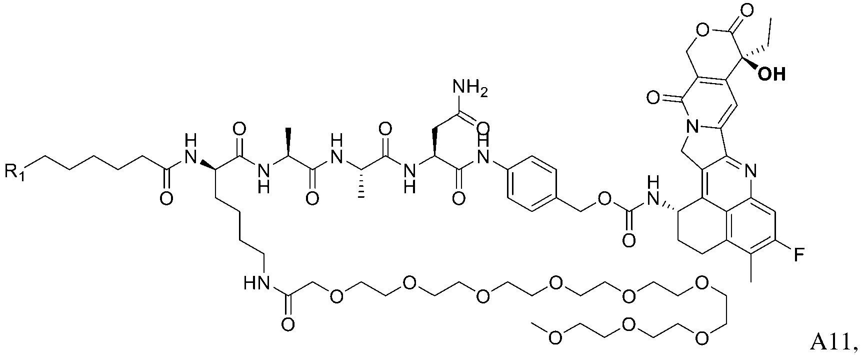 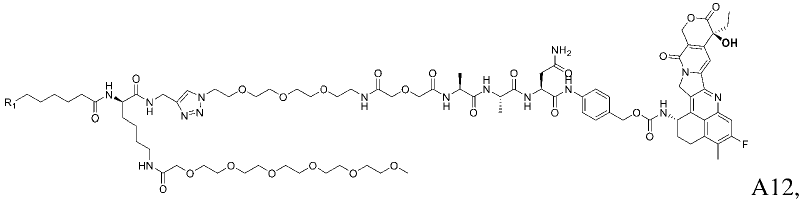 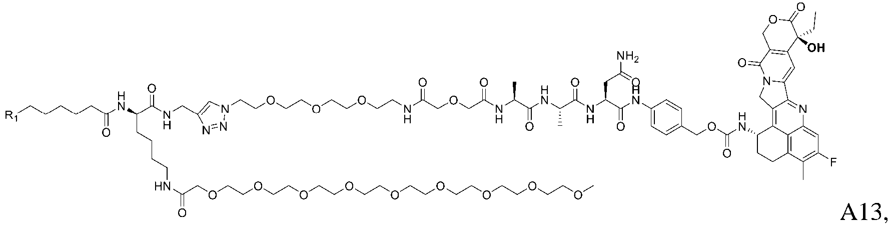 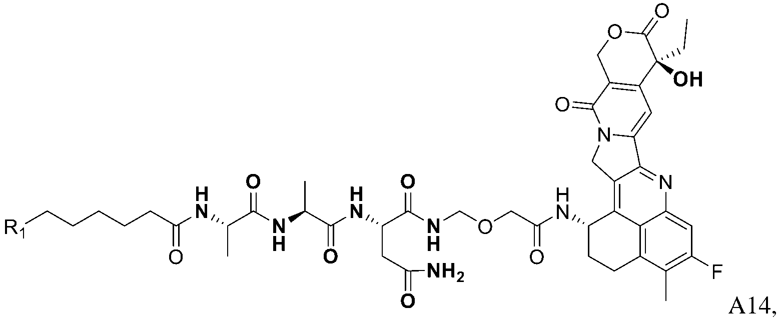 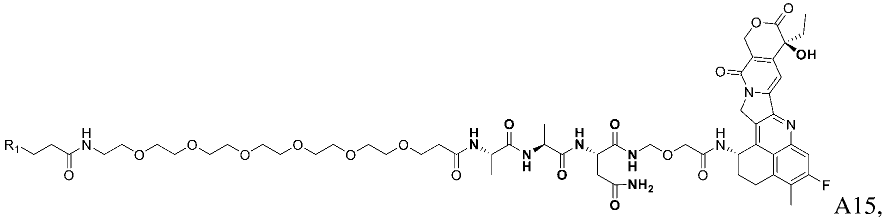 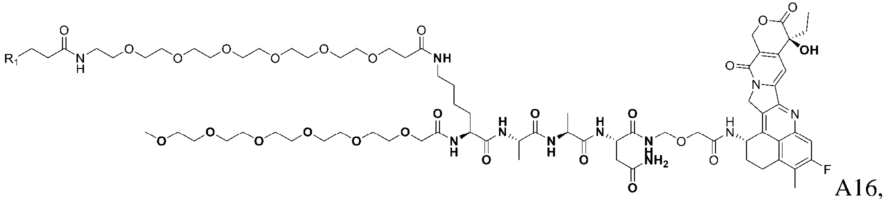 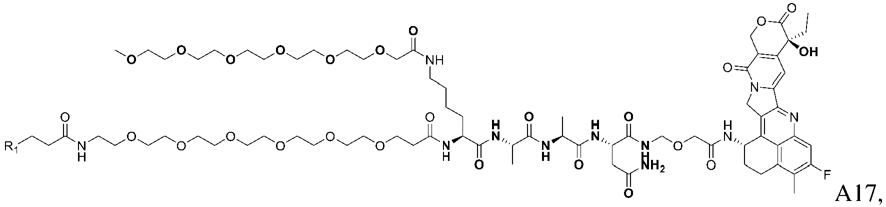 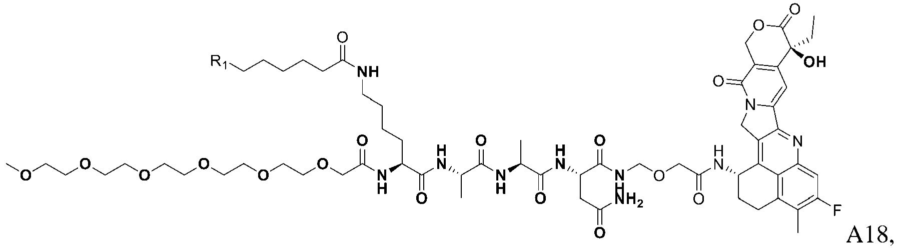 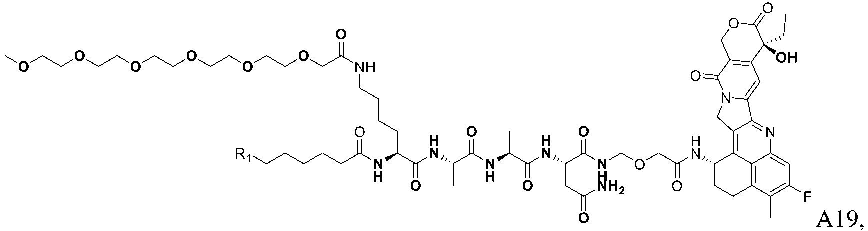 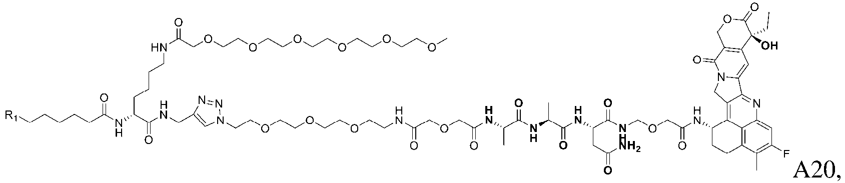 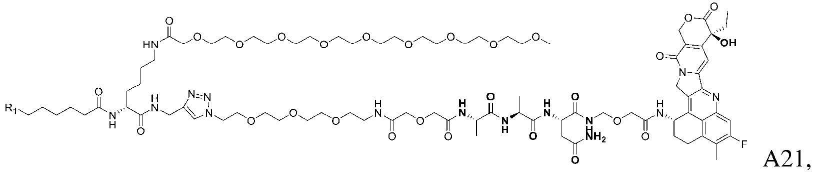 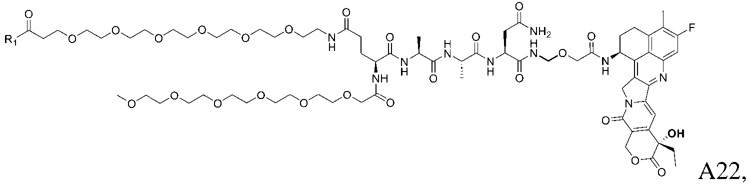 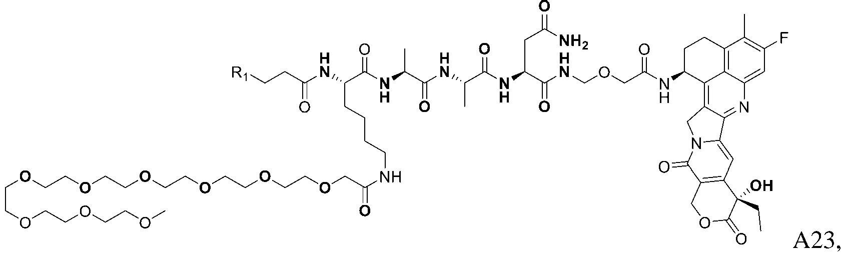 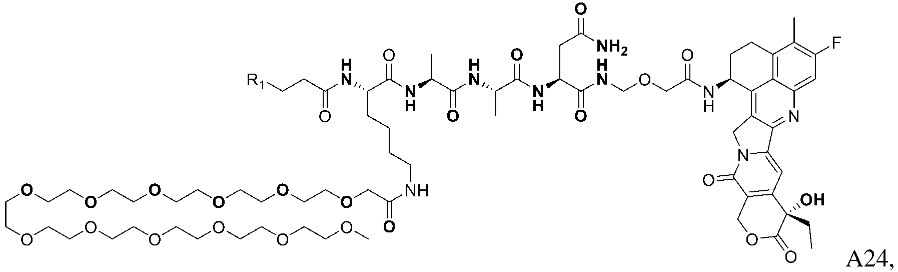 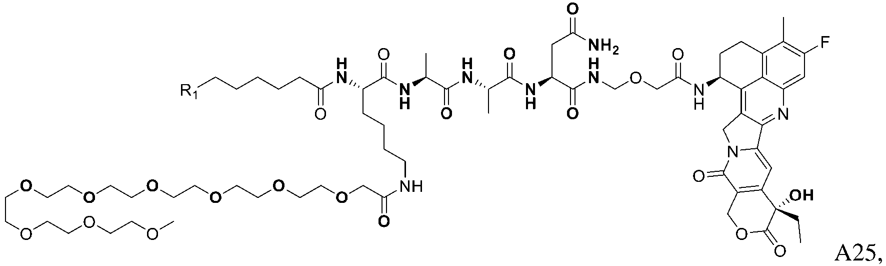 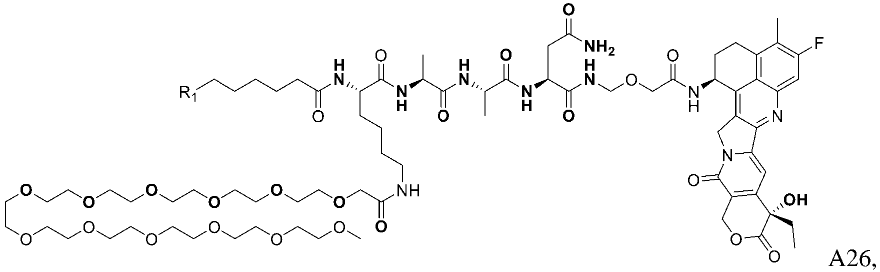 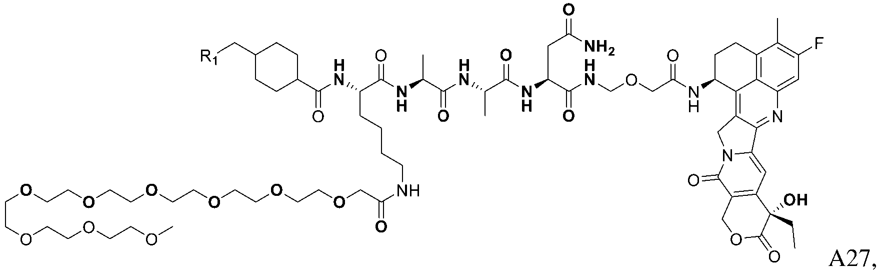 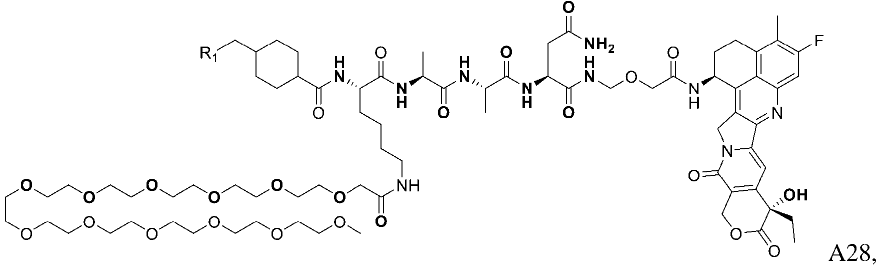 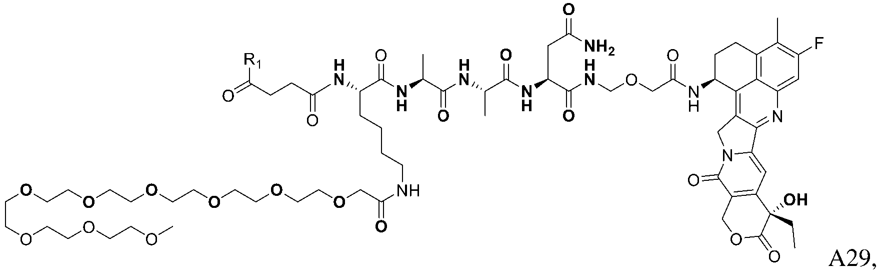 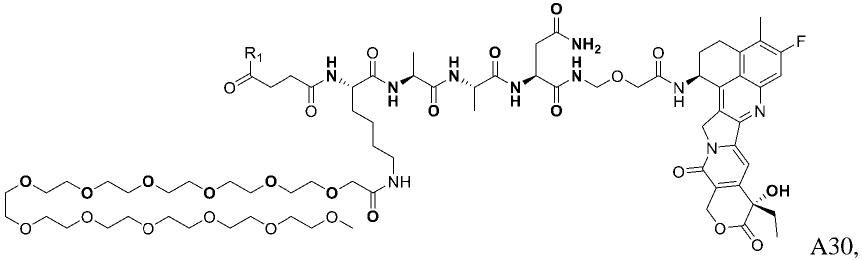 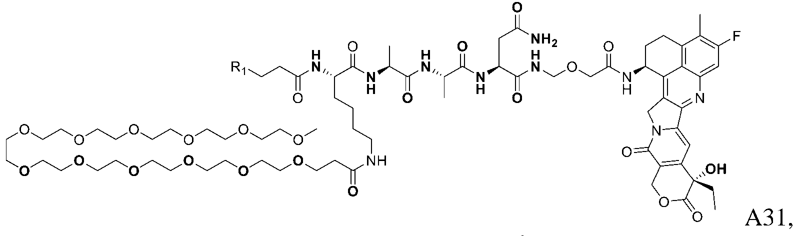 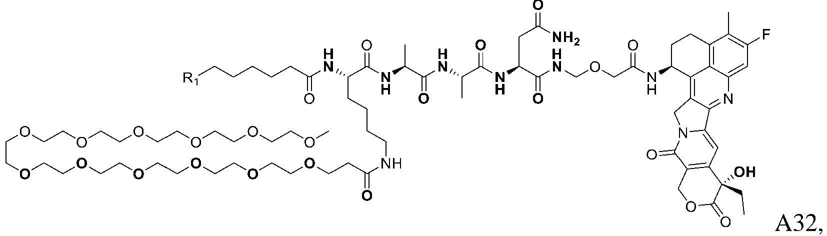 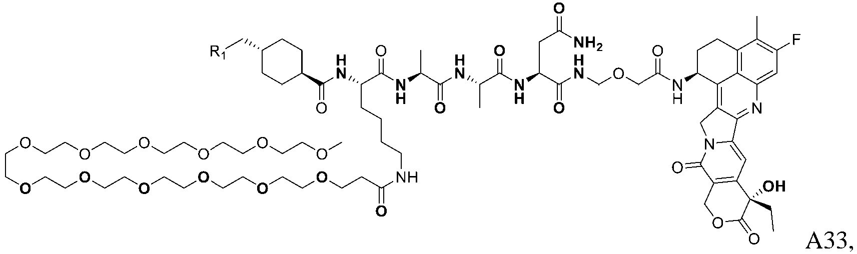 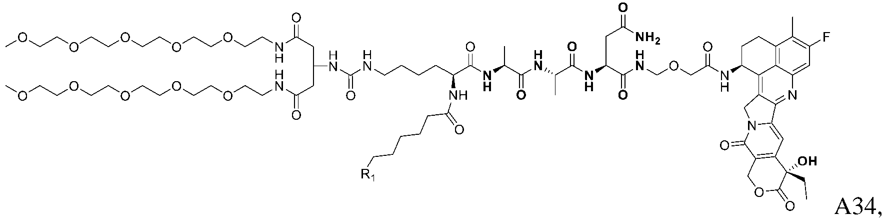 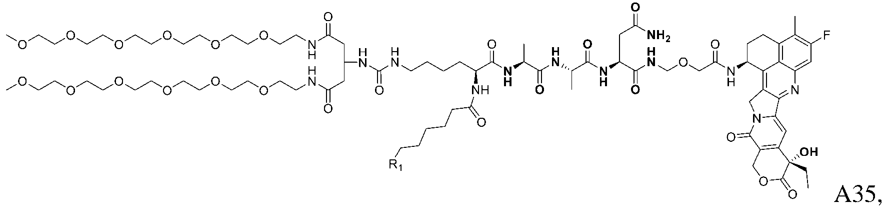 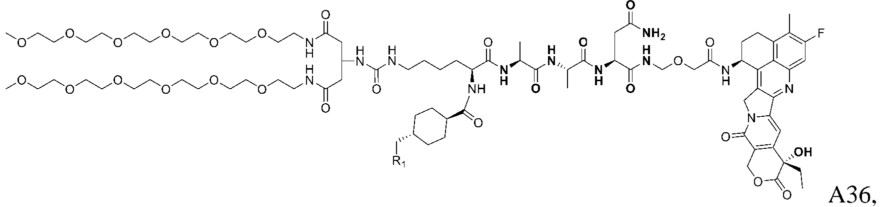 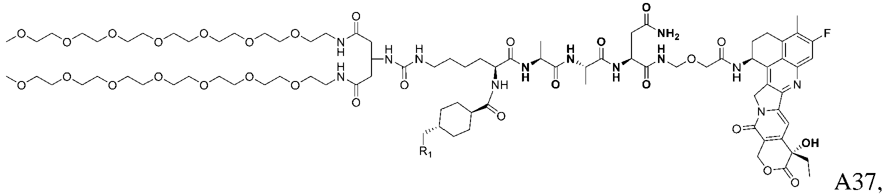 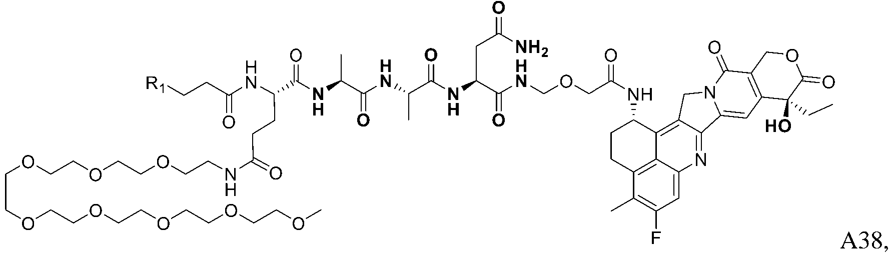 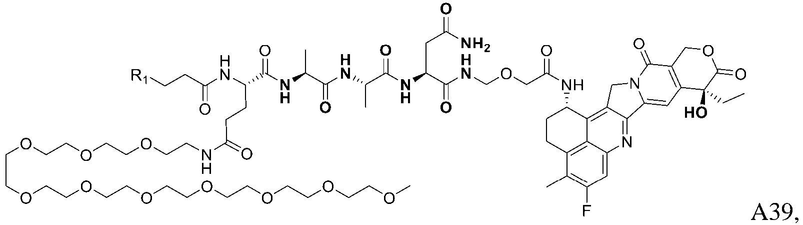 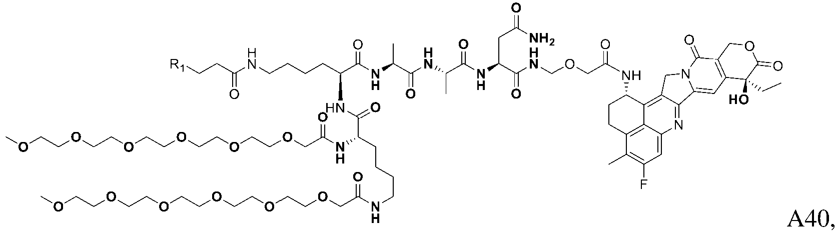 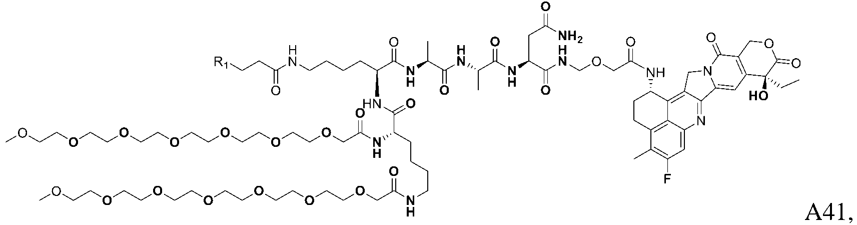 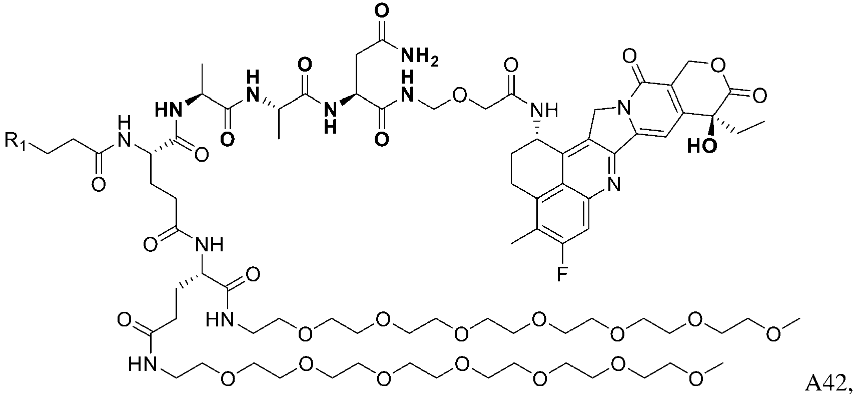 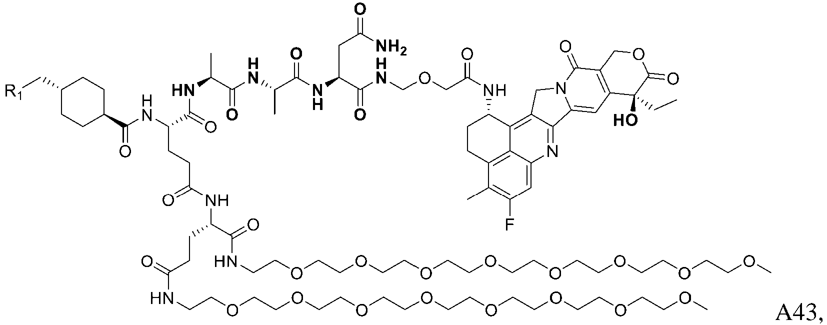 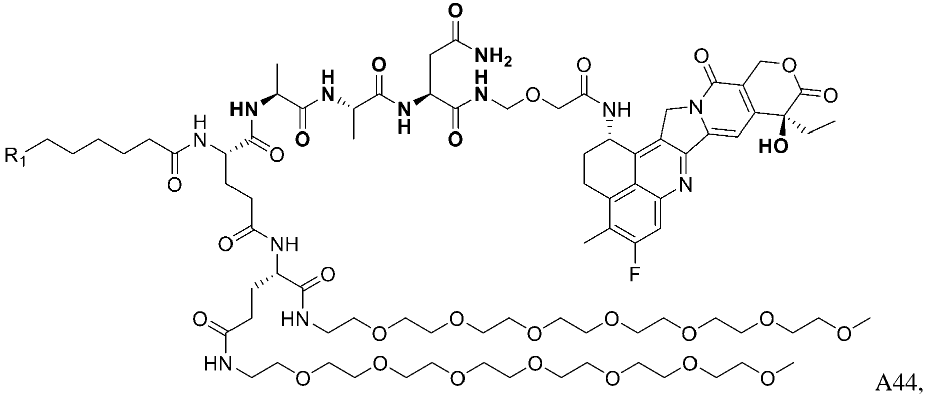 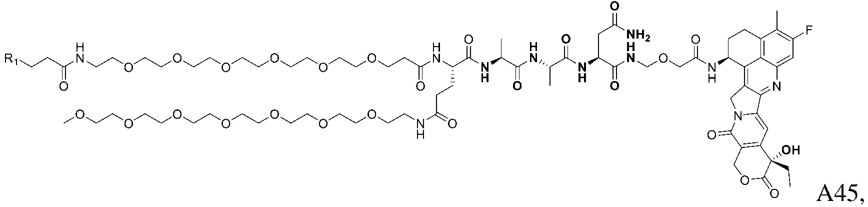 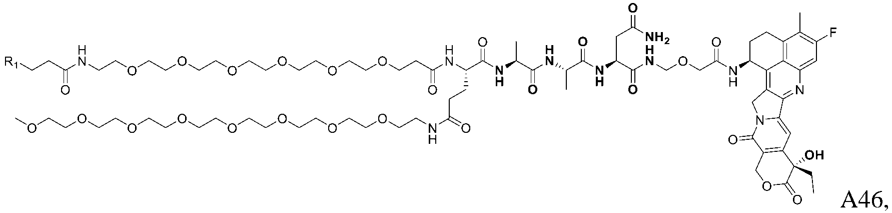 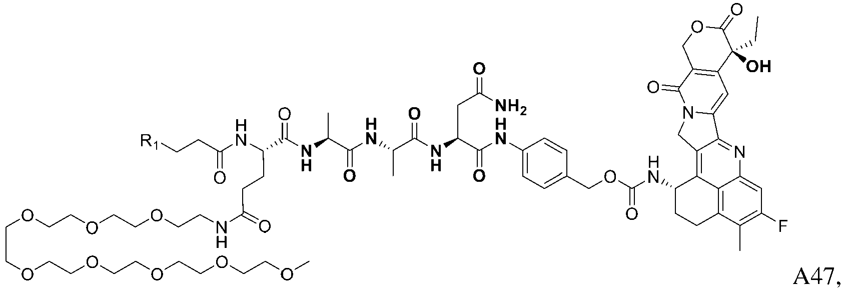 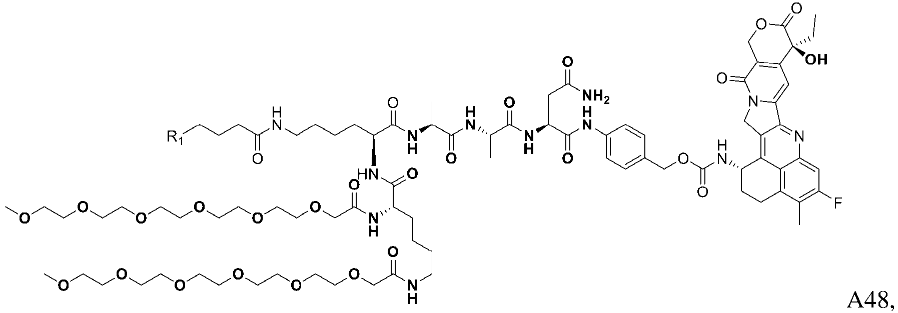 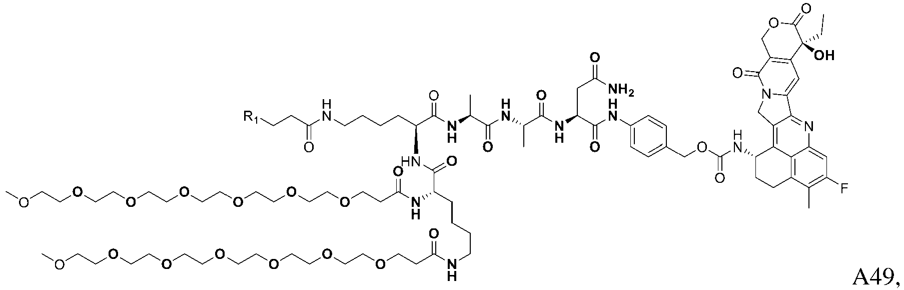 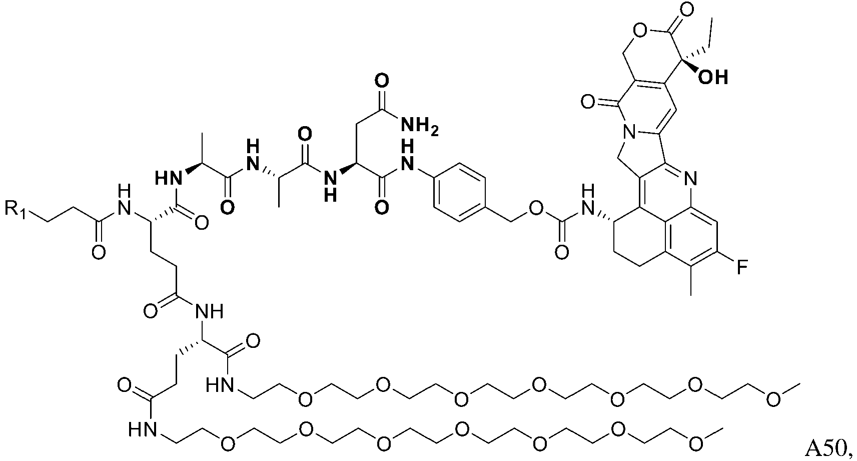 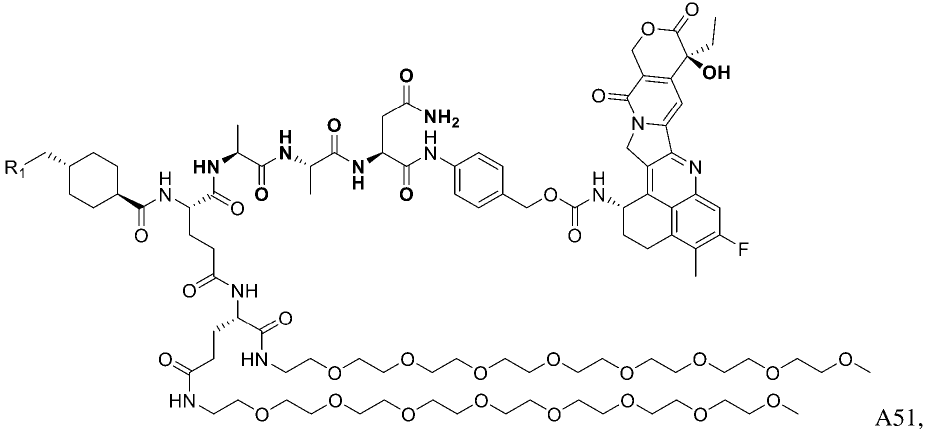 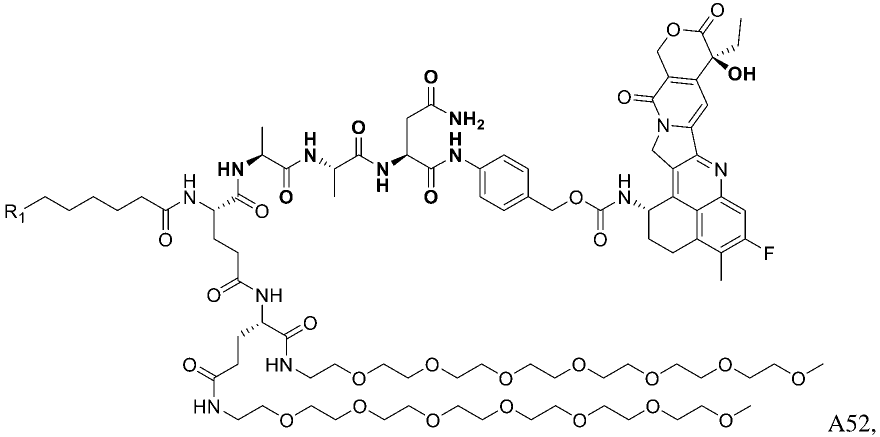 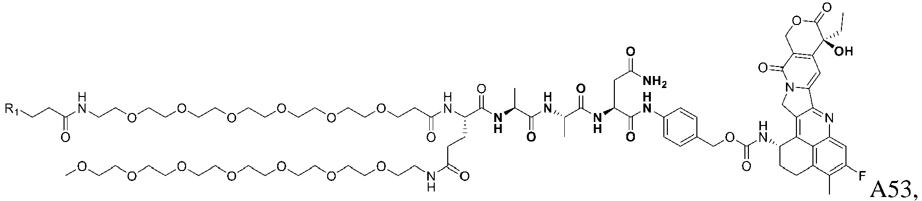 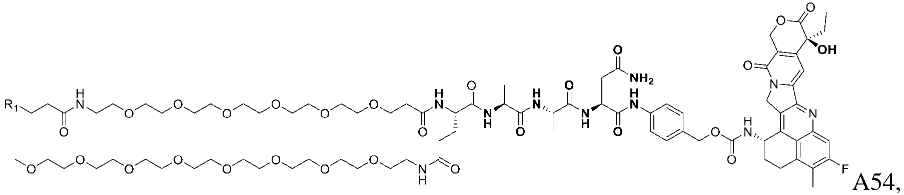 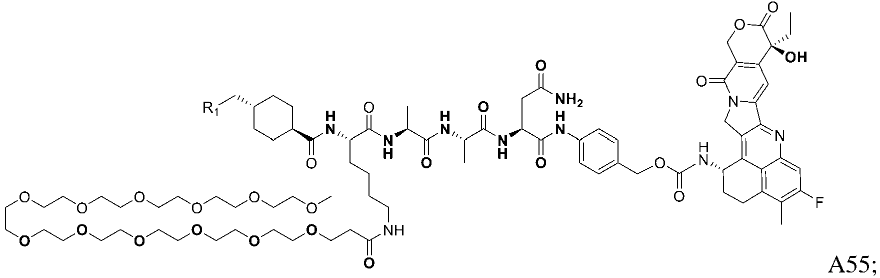 wherein in each formula, R₁ is as defined in claim 1 or 2.

5. The drug conjugate of claim 1, a stereoisomer thereof, or a pharmaceutically acceptable salt thereof, wherein the drug conjugate of formula (I) is selected from:

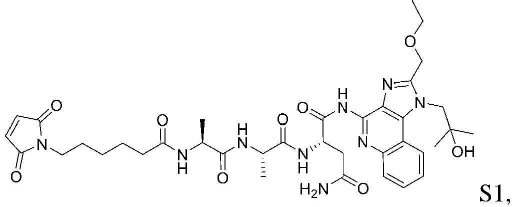

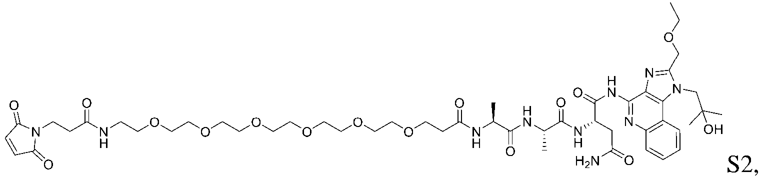

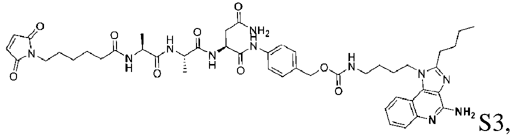

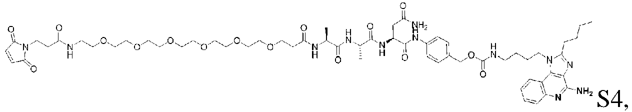

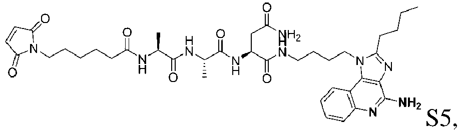

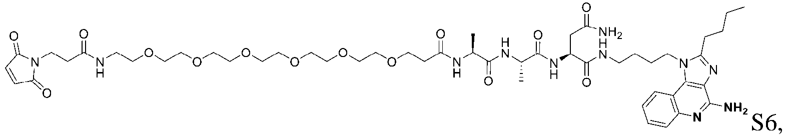

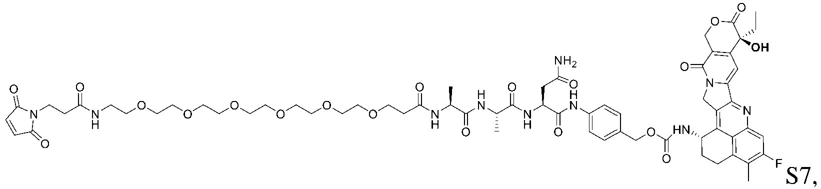

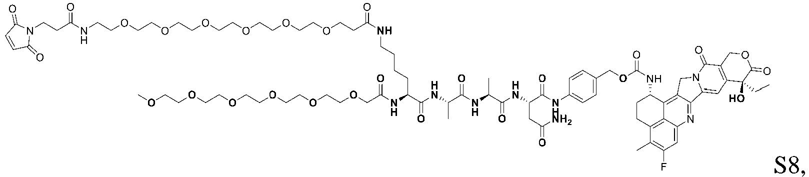

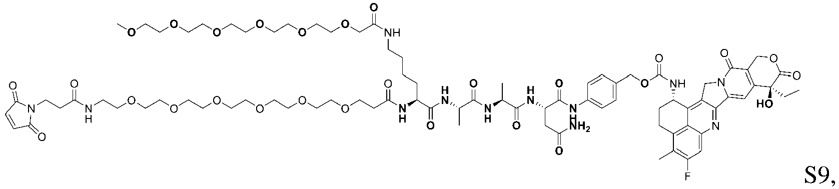

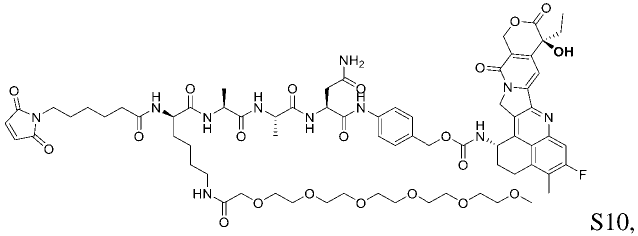

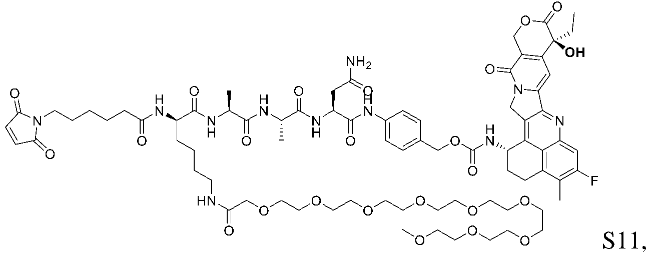

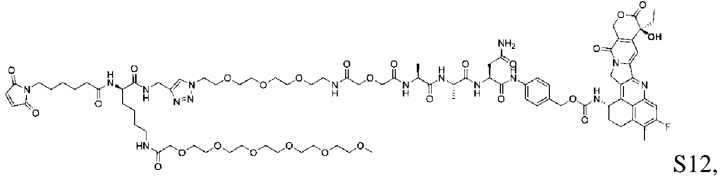

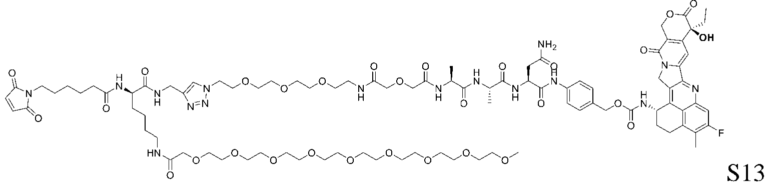

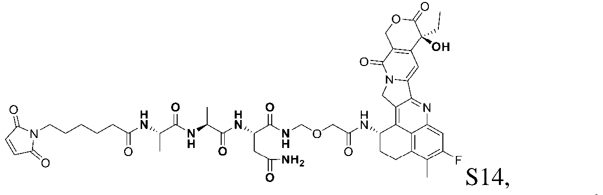

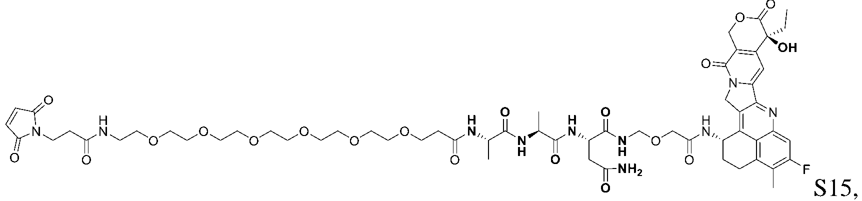

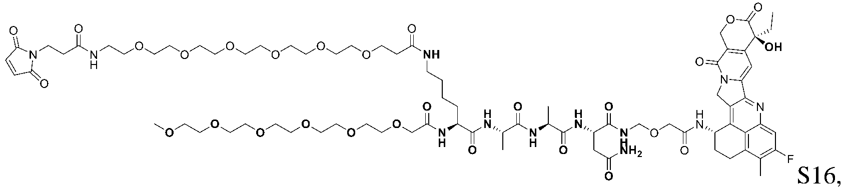

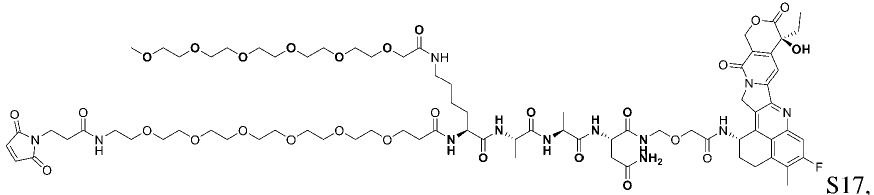

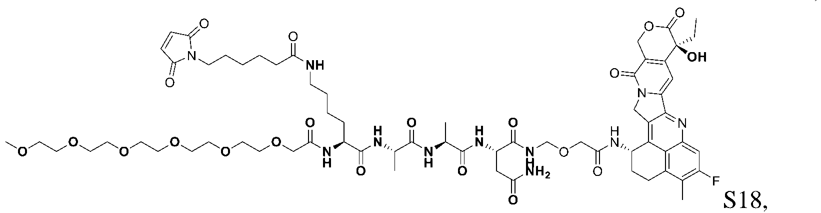

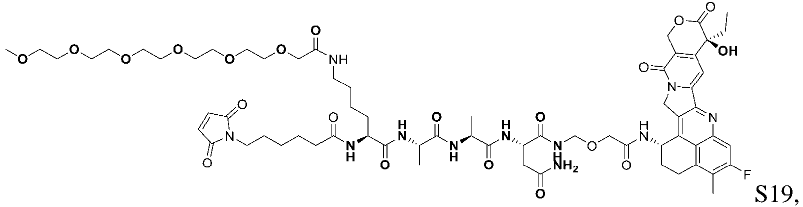

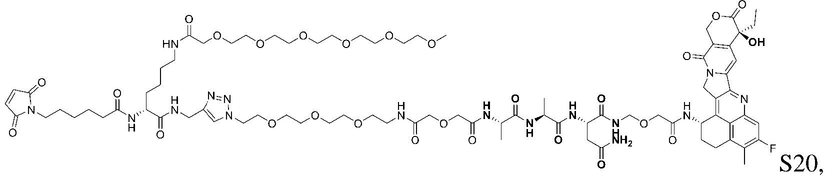

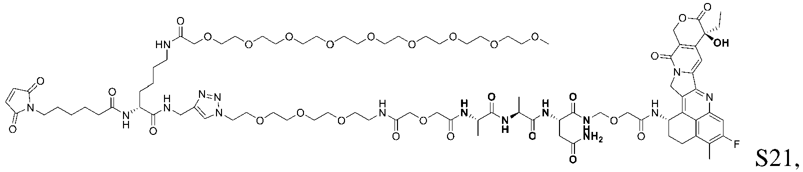

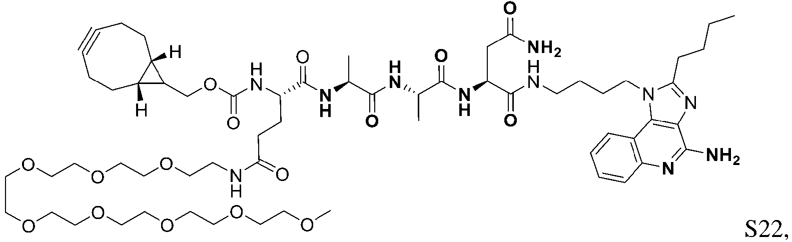

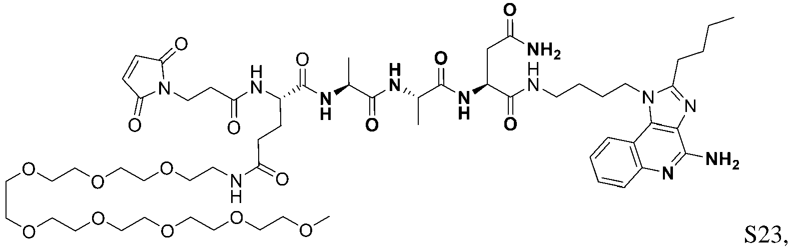

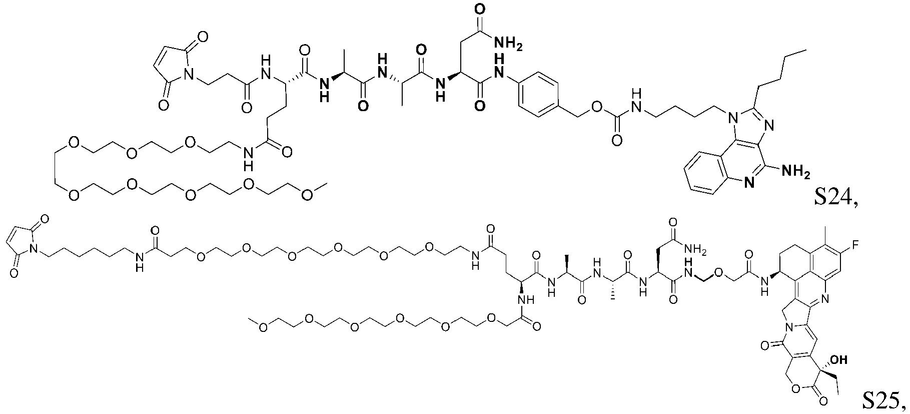

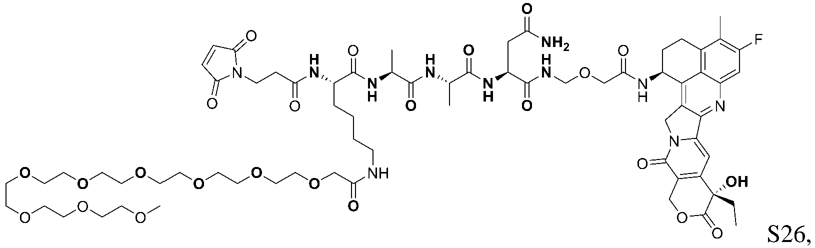

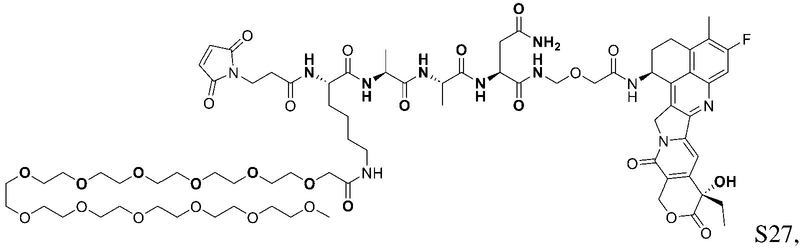

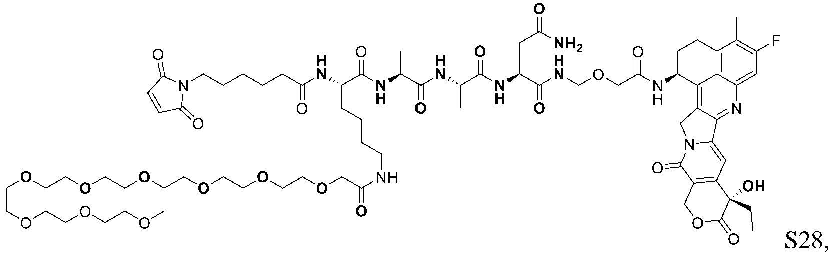

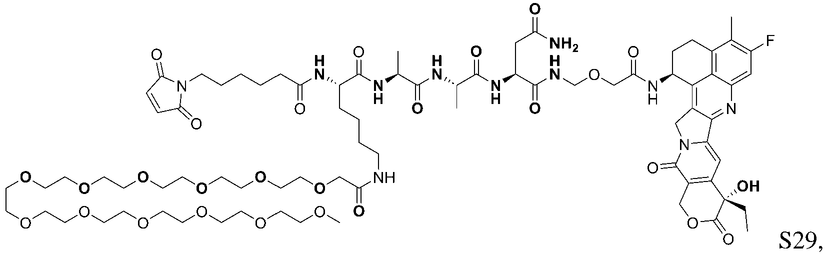

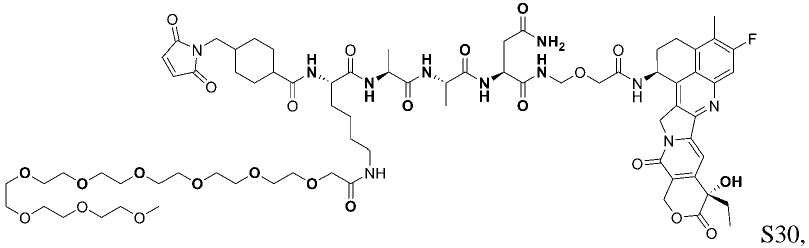

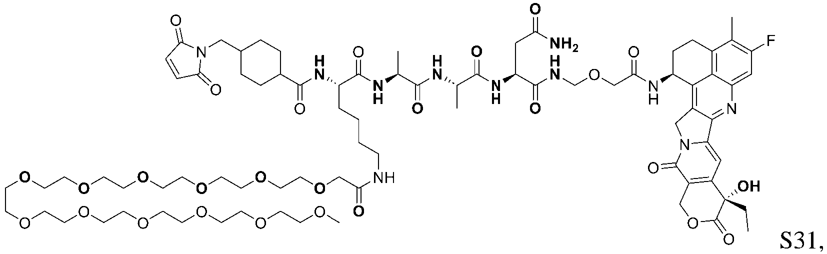

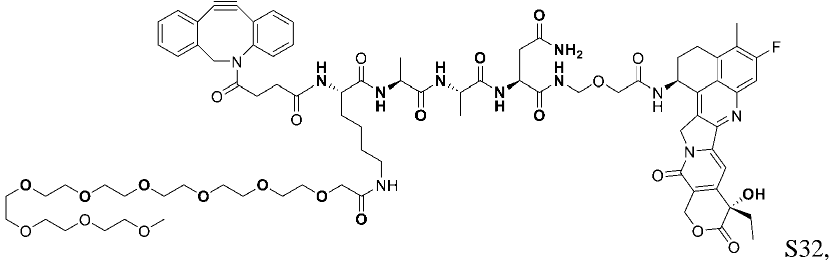

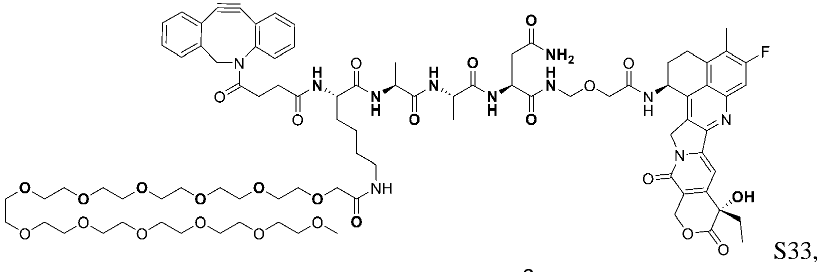

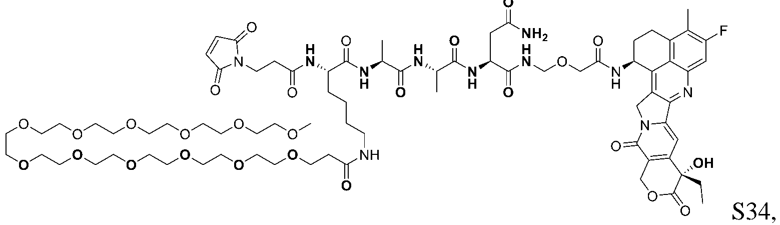

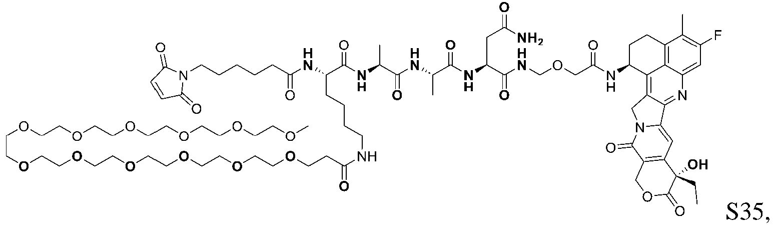

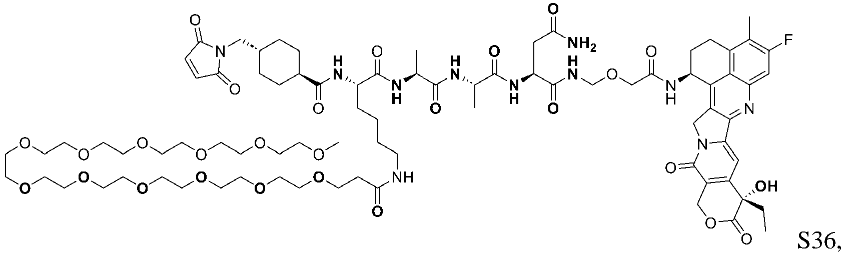

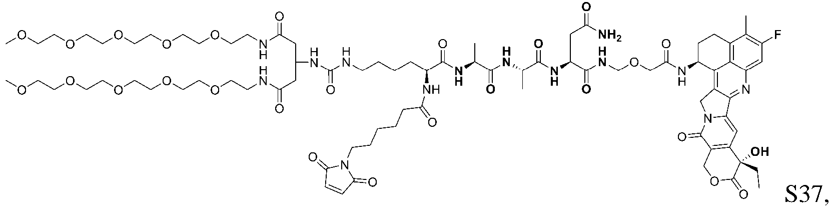

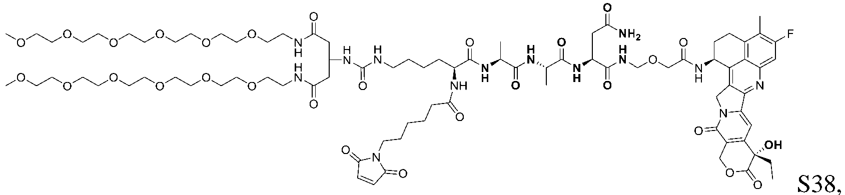

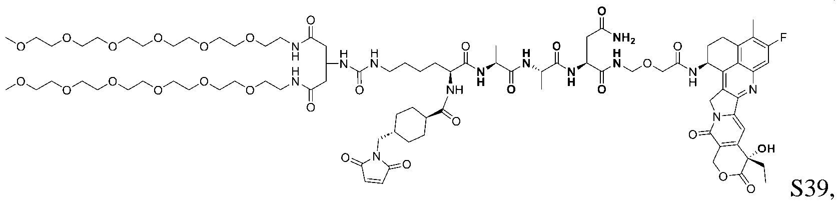

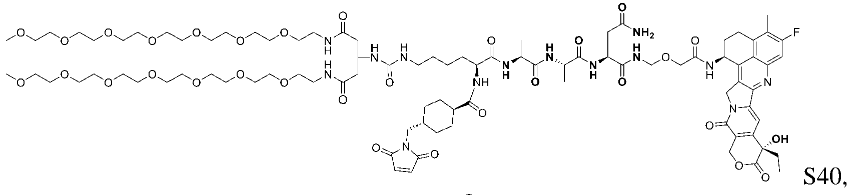

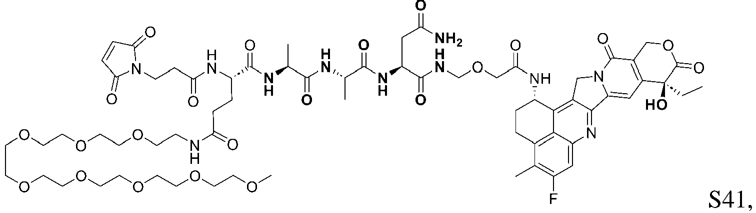

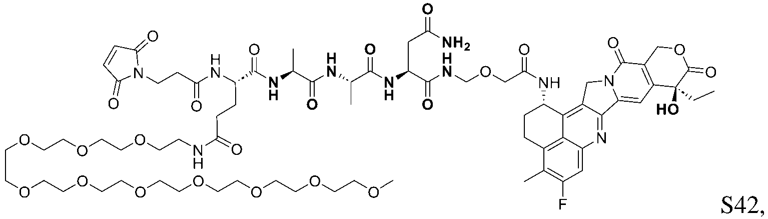

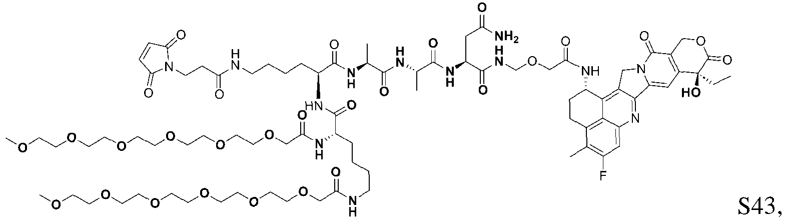

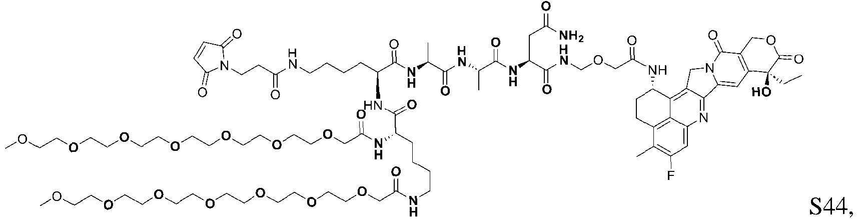

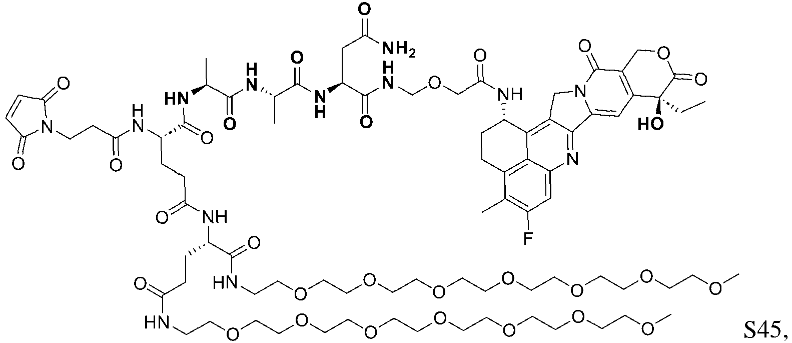

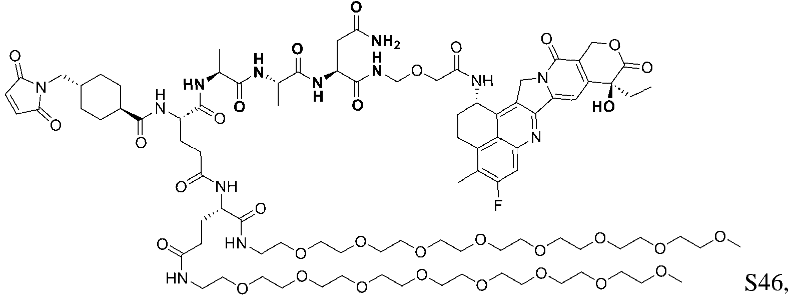

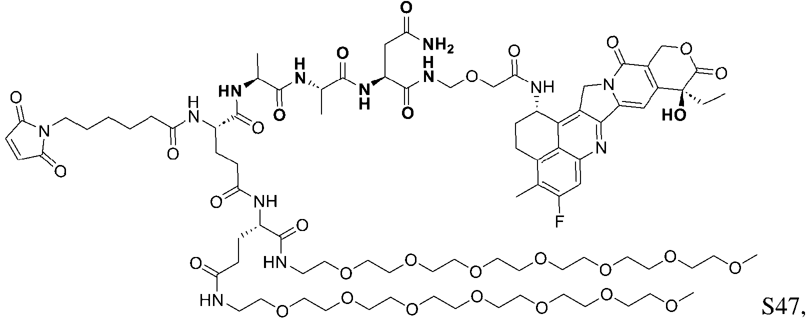

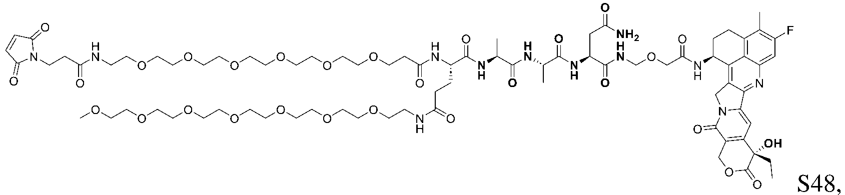

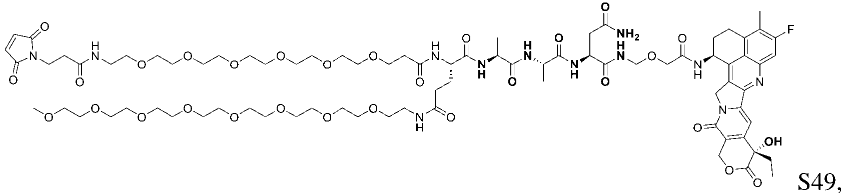

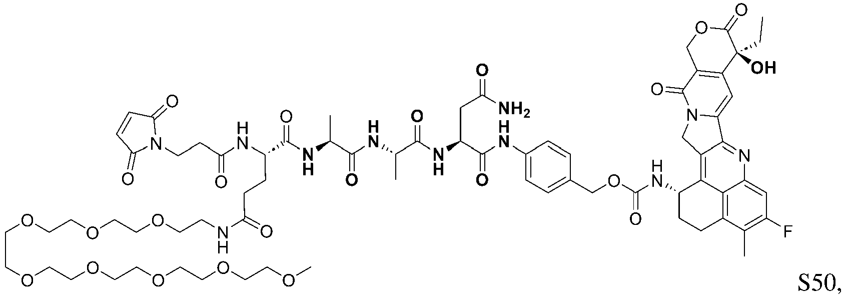

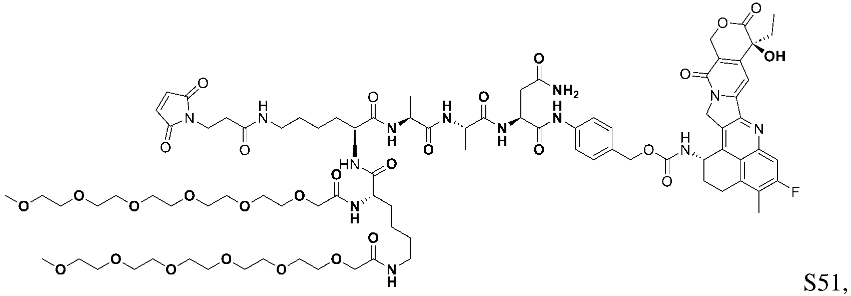

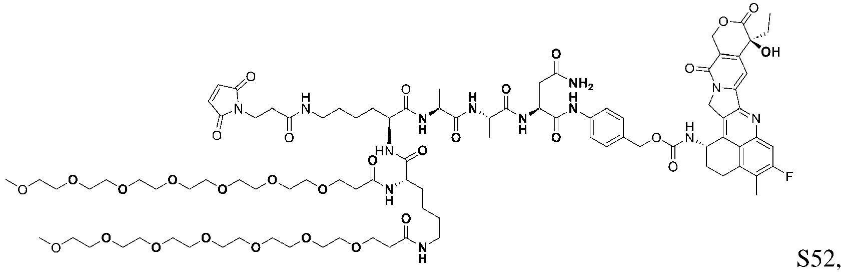

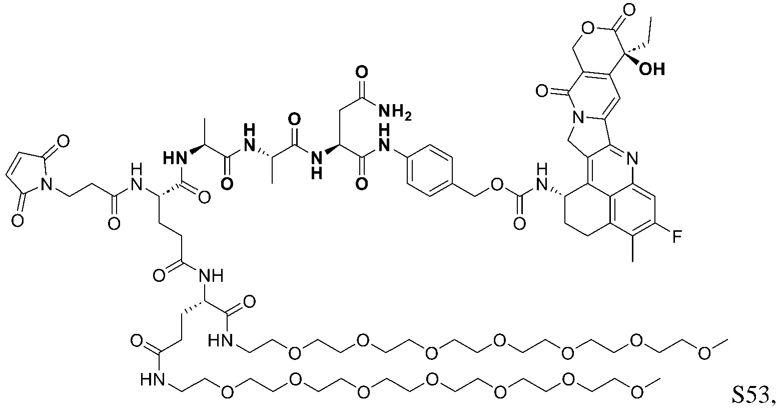

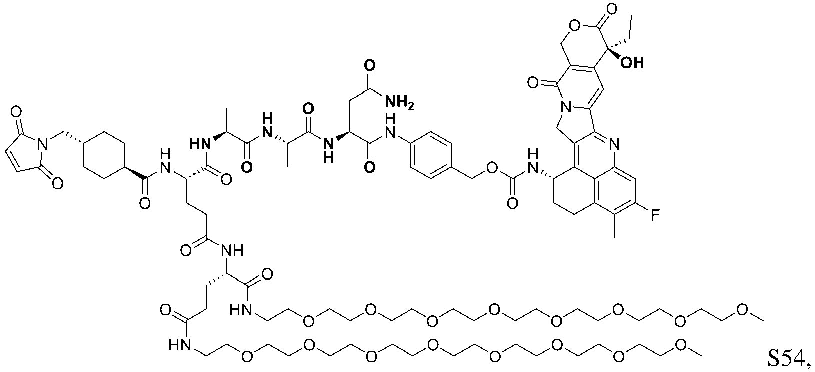

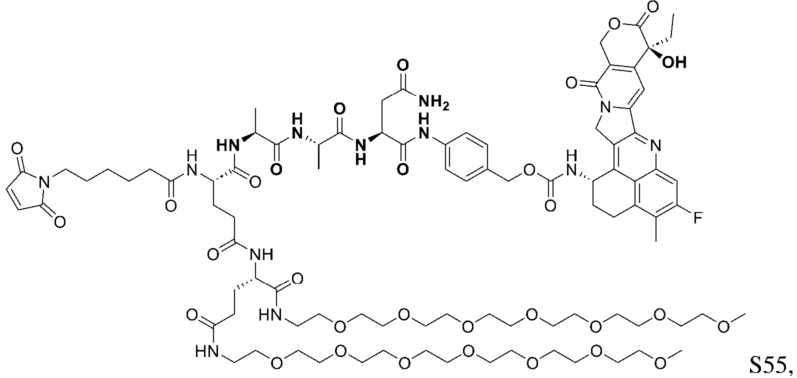

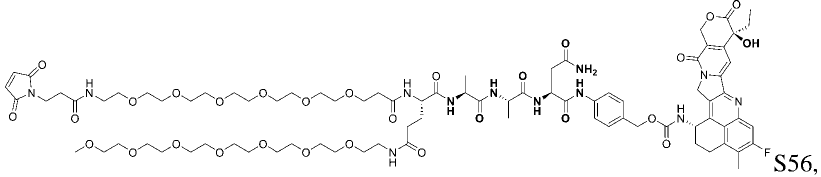

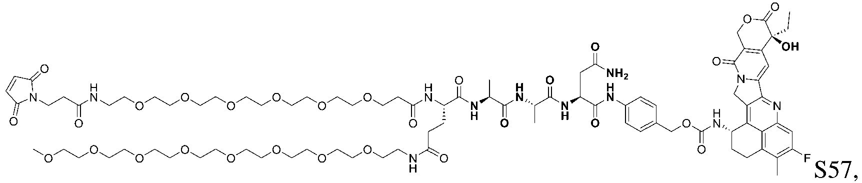

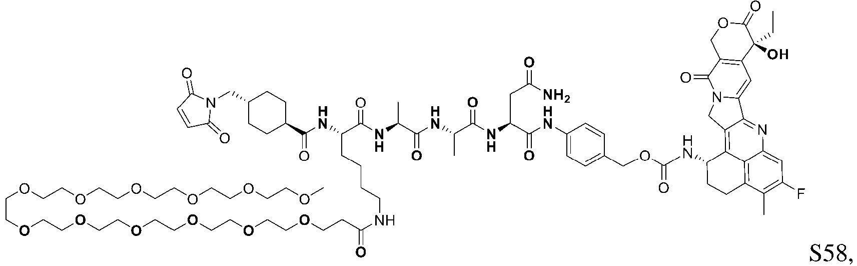

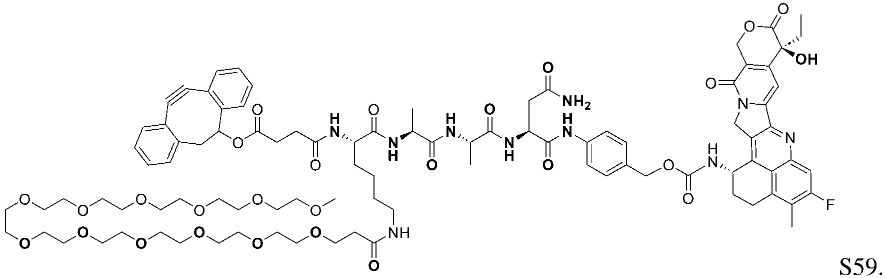

6. The drug conjugate of claim 1, a stereoisomer thereof, or a pharmaceutically acceptable salt thereof, wherein R₂' is selected from:
C₁₋₆ alkyl-CO-*;
C₁₋₆ alkyl-O-*;
C₁₋₆ alkyl-OCONH-C₁₋₆ alkylene-(O-CH₂CH₂-O)ₙ-C₁₋₆ alkylene-CO-*;
C₁₋₆ alkyl-OCONH-C₁₋₆ alkylene-(O-CH₂CH₂-O)ₙ-C₁₋₆ alkylene-O-*
C₁₋₆ alkyl-OCONH-C₁₋₆ alkylene-(O-CH₂CH₂-O)ₙ-C₁₋₆ alkylene-NH-*(O-CH₂CH₂-O) ₙ-C₁₋₆ alkylene-CO-*;
C₁₋₆ alkyl-CONH-C₁₋₆ alkylene-(O-CH₂CH₂-O)ₙ-C₁₋₆ alkylene-CO-*;
NH₂-C₁₋₆ alkylene-(O-CH₂CH₂-O)ₙ-C₁₋₆ alkylene-CO-*;
NH₂-C₁₋₆ alkylene-(O-CH₂CH₂-O)ₙ-C₁₋₆ alkylene-NH-*;
NH₂-C₁₋₆ alkylene-(O-CH₂CH₂-O)ₙ-C₁₋₆ alkylene-O-*;
NH₂-C₁₋₅₅ alkylene-CO-*;
NH₂-C₁₋₅₅ alkylene-NH-*;
NH₂-C₁₋₅₅ alkylene-O-*;
HOOC-C₁₋₅₅ alkylene-CO-*;
HOOC-C₁₋₆ alkylene-CONH-C₁₋₆ alkylene-(O-CH₂CH₂-O)ₙ-C₁₋₆ alkylene-CO-*;
HOOC-C₁₋₆ alkylene-CONH-C₁₋₆ alkylene-(O-CH₂CH₂-O)ₙ-C₁₋₆ alkylene-NH-*;
HOOC-C₁₋₆ alkylene-CONH-C₁₋₆ alkylene-(O-CH₂CH₂-O)ₙ-C₁₋₆ alkylene-O-*;
HOOC-C₁₋₆ alkylene-NH-*;
HOOC-C₁₋₆ alkylene-NHCO-C₁₋₆ alkylene substituted with -SO₃H-NH-*;
N₃-C₁₋₆ alkylene-CO-*;
N₃-C₁₋₆ alkylene-(O-CH₂CH₂-O)ₙ-C₁₋₆ alkylene-NHCO-C₁₋₆ alkylene-O-C₁₋₆ alkylene-CO-*;
HOOC-C₁₋₆ alkylene-O-C₁₋₆ alkylene-CO-*; 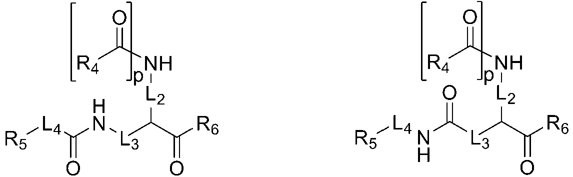 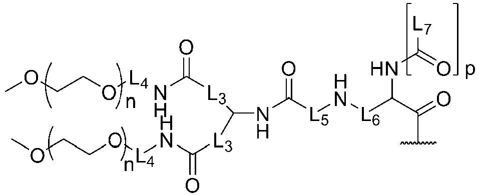 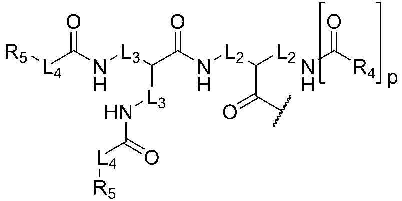 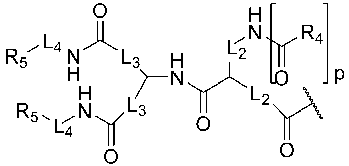
wherein:
* indicates the position of attachment to D;
R₄ is selected from: C₁₋₆ alkyl; C₁₋₆ alkyl-C₃₋₈ cycloalkylene-; COOH-C₁₋₆ alkylene-; C₁₋₆ alkyl-(O-CH₂CH₂-O)ₘ-C₁₋₆ alkylene-; NH₂-C₁₋₆ alkylene; C₁₋₆ alkyl-CONH-C₁₋₆ alkylene-(O-CH₂CH₂-O)ₙ-C₁₋₆ alkylene-; and NH₂-C₁₋₆ alkylene-C₃₋₈ cycloalkylene;
R₅ is selected from: C₁₋₆ alkyl-(O-CH₂CH₂-O)ₘ-; C₁₋₆ alkyl-CONH-C₁₋₆ alkylene-(O-CH₂CH₂-O)ₘ-; COOH-C₁₋₆ alkylene-(O-CH₂CH₂-O)ₙ-;
R₆ is absent, or is -NH-C₁₋₆ alkylene-triazolyl-C₁₋₆ alkylenecarbonyl, or -NH-C₁₋₆ alkylene-triazolyl-C₁₋₆ alkylene-(O-CH₂CH₂-O)ₙ-C₁₋₆ alkylene-NHCO-C₁₋₆ alkyleneoxy-C₁₋₆ alkylenecarbonyl;
L₂ and L₃ are each independently a bond or C₁₋₆ alkylene;
L₄ is each independently C₁₋₆ alkylene;
L₅ is a bond, C₁₋₆ alkyleneoxy-C₁₋₆ alkylenecarbonyl or C₁₋₆ alkylenecarbonyl;
L₆ is C₁₋₆ alkylene;
L₇ is C₁₋₆ alkylene or -C₃₋₈ cycloalkylene-C₁₋₆ alkylene;
each n and m is independently an integer from 1 to 50; p is 0 or 1; when p is 0, the -NH-[CO-R₄]ₚ group is -NH₂;
preferably, R₂' is selected from: 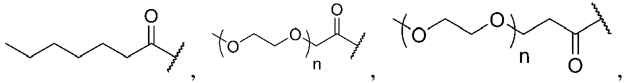 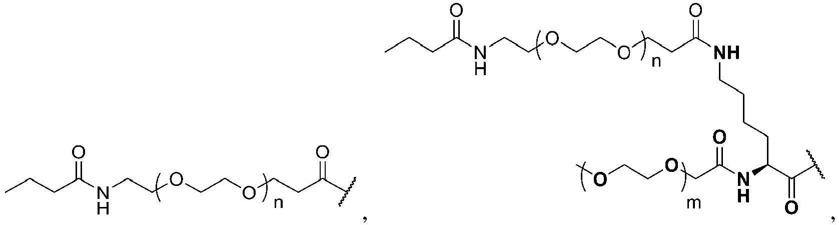 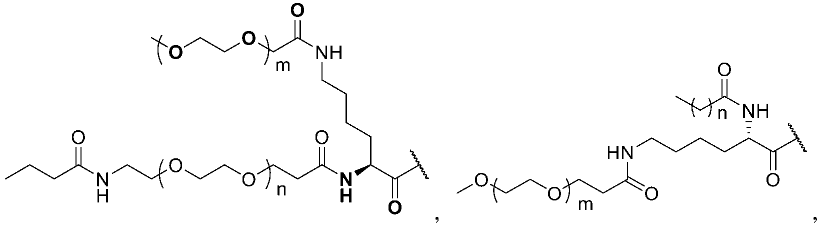 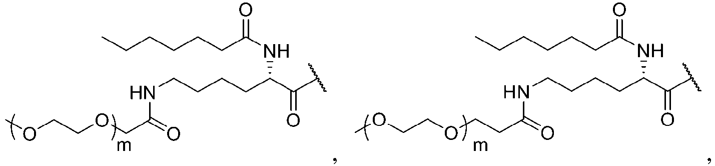 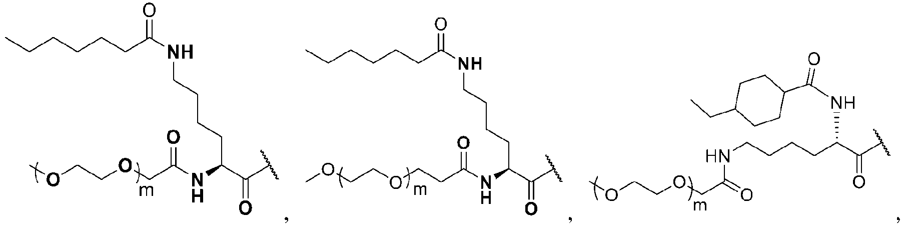 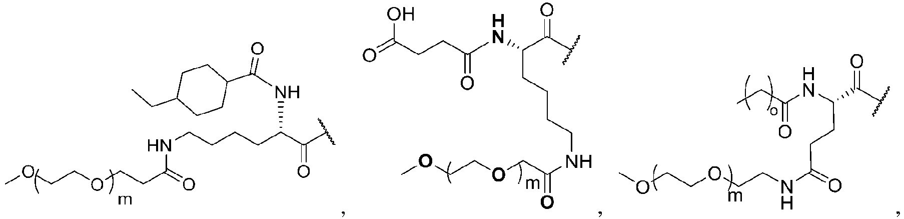 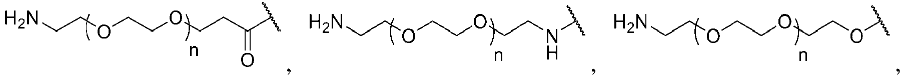 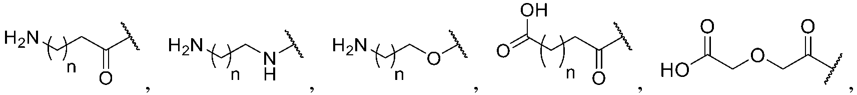 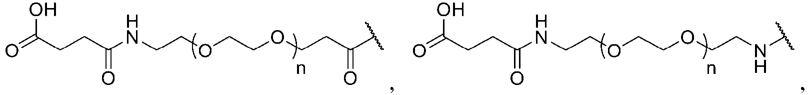 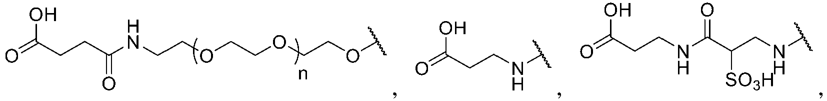 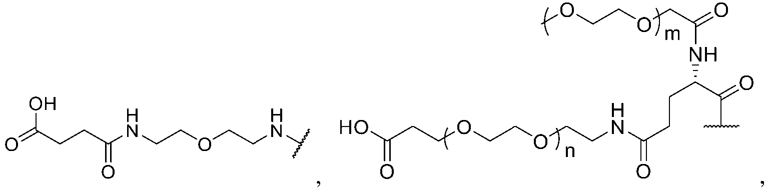 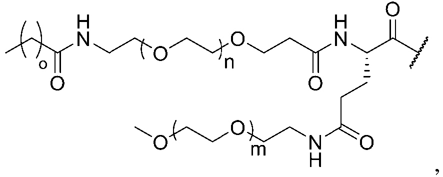 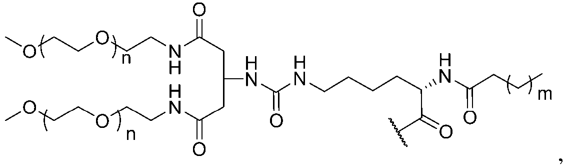 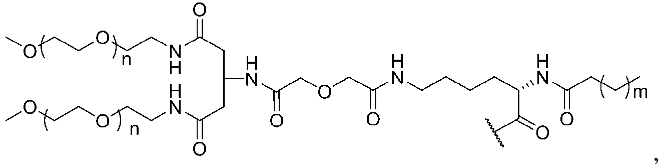 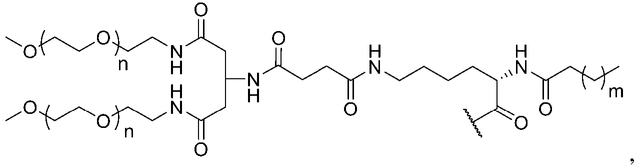 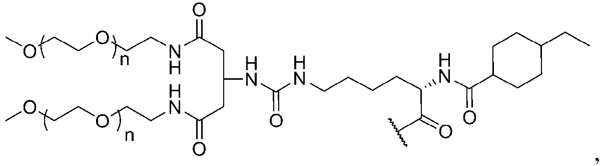 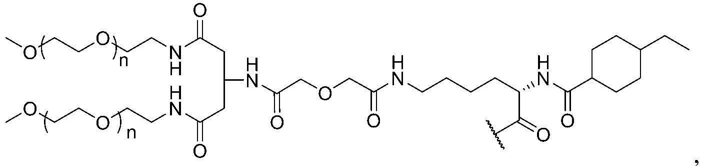 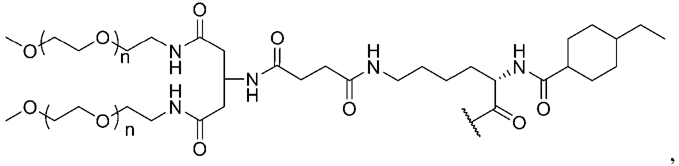 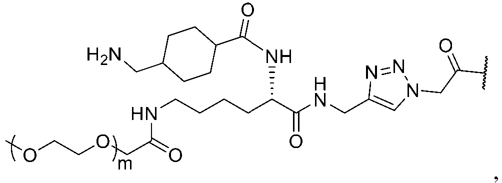 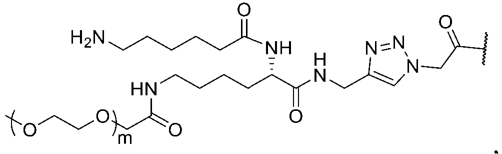 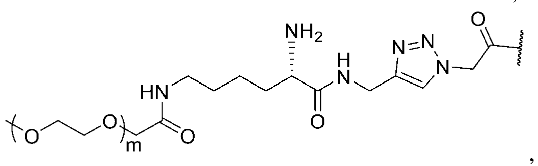 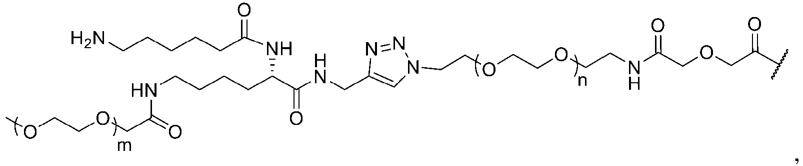 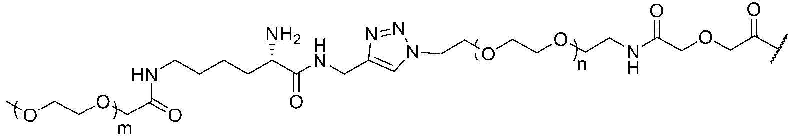 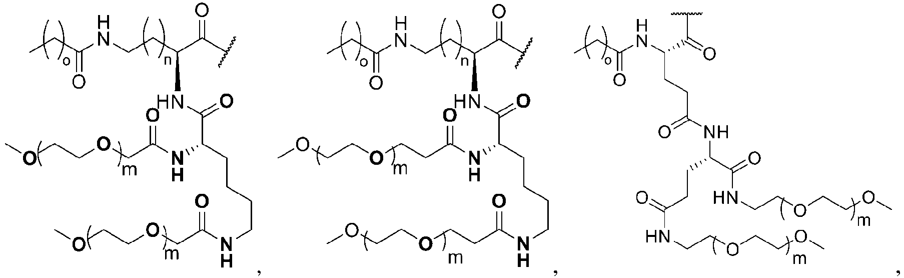 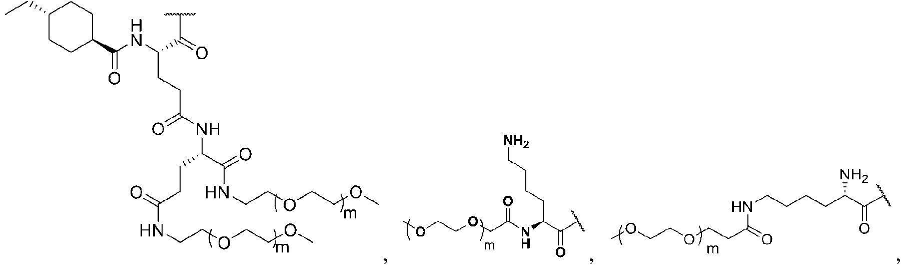 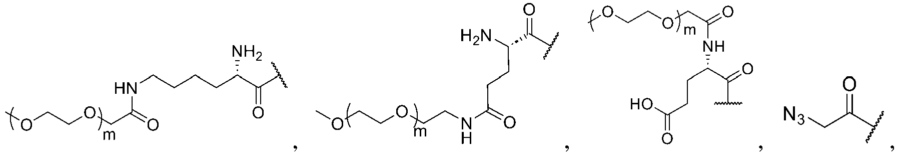 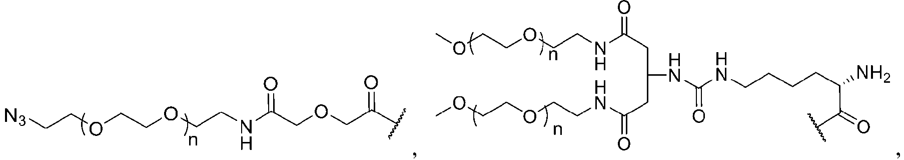 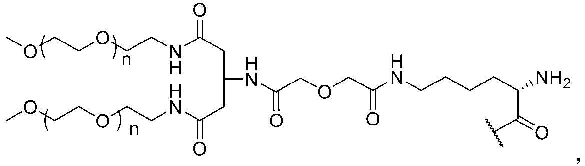 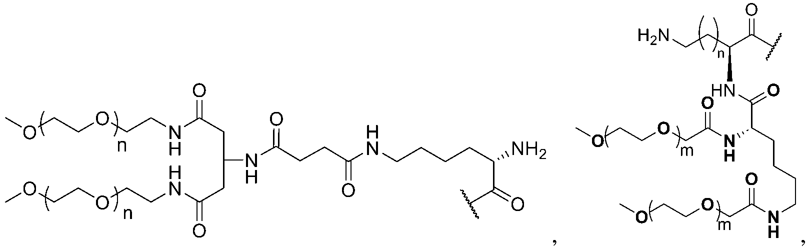 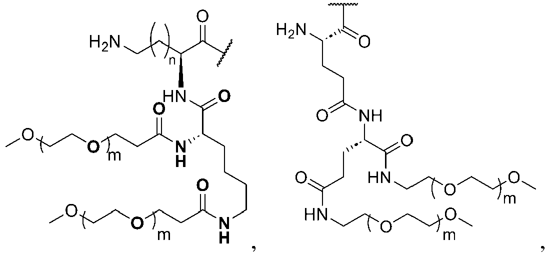 wherein o is independently an integer from 1 to 20, and m and n are each independently an integer from 1 to 50;
more preferably, R₂' is selected from: 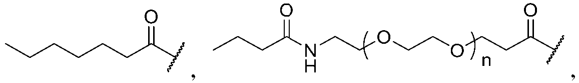 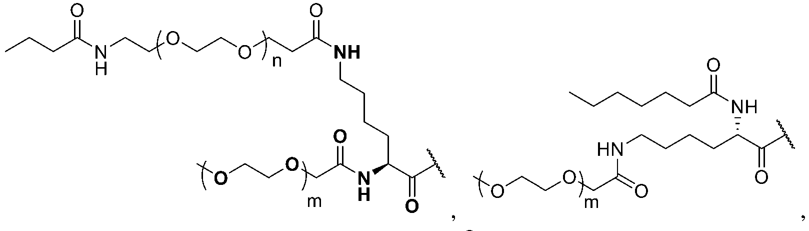 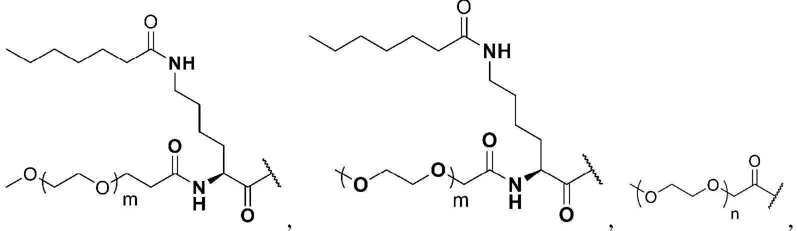 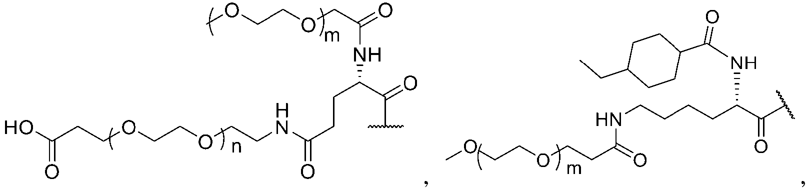 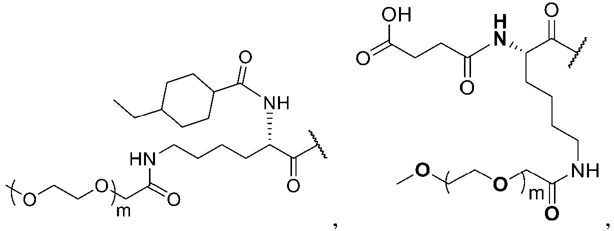 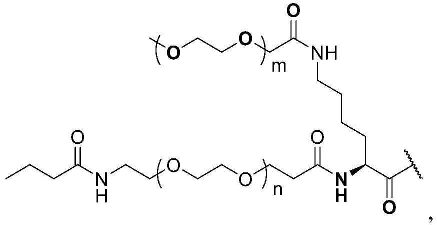 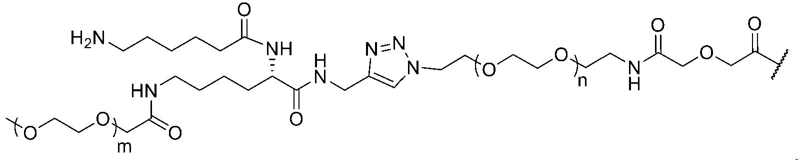 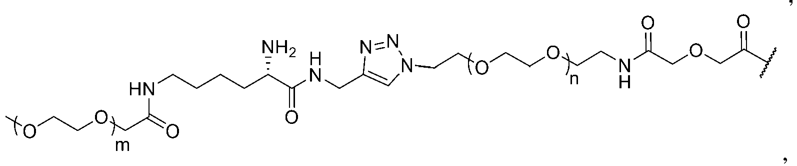 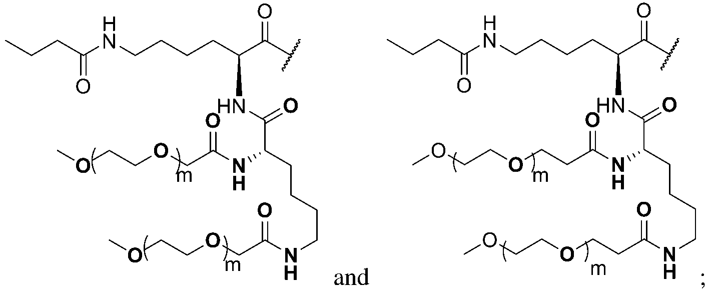 preferably, n and m are each independently an integer from 2 to 20.

7. The drug conjugate of claim 1, a stereoisomer thereof, or a pharmaceutically acceptable salt thereof, wherein the drug conjugate of formula (II) is selected from:

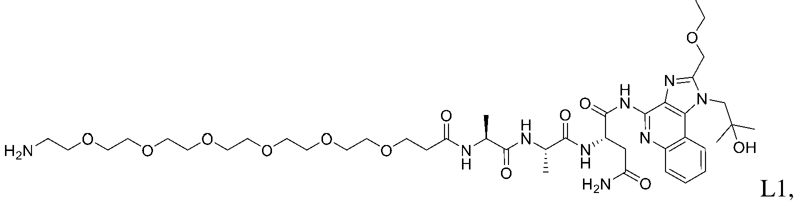

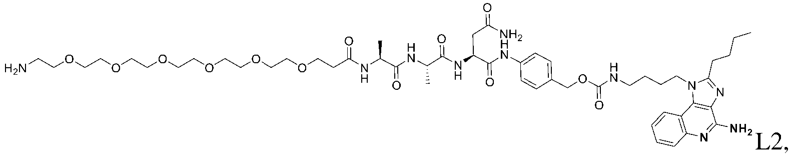

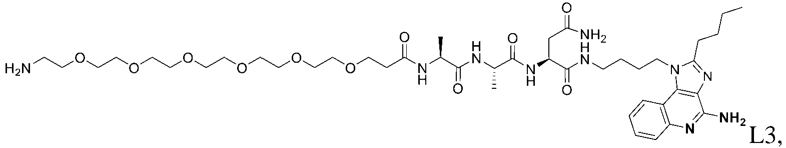

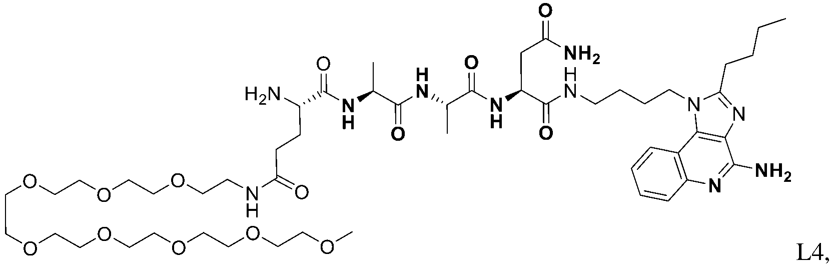

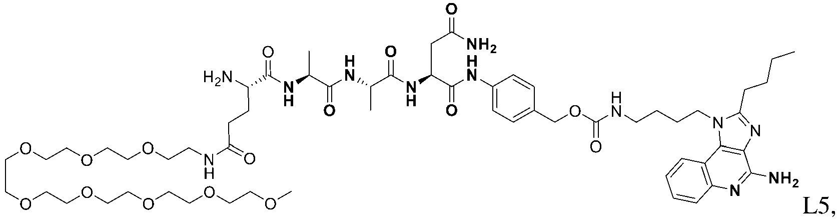

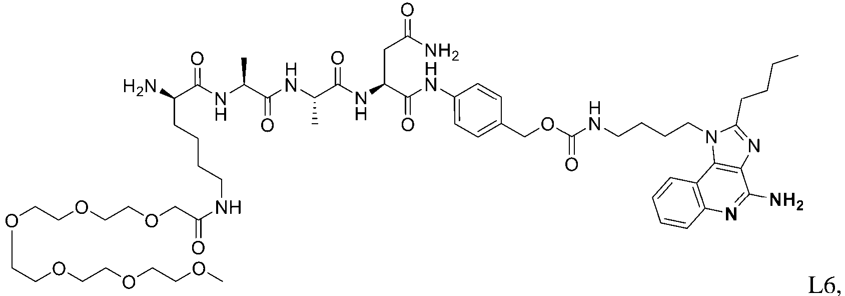

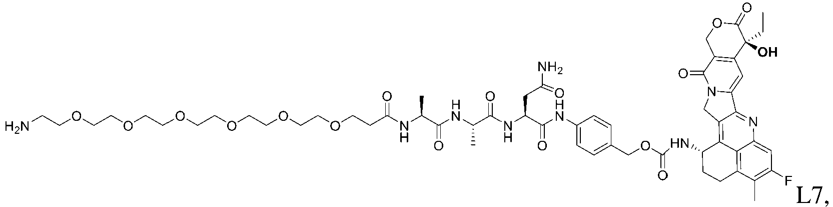

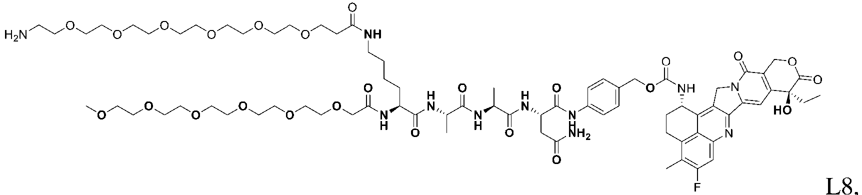

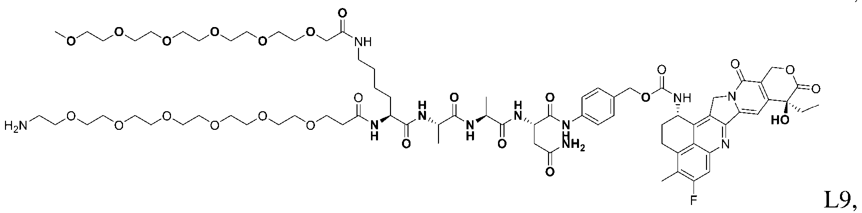

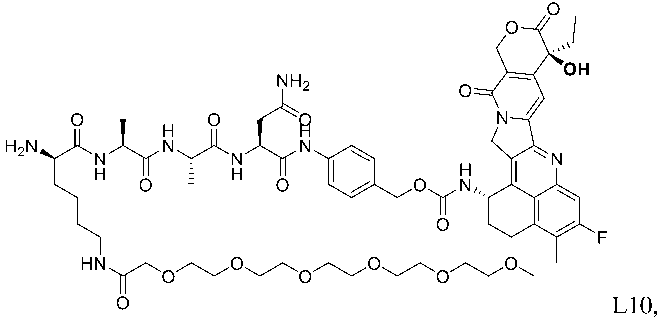

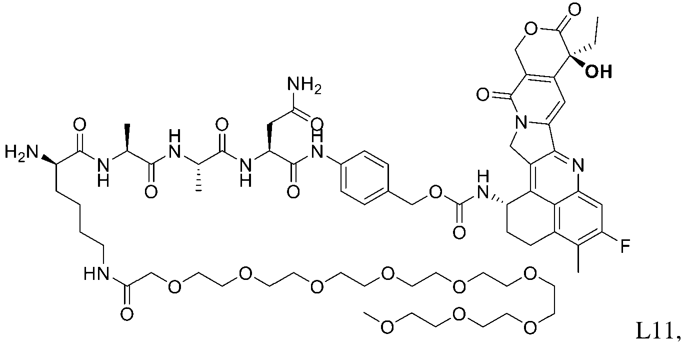

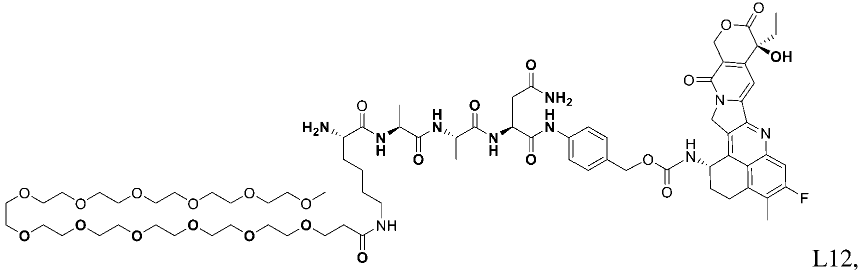

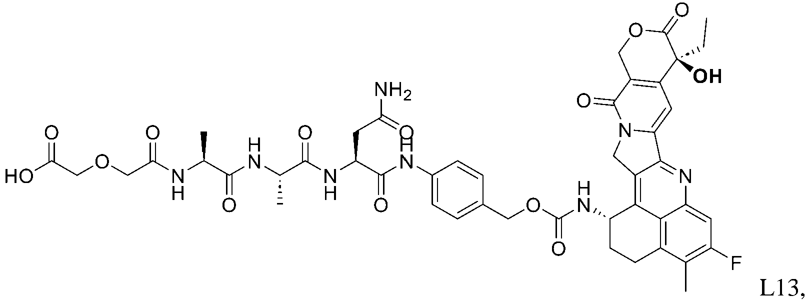

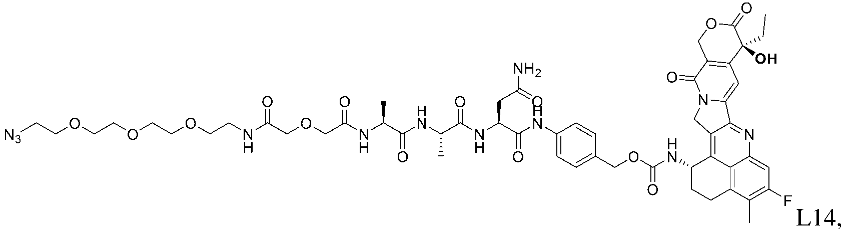

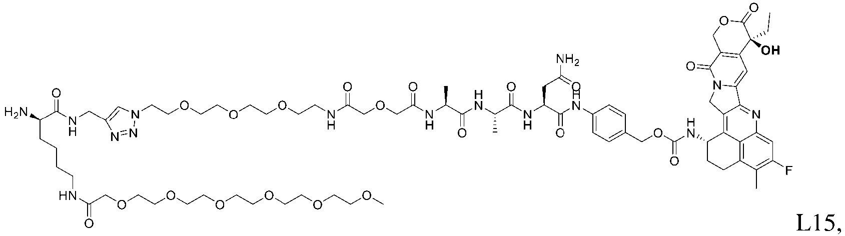

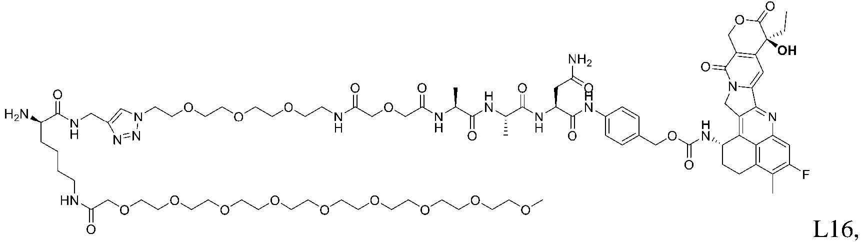

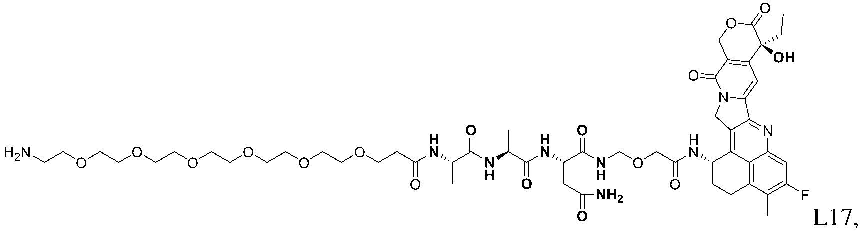

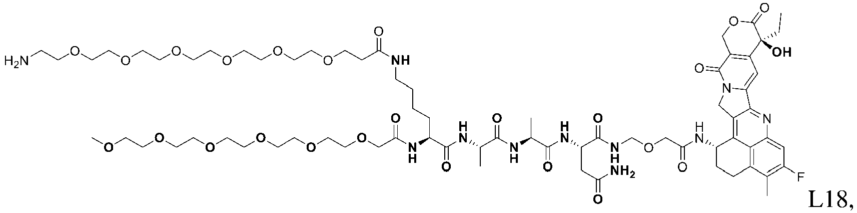

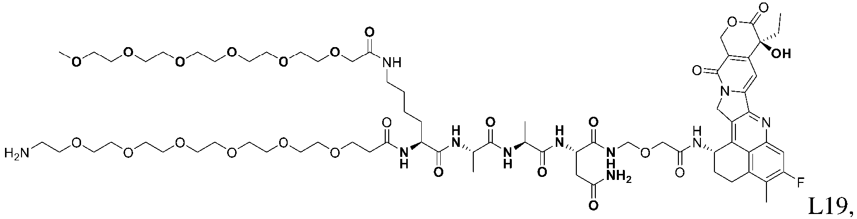

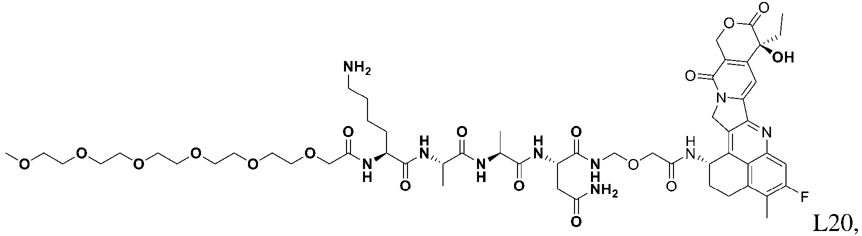

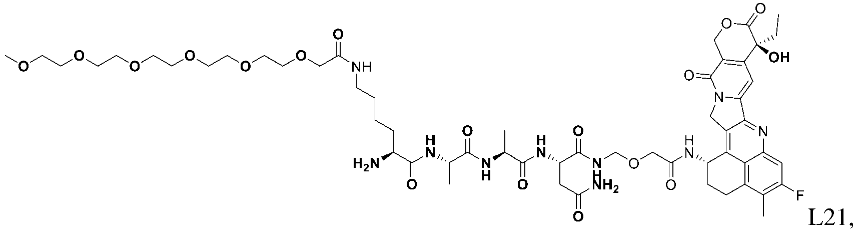

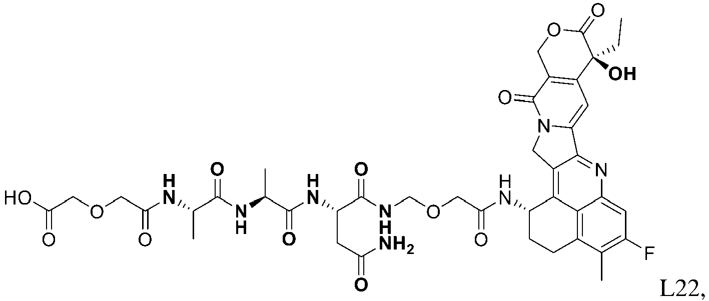

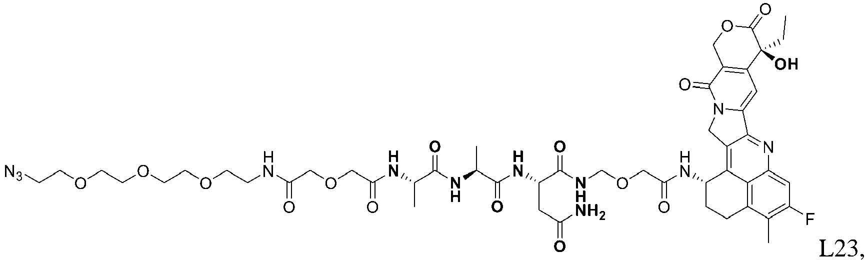

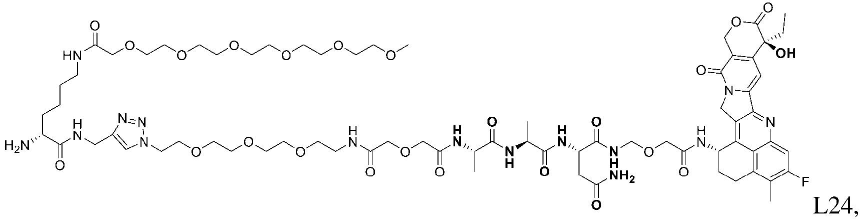

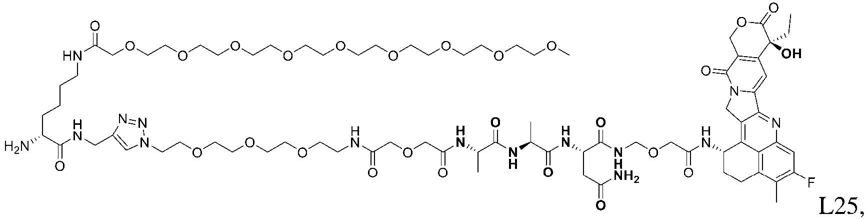

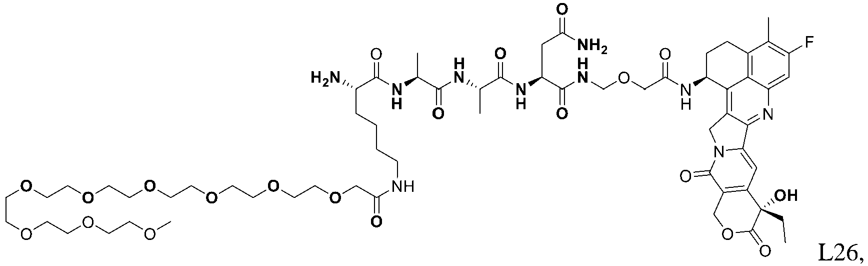

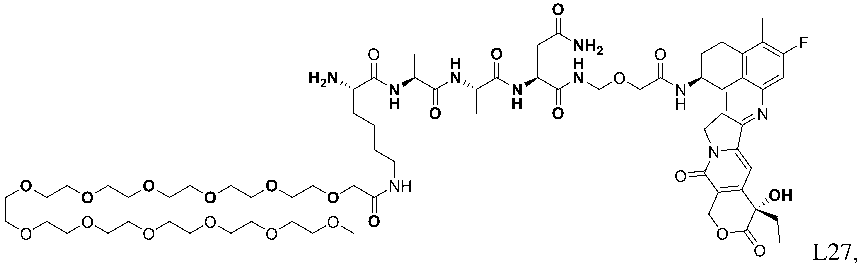

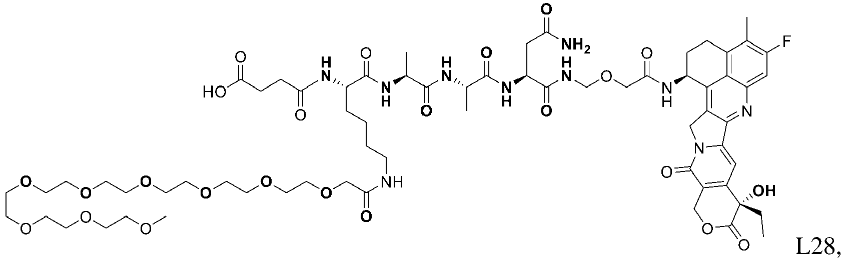

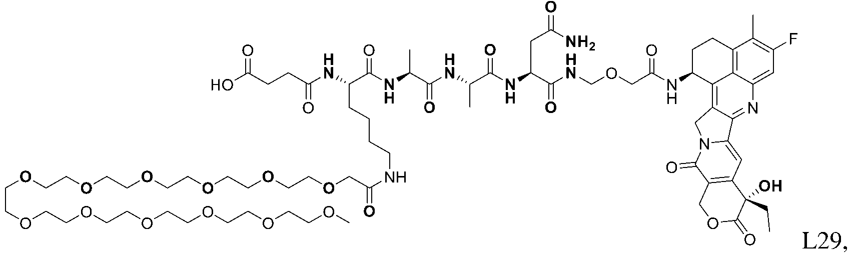

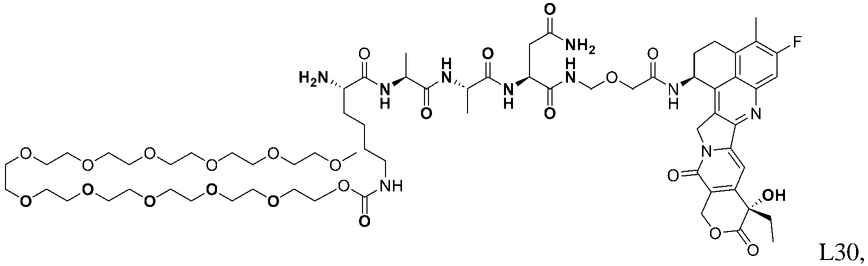

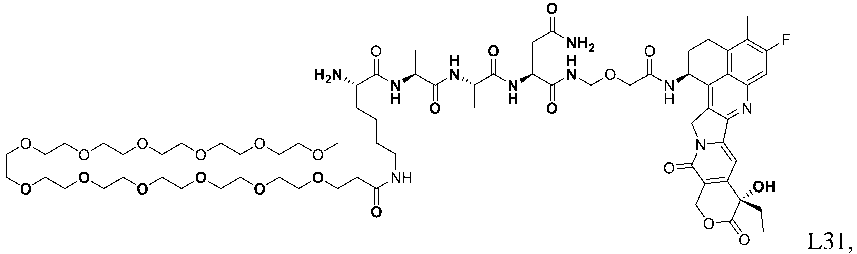

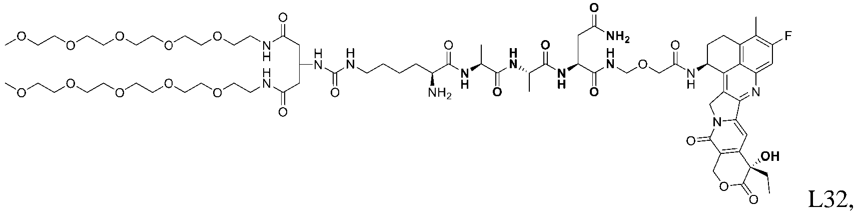

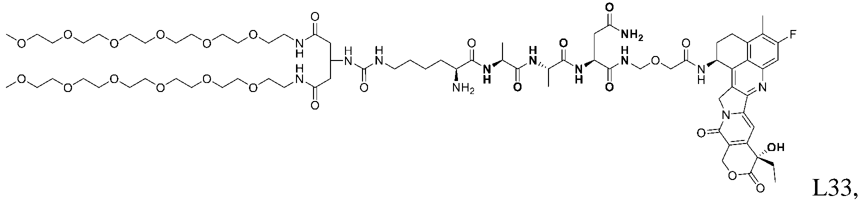

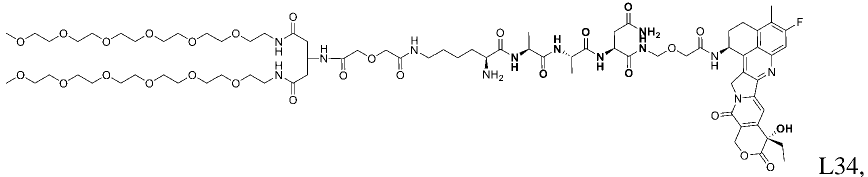

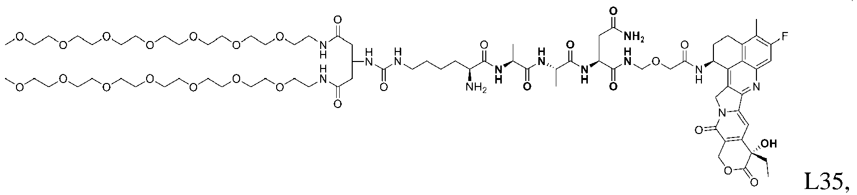

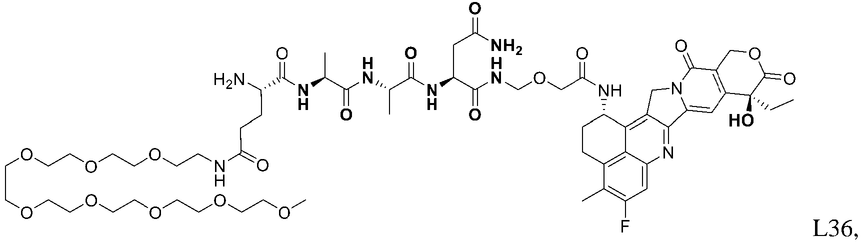

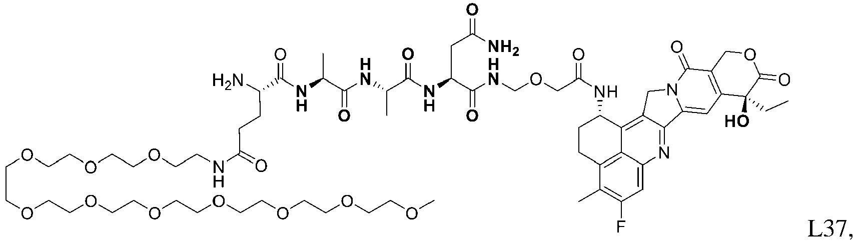

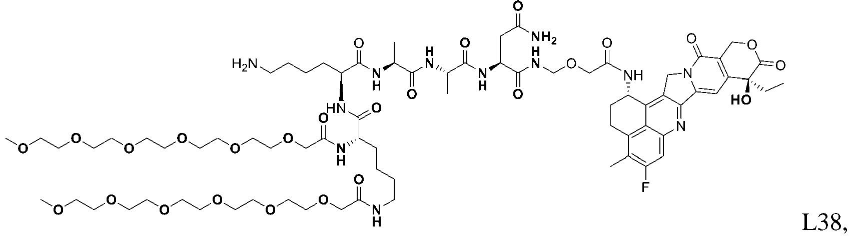

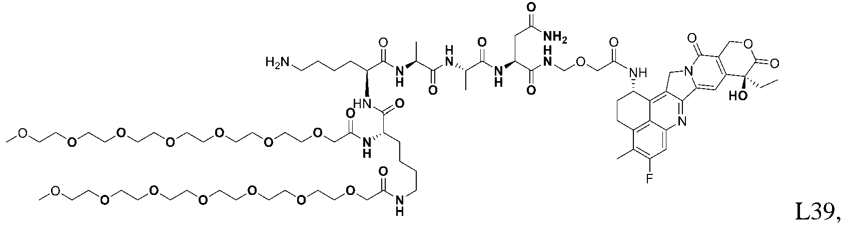

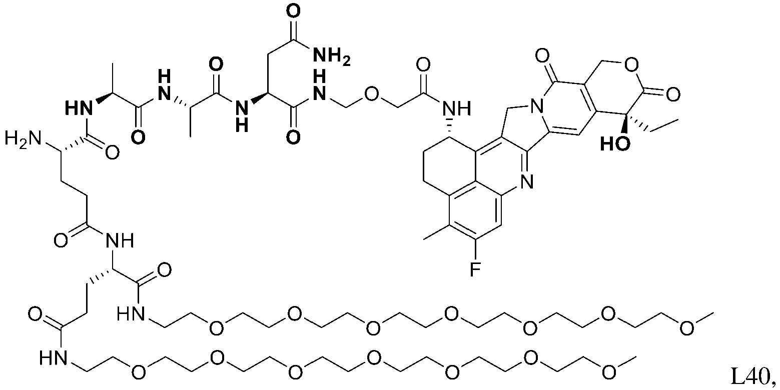

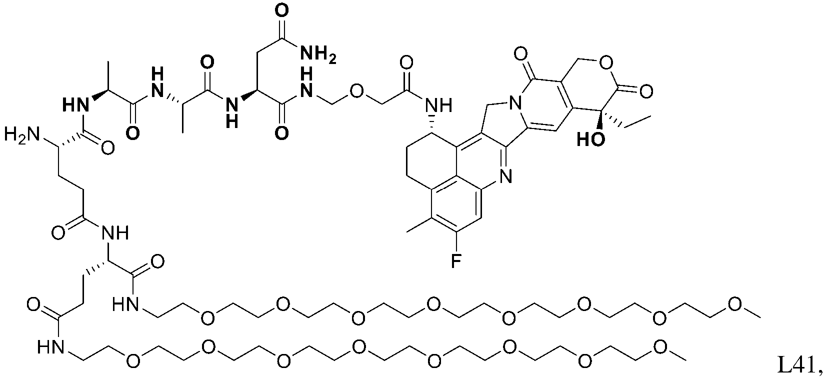

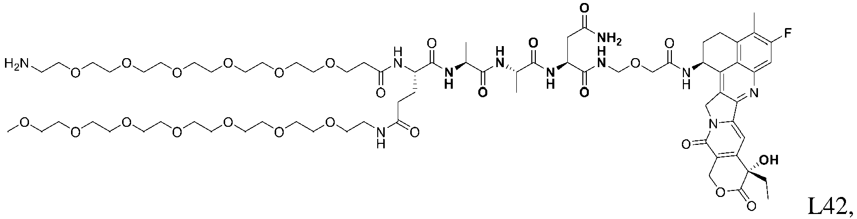

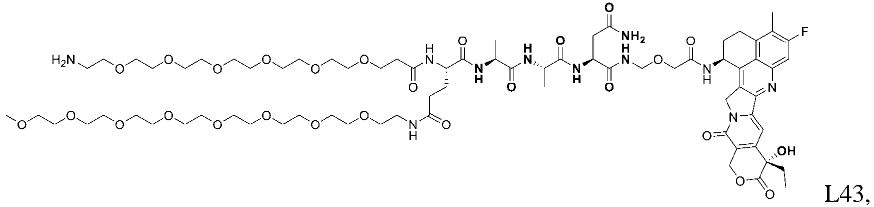

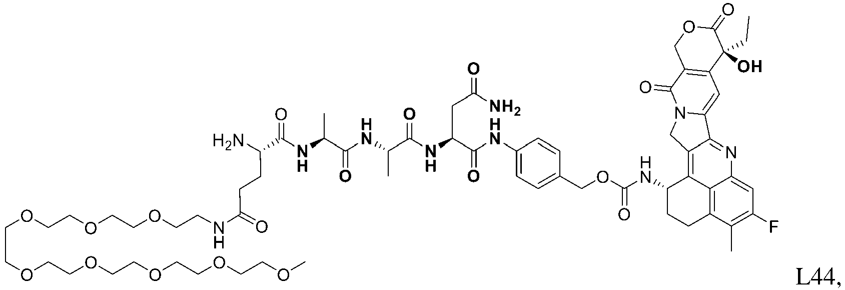

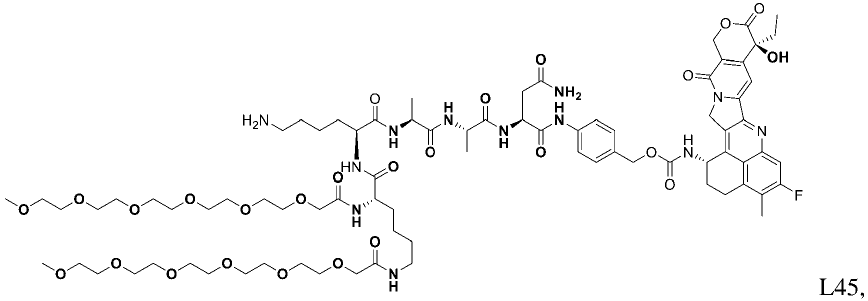

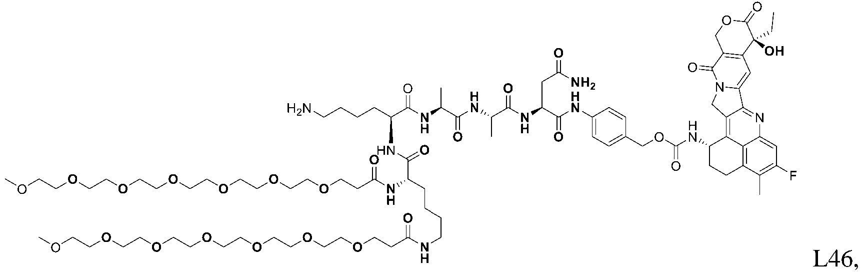

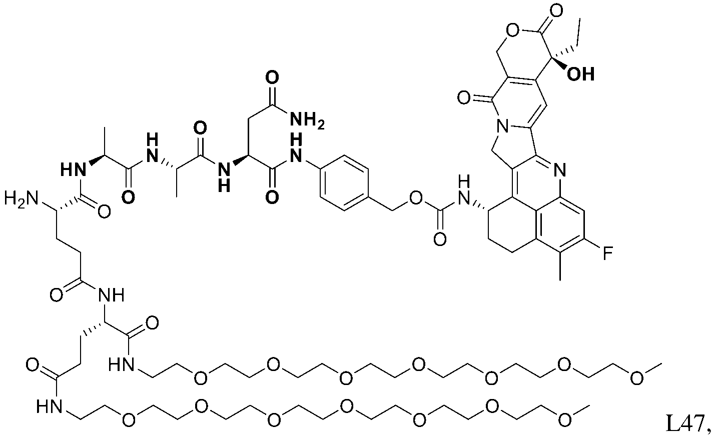

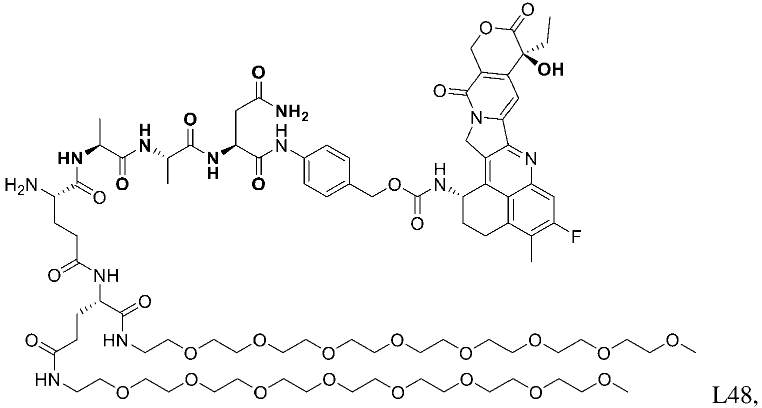

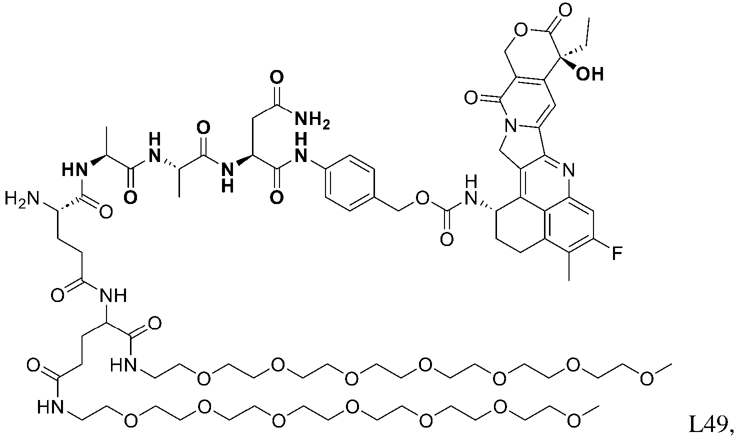

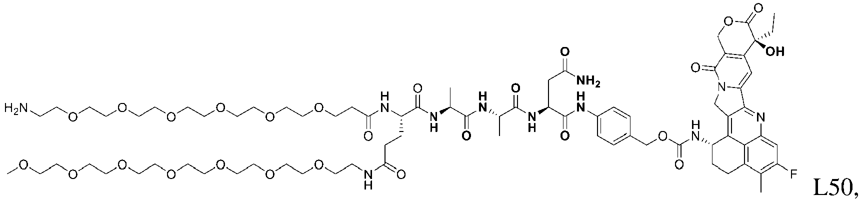

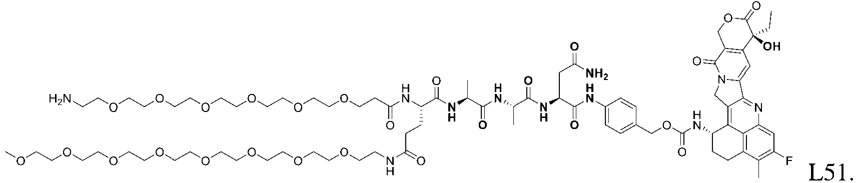

8. An antibody-drug conjugate, a stereoisomer thereof, or a pharmaceutically acceptable salt thereof, wherein the antibody-drug conjugate is shown in formula (III),
R₃-(R₁-R₂-D)ₓ Formula (III);
wherein R₃ is an antibody;
R₁, R₂, and D are as defined in any one of claims 1-4; and
x is an integer selected from 1 to 8.

9. The antibody-drug conjugate of claim 8, a stereoisomer thereof, or a pharmaceutically acceptable salt thereof, wherein:
R₁ is: 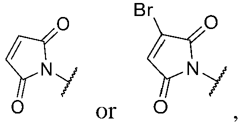 the attachment mode of R₃-R₁ is: 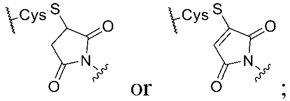 wherein S is the sulfur atom of a cysteine residue in R₃; or
Ri is: 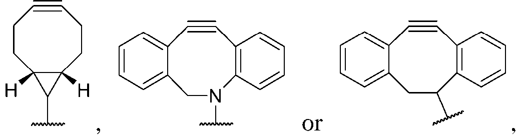 the attachment mode of R₃-R₁ is: 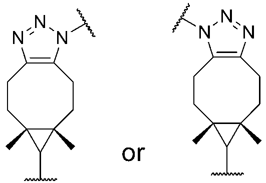 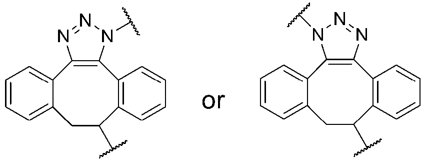 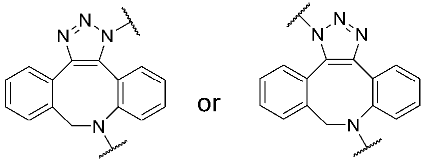 where R₃ containing an azide group is conjugated to the alkyne group in the R₁ moiety.

10. The antibody-drug conjugate of claim 8, a stereoisomer thereof, or a pharmaceutically acceptable salt thereof, wherein the antibody is a monoclonal antibody or a multispecific antibody;
preferably, the antibody is selected from the group consisting of: anti-Her2 antibody, anti-EGFR antibody, anti-VEGFR antibody, anti-CD20 antibody, anti-CD33 antibody, anti-PD-L1 antibody, anti-PD-1 antibody, anti-CTLA-4 antibody, anti-TNFα antibody, anti-CD28 antibody, anti-4-1BB antibody, anti-OX40 antibody, anti-GITR antibody, anti-CD27 antibody, anti b-CD40 antibody, anti-ICOS antibody, anti-CD25 antibody, anti-CD30 antibody, anti-CD3 antibody, anti-CD22 antibody, anti-CCR6 antibody, anti-CD38 antibody, anti-CD52 antibody, anti-complement C5 antibody, anti-RSV F protein antibody, antibody-GD2 antibody, anti-GITR antibody, anti-glycoprotein receptor lib/Illa antibody, anti-ICOS antibody, anti-IL2R antibody, anti-LAG3 antibody, anti-α4 integrin antibody, anti-IgE antibody, anti-PDGFRα antibody, anti-RANKL antibody, anti-SLAMF7 antibody, anti-LTIGIT antibody, anti-TIM-3 antibody, anti-VEGFR2 antibody, anti-VISTA antibody, anti SSTR2 antibody, and anti LY6G6D antibody, anti GCC antibody, anti Trop2 antibody, anti Cmet antibody, or a multispecific antibody formed from any two or more of these antibodies.

11. A pharmaceutical composition, wherein the pharmaceutical composition comprises: (i) the drug conjugate of any one of claims 1-7, or a stereoisomer thereof or a pharmaceutically acceptable salt thereof, and/or the antibody-drug conjugate of any one of claims 8-10, or a stereoisomer thereof or a pharmaceutically acceptable salt thereof; and (ii) a pharmaceutically acceptable carrier.

12. A drug conjugate, or a stereoisomer thereof or a pharmaceutically acceptable salt thereof, wherein the drug conjugate is selected from: 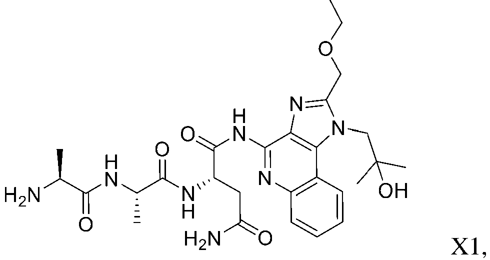 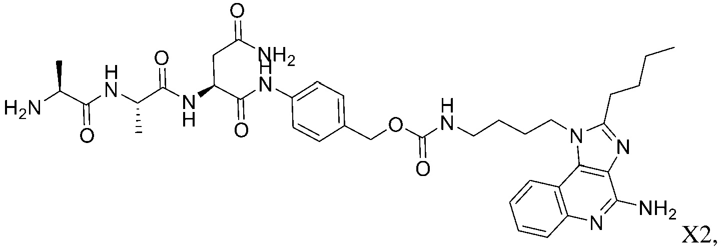 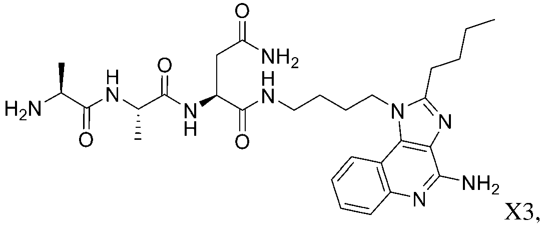 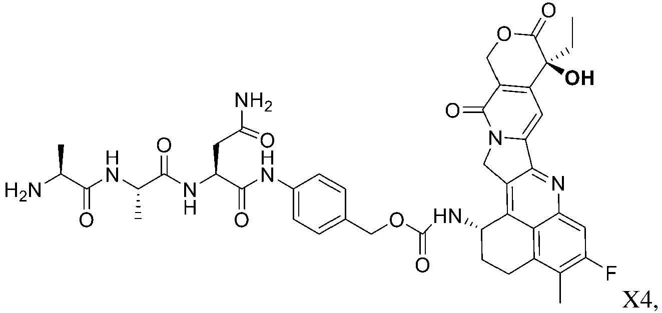 and

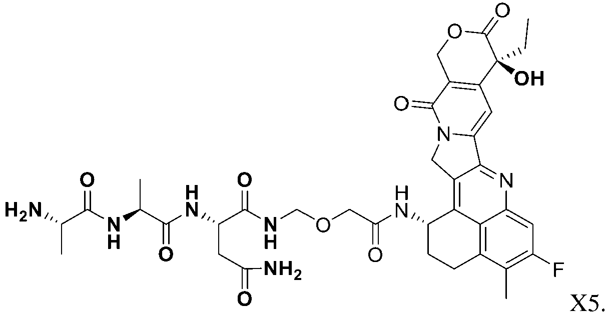

13. A pharmaceutical composition, wherein the pharmaceutical composition comprises the drug conjugate of claim 12, or a stereoisomer thereof or a pharmaceutically acceptable salt thereof, and a pharmaceutically acceptable carrier.

14. The pharmaceutical composition of any one of claims 11-13, wherein the pharmaceutical composition further comprises one or more other anti-cancer drugs; preferably, the one or more other anti-cancer drugs are Legubicin.

15. Use of the drug conjugate of any one of claims 1-7, or a stereoisomer thereof or a pharmaceutically acceptable salt thereof, or the antibody-drug conjugate of any one of claims 8-11, or a stereoisomer thereof or a pharmaceutically acceptable salt thereof in the preparation of a drug for the treatment and/or prevention of tumors and/or inflammations; preferably, the tumor is a hematological tumor or a solid tumor; more preferably, the tumor is selected from the group consisting of sarcoma (e.g., fibrosarcoma), bladder cancer, brain cancer, breast cancer, cervical cancer, colorectal cancer, esophageal cancer, renal cancer, liver cancer, lung cancer, nasopharyngeal carcinoma, pancreatic cancer, prostate cancer, skin cancer, gastric cancer, uterine cancer, ovarian cancer, testicular cancer, and blood cancer.
